# EUROPEAN PATENT SPECIFICATION

(11) **EP 1 651 674 B1**
(45) Date of publication and mention of the grant of the patent: **29.09.2010**
(21) Application number: 04781084.1
(22) Date of filing: 12.08.2004
(51) Int. Cl.: C07K 16/00, C07K 14/435, C12Q 1/00

(54) **UPS AS MODIFIERS OF THE BETA CATENIN PATHWAY AND METHODS OF USE**
UPS ALS DEN BETA-CATENIN-PFAD MODIFIZIERENDE SUBSTANZEN UND ANWENDUNGSVERFAHREN
UPS UTILISES COMME MODIFICATEURS DE LA VOIE DE LA BETA CATENINE ET LEURS PROCEDES D'UTILISATION

(30) Priority: 14.08.2003 US 495172 P
(43) Date of publication of application: 03.05.2006
(73) Proprietor: Exelixis, Inc., South San Francisco, CA 94083-0511 (US)
(72) Inventor: FRANCIS-LANG, Helen, San Francisco, CA 94109 (US); WINTER, Christopher, G., Needham, MA 02492 (US); VENTURA, Richard, Benn, Abegania, Daly City, CA 94014 (US); HEUER, Timothy, S., El Granada, CA 94018 (US); LICKTEIG, Kim, San Mateo, CA 94401 (US)
(74) Representative: Main, Malcolm Charles
(86) International application number: PCT/US2004/026339
(87) International publication number: WO 2005/017119

(56) References cited:
- WO-A-01/60985
- WO-A-03/052068
- US-A- 6 066 500
- US-A1- 2002 015 943
- ASHOUR ET AL.: 'Enhancement of 5-Flouro-2'-deoxyuridine Antitumor Efficacy by the Uridine hosphorylase nhibitor 5-(Benzyloxybenzyl)barbituric Acid Acyclonucleoside.' CANCER RESEARCH vol. 55, 01 March 1995, pages 1092 - 1098, XP008064970
- FINDENIG ET AL.: 'Modulation of 5-Fluorouracil Resistance in Human Colon Tumor Cell Line sby Azidothymidine.' ONCOLOGY RESEARCH. vol. 8, no. 5, 1996, pages 189 - 196, XP008064914
- LIU ET AL.: 'Expression, Characterization, and Detection of Human Uridine Phosphorylase and Identification of Variant Uridine Phosphorolytic Activity in Selected Human Tumors.' CANCER RESEARCH vol. 58, 01 December 1998, pages 5418 - 5424, XP008064971
- MONGA ET AL.: 'Beta-Catenin Antisense Studies in Embryonic Liver Cultures: Role in Proliferation, Apoptosis, and Lineage Specification.' GASTROENTEROLOGY vol. 124, January 2003, pages 202 - 216, XP002993318
- LIU M. ET AL: 'Expression, characterization, and detection of human uridine phosphorylase and identification of variant uridine phosphorolytic activity in selected human tumors' CANCER RESEARCH vol. 58, 01 December 1998, BALTIMORE, MD.; US, pages 5418 - 5424, XP008064971

### SEQUENCE LISTING

<110> EXELIXIS, INC.
<120> UPS AS MODIFIERS OF THE BETA CATENIN PATHWAY AND METHODS OF USE
<130> EX04-058C-PC
<150> US 60/495,172
   <151> 2003-08-14
<160> 11
<170> PatentIn version 3.2
<210> 1
   <211> 1796
   <212> DNA
   <213> Homo sapiens
<400> 1
   ggtcagctga gttcgccggc ccagggcagg cggggcccga gcctagcggt aacccccggg     60
   cagggcgggg ccgctcgcag actccatatg agattcacct cgcaggtggt tccctcattc    120
   gagtgctccg gcgcacagac ccgcgccccg ccgtctgcga gcctcccgag agccgtccct    180
   tcgtccggcc ctggagcatt gcgtttgtcg ccggtgtcgc agtgcgagga tggcgccgcg    240
   ggtgtagcgg ctctctgcgc aggccgagtg ggcccagaga agcgaggaac tccgcagctc    300
   gtcgacacgt ctcgtctcct gtcccaattc agggcttggt gaggtgactc gcggtcgcgg    360
   gtgactcgcc ggcaggacac tgcctggaac gcctggagcg cctcccactg cagacgtctg    420
   tccgcctcca gccgctctcc tctgacgggt cctgcctcag ttggcggaat ggcggccacg    480
   ggagccaatg cagagaaagc tgaaagtcac aatgattgcc ccgtcagact tttaaatcca    540
   aacatagcaa aaatgaaaga agatattctc tatcatttca atctcaccac tagcagacac    600
   aatttcccag ccttgtttgg agatgtgaag tttgtgtgtg ttggtggaag cccctcccgg    660
   atgaaagcct tcatcaggtg cgttggtgca gagctgggcc ttgactgccc aggtagagac    720
   tatcccaaca tctgtgcggg aactgaccgc tatgccatgt ataaagtagg accggtgctg    780
   tctgtcagtc atggtatggg cattccttct atctcaatca tgttgcatga gctcataaag    840
   ctgctgtact atgcccggtg ctccaacgtc actatcatcc gcattggcac ttctggtggg    900
   ataggtctgg agcccggcac tgtggtcata acagagcagg cagtggatac ctgcttcaag    960
   gcagagtttg agcagattgt cctggggaag cgggtcatcc ggaaaacgga ccttaacaag   1020
   aagctggtgc aggagctgtt gctgtgttct gcagagctga gcgagttcac cacagtggtg   1080
   gggaacacca tgtgcacctt ggacttctat gaagggcaag gccgtctgga tggggctctc 1140
   tgctcctaca cggagaagga caagcaggcg tatctggagg cagcctatgc agccggcgtc 1200
   cgcaatatcg agatggagtc ctcggtgttt gccgccatgt gcagcgcctg cggcctccaa 1260
   gcggccgtgg tgtgtgtcac cctcctgaac cgcctggaag gggaccagat cagcagccct 1320
   cgcaatgtgc tcagcgagta ccagcagagg ccgcagcggc tggtgagcta cttcatcaag 1380
   aagaaactga gcaaggcctg agcgctgccc tgcacctccg cagacctgct gtgatgactt 1440
   gccattaaaa gcattgtcca aaatcccctg ttgtgtggac tttgagcaca ctttacacaa 1500
   gaatctagaa aatcagatcg cgattaagag acagagaatc ttggattaac cgcatgggag 1560
   atgttcttcc ttttgaagtt tcattggagc attttcaatg atgttagcct gatttggggt 1620
   ttcttcaaga acattctacc aaatttttgt actatttcta gggaaatttt tcagacttta 1680
   aaattctaat ggtagtcaga tttcatgtca ctaaacaaga aatctgacaa tagtgccagg 1740
   aaactaattt cctgatacat taaaaaaatt ccatgcaaaa aaaaaaaaaa aaaaaa 1796
<210> 2
   <211> 1399
   <212> DNA
   <213> Homo sapiens
<400> 2
   tgagattcac ctcgcaagtg gttccctcat tcgagtgctc cggcgcacag acccgcgccc 60
   cgccgtccgc gagcctcccg agagccgtcc cttcgtccgg ccctggagca ttgcgtttgt 120
   cgccggtgtc gcagtgcgag gatggcgccg cgggtgtagc ggctctctgc gcaggccgag 180
   tgggcccaga gaagcgagga actccgcagc tcgtcgacac gtctcgtctc ctgtcccaat 240
   tcagggcttg gtgaggtgac tcgcggtcgc gggtgactcg ccggcaggac actgcctgga 300
   acgcctggag cgcctcccac tgcagacgtc tgtccgtctc cagccgctct cctctgacgg 360
   gtcctgcctc agttggcgga atggcggcca cgggagccaa tgcagagaaa gctgaaagtc 420
   acaatgattg ccccgtcaga cttttaaatc caaacatagc aaaaatgaaa gaagatattc 480
   tctatcattt caatctcacc actagcagac acaatttccc agccttgttt ggagatgtga 540
   agtttgtgtg tgttggtgga agcccctccc ggatgaaagc cttcatcagg tgcgttggtg 600
   cagagctggg ccttgactgc ccaggtagag actatcccaa catctgtgcg ggaactgacc 660
   gctatgccat gtataaagta ggaccggtgc tgtctgtcag tcatggtatg ggcattcctt 720
   ctatctcaat catgttgcat gagctcataa agctgctgta ctatgcccgg tgctccaacg 780
   tcactatcat ccgcattggc acttctggtg ggataggtct ggagcccggc actgtggtca 840
   taacagagca ggcagtggat acctgcttca aggcagagtt tgagcagatt gtcctgggga 900
   agcgggtcat ccggaaaacg gaccttaaca agaagctggt gcaggagctg ttgctgtgtt 960
   ctgcagagct gagcgagttc accacagtgg tggggaacac catgtgcacc ttggacttct 1020
   atgaagggca aggccgtctg gatggggctc tctgctccta cacggagaag gacaagcagg 1080
   cgtatctgga ggcagcctat gcagccggcg tccgcaatat cgagatggag tcctcggtgt 1140
   ttgccgccat gtgcagcgcc tgcggcctcc aagcggccgt ggtgtgtgtc accctcctga 1200
   accgcctgga aggggaccag atcagcagcc ctcgcaatgt gctcagcgag taccagcaga 1260
   ggccgcagcg gctggtgagc tacttcatca agaagaaact gagcaaggcc tgagcgctgc 1320
   cctgcacctc cgcagacctg ctgtgatgac ttgccattaa aagcattgtc caaaaaaaaa 1380
   aaaaaaaaaa aaaaaaaaa 1399
<210> 3
   <211> 794
   <212> DNA
   <213> Homo sapiens
<400> 3
   ggatggcgcc gcgggtgtag cggctctctg cgcaggccga gtgggcttgg tgaggtgact 60
   cgcggtcgcg ggtgactcgc cggcaggaca ctgcctggaa cgcctggagc gcctcccact 120
   gcagacgtct gtccgcctcc agccgctctc ctctgacggg tcctgcctca gttggcggaa 180
   tggcggccac gggagccaat gcagagaaag ctgaaagtca caagtctgga gcccggcact 240
   gtggtcataa cagagcaggc agtggatacc tgcttcaagg cagagtttga gcagattgtc 300
   ctggggaagc gggtcatccg gaaaacggac cttaacaaga agctggtgca ggagctgttg 360
   ctgtgttctg cagagctgag cgagttcacc acagtggtgg ggaacaccat gtgcaccttg 420
   gacttctatg aagggcaagg ccgtctggat ggggctctct gctcctacac ggagaaggac 480
   aagcaggcgt atctggaggc agcctatgca gccggcgtcc gcaatatcga gatggagtcc 540
   tcggtgtttg ccgccatgtg cagcgcctgc ggcctccaag cggccgtggt gtgtgtcacc 600
   ctcctgaacc gcctggaagg ggaccagatc agcagccctc gcaatgtgct cagcgagtac 660
   cagcagaggc cgcagcggct ggtgagctac ttcatcaaga agaaactgag caaggcctga 720
   gcgctgccct gcacctccgc agacctgctg tgatgacttg ccattaaaag cattgtccaa 780
   aaaaaaaaaa aaaa 794
<210> 4
   <211> 1951
   <212> DNA
   <213> Homo sapiens
<400> 4
   gcgggaactc ccaggatcag gaaggtccca gagaaactcc ccagggggga agccgaggcc      60
   gtccagagag atccagggta cacacagtga cagtgccagc tccacattcc cgaccaccgg     120
   gccagggctg gttccacgct gtcatttctc tccgctggcc ccagccgcgg ggccaccgcc     180
   agacagacag accggggatc ctgcctggcg gcgtgacctt gggttagtcc tagagccgct     240
   tctagcccgg tttcctccgc cccaggttag gaccacgtgg ggcggggccg ctcgcagact     300
   ccatatgaga ttcacctcgc aagtggttcc ctcattcgag tgctccggcg cacagacccg     360
   cgccccgccg tccgcgagcc tcccgagagc cgtcccttcg tccggccctg gagcattgcg     420
   tttgtcgccg gtgtcgcagt gcgaggatgg cgccgcgggt gtagcggctc tctgcgcagg     480
   ccgagtgggc ttggtgaggt gactcgcggt cgcgggtgac tcgccggcag gacactgcct     540
   ggaacgcctg gagcgcctcc cactgcagac gtctgtccgc ctccagccgc tctcctctga     600
   cgggtcctgc ctcagttggc ggaatggcgg ccacgggagc caatgcagag aaagctgaaa     660
   gtcacaatga ttgccccgtc agacttttaa atccaaacat agcaaaaatg aaagaagata     720
   ttctctatca tttcaatctc accactagca gacacaattt cccagccttg tttggagatg     780
   tgaagtttgt gtgtgttggt ggaagcccct cccggatgaa agccttcatc aggtgcgttg     840
   gtgcagagct gggccttgac tgcccaggta gagactatcc caacatctgt gcgggaactg     900
   accgctatgc catgtataaa gtaggaccgg tgctgtctgt cagtcatggt atgggcattc     960
   cttctatctc aatcatgttg catgagctca taaagctgct gtactatgcc cggtgctcca    1020
   acgtcactat catccgcatt ggcacttctg gtgggatagg tctggagccc ggcactgtgg    1080
   tcataacaga gcaggcagtg gatacctgct tcaaggcaga gtttgagcag attgtcctgg    1140
   ggaagcgggt catccggaaa acggacctta acaagaagct ggtgcaggag ctgttgctgt    1200
   gttctgcaga gctgagcgag ttcaccacag tggtggggaa caccatgtgc accttggact    1260
   tctatgaagg gcaaggccgt ctggatgggg ctctctgctc ctacacggag aaggacaagc    1320
   aggcgtatct ggaggcagcc tatgcagccg gcgtccgcaa tatcgagatg gagtcctcgg    1380
   tgtttgccgc catgtgcagc gcctgcggcc tccaagcggc cgtggtgtgt gtcaccctcc    1440
   tgaaccgcct ggaaggggac cagatcagca gccctcgcaa tgtgctcagc gagtaccagc    1500
   agaggccgca gcggctggtg agctacttca tcaagaagaa actgagcaag gcctgagcgc 1560
   tgccctgcac ctccgcagac ctgctgtgat gacttgccat taaaagcatt gtccaaaatc 1620
   ccctgttgtg tggactttga gcacacttta cacaagaatc tagaaaatca gatcgcgatt 1680
   aagagacaga gaatcttgga ttaaccgcat gggagatgtt cttccttttg aagtttcatt 1740
   ggagcatttt caatgatgtt agcctgattt ggggtttctt caagaacatt ctaccaaatt 1800
   tttgtactat ttctagggaa atttttcaga ctttaaaatt ctaatggtag tcagatttca 1860
   tgtcactaaa caagaaatct gacaatagtg ccaggaaact aatttcctga tacattaaaa 1920
   aaattccatg caaaaaaaaa aaaaaaaaaa a 1951
<210> 5
   <211> 2261
   <212> DNA
   <213> Homo sapiens
<400> 5
   gccgcgcggc acgggtgagt gcccagggac ttcaccagtg ctgccatgct ggccccaggc 60
   tgtgagttgg acccagacca agaagtggtg aggacaaggc ctgaagatgt gcctgcttcc 120
   cattcaactt ccaccatgat tgtaagtgtc ctgaggcctc ccagccatgc ttcctgtaca 180
   gcctgtggaa ctgtgacttt tcacatagta gagagaatgg cttcagttat acctgcctcc 240
   aataggtcca tgagatctga caggaataca tatgttggaa aaaggtttgt tcacgttaaa 300
   aatccttact tggatttgat ggatgaagac attctctatc acttggattt gggaacaaaa 360
   acacacaacc taccagcaat gtttggagat gtaaagtttg tctgtgtcgg tgggagcccc 420
   aacagaatga aagcatttgc actgtttatg cacaaggagc tcgggtttga ggaagctgaa 480
   gaagacataa aagacatctg tgctgggaca gacagatact gtatgtacaa aaccgggcct 540
   gtgctcgcca tcagtcacgg catgggcatc ccctccattt ctattatgct tcatgaactc 600
   atcaaattac tccaccatgc acggtgctgc gatgtcacca ttattagaat cggtacatca 660
   gggggaatag ggattgcacc agggactgtt gtaataacgg atatagctgt agactccttc 720
   tttaagcccc ggtttgaaca ggtcattttg gacaacattg tcacccgaag tactgaactg 780
   gacaaagaac tgtctgaaga actgttcaac tgtagcaaag aaatccccaa cttcccaacc 840
   ctcgttggac atacaatgtg tacctatgat ttttatgaag gccaaggccg actagatgga 900
   gcactgtgct ccttttccag agaaaaaaag ttagactact tgaagagagc atttaaagct 960
   ggtgtcagga atattgaaat ggaatctaca gtgtttgcag ctatgtgtgg actctgtggt 1020
   ctaaaagctg ctgtggtctg tgtgacactt ctcgacagac tcgactgtga tcagatcaac 1080
   ttgcctcatg atgtcctggt ggagtaccag caacggcctc agctcctaat ctccaacttc 1140
   atcagacggc ggcttggact ttgtgactag acgtcctaac tgggcagccc aaccctcccc 1200
   tgcaagtttg tagctcaagt tgtaatgtga aagtcatatt ttatttgtgg catttttata 1260
   tagttctcat ccacatgcta aatggaaaga ctttatgaaa tccttctctc ttaaaaagga 1320
   atttattgta aaagaatact cacactaaat taaattcaaa tttcatttta gaataagtta 1380
   actaaatcag tctaataatt aaaattttaa aactcctgga ttatgacatt tggagtttca 1440
   tatgcagcat taattaagct cacatcaaaa tagaccagcc atttgcacat agttaccagt 1500
   cactctgttt ctgaaaccaa tccagaaatt catgttagaa cattgtcagg cacttctagg 1560
   tattggacaa gtgacagaaa ctgatcatcc atattcaaaa tatttacaga aaaaagtgtc 1620
   agtgaaatgt aggtctatat ggaaaagtta ccattaggaa aatatggcca tactgctttt 1680
   ttacccctgc actatttcag tgcaccacag aataggattc gagatttaca tatgcctgtt 1740
   tgtgatttta tctcagaata taacaactct ggattacatt ttcctctcaa ctgaaaaccc 1800
   tcagtaatgc aaagaaaggc cttccttttt cttcagaatc acaagtgact aaattgcctt 1860
   ctagccattt tttcttgtaa aaaaagtgtt ttgaggaggc tgtatttttt attccatttc 1920
   aatattaacc aaataggctg agaaattcat agcaattgaa ttaagacttg agttcaagat 1980
   gttaaaggaa tccttttgta tgacttgtcc agaccggcct attgccctac ccgtttgatt 2040
   ctctgggagg catggtgctc ctcctggctg cacatgctct ctgtgaactg atcgcatctt 2100
   gggttcttct caatttgaca tacttgggag tcggaatgac tggatggggg atgggtagag 2160
   gatgactggc acacttgtat agtatgtaat gttgtgtaca tattatttat atggttgtgg 2220
   ctcaaataaa gtttaattcc aacacaaaaa aaaaaaaaaa a 2261
<210> 6
   <211> 2261
   <212> DNA
   <213> Homo sapiens
<400> 6
   gccgcgcggc acgggtgagt gcccagggac ttcaccagtg ctgccatgct ggccccaggc 60
   tgtgagttgg acccagacca agaagtggtg aggacaaggc ctgaagatgt gcctgcttcc 120
   cattcaactt ccaccatgat tgtaagtgtc ctgaggcctc ccagccatgc ttcctgtaca 180
   gcctgtggaa ctgtgacttt tcacatagta gagagaatgg cttcagttat acctgcctcc 240
   aataggtcca tgagatctga caggaataca tatgttggaa aaaggtttgt tcacgttaaa 300
   aatccttact tggatttgat ggatgaagac attctctatc acttggattt gggaacaaaa 360
   acacacaacc taccagcaat gtttggagat gtaaagtttg tctgtgtcgg tgggagcccc 420
   aacagaatga aagcatttgc actgtttatg cacaaggagc tcgggtttga ggaagctgaa 480
   gaagacataa aagacatctg tgctgggaca gacagatact gtatgtacaa aaccgggcct 540
   gtgctcgcca tcagtcacgg catgggcatc cctccatttt ctattatgct tcatgaactc 600
   atcaaattac tccaccatgc acggtgctgc gatgtcacca ttattagaat cggtacatca 660
   gggggaatag ggattgcacc agggactgtt gtaataacgg atatagctgt agactccttc 720
   tttaagcccc ggtttgaaca ggtcattttg gacaacattg tcacccgaag tactgaactg 780
   gacaaagaac tgtctgaaga actgttcaac tgtagcaaag aaatccccaa cttcccaacc 840
   ctcgttggac atacaatgtg tacctatgat ttttatgaag gccaaggccg actagatgga 900
   gcactgtgct ccttttccag agaaaaaaag ttagactact tgaagagagc atttaaagct 960
   ggtgtcagga atattgaaat ggaatctaca gtgtttgcag ctatgtgtgg actctgtggt 1020
   ctaaaagctg ctgtggtctg tgtgacactt ctcgacagac tcgactgtga tcagatcaac 1080
   ttgcctcatg atgtcctggt ggagtaccag caacggcctc agctcctaat ctccaacttc 1140
   atcagacggc ggcttggact ttgtgactag acgtcctaac tgggcagccc aaccctcccc 1200
   tgcaagtttg tagctcaagt tgtaatgtga aagtcatatt ttatttgtgg catttttata 1260
   tagttctcat ccacatgcta aatggaaaga ctttatgaaa tccttctctc ttaaaaagga 1320
   atttattgta aaagaatact cacactaaat taaattcaaa tttcatttta gaataagtta 1380
   actaaatcag tctaataatt aaaattttaa aactcctgga ttatgacatt tggagtttca 1440
   tatgcagcat taattaagct cacatcaaaa tagaccagcc atttgcacat agttaccagt 1500
   cactctgttt ctgaaaccaa tccagaaatt catgttagaa cattgtcagg cacttctagg 1560
   tattggacaa gtgacagaaa ctgatcatcc atattcaaaa tatttacaga aaaaagtgtc 1620
   agtgaaatgt aggtctatat ggaaaagtta ccattaggaa aatatggcca tactgctttt 1680
   ttacccctgc actatttcag tgcaccacag aataggattc gagatttaca tatgcctgtt 1740
   tgtgatttta tctcagaata taacaactct ggattacatt ttcctctcaa ctgaaaaccc 1800
   tcagtaatgc aaagaaaggc cttccttttt cttcagaatc acaagtgact aaattgcctt 1860
   ctagccattt tttcttgtaa aaaaagtgtt ttgaggaggc tgtatttttt attccatttc 1920
   aatattaacc aaataggctg agaaattcat agcaattgaa ttaagacttg agttcaagat 1980
   gttaaaggaa tccttttgta tgacttgtcc agaccggcct attgccctac ccgtttgatt 2040
   ctctgggagg catggtgctc ctcctggctg cacatgctct ctgtgaactg atcgcatctt 2100
   gggttcttct caatttgaca tacttgggag tcggaatgac tggatggggg atgggtagag 2160
   gatgactggc acacttgtat agtatgtaat gttgtgtaca tattatttat atggttgtgg 2220
   ctcaaataaa gtttaattcc aacacaaaaa aaaaaaaaaa a 2261
<210> 7
   <211> 209613
   <212> DNA
   <213> Homo sapiens
<400> 7
   gaattctttt tcagataaat cagggatttc ttcttggttt gggcccattg ctggtgagct 60
   agtgtgattt tttggcaggt ggtaaagagc cttgttttgc cattttacca gagttggttt 120
   tctggttctt tctcatttat gtaggctctg tcagagggaa ggcctagggc tgaaggctgt 180
   tgttcagatt cttttttccc acggggtgtt gccttgatgt agtactttcc cccttttcct 240
   atggatgtgg cttcctgaga gccaagctgt agtgattgtt atctgtcttt tggatctagc 300
   cgcctagtga gtctaccagg ctcctggctg gtactggggg ttgtctgctg agtcctatga 360
   tgtgaaccac ctgggtctct cagccatgga taccagcacc ttgttatata taaagttttg 420
   gtgctgcaaa agaaatagca ctcgaatata aaattttctt tttaattctc agcaaggcaa 480
   ggtacttctg tagaagcgtg cacccttaca gatgaagcaa tggtgagtac acacttggac 540
   aagggagggg aaggggttct tatccctgac gcgcgtggcc cctgctgctg tgtccttccc 600
   ctcttggcta gagttagacc acacaagcta aactaattcc gactggctaa tttaaagaga 660
   gtgacggggg tgagtggttt ggcgggaaaa atggttatga cacagcaggt aatcagaatg 720
   agtcaggatg gaacaggtaa ctgaaatgag tcagggtgga gcaggtaatc aaaaaaggtt 780
   gctttatgag aaagttaagt ttaaaagtag acggtaaaga attgaacata ctgacatatt 840
   gattctttga aaagaaattt agaactcata tctaacaacc tgttccagtg gaggtggcag 900
   aggggtgaaa tggactctgg ggggttctta gcttcggtgg tttaatgcac cggttttgcg 960
   ctggttggcc tctggccagg aggtggtgct ttccagagag catcagctgt gataggatag 1020
   gagtaacagg tggtgggcgg ggcctcagaa ctcccaagag tataggcctt ttgtcttcag 1080
   ctacctgagc tgagaacttg ccccaggcta cctgcctccc agctgcaaaa gcaactatgg 1140
   ctttctttgt tcccccacct gtggagtcag cacaccagat agattgacgc gctgccccaa 1200
   gttctggcca ggagacttct ccctcagttc aaattgttac aaaattcagc tggagatttc 1260
   cttctccctg tggccttttc ccagcacctt tggccatcct cccaaagcgc ctctgtgagg 1320
   ccaggcaaaa acagcttgcc aggggaccca gcgagctccc acggcttttc ccactgcttc 1380
   ctctacccct gtatttcact ggctttctaa atttactcag ctccaggtaa ggtcaaaatc 1440
   ttctcccata atctaggcct tcagtttctc cagtggggag gtgtgtttgg gggcagacaa 1500
   tctccctttc ctacttccag tttgggcacg cagagtattt gggttgtctc ccgggccctg 1560
   caggagcaat ctgctttctt cagggggttt gtgggtcctc cgaagttttc tgatttattc 1620
   ctgcagtcgt tgtggagcaa aaattcatga tgcgagcctc cacacacctc tgtctgtccc 1680
   agtcggagtt gcaatctagt cttgcctcgc gtccgccatg atcctcacat gtcttttcac 1740
   atttttattt cttgtgtttt agttacttct ggtatagctt tgatgatttt aaagctatta 1800
   tatgacagtt gctttcttta aggcccaatt ctagtcacat tcttgggtct aatcctcctg 1860
   ttgactgtgc ctcacacaaa tattgttact tgcttattct gtgactttct atcatgagct 1920
   tatcttcaac gaggctggct attttgtgag aatcatgtgt cacctgggtg tgtctgcttc 1980
   cccatgtggt tttgcattta ttatgctagg agcaccaggg gtgtctctgt tccatgaccc 2040
   aattttctgt gtatctttta aaatgagagt tcctgaaata aattcaaatt ccaagccaga 2100
   ctcaggcatg gagtttgact ttcttaggga attgtatttt tttcctattg cttgttaaac 2160
   agagacaaac tagattcact agaatctcct gtgtaggtgg gaggatttct tcagctgggt 2220
   attagttcca atatccacct cacctgacat gaggcctcat gtctacatga gctttaaacc 2280
   ttagccttca tgtttagtat cagctgtaag ccccggtcac aaccctggct ttaagttttc 2340
   tctttttttc tggcatgttt tgattctctt cccaaaatat ttcatgcagc cttcgtagat 2400
   gtttgcaatg ccttggggta taatagctct ccctcttgca gaacccccag taaacttctt 2460
   ctcataatat ccttccacaa ctgttctcct tgcttactag tctcccttcc ctctcaatat 2520
   ttcctttcat ttttttctta ttatttccct tcacttcatg ataatttcta tctttctcta 2580
   gcaacatatt ttgaccccac agagtcccat gtatgctatg acaacctgaa aagataaatt 2640
   aacttattag ttgttcttcc attattgaga caccagagta tttataccca gggtaatgcc 2700
   tattggattg atgcaggaaa attccatttt aaatttacag cctttgatcc tgtagaaata 2760
   ctccctggtg tgtctacctt cttctcttct ctctagtatt ttagatttaa tcattctttc 2820
   tgatagctat tgatgtgtca gtcatatctt ctcactgcat tcctgagtct gataaactgt 2880
   gctttaaaaa taacttcagt cagctatgct gtcttgagca agaatgcctg aactgccgga 2940
   ttcaaagatt taggacttct tggcagatac agtcttatat tggagataag aacatggggc 3000
   tacacggtct ccctgctggt agccattacc atactcaact cctttgctga gtgactcatc 3060
   actcaccaca gaaccttctt agagccactt aactcctgta gatcagagaa gcccacgtag 3120
   aaatgccaac cagcaggcat gagtatcagc aatgagtaaa ttaataaaat tagcaaatta 3180
   gaatgtgtta ttataaaaac cacaaaatag acctcactga gcctttacat ttttgtgata 3240
   gtgaaactta cataacattt atcattttaa cacttttgca tgtacagttc agtagcatta 3300
   aatacattca cgttattgtg caactatcac cattatccat cttcagaact ttttcgtcat 3360
   cccaaactga aactctgtgc tcataaaaca atgaatccca cttcctcttt ttctcagccc 3420
   ctggtaacca gtatcctact ttctttctct gtgaatttgt ctactctaag tacctcatat 3480
   aagtgaaatc gtacaatatt tatctgtatg tgtgtagtat ttcacttagc ataatatcct 3540
   gaggtttatc catgttgtag catgtatcag aatttcactg agactttact ttttaatgaa 3600
   ttaaatttta tacatttttt ttggaaaaac taatttttta ttagccacaa aaccgtaaaa 3660
   ggttttatct gcatagatgc tgagatcatc actgacatct ctcactcatc agtcatttca 3720
   acaccactgt ctcccacagc ctctattcag gctgtgtcat atttgaactc tccttagcct 3780
   tttaacataa gaagactttg gaagcacatg atctttcacg tgaaaaggca atttttattc 3840
   tgaaatatgt aagcggcttg actttttagg taaaaatttg aagccactga ccttcacaag 3900
   taagggaact gactaacaag caaatagtgt ctttggaggc ctcatcttat ctttaatcca 3960
   tctccccctg accacctacc accaccttgg aaattgtgaa atgtagccga tcaagtattg 4020
   gtcatctaca tccaggtgtt tcttcttagt gatctttatc tgaaaaagaa ccagtgcttt 4080
   tacacttttg aacaacttcc cattgcctgg ccaatatggt cccatcttct taacctgact 4140
   tttaggaccc tacctaatag actcccaaac tacatttcca gattcagcca accaggattc 4200
   ttcaccattc tagtcatatg tcctgtcttt tcctcatttt ttcttaatgc ttttcctttt 4260
   agtgacaaca cctccgctag aacctctatc aaaataccct taagtaaagc tcatctcaaa 4320
   tatccacctt agccaagaaa ccctttcaaa ccctctcagc tccatagtat cattttcttt 4380
   gtttaacctc tcaaaacact tgtatgtcta cctatttttt ggagtctatc agattatcct 4440
   ttgagttcta gtcattcaca catttgtctt agtgccttga acttactagg ttgcaaattg 4500
   cttaatggag ctggctgtat cttgttctct cataacacct agtcttgtga cttacagaaa 4560
   gtagattctt tataaatata tttgttctgt catttgcttg tttacttgga gcgagttcat 4620
   agcattgtag ccttaaacca actttggact ggctctttaa gaggtgagtc ctctggggtc 4680
   ctagtagaag gaagaagata gcactgaaag taggactgtg aagtgtagga aacagatctg 4740
   agtagagggc tcgtggtcac tgctgatcat ttacctagag taagtggaac ctgggtgtca 4800
   ttttatttca tctcatcatt ggtttttgtt ggttggaatg ctggtaggaa atgtacaaac 4860
   ttgaactcaa atgaaaaaat aagttgattt ggatatgaaa agcatctaga tttttaaaga 4920
   tccatcttcc tgttttaaca aggaatgggc aaaatctgat gacccagata atgtctgtaa 4980
   ccttggaaca tccacagtgc ttcacggtgt taacaattaa gctactatag gtggaatagc 5040
   ttttccaaga atattagaaa tttagaaaaa tagacacaac cctaaattac tactttttgg 5100
   taggttaatt ctagaccagt cctctgcaaa atatgatact ggtctaagtt gtttgcctaa 5160
   tgaatttcta aactacttta tactaataaa aaataatatt taaatcaggc ttaatgtttc 5220
   aaggggaaaa agtggtttaa tggctcttgt tctcaagtga aaactgctat gctcagtttt 5280
   ttagaaaaac gaatgttaaa ttgattctag attcaatgac ttctgtttgg gaggagtgaa 5340
   aaaacaaatc agaaattgag aaaatcattg agaagtgaat aacgaagcag gctgcaaatg 5400
   gtaagttttt ttccagttat tcattttagg agacagtggt ttgcagagtc actggctata 5460
   gttttaggat cattaaaaga cagagaaagt cctggaggat tgcgaaagaa aaaaacatgt 5520
   ataattcatt tatttttaaa aggaacaaag ggagatctta acaattaaag attagtttac 5580
   acagctccaa ccctgaggtg gaggttgttg atcaaatcct caaaaaaaaa aaaaaaatct 5640
   atggtataga catctagagg cacacaggat cctggagagg aacagtgtgg ctttatgaaa 5700
   ctcagtatgt atcagacaat tttaatttcc ttccatgaca ggatgacagg acttgtagct 5760
   tggaggcaaa cagtagaggc cattaatctg tcttttactc aagttttcaa ttctatcata 5820
   catgtgatgc ccagcagcca gctaggaaaa taagttgagc accatgtttc tcaagctgca 5880
   ggccctggag agttcacgag aatctctaaa taaaagaata cattcttgta cagaagatta 5940
   tatttttaaa agtattttac cagttaaaga attagcttct gttttgcaat ttaaaaaatg 6000
   cataattgaa agatcttcat atagaatgac ttcagttcct taagtcagtg attttcaaaa 6060
   tggttttggt ccccagatgt attttggagg ctgtattact gggtctcagc aggaaccaga 6120
   tggcataatt aaacagggaa ccgagagaac ttactgaagg gactgtttac aaggggtggg 6180
   caggatttag ggagcaacaa gggatgatgc agctggaaac aactgttacc agccctgatc 6240
   cgagagggct gaagcagggc aattaccaga acccagggag gacagaagtg tggcaagggc 6300
   cacccacagg agctatggcc tttggtagag gacacaggtg tcctgccaca acctgaccta 6360
   aggaaggtgc acaaacccag gaaattgtat gcagatttgg gggcatatgt atgaatgtgt 6420
   gttttcctga agatcgggtt catagatttt atcaggggtc tccaaaatga atcccaaatg 6480
   ttaaaactgt gtttgtaaaa ggaataaata aaattcaaac tattatattc ctgaagttga 6540
   tagtttacct cccgtatgaa gcctctacag gatgcaacac actaggtaac attctatagc 6600
   tgataaaaat aagtcaaaaa tgttattttg tgtattttgg attacagact gagtttttct 6660
   aagcaatgat taaacttaac tgtttcctgt gctgatgttt gggaatgaaa atggaataca 6720
   aaagcaaatt atattgacta atatatacat gtcgacctga agacttgttc aggtgataac 6780
   atttggataa aattaccctg agagatagtc agctattttt tgttattttt gtgattttgt 6840
   tcatttttgt ttcatctttc attggggtat tttagttttt tgtatgtttt tgtacataat 6900
   tttatacata ccagcaaata tgtgtgtgta catgtgtata tgtatgaata cataaacaca 6960
   tagtaagatt gaatgaatta ttttattaat agataatata tagtatataa ttacatcatt 7020
   ataattatat ataattttta atatatcaca tatgttatat aaattataaa ctgtttcata 7080
   tattttatat ataatataaa aatgtaaaat cctttcagtc ttactatgtg ccaggcactg 7140
   tgcaagttgc tggaatataa aatgagtaag atactgttgc tagcatcgag gagtgagtca 7200
   agtaaaagaa cttgatcttt atataactat tccagcggag acaaggccta aagagtttta 7260
   gaagttcagc tggaagagca catgccagag tcacagactg tccattcttc taaattggta 7320
   actctcaact taggctgcaa aataaaaaat agagcttccc agtcccactc ccagagattc 7380
   ttgttgaatt ggtctgagat ggacctctga acagtgtttt caaaaggctt ccctggtgat 7440
   cctagtatgc acctgtgttg agaacctcag ggcggtgtcc tggaatgaac acactgacgc 7500
   ccaactgggg ctcatccccc caactccaca cccaggcggt ctgagatcca cactcatctc 7560
   tgcacactgt ggcctcttag aatcaggact agttgttctt ctcctgatca ttagtttgca 7620
   tttttatttt agagatgctg taaatttgtt ctctttacga ggcaaccact agctggcccc 7680
   aagccaattc agagcccacg ttctgctgat gcacagactc tatggaatgc acagtagcag 7740
   aagcttcagg cttgtaccag ttgatgcccc ttgcctttgc ttttcctttt cttgcccagt 7800
   tagttctatc ttatactatc tcgatgtgct attattcaac cttgtaagct gtctcagatt 7860
   ctactgggga agaaggagga atatatattt ataatgaatg aatgaacaaa ggaagaaaca 7920
   tacaaaaaat gtacacaaat atacatagaa atgacaagaa gagggttttt gcaggccaat 7980
   ctgcttcttg aagggtgttc aaagagggca agaaagaatg agtagagaag cttgttacag 8040
   gtgaggtaaa aagccggtgc ccagacctag agtgtgcata ggtaagcagt cagaagatac 8100
   ctgtagagaa caaaactcta tgacacgcta agagtacaaa ggaaatgtga gcctcccaaa 8160
   tgaagacagc ccaggaacat gaaaaaggat aaatgttctc aatacggcat ctgcatgaga 8220
   acaaattcct gtcttcccag tgcaaatcta gttctaaagg aaggagggaa gaagtgatga 8280
   cttgagggaa aattgaagtt ggagacctcc ccaaaggagg tgaatttggg gcttggtttt 8340
   gacagagggg aaagctgtgt gtatgtgaga aaacaagagg ttctagacac tgccacagat 8400
   atacactgat ggtacagagg actttcctcc ccacagagtg agatgtagtg ggaggaacag 8460
   ccagggagag ctgtgctgac caggactgaa taaggaaagt atgagtcttg caaagattgt 8520
   gagattatag tgagagttta caagggaaat gctatgaaaa ggattcattc ttcacttgac 8580
   aagtatttat tgagtgccta gtatgtgtct ggtactgttt taggtgcttg aagcgataga 8640
   agcaaataaa atatgtgcag tctttgttct ccagaacctt atagtgtaga cagtaggcat 8700
   taattaaata atgaccccaa tcatgacata gttgcaagct gcatactttg aagaagtagt 8760
   cgagggggct ctggaggggg actacaggtg agcatggaag gaatctggtt tgaggattag 8820
   aaagactttc taagggaagg gatctctgag ttgagattta aaccatgagc atgagttaag 8880
   cgagggccct tcagggagag tagacagcaa aaggctctga aatgggaagg gacattggca 8940
   ctaagagttc ccactatccc atataacacc attgtataaa tcaggaaaag gcccctctca 9000
   gtcactgtgg ctcagggtgg actgagattc accctgtcca ggtctcacag ctagctggac 9060
   agggtggatc tcagtcccac ttgtccccgc agctaagcac ccccattgga tggatctgtt 9120
   ggcagtcctt cccaagggat tgtgcctttg tgaatttgtg ccttgggtaa gactgaagga 9180
   ccaagggtga tgtgcctgga gggcagacac aaggggaaga agtgggtgag gtgagctggg 9240
   ctaccaagct gggcccagtc aggaatgcct aagctgcaca gagggctttg gtcttcattc 9300
   taagaccaat gtgaaggctt tgaagcagga aaataaccca atcaaacttc aagaatggag 9360
   gcagggtgat caggagtgag gcttcctcag tgacctggag gagagatcat ggtggcctgg 9420
   actaggatgg gtgccaaaaa gatggaagga aatgaacaga ctggggaaat atttaggagg 9480
   atcaattaat aagacttagt gattgactgg ctgtggaaat taaggagaaa gaatcatcac 9540
   aggggtttcc caggtttctg gcttgtgcaa gtgatatggt ttactgagat agaaaatacc 9600
   agtgaggaag acacagacat aaatgcatgc atgcatgcac ctgtgcgcac acactccttg 9660
   caggagttga agagaagatt aactgtaatt atttcattct tctccctctc atagcctctc 9720
   cttctccgtg taaaaaaaaa aaaaaaaaga ttgtccagga gcaggcagat aaaagttcaa 9780
   actgaagcct ctctgaggtg gttttcacta catggtctaa aaaaatatga aacaaagtaa 9840
   aaatcccatc agaaaatgac ggcaatgcta ttctagcttg cttttagctc tgatctttga 9900
   atttgatggt caattattga gataccctaa aaatggcact taaagtgtct ctagtgccaa 9960
   aaagaagttc acaaaccaat gtacctcttg ggggattgtt tattggtgat gataaatacc 10020
   tcttgtttat tgtcattgct tgaaagagaa agacattccc cacccaggag caggccaatt 10080
   cagactcaac cggcctagga cctatttcct gccccaagcc tccattcggt catgtgtgac 10140
   aatggctctg ggatccaaat aatctgtact gtgttggaat taaactttat ttgagccaca 10200
   accatataaa taatatgtac acaacattac atactataca agtgtgccag tcatcctcta 10260
   cccatccccc atccagtcat tccgactccc aagtatgtca aattgagaag aacccaagat 10320
   gcgatcagtt cacagagagc atgtgcagcc aggaggagca ccatgcctcc cagagaatca 10380
   aacgggtagg gcaataggcc ggtctggaca agtcatacaa aaggattcct ttaacatctt 10440
   gaactcaagt cttaattcaa ttgctatgaa tttctcagcc tatttggtta atattgaaat 10500
   ggaataaaaa atacagcctc ctcaaaacac tttttttaca agaaaaaatg gctagaaggc 10560
   aatttagtca cttgtgattc tgaagaaaaa ggaaggcctt tctttgcatt actgagggtt 10620
   ttcagttgag aggaaaatgt aatccagagt tgttatattc tgagataaaa tcacaaacag 10680
   gcatatgtaa atctcgaatc ctattctgtg gtgcactgaa atagtgcagg ggtaaaaaag 10740
   cagtatggcc atattttcct aatggtaact tttccatata gacctacatt tcactgacac 10800
   ttttttctgt aaatattttg aatatggatg atcagtttct gtcacttgtc caatacctag 10860
   aagtgcctga caatgttcta acatgaattt ctggattggt ttcagaaaca gagtgactgg 10920
   taactatgtg caaatggctg gtctattttg atgtgagctt aattaatgct gcatatgaaa 10980
   ctccaaatgt cataatccag gagttttaaa attttaatta ttagactgat ttagttaact 11040
   tattctaaaa tgaaatttga atttaattta gtgtgagtat tcttttacaa taaattcctt 11100
   tttaagagag aaggatttca taaagtcttt ccatttagca tgtggatgag aactatataa 11160
   aaatgccaca aataaaatat gactttcaca ttacaacttg agctacaaac ttgcagggga 11220
   gggttgggct gcccagttag gacgtctagt cacaaagtcc aagccgccgt ctgatgaagt 11280
   tggagattag gagctgaggc cgttgctggt actccaccag gacatcatga ggcaagttga 11340
   tctgatcaca gtcgagtctg tcgagaagtg tcacacagac cacagcagct agaagagcaa 11400
   ttagcacaac tgatggaatg aatgggttca tcttttctat agaagcatta gccaaacaca 11460
   ccagcatccc tagcacatgt acttttgtgt atttttgttg ttgttgttgt aatgtagatg 11520
   caacctactg aaccttctgg tcttcaatat ttttgtggaa tcccaccaaa cacttcattt 11580
   aaataagtta actaaaattg ttgtgaacac tcctccttcc tcttttcctc cacaattgac 11640
   tagattaagg ctcaggaaag catcatgttc aagataaagg agaaagcttt gcaatctgag 11700
   atatgccatc tcctgctttc tatcctattt ccagttctta gaggagggtc ttttacggga 11760
   ggatataact ggagaactag ccgtaattcc caaggtgaca tttttctcct tggtgcaatg 11820
   gttgagtttg cctgctgcat tgcagccctt gctgaagaaa aaagtaaata cagaagagat 11880
   atagaacgct gacctcagga aataccaata aacgtgagta ctggctctca tgaaatttga 11940
   gaggaaaaaa catgatatga aatgcttaaa tgtaagaagc agcaccatga aagaggaaat 12000
   gtattagcat ttgatttcta aagaatgaga atgaaatctt ataggaaact ggaggaaaaa 12060
   aaacaaaagt cttttttaaa aaatgaaact cttatttttg aaaagttgtc acaagggcaa 12120
   tgtctttggt ttcatcagca agaaaaattg agttccatat gaattcttta ttgtcagttt 12180
   catatcaaat ctttgatttc aaggaatgtg gttacccaca ttgaaatcag tgttagccac 12240
   tttgaattgc taacggtgta ataatctcca tctgaatctg catcatttaa aaaaggattt 12300
   cagtacatat gtgattggat tggatgtgtc tgtattaaca aacttggaag gcatggagag 12360
   agttgggcat gagggtagtg tttatttctt tgacctctct gctactccag acttctttgt 12420
   aagatctaga tttgggaaga agtagtatac tgtaattata atagtacatg ctatctttat 12480
   ttttaaatga caaatagtaa ttatatataa ttatgaggta caatgtgatg ttttgatata 12540
   tgtatacatt gtgaaatgat taaattagcc taattcacat atctatcatc tcttatactt 12600
   gcatttttat ggtgaaaaca attaaaatct actctcttaa agatttttca ctatacgtta 12660
   ttacaaactc tcatcactat gctatgcaat agatctctcg agcttaatcc tccttgtaac 12720
   tgaaattttg cacccttcga ccagtttctc tccgttcccc atcctgctcc cctatactct 12780
   ggtaaccacc attctactct ctacttccat gagtttgatt tttctagatt ccacatataa 12840
   gtaaaatcat acagtatttg tctatctgtg cctggcttat ttcacttaac aaaatggcct 12900
   ccaaattcat ccatgttgtt gcaaatgaga gaatttcctt ctttttaaag gctaagtagt 12960
   atttcattgt gtatatatac cacattttaa aagtctattc atctgttgat ggacacttgg 13020
   ttgattccat atcttggctg ttgtgaataa tgctgcaaca aacttgggag agcagatgtc 13080
   tgtttacagc acactgatac cagttccttc agatatgtac cagaggtggg attgctagct 13140
   tatatgggaa ttcttagttt tttgaagaac caccatactg tttttcataa ttgatgtact 13200
   aatttacatt cccatcaaca gtattcaagg gttctctttt ctccacatcc tgccaacact 13260
   ttttatcttt cgtctttttt gattatggcc attctagcag gtgtgaagcg acacctcgcc 13320
   attgttttaa tttgcatttc cctgatgact agtgaggttg agtgtttttt ttaatatatc 13380
   tgttggcctt ttgtatgtct tcttttagaa cctattcaga tcctttgccc atttcttaat 13440
   ttgttttctt gctattgagt tgtttgagtt ccttgtatgt tctgtgtatt acctcttatc 13500
   agatgtatca gtttagaaat gttttctcca tgcgtttctc cacatttctc caatccatgt 13560
   atcttttttg tttgacttag ttattgctac atgccaagca tttatgatat cacttaatcc 13620
   tttacagcta ttccttgagc taggattatt tttgtcctct ttcgacagat gattaaatga 13680
   gggcttagaa tgatcaacca aatggcccag tattacacag ctggtcagag ccaatcaact 13740
   cctagcttcc taaactgatg agagaagagt tctctacctt ccatccactc ctcccctgag 13800
   ctagcacatg gtgaaccatt aggcaatcct gccctatcgg ttatactgac ccatcggtca 13860
   gactgaccct cttgttcagt gacgtgaaaa gcaatgaaaa tattcaactt gagctgcggc 13920
   aaatatttca tcacctagga ccctatttat tcaaaactca gtcagttgta aagcaacttg 13980
   gctgtacagg taaacaagca aatggaagag ctagaaactt cccacagctt ttcaagttaa 14040
   tgatcagcaa atctgtgaag cctgacatgg gctggcagtc gcacagaaaa gttgaaagtc 14100
   cttctctaat ccttcacttt ggcagaagga taaaaggaaa ccagtgctct atacttttta 14160
   cagagtggga accgacttta ctaaatgtct tttctggatt tcaaggggaa tgccaaagat 14220
   agaaaagaac aggttatagc aatctcagtg gtgttttctt gaccctggag aatccactag 14280
   cgagcagcat ataataatct tgtcacttaa aattactatt tccatgagca attaaaaggg 14340
   actaattacc caaaacaatt tgctgaaatt aagcctgaca gctttgctag caaaatcctc 14400
   agattaggca gggagggtgg ctgctgggga ggaagagata cttagtgacc cagctgccac 14460
   caaggagatt gtcagtgaac tgacctgagg ctagaatgtt ctgggaacac acagacctga 14520
   acatggagct tggatgcacc ctgagctgaa ctaggactgc ccagtaattg tcaacccttt 14580
   gggaacattt atggctgctt tgaaaatctg atgaaagtcc taattggaac ctcttttcac 14640
   acaaaaatac acatctgcca atacacacaa aatatttgtg tataacttca ggggtcccag 14700
   ggcccctgaa aattcactca tgaatcctta ggaatccacg cacccctatg gaccaccact 14760
   ttaaccccac ctctctgatt cagaatgaac tttcaacaag gttttcccat gaacagtggg 14820
   caaagatagc agggctgggc acaaatgccc attgcacagc agaagtcaaa cagggctggt 14880
   ctttggaaaa aggaaaagca gcaaagatta gctttggagt tcccagcctg gcctttcttc 14940
   tacctcttcc tggttatgtt cataccctcc cccattgctc ctcaagggtt tgagtttatt 15000
   aagcaaacac tacctttgct ccctatatgc ctggtatgta aagcacagga aatgtttttt 15060
   aatcaacaac ccagagtctt tgcacttcct gtatctccgc tgaaacatta atctcaggct 15120
   attggctgct tattaaaaac aaggtgggga aaatttgtat tcaaaggggc tggtcaatgg 15180
   taagagacat agaaacaatt tgttcttcta gagtcatgtt tctcaaaatg tgctccactg 15240
   gcctcttgca tcagaatcag agatacttag gctttattcc agacatacta aatcacaatt 15300
   ggggatgggt gttgggaaag cgcagtgaat ctgcatgaaa acatcttccc agttaaatta 15360
   tttttacatt aatatttgaa aatgactgct tcaaagactt taaactctat ctttgaatca 15420
   tcattaccac aaatatacat acctggaccc cagttctttg ggtgcccaaa caatgctgaa 15480
   ttagaaccaa ttcattatac ctatgaagaa ttgtcccata ccttttataa aggtgctatc 15540
   aacgacactg cccattataa tatagctgat aatttcattt aagtttaaca tttggatcaa 15600
   gattttgtga caaaccgcaa atgtttttac tagctaatgg tggctgtata atgcacagtg 15660
   gttttttttt tttgtttttt tttttgagac ggattcttgc tctgtcaccc aggctggagt 15720
   gcagtggtgc aatctcagct cactgcaagc tctgcctcct gggttcacgc cattctcctc 15780
   cctcagcctc ccgagtagct gggactacag atgcccacca ccatgaccgg ctaatttttg 15840
   gtttttttcg tatttgtagt agagacgagg tttcaccatg ttagccagga tggtctcgat 15900
   ctcctgactt cgtgatttgc ccatctcggc ctcccaaatg cagtgttttt gtttacagct 15960
   tggcttccag gaaactcaga gctcaatcta agtgtttaaa ttaagtattg tactcctggg 16020
   tggcctcttt aatactgaga acttctagcc agacacagtg gctcatgcct gtaattccag 16080
   cattttggga gggtgaggca cgaggattgc ttgaacccta ggagttcaag acagcctgag 16140
   caacaaagtg agacctcgac tttgcaaaaa caacaacaac cacaacaata aaaacccata 16200
   cacacacaaa ttcgtggttg tatgaacctg tggtcccagc tactctggag gctgaggtgg 16260
   gaagatggct ttagcccaag aggtggaggc tgcagtgagc tgtggtcata ccactgtgtt 16320
   ccaatctgag ttacagacag agaccttgtc tcaaaaaaaa aacaaacaaa aaaaaacctt 16380
   cctatctatt tccttaaata aatgcctcct attttttttt ttacctctgt ttaaattata 16440
   ttattatgca ttcctctttc attgaaagct gccacaaatg cttttttaaa tagaggcagt 16500
   acaggtcatc tttcgataga tgaatatatt acttttttgc accttatgga atagtggaga 16560
   gagtggtgat caggagacag gagatgtcta ttctaattca tctctgtttt agtctgtttc 16620
   ctcaggcagg tcaattacac tctctagacc tcatttcatc ctctgagcaa ggagcaggtt 16680
   agatctcaaa ggcttcttgc aagtccacat cctcagctta catgcctcac atgatttggt 16740
   tcgttcaaaa acctgctgtc ttctccagga ggtccagatg taatctcgag gacttgagct 16800
   tctgccagat gtgggaccgt taaccagaga tgctacagga taggtagacg atggagcctt 16860
   gtgtacttac tggaggtggg ggtgagaggc tcaccagggg aatccagata gcctccctgg 16920
   ggtcatagca ggggagagga gggccacaaa taatgacaac cacagacagg gtggaatcaa 16980
   tggcgctaaa tgatccctaa tgtgtgagtt gttggcagtg ggaagggaag agtagaatag 17040
   aggctaggag ggtggaatta aaatgaatca aacaaaacta tagacactga ccttgattaa 17100
   aatatgtgaa aaagcaaaaa gacacctttg taaaaatctt gtccctgtaa aagtaataag 17160
   tgaattatat accatttata cctgattttc cagctacaat gactgatttt cttggcaaaa 17220
   tgagggctct gcatactata aaataagtat ctgcttaatg ttttatttga tcccatcact 17280
   aagtcaattg gtatgtgctc agcccttagg gtttgcttgt ggcagtggtg caactatctg 17340
   ctcaccacac ttaaaatgga aaaatctgca ttgactttta gtggtgcaca ttttggctct 17400
   tccgtccaga gactatgaac atgttgtgtc cacacggagg ctgctggcaa gaaatggcag 17460
   gcagggtgca aagctgcctg agtcagccaa gctgggttca aagtccagct cacctgtttc 17520
   ccagctctga gaaggtggga aagttattta acctccctga gctacagtca tgtagaatac 17580
   atacagaatg aagtttttct cttgtaaagg tgaagagccc tgcaattggt ctccttgtac 17640
   cctggcctaa agatttttca aaatctggat ttatctccat tttagggtta agaaatactc 17700
   tagctccaaa ctgattataa cctttaatcc ttgtggtatc tttttacctt tatcaatgat 17760
   cctcatgcag ttctcagagt ccaatatcct tcattgttgg gaacagcaga agaatataaa 17820
   catggaatta aagtcaagat tctgctccct ctcattgaac tgtactaagg taatattata 17880
   aagactaaat atttccctaa gttaccagtg tctgtccaag gaaataacac tgaattcttt 17940
   aaagaaaagc cactgtgagt gctgcaaaca actagacata gaagaggatt taagcagaac 18000
   ctaagcgtag acaaatatta ggcttcatat attgtcaggc aagcctgtgt caaaaggcag 18060
   cactgtcttt aaaacacgaa tacaagttaa acatacttac cacaagttca cagtacaaga 18120
   ggattatcat ctttcatcag aatgtttggg gcttaattaa agtagatttt gctttaagag 18180
   agacacagat atgtgcatat tataaagtga taggagaatg catttattga gcattgcaca 18240
   gtgcttccat tgaacaggtt tacattatgc tgaaaattta gctttggaat aattgggtga 18300
   attccactat ttcccttcac ctgaatcttt gttaaaacag cattggattc ccaactctct 18360
   tttctaaaat aatgagatca ggatagcaac agtgacttct gggtgtcagt ggccttatta 18420
   taggtagttt gtccacctat caaatgttga cagacccaga ggttgtagat ctagcaaaat 18480
   aaatatttgt atagacctta gagcaaaggg aggcttgact ttaaggcttt atttccccct 18540
   ttgtggacag ttaggagata ctcctaagtc ctttagacaa ttctaagcca cttatatggc 18600
   tggggaataa aggttaaagt ttgcaatggc cattttccat agtcaaaaag agtctagtgg 18660
   cccttttttt tttcattcac tcactcgttc agtaaatatt gaaagcccat atggcaggca 18720
   ttattctaag tactggggat acagcaatga caaacaaggt ccctgttctt atggcactaa 18780
   tggagagaaa taatcaatat atgcctttat gcactaatat gcatgaaata atatataagt 18840
   taacatataa acagactgga aatatagtca gtagtggtaa atgtgatgca gataaatgaa 18900
   atagaatgac accattgtga gtgagtgagt gtaggtagtc atgcaagact tttttgagga 18960
   ggtgagattt agggtgagat gtgtaatact aactgcaaag ataataagtg gtaatccacg 19020
   tggacccaag gcctccttgt gctaagtaga tctagaaaga tctgtttctg caatggtcca 19080
   aactcagtga gtaattcatc taagttagta caatggttct ctagaagacc tttgtttatg 19140
   taagtatttg gtatttataa gccatcacct tagtatgaat tctgattggt tatccagtca 19200
   tctggtgtac taaaggaaca gaagaggtgt cagaaggctg tctggagaga taggataggg 19260
   tgggccataa ctaacagtgg cagggagggg gttagggaga catgcctatg ggccctgggc 19320
   tcttggtgcc aggtgaaatc ttttttccca tattgcttac catcagatgt cctctctaac 19380
   ttcctctgtt tcagcagctg tccatgaatg cattctcagc ccttccccaa gcaagctttt 19440
   ctggctcaca caaagtgcgt tataaatggg ttaaaatcaa aaggtgccag atgccagctt 19500
   ccaagcttca gtgttacggg aacaggatgt tatgaagtag taatgaaaga agagaatatg 19560
   atagcaccct atccatgggg ttttcggtct ctatacttgg tggctgatct tccaggggac 19620
   agcataccag gaatccacct tttagaacag taaatctgag ggagataaag agccgctggc 19680
   accatgaaga cattcagata gaaatactcc caagaactac agagctgcag atgcaacttt 19740
   tagtgtgaat gttggcagcc tagaaggggt ctactccatg aaaggctgca gcagtggcct 19800
   gggggagagc tatttgcagg cggaaatcag ctaccagcag gtccagcagc agcataagaa 19860
   agaaaatgca gtggagggca gtaactacag aaatgtagag aagaaagcat ccaggaggga 19920
   aacctgcagg tagcagagag agcaaggtta caaagatttg cagcaaggac agaaaggctc 19980
   ttagaataac ttcacttagg tttctgcaac atcctaaaca gagaaagaaa gactcagagt 20040
   ccagggtcca caagcctaga actcttcttg gctgccagtt ggtgcctctt aagaaagaac 20100
   tgctggtcat cacccggttt tcatgaaggc aacatgttga taactcggca ttttatgttt 20160
   aactattaat aaagtggtta acaaattatg ttacacttac acattaggtg gagacatgac 20220
   tcaagtgatc acttgtttat gtaataaaga aatgtgagga ggcatttcct ggagcagaac 20280
   catctgaatc caggtgtccc tgcaggctgt gtggggtgct gctttcccac cgtcactaac 20340
   ttgtagagag aaaagcagat gctcttgttc tctaaaagag gatactagca atgaccttca 20400
   aaagaataca aagatgggct gggcgtggtg gctcactcct gtaatcctag cactttggga 20460
   ggccgaggca agtggatcac ttgatgtcag gagttcaaga ccagtctggc caacattgaa 20520
   accccatctc tactgaaaat acaaaagtta gccgggcatg gtggcacaag cttgtaattc 20580
   cagctatgtg ggaggctgag gcaagagaat tgcttgaacc caggaggcaa agcaaagatt 20640
   atagtgagcc gagattgcgc cactacactc agcctgggca acagagggag actctgtctc 20700
   aaaaaaaaaa aaaaagagta caaaaatgca ttgcgtgttc ttcccacgtt tttaccaata 20760
   atttacacgt acatatattc tgacagatgc aagtagttaa ggatagtggt tcaacaatta 20820
   atctccttag aactacagct tttttgtaaa atgcattctt agaaatatga ccacatatat 20880
   tctgtttgta atgtgtagat taaaattcat tgactataag gacaagtttt gcaagtcaaa 20940
   ttccacttat aaaaatgaag ctttattgtg ctgtaattct ttacttcttt gaagccttcc 21000
   tctttgcata tcaggatgcc acacttccct ggtgtactgc caacttcatg ggccactcct 21060
   ccttggtctc tcccatctca attaactttt aacgtttaag caaggacctc ttgtcatctg 21120
   tgcctagact cattgtttgt tatcttaccc attgcatagc tttaaatgcc atctatatgc 21180
   tttcaactta gaattttatg tctccagtgc aaacgaccaa cctaaactcc agattcataa 21240
   attcacctgc ttgcttggcc tccattttga tgtctactac cctaatgtct tcaccccaat 21300
   ttcctctgtc ccagttatgg caacctcatg cttccagtag tttagaccat aaacatggga 21360
   gtcattttta actcctgcct ttctcaccaa atttacatca agttgtcagc aaatgttgtt 21420
   gggtttgtct tcaaaatgtt tctacaatct gactacttct cactacctgc gctgctatca 21480
   ctttgtccat caactctcac tagactactg cgttcgcctt ctcgttcttc tccctgcttc 21540
   tgcccttgac cccattcggg tcttcccagc tgagccgcca ctgtgattct gttaacacac 21600
   aggtcagatc cagccacctc tctattcaga gctctccagt ggcttctcat ttcacccaga 21660
   gtgaaagtca ttgttctcac agtgtcctac acaatctgta catcttccct ctctcctccc 21720
   attctccacc actgcctctc ccctcctgat ctcttccctg ttcctccaat tctccttgaa 21780
   tgctcctgtc tgtggccttg gatttgcatt gaacagaatg ctcttccccc agatatccaa 21840
   gtggcacatt cttctcccat ccttccctga ccatgctctt taaaattatg attgatggac 21900
   tattttagga atattttgtt tgtatcttgt ctcaataaat tcaattgtaa tagaaactac 21960
   tagccaccac caggatgaag gaaactgaac ttgctgtcaa gccctgaaca tgtcaccaat 22020
   tagctgttga accctgaggt ttagtttcct tatttgcata ataagaataa tgtcctggcc 22080
   actcacaaga tattatgaga taatgtatat tatatacaat tataagtcat gccttcagcc 22140
   caaggtaata tatagctgga cctctttttc ttctcctaag gtgtaaaagt agcttcatga 22200
   aagaggagaa tttgacccta agcattcacg aaaagcttac cttttagacc acagagtcca 22260
   cacatagctg caaacactgt agattccatt tcaatattcc tgacaccagc tttaaatgct 22320
   ctcttcaagt agtctaactt tttttctctg gaaaaggagc acagtgctcc atctagtcgg 22380
   ccttggcctg aaagggaggg gagaacgttt agtgacttga tgtcccaccc cactgacagt 22440
   ggcctcatag gaggcttgct gttggctgga gcacgctgcc gctgtctaac tctacagcgt 22500
   gctccccgtg taggataaac tgagctgtgt gactcttcct accagaggac tgtggagcga 22560
   tgctgtccct agctgaggag tctcatacgt aggaaggagc cttcatggcc gaggacctca 22620
   gttctggggt cagtaactgt tgagtggctg cagcagccag cactgtgccc agggaaatca 22680
   ggaagcgggg ggttgctgtg agagctgccc tttgaggaaa atctcaggtt gctccaatga 22740
   ctggggaatg gcccagggat gggtgctggg agaaaaaaaa agaggtttcg atgatgctaa 22800
   gctacttgag gatcaggaca gtgtttggac attgcgcctc tccatatgcc tttgcgacat 22860
   tgtccacctt ccccctttcc gcacacttct caggctgcac gccacccctt gtgatcacat 22920
   tttcctttcc aggctgctcc cagtgttcat tcatcactga atttcagctg tatctaccag 22980
   tgcggacaga agcaaggaaa ggaaaccttg atgtcattac tccttccgta ccctgtgcac 23040
   acccagaaat agacctccta atgcctcctc actcctgaga agacctggat tcaagcaagt 23100
   gggctaatag cactctgttg ccatccatta ttttcacaat cccctttttg gtgctctttc 23160
   ctctcttttt ctccctttcc tctatctctt tcacgctact ttcacccctg gcagtctgct 23220
   tccccttgtt ccttctcttc tcattagagc tgcgtgccac cacctctatt taagctgatt 23280
   caaacacaga catcgcagaa gccaagcagg taatgtcgat gcgagtgtct ggtctgaggg 23340
   gagcagcttc acctcacctt cagcaaatgt agccccagat gtgggtcagg gcttgtcaag 23400
   gctgtcatgc atatactggt gtacattttt aaatgctagt tatttcaaaa agccagaaat 23460
   gtgggtgtta atataaaatc tccagatatt gtaaatgttg gcaaaataat tcacattttt 23520
   aaaaatactg tgcaggccaa aggaaacatt gactttaaca agcttccatt tgcaaattct 23580
   gctttaacat atcagtgagt gaccctcgag atggactatg agtcagtggc cttgaacaga 23640
   gggtcccaac cctattggac cccaacagcc cttctatttt acaacaaata tgtggtgaaa 23700
   ccacctttac taaccggaag taaaatgtat agataatatg acctatctcc aaacataatt 23760
   ttttaaaatt caatatatct taagtttgag ctaaagggaa aataaaagta atttataatg 23820
   aaatagtatg gattttgaaa agaataaaac tactgccaga ttttcagaat gcccttcaag 23880
   gcatggtacc aatactgtgt agattctttc ttctgggacg agaaagaatc gggggtctgt 23940
   ctttgaaggt ttcttcccct tggcccttca tccctagtag caagtgctgc tacatagcag 24000
   tcatcttcaa tttgtctcta aacttggttt ggggacccct tactcatggc tgagagataa 24060
   aaaccccgct ctgagatcat gccatcccgt gggatttgta ccccctcccc aagcatttct 24120
   ccccattttc tggaccactg tttccggggg acaggatcat ttttgaggta cagatgaatg 24180
   gcagaaacat agaatcagca tacttccctt ctcttagagc tgctaattta agttaaaggc 24240
   ataaacagag ataagattct gtgaacatca ctaattgcat tttagtgttc aatgtgttct 24300
   tggaggatta aatccaccct caatataaat tcattctgct gctcattatg tccttgtgtt 24360
   tctatgcttt agtaagtaaa aaaggctgtt tcatttcctt tttaacccac aaatattaat 24420
   tttcttaagt agagttgtta atataaatta tgggtttgaa taacaaagag ttgctggcat 24480
   ttttctgttt ccttcacagt ttataaattg ttctcacctt cataaaaatc ataggtacac 24540
   attgtatgtc caacgagggt tgggaagttg gggatttctt tgctacagtt gaacagttct 24600
   tcagacagtt ctttgtccag ttcagtactt cgggtgacaa tgttgtccaa aatgacctgt 24660
   tcaaaccggg gcttaaagaa ggagtctaca gctatatccg ttattacaac agtccctggt 24720
   gcaatccctg caatgtggga ataaatgatt acagaagaat atcgtttaga gtttactttt 24780
   gacacatagt atgtaactta ttctagtatt aatgctagct gctaatgagt ctccctgttt 24840
   tcaactttaa aattattttc ttttccaaat gcaattaaat aacattcaga agaggcattt 24900
   acatttgttg tcttttcaaa tgcacattta caatgtgatg gggtttagat ttcaagacaa 24960
   acatgactct tggacatgca aattagtgcc agactttggg ctcaaatttg aataaattag 25020
   gtcaggaaaa aactaaataa gcctgccagc aagtagcttt cataaagtga tgtgacttct 25080
   taatatttaa tcttctctgc gacttcttga ttactgtaat ttctttgcat ctctatcctc 25140
   tttttcttct gattcttttc acatttgttc tctcgccgac ttcatgtaaa tcagggctct 25200
   tggctgacct tccatggcta caaagtctga gttgctcaag ggacttgggc ttacttaaga 25260
   aactattttc tgccaactgt tagtctccct gcctgccatg ccatctcctc ctccccccac 25320
   cttttcacca cacaccaagt cactcttcag gtgtcagctt aatttacttc ctgaaaaagg 25380
   tctgaatcca cagaagggaa gcaaacaaat gaaaaaatta aaaggcaaca cagataggtt 25440
   acactaaatt tccccaggcc catttttgac ccagcttttg acccagtctg gtttctattt 25500
   aaagcctgaa taagaagata tatcttcata aatgccctag actctctctg tcctaacact 25560
   ttttataatt acccatttgt ttatcttttt ctctgataga acataaactc tataaagtca 25620
   agaactatgt ctgttcactg ctctctcctg gtctagctct gtgcctgcca cattataggt 25680
   actcaataaa aatttgtcaa atgagtgaat gttttcacaa agatgccata gattatagct 25740
   ccgcaagcct atggttcata aaccaaatcc tgtctttgta aataaagttt tattagtcac 25800
   acagccacac ccatttattt acatattgtc tatagctgct ttcaagctct agaggcagag 25860
   ttgagaagtt gcaatagaaa tcacacagtc agcaaatcct aaaatatgta ctgtcttgtc 25920
   ctttacagaa aaagcttgac aatcctttct atagagtata gacttatcct tcagggtggc 25980
   ataaattttg atattgatca aagtagactt aattacaaaa tgctttagaa gatccaacta 26040
   gagacccaag gtggccactg ataatagagc cttgttgggt gaaatatttt tttttaattt 26100
   atttttattt ttattttttt tttttgagat gaggtcttgc tctgtcaccc aggctgtaat 26160
   gtagtggggt gatctcagct cactgcagtc tctgcctcca gggttcaagc aattctcctg 26220
   cctcagcctc cagaatagct aggactacag gcacatacac ccacacctaa ctaatttttt 26280
   gtatttttag tagagatggg gtttcaccac attggccagg ctggtctcaa actcctggcc 26340
   tcaagtgatc cactgcctcg gcctcccaaa gtactgggat tataaatgtg agccaccatg 26400
   cctggccaga tagtttttaa acaatccaag taagtggctc cttgaattgt tcacaatgtc 26460
   ctaagaaagt gattgagagg gaagttgaaa atatatacat aaaaatgatg ttatttatat 26520
   gccatcttaa tatttaaatg tgttagtgta ttatgtcatg gttaaggttt ctcaaaggtc 26580
   aacatactat ttaactgagt agtttatgct tgttaaggtg tgttggctca tgtaggaagg 26640
   gatcatgtga gcgacaatac tgtgtacatc tttcatctcc aaacgcaata ctgcttaaaa 26700
   gaaaataagc aaatacatct ttttggtaag ctaagagttc ttaatgagga taaaacttca 26760
   aaatgaaggt gtccacacca gtacagtggg ttaatttgcc accatccatg ggccttcatt 26820
   gtgtgctata tgtatcgtgt tgccatgaaa taaaataccc taatcaagag ctggatgatc 26880
   attggagcac cttgcccatg ctggtgtttg ttaatatcaa gtggtaactg tagcagtcag 26940
   gactttacaa tgcatgaatg ttggctatga aggcagatcc ttgtaaccca caacccaaga 27000
   attatgagca actttaatag ctatgttatc tgaaattcat gcccaaatga gcagttccct 27060
   actactgatt atttggggta ttgttggata aactacaatc tagaaggatg cctgatctga 27120
   cacttattgc tagcactcta cttctctcta tggatgaaca tccacaaagg tcctactttc 27180
   tcccagatgt aagggccttg gctggacttt attcctggac cacggctgtg gaaagccatc 27240
   atcttttcca acatgtctga tttctagtga agcatggctc agccatggtg gctttgtgat 27300
   gttgaacaca ccacttctcc acctacttgt gtgaggattt ctgacatccc tttttgctcc 27360
   ataaatgtct aattctacca ccatttctct tcccaaatct cagccctttt tataatgccc 27420
   attcagttct atcgccctgt caaccaaact tctccatttg ccatgtgcag tctgtatttg 27480
   cactttggtg acatggtaaa atcataccct gacaagatgt gctccttaag aaaaagctga 27540
   aagttttgag gaaacctcaa aacttcatca taacacattc tggaagcaaa accaaggcct 27600
   ctatacagct gactgacagg cctaagaaga agggaggttg ggaggctctg tgaaaactat 27660
   aggaagcatc acttaagcct tccctgaaag cctggcctgg tttctttcta gtgtactgat 27720
   gaggaaaaga tacagccagt agaggtgacg tagtaactga tgtgagcaaa aagcaatgtt 27780
   tttctaagag ctttgcagtt tgctcactta gctgataaaa ctgggacatt tttatgattt 27840
   ttttttttac ttgaacacac ttatttctgt cactttaaaa aaactcctaa gacttgaaat 27900
   ttacccctac cctcaaaaca tatcttctaa caatttttct actcttccct ttataaacac 27960
   ctaagactat cccgcaatag gcagtgagat ctctccctgt gggtttccat aaagaaaaaa 28020
   ggaataaaag aaaaaggcaa tgtgggcaga tttccttaag tttcctcaag tccagccttc 28080
   tgacactttt tagcccatct gggcttttgc tgggctctga ataggatgtt atattttggc 28140
   agctaccatg tatgtaagga tcactcagtt ctaatagtag acatcaatcc gtctcaccta 28200
   ttccccctga tgtaccgatt ctaataatgg tgacatcgca gcaccgtgca tggtggagta 28260
   atttgatgag ttcatgaagc ataatagaaa tggaggggat gcccatgccg tgctattgag 28320
   agaaagagaa agtcatcata catcttgcaa agtgatacat gaacaggaca taatttcttc 28380
   catcattaca agttaacagg cctcagatac atttacataa tcattaagac tttgccaatg 28440
   ctgaggaaaa tgtaagtcct ccctaattgg cacctacatg acatgctgac agatgtgtcc 28500
   atctgtgaag atcagaggga ggcatctcac tgccagttct gttgacttca gccctcatca 28560
   gcaagagcag aaggtgggga tggtgtggta tacaggtgtg tcagggcggt gccatttccc 28620
   atcttcccac tgggctcttc atgatgagcc tttgaaaaag agctttttgc acgattgact 28680
   tgattttgag gaaatataat tttggttgcc aaatcatcag tctttttcaa ccatactaaa 28740
   tgaaatagtg aatccatttc atttgctgtt aattttgtgt gctctgaaag gtactagagt 28800
   agaaacgtgg gttagattcc ccacttaaca attttaaact taaactgaac atggaagtga 28860
   gtttaaatta ctatagatga tgaccatagc cctgtttttt tcagtattca ggacagtgga 28920
   atcaatgtca ccagaaaagg gatcaacaca ggctacagga tttagcaact cctggtggct 28980
   acacaatgtc ctaataactc tccagaaaca tcctccattc tgtaatttgc agaaactgga 29040
   actgatttca aaactggtag taacttaatt cctgacataa cagcagagag tcaaatgtgc 29100
   tcttcaatgt gtcctacttt tggacacaaa gactttggaa aaccattggc cgtgaccatg 29160
   atgttttcac ctgagcttcc tccgtctatc aagaatgttg agctatggaa acttcgaacc 29220
   tctgcgttac ccctttctgg ttcccattgc atatgccctg actttcaaat gtttagtaag 29280
   catcctggag ccaggcatcg aacttgtatt gatttgtata tccaagatta acaagtttag 29340
   gactttggaa ctggctctgt gactaatgaa taaatgctct aagtattatt ttttcttacc 29400
   tgcaaaataa gaagatgaac tattatttga tctattagat tccttctagt tcagacattt 29460
   agtaagttaa tgattgccac ttattatgtt acgcattgat aaaatgacaa atggacttgt 29520
   gctctctttc acacacacgc accccattca accactactg gagaggaaga attttcaaag 29580
   aaggaatcac atttgtcaat cccctgcctg cctagtaagg cccatttcta attggtaacc 29640
   ttggcatcac acatatttca ggtctttttc ccagacagga caggatcagt ctattgctgt 29700
   tttcatttct tactgtgaag ttttactgct tatttatggt agtgtcacat ttttaagaaa 29760
   cttacttttt tgtccttctc catttactat acaaaaaaca gttatagtct aaaggtccac 29820
   taaatgttgg taagaaaaaa tttaccaaga tcagtggata ttcagcttcc ttctaatgta 29880
   gatattctgt aatggtttat tgaatgcaca agtgaataac tgaatggact ctcccatttg 29940
   agcaggtttc aggcgctctt ggcccagaga ggagagcaat ttagctgcct tattggtgtt 30000
   gcataatttt cccttcttta ccgcaggttt tcatcaatga ttgtccacac tgtagggcaa 30060
   tcaatggatg atgcctattc cctatggtag gagccactta cagcttagtt ccttagaatt 30120
   gccaccctgt gcaggagact cttcccctgt cccctggcat gcctgcctgt gcctcttttg 30180
   agggttgcag tgcccagaaa tgtgatggct tcagagttat tgcttcagaa tacagccctg 30240
   tgcagagaga caaacatggg aaggcaaatg ggatgcctat gcacatacgc cctcatcagt 30300
   gtgtgcccag ctgagtccaa gggggaaatg gtctgcatct tccctaaaac tgcaaatcat 30360
   tcccctttca ttttgtctta gggcactgtg tgcttccata actgttcaca tgttctaaat 30420
   gcaggagagt aatttacaag agctcagtag aaatgcatta gcagcttttc agcgagctta 30480
   ttatggtgtt aaacagcagc actttagcag tcctgaaaat tagcctctgt gtactaaaag 30540
   ctcaacattc ccaaagctgg gtaagggata gaagaaggaa aattgccgtg gcgggggtga 30600
   ggggtgttaa ccagttacaa agtaaagaga aaggaggggt gctttgagta ttttccccct 30660
   aatttgaata cctaagtggt aactaataaa caaaaatgat gctgcatgct gtgttctgcc 30720
   ctgggggctt tttccaataa tgaactcata cctggacacc ttgtttgatt gtgtaagaat 30780
   gcgaagctgg agggaaaaag aactcctaca tgtaaaagtt cccacttttt tctctagcct 30840
   gcaatgacta gctgaaatgc aaccatggat acttacactg atggcgagca caggcccggt 30900
   tttgtacata cagtatctgt ctgtcccagc acagatgtct tttatgtctt cttcagcttc 30960
   ctcaaacccg agctccttgt gcataaacag tgcaaatgct ttcattctgt tggggctccc 31020
   accgacacag acaaactgtt aataaaaata aacaagggcc tgccatagta actgggataa 31080
   gaaccacggg tcagtttcaa ctctaatgtt tttattttac tttttagtta atttacttaa 31140
   gaacacgtgt agatgtcatg gtttatgttc catgaattag ctaatttgca tcatcaacct 31200
   gtaaataaag caacagatgc aaagtttttt gcactaaatt tgtaaagaaa atcacaaaat 31260
   caaaataaat gttgatatta ttcaaggagg tgtttcctgg tgtttttgtt tatagtgaac 31320
   accaaccagc atcttaaatt gacatagaca gcctattgtt gagcattacc atgacatatg 31380
   cttaggatgt aagttctgag gccacggcac gtgagtgatg taagagcaaa aggagaacca 31440
   ccagttttag gtatgttttc tgattcccca tcccccatca taaatttgta ttgtgaaatg 31500
   gagtatgtga acttaaaatt ttaaaggaat catgtcaaga cttttaaatt tcttattcag 31560
   aagaaattct catggaacaa gccttattat tgtaaatgtg ggatctccct gtggcatttc 31620
   atcaattcca gtgatttatt tttgtttttg ttttattcac attttacctt ctctgaaatt 31680
   aggataggtg ttgcagtcaa cggtgtctaa tagaagatgg taatacaatt gtctggcagt 31740
   tactcataca acagtctgtt taggggtacc ctagccccag gtaccaggag tgggagaggt 31800
   ggacgccagg gagaggagca gagaagcact acgggtcagc aacaagaggg tgagcagtag 31860
   cagactctca cacccaggag gaagcacagg ccacagcaag gtggcaagag tcaagtcctc 31920
   ccggattctg aagtaagtgg tgaggaaaat gcagagagag agaagacaac atagtggagg 31980
   gtctgccctt tgaggttagg taaacctggc tttgaactcc aaccccatca gtgagtaaga 32040
   aacttgctca gtgtcacaca aataatcgat gtgcctcaga acatctccaa aataggaaaa 32100
   acacagtacc tctcagagag ttttgaaaga agaacgtgag attgcatatg gcaaaggaca 32160
   ttgcacaagt ccagacacgt tctcagtgat gtgcaaatgg taaccattat tttttatatc 32220
   atgtagcaat tttctattcc tgcttctgag cttgttactt ttcctgtatt gcagaattta 32280
   gtacacagga agctggtttg agtcctctag tgaggaaaag gcagttggtg gtatctggct 32340
   gatgtaggca gcattggaca gaggactggg agggctgaca aggtccatca cagggataaa 32400
   gaatatctgt ctaacgaaat acctaactga ggttatagaa taggcggccc cattcacaaa 32460
   tgatttactc taactattgg gtggtctgag tacatgatgc atcttgtttc catttagcaa 32520
   taattcctga gctattctct ttctcaatta ggggtgtaat taaccctgta tccacgccct 32580
   ctgcttctct tctaccctta atattgtctt ttctctgcct tcttatgagt ttctctgatg 32640
   aagccttaag ctagtaatga actgcaatag aagtctgcca taaagactgg tagcaaagag 32700
   gttaagacca tggtttcagg tgagttttaa actagttatt gataattctt agaattttct 32760
   tctttgttgg tatggatagt taagacttgg ctcacgatct aagtccaagg aaatttttta 32820
   acacagtacg gtagtttgaa aataacctca ttgtgtggtg ccagtgaatg taaattacaa 32880
   ctattcagat tgaccataaa accaaaacag atgttagaat agcttgtggt ttgaattctc 32940
   ctttgaactg tagatattgc tataatcgac aggaatttct gaatatttct ggagacataa 33000
   agatcatatg cacagacaga agcagcttgg ttcttaaagc cttccttact cctaaaccag 33060
   actcactaaa caccacctct ggacacaatt ttcggtctgg ctgctttaat gagaagtaaa 33120
   atatggctga agaccaccaa ccaacattga ccttaagtga cacttactgt gtttatgtct 33180
   caaacaaaat gatcaattgt cctggtttta tcaaagaagt tagaaacaca ctgggaagga 33240
   gtcagaggca ttacaaaaat atgttaagga ttttgcctaa tgattgccag aaaaaaactc 33300
   agctttagat atccctagca tcttaaaagg cttaactatg ccatgttgta ttaatctggt 33360
   ctattgcatt ttatgcattt tctgtgttaa caagggaata taaatacatt caaaatgcat 33420
   tgcatattac taaaatttct agtgagagat tagaggatac cagttagtga ttttataaaa 33480
   gacatattta gatgttgatt ttgaatagat aacttccttt tttttttaga gttcaattta 33540
   tttattttta ttttattgct tgtgttttgg tatcttacat aagaaatcat ttcctaatcc 33600
   aaggtcacaa agatttatgt ctatgttttc ttctaacatt ttatagaatt aagccttaca 33660
   tttaggtctt tcatccattt tgagttaatt tttgaatatg gtgtgagata gggatctaac 33720
   tttattcttt agcatgtgga tatctagttg tcccagcacc atttgaagag atgattcttt 33780
   tcacaataaa ttgtcttagc atgcttgttg aaaatcagtt gtacataaag gtgagagttt 33840
   atttctggtc tcttggttct gttctagtga tctttatgtc tatccatatg ttagtaccac 33900
   actgtcttta tcattataac tttgtaataa gttttgaagt tgggaagtgc gagtcctcca 33960
   acttttttct cctttgtaaa aactattttg gctatcctgc attccaggca ttttgacgtg 34020
   gattttatta gaagcttgtc aagttctgca aaataggcaa ctggaatttt aatggggact 34080
   gcattgaatc tgtagaatat ttttggtagt atagttatct taacaatatt aaatcttgca 34140
   atccataagc ataggatgtc ttcccattta cttagagctt tcaaaatttc tttcaataat 34200
   gcttgttgtt ttcagagtat aagtttgcac ttcttttgtt tatttgattc ctgtatattc 34260
   ctgtaaggtc tatatgtccc acttcaagtc gtacaacatt gattaaaagt agatttttaa 34320
   acattaaata tgtttactgt aatccttaga gcaaccacta aaacaaataa gcagagaaac 34380
   aaaactgaaa caaaacaaag agatatagtc aaaatataac agataaataa aaatggaata 34440
   tttaaaaaat gttcaactat cccaaaagaa agctggaaag aggaataaag aaatggaaag 34500
   cagagcttaa tacacagaaa tcaaatataa aatggtaaac ctaaatccaa acatatcaat 34560
   aattacatta aatgcaaagt gtctaagcac ccatttaaga cacagagatt ttcagaatga 34620
   ataaacaaac atatgatcca attatatgct gtctataatg ataatgatac aggcagatta 34680
   aaagtaaaag aatggaaaaa tatagaccat gcaagcttta atctaatgct ggaatgacta 34740
   tattaatatc aaaataggct tcagagcaaa gaaaattacc agagacagag ggatattcca 34800
   taaccataag agtcagttta ccaagaagac ataacattca aagtgtataa aaactaaaaa 34860
   cagagcttaa aaaataaagc aaaaactgtc agaaataaag tattaataaa ctcacaagca 34920
   tagtttgaga cttcacctct ctcagtaatg catagaactt atggaaaggg aatcggcaag 34980
   acttaaatgt tagacctaaa accataaaaa ccctagaaga aaacctaggc aataccattt 35040
   aggacatagg catgggcaag gacttcatgt ctaaaacact aaaagcaatg gcaacaaaag 35100
   ccaaaattga caaatgcgat ctaattaaac taaagagctt ctgcacagca aaagaaacta 35160
   ccatcagagt gaacaggcaa cctcagaatg ggagaaaatt tttgcaatct acttatctga 35220
   caaagggcta atatccagaa tctgcaaaga actccagcaa atttacaaga aaaaaacaac 35280
   cccatcaagt gggcgaagga tatgaacaga cacttctcaa aagaagacat ttatgcagcc 35340
   aaaaaacaca tgaaaaaatg ctcatcatca ctggccatca gagaaatgca aatcaaaacc 35400
   acaatgagat accatctcac accagttaga atggcaatca ttaaaaagtc aggaaacaac 35460
   agatgctgga gaggatgtgg agaaatagga acacttttac actgttggtg ggactgtaaa 35520
   ctagttcaac cattgtggaa gacagtgtgg cgagtcctca aggatctaga actagtcctc 35580
   aaggatctag aactagaaat atcatttgac ccagccatcc cattactggc tatatactca 35640
   aaggattata aatcatgctg ttataaagac acatgtacat gtatgttcac tgcggcacta 35700
   ctcacaatag caaagacctg gaaccaaccc aaatgttcat caatgacaga ctggattaag 35760
   caaatacacc atggaatact atgcagccat aaaaaggatg agttcatgtc ctttgtaagg 35820
   gacagggatg aagctggaaa ccatcattct cagcaaacta tcacaaggac aaaaaactaa 35880
   acaccgcatg ttctcactca taggtggaaa ttgaacaatg agaacacttg gacacaggaa 35940
   ggggaacatc acacactcgg gcctgtcatg gggtgggggc agtggggagg gattgcatta 36000
   ggagatatac ataatgtaaa tgacgagtta atgggtgcag cacaccaaca tggcacatgt 36060
   atacatatgt aacaaacctg caggttgtgc acatgtaccc tagaacttaa agtataataa 36120
   aaaatatttc ttcttttcca acattagcct ttaattctat aaatttcctc tcagcactct 36180
   acccacattc tgtagatata atatgttgta ttttcatttt tcctcagttc aaactatttc 36240
   ctgccttccc cggaaatgta tatcctgctt tgtttgagtg gagaatttta taaatgtcca 36300
   ttaaatttga atagataact tctaaggatc ttccaccgta tgtattaatt gtctgaatct 36360
   ttctagtgag tatggcagag attgattagc tgttcaccta aacagtttgc tcttctttct 36420
   gggcacccag ctaaacttaa catctcagcc tcctttccag ttagacaagg tcatttgtct 36480
   gggttccagt gactagaatt tgagcagaag taatgggtac catttccagt cctagcccat 36540
   agaaatctcc catcctacaa ggaggatgta ttggtgaccc tacatattgt cgctgtcatc 36600
   tgctggacag gtgttgacac actgggaaac agcaatttat gccttgaagg aaacagtaac 36660
   aattatggaa ggagcctggg tcctcgaatc accagatgta gggaaaccat ccatcaacta 36720
   agaactccca ccttgtacct aatgtgagtg agaagtaaac ttaaattgta ttaagctact 36780
   gagattttgc agtagcaagt agcaaaaatt tttaaatatg gtaagaatat gaaggaaata 36840
   ggagtgattg ctggagtctg aagtttcagc tttatacacc cagagttctc ctttcttgga 36900
   agataatggt attaataatg attatcatct ctgtgaagac aagcctgagt tttcaggtac 36960
   tgatggatta tgtcctcttt tcttagccag gactgctgcc ctgggattta aggaactcaa 37020
   gaaattgaat gtagaatgtt ctcattcaaa gaggagaaca tggctggagg aaggcaagtt 37080
   tctgaggaaa ccatctatga gaataaatca gactttacac gctgccttga aaaaattcta 37140
   cttcagatta aatggccagt tctgaacatt tggagctagg ttacctgctc ctcaatttac 37200
   aaacacacac acacacacac acacacacac acacacacac acacacacgc ttttgcctcc 37260
   tattcatcaa aatggctcaa tcagtagagt tgctgtttga tgggataaaa catgtttgaa 37320
   acagagcatg tctttttatc caaaagataa tttactgaag gttcacaacc aggtgggtgg 37380
   tgaatgtata aggttggata gtggttatga cagacagtgt gtgagaataa gtgctgaaca 37440
   aaatctggac aggcagacgt gaaggtctgt ttcctgggat tataaccgag atgagacaac 37500
   tatgtgtgtg tgcgtatgtg tgtgtgtgtc tgtgtgtgtg cgtgtgtgag agagatacat 37560
   acatgtgagt ccccttattt ttatctctaa aacaaagctt tccatcccaa taatttccca 37620
   gaaactaata cttggagtac taaaaagtac taaaacatag tgcttttgtt tttttgtttt 37680
   tgtttttgtg ttattttgtt ttgtttgaac agagaaagag ccattatttt tttatcggag 37740
   aaacctaagg tttaatctca gctatttgac ttaccagctg tgttgtgcct attacagaca 37800
   aacatatatg tgttataaca ctatgtatat atttgagcct gtgttttctc atctgtaaag 37860
   tggagataat aactatagtt gctgtgggga ctgaatgtac catttgcatg tgccacacag 37920
   agtctgatat atagaaagcg caccataaat ggtagaggag acacagacac ccaagatatg 37980
   tagatagcaa atcactttct gtctcaaata tatatctcaa gtcttttaaa tgtattaacc 38040
   agctgtgtgc cttagttcat taattgtgaa aagtctttca attaatcaga gtcatcccta 38100
   gacaagggtc ctgggatatt attgagaggc atgaacctca aatctaacat ttgaattctg 38160
   gtacaatctg gttattgagt attaaagacc gccttgatca atgactcttg gagcataagt 38220
   aaaccaaaac agcagcaggg gaagaggtta cattgagaag acaaagcgca aaatagaggc 38280
   aggccacaca aaggcaagga gcacagcctg gagaggagtg aagaaagggt gggtcacctc 38340
   actgccagga cacccaccca tgccctcaca ccttccttcc taggagagag ggagcaggag 38400
   aaggccactc agcacctact aatcataggc ataatagatc tatttcttct cattttccca 38460
   tctttttttc tttccttccc tccttcctta ctttcttgtt cccttccttt tcctctgcct 38520
   ccaacctctt cctcctcccc ttttctcacc tgcctcttga cccactgggt aagtgaaaga 38580
   gctaggatta aataattatg gactatcctg agttactgtg actcaaaggg tcctcctagt 38640
   gcaatgagca agaaccctag agattggact tggaagaact taatccaggt ctcagcacta 38700
   ccactctatg gtgtaactgt aaccttcctc cacctctctg aacttaattt ccttgtttta 38760
   tggagagagt taatatctgc taaacccatc caaaagagct aaatctgcag atgttagtga 38820
   gatagtagat gtgacgcttt tatttttgtt ctcgtaattg ctaaagaaac gaaaaagtca 38880
   gatgaaaaca gcgcgattct ccattagtgc aactgtaaat agatacagtc ttttggaaaa 38940
   caaaagttct ttaaaaattc agtctctttg accatttctg tgaatcaccc taagcaaatt 39000
   ggcctaaatg tagaaaatct ttagttacaa aatattcatt tgattcctgt ttataatgga 39060
   aaatatttta acataaaact cagtaatagt ctctgtaatg tgtggtacac cacttgacac 39120
   ccataaatga tatgtttcaa aagaatgtaa taatatggag aaatgttaat gatgtaaacc 39180
   taaatgatga agtgcagaat gcagttatat aattacaact attttaaagt ctattcatta 39240
   aaaatgattg aaagaaaaaa atgagttgat catgtctatg tttaggtggc aggactattg 39300
   ggtgtttttt aacaatcata ttcttttttg tattccctat tttaaataat gtacacaaat 39360
   tgcttatata attaaaatat gtcagataaa ataaataatg cagaagtaca attttatggt 39420
   agtatttaat aaataatttg tatgaaatat ttcttatgaa acaaaattct taatatgaaa 39480
   tattagataa tggttatttc ccagaagtga aataatatta tattgttagt tttgttgggt 39540
   tatttttatg agttaataaa acgagaacta gtgagattaa gctataaatg tagatgctaa 39600
   atagtgagct gaatctgtta aaacataatg aattctcaac acccagcagt attgtcaata 39660
   aaattatatt tatctccagg ggccagagta gccaaataac atgtttgcat tagatttagt 39720
   ttgggaacct ttatgtcttg ttgaggtgga aatgattgtt catgatatac acattccaaa 39780
   ttgtttcaga gaatgagtgt gacttcagtt cctattggga ctgtgctagc caaaggtgta 39840
   tagattattt tggcaaggta agaagaaaaa ttagtagaga aaactcaatc atttttcctg 39900
   aaattagaaa tgtttttacc tttacatctc caaacattgc tggtaggttg tgtgtttttg 39960
   ttcccaaatc caagtgatag agaatgtctt catccatcaa atccaagtaa ggatttttaa 40020
   cgtgaacaaa cctttttctg gaaaaaaaaa ttatacaaag aagatataaa agaattttga 40080
   gagaaagctc acaagaaaga aagtctgaac ataaaccttc ctttagggtt aaattatttg 40140
   ttgagaagca atgtagtata acagtaatta tggctaatat ttgcccacac tatgtgccaa 40200
   gcatagcact gagcatgaaa atggcaagaa aactgcatat aacaaatgca ttaactcatt 40260
   tgtcccataa ctgccctctt agctgggtaa gattaataat ttagtttaca aagaagccaa 40320
   ggcttacaga agttgagaaa cttgtgcaag attaacaacc agtaaattac aaaacagcca 40380
   tttctattca gttctgactg taacggtcat ctaatttctt tgcatgggag gctgtggaat 40440
   ataaactact gggctctgga ttcagattgc ctgggttcca gtacaagttc aaccatatat 40500
   atcctaattg tgcaaccttg agtaagttat ttgtatttta ttttatcttc tcaaagtgta 40560
   gcatgcataa caatcagccg gacacttcat ggactcttct tctggaaatt ctgattcagt 40620
   gagttgctga tgtgtgtccg aatttgcatt tctagcaagc tcctcattgg tcctgatgtt 40680
   gatggtctat ggacaacact ttgaatagcg ctgccattga ggactccacc aaccatgctg 40740
   aacacaccac tttctttcta ccccactggc catggccctg gctcaaaccc ccatcacggc 40800
   agtgctagct agaacaccaa tccttcctcc cttttctttg tgaaatacca cgaaattcaa 40860
   gacagtgagc tgatgacggc tataagaatg accttaattc aatctagttc atatttatga 40920
   agcatatcat ttgtagcttt cggttatttg gatagtaatc atatgccact agagttgtta 40980
   gtgataaaag ttatctaccc ccacaccaca gtgggtcaag acgtctttgc caagaactgt 41040
   ctgagacttc tttgttcttt gttcctccat ttctctccac atgcctgccc cctcccctcc 41100
   cctcccctcc cctcccctcc cctcccctcc cctcccctcc cttcccttcc cttctcttcc 41160
   cttcccttcc cttcccttcc cttcccttcc cttcccttcc cttcccttcc cttccctttc 41220
   agagtttcgc tctgtcaccc aggctggagt gcagtggccc aatctcagct cactgcaacc 41280
   tccgcctcct ggttcaagcg attctcctgc ctcagcctgc tgagtatcca ggattacagg 41340
   tgcctgccac catacccagc taatgtttgt atttttagta gagatggggt ttcaccatgt 41400
   tggccaggat ggtctcaaac ctctgacctc aagcgatcca cccaccttag cctcccaaag 41460
   tgctgggatt acaggtgtga gccatcatgc ctagcctttc cacatggctt tcattccata 41520
   cacctcaaaa cattttacca ggccttgtgt ctttctatat ctttatcgat tctcacttgg 41580
   gtttgcatta aaattaccag ggagctttaa aaacaaaaac aaaaacaaaa aaacactggt 41640
   gaccaggcac taattaattt agacactctg tattttaaat ctaaagattt taagtatttt 41700
   taaaatctca aatgatcttt ggtgaagcca aagttgagaa cccctgcttc aggcctttgt 41760
   ccttcaaaca tccttccccc tttcttccac ttttcaccta tcaaattcca attttccttt 41820
   atggtccaat tcaaatatta cctcttccgt gaaggctttt ccaagcatct tctctccttg 41880
   atcagtatta atttttttct cactgtgttc ccaaagcatt tgtttcatgc taatgctggt 41940
   agatagcatt tcacattgca atatagttaa gtttccatcc ctctagaaag actgcgaatc 42000
   ccctgatgca agctatctca aatatccttc ttgaatccct aagttttaac aaattctatt 42060
   gttgaattaa atttaactgt tcagaaattg gaacaaaata ctattaattt ccttctcaaa 42120
   aacatctaga gttatagatt tttaattagt tactgggtca tacgtaccta gtttcaaaaa 42180
   taaagtattc cgtggtgtta gtacacatta ctactacaga acaagcttca aaatctcaaa 42240
   gcaaagggac atttcctttc ttctcttgtg aggcaagaat ttatccctag tcccttgtta 42300
   tacagtgtgt ggtcagtgaa ccagcagcat caggaacatc tggggagctc attaggaatg 42360
   caaagtctta gattctaccc catacctgct gaatcaaaat ctgcatttta acaaactctg 42420
   tgtgattcac atgcacatta aagtttgaca aacactgcca taaacttttt cttccaaatt 42480
   ctattacact ttctgtttca ctctgccaag actactttca cgacctggaa atattccttt 42540
   ggattcctaa ctgaatctat gaatcattac tctataaaag agtctcattg ctctacccta 42600
   gtgattccaa actccagcta catatcagaa ccaccagggg cactttaaag agtacttatc 42660
   cctagaccct atctctagaa atttggattg acttggtcaa ggagacctgg gctttggtgt 42720
   ttttaaaatc actcatctat acacacactt cctatcttca aggagaaatt ctccaaccag 42780
   cagagccatt aattattttc acttatatta attataatac tattaattca ttaggattag 42840
   ttttccttga tgccctgtcc caacgtctaa tgatttctcc gtcagaaaat ttgggggact 42900
   gtctatagaa gtttcttgtt tcattgggct catccagaag cacttctaag atgatcactt 42960
   tatcagtacc aaagaaaaaa cttgtttggg atttcatgcc aaatttctac ccaatatgaa 43020
   tgtagttttt ggaatatttg atttttccaa aatatataat ttttactaaa taggtttata 43080
   tgtgttgttg aatcttcaaa ctcttcttcc ttttcctaac acatagaata gtctttttag 43140
   taaatcgtga caaattacac tacatgatga gtattaaata ttggtaacca aaagaaagtg 43200
   ctggctgagc agaagtcttt ccatttgcca ttaccttcat cttcataatt tttccttttc 43260
   tttttctcct gggcttccct gaagaaggga acatgcagga ggctacctct gtttgggatg 43320
   ggcaaatgga gtgttttctg aaatgctgac ataatggagg ctctaagcaa gacatttcac 43380
   acaactctca ggaaatcaga gggaaccagc aaaatctcct tatgcctcat gatgattaat 43440
   gctctgttgt gttctgttaa cctggggcag gaactggagc taaatttgta gacagcttca 43500
   gcaattttat atctacccat gagatgaatc acatagacgg tatttcagaa gtaaattcta 43560
   tgtaaggtcc atcatgaagc atatgaaatc actccacaat atcctgcaat acaattaaac 43620
   ctccctcagg cactgggagg cccttttaat tatttctcat ttgcagccca tggtcttaga 43680
   attaagggct gaactctata gtgagtccca ggctgaacct tgaatttgtt tactactgaa 43740
   ggttggctaa acttttgcat gttttttata atttattttg ccccattttc ttagatgagc 43800
   caaaccctaa cccccaaaac caaggagtaa aaatgtcatt gaaaatacat tttccactgt 43860
   ttttattgct ttatgagctg catccagtga tataatctct acatttaaga aatttgctta 43920
   actgtagagg cccattatct aatcatttaa tacaaaatcc aaggagcaac catctgatca 43980
   aaacaaattt acaaaatcaa aattactcac ccaacatatg tattcctgtc agatctcatg 44040
   gacctattgg aggcaggtat aactgaagcc attctctcta ctatgtgaaa agtcacctta 44100
   aattgatgtc aagaaagaga gatattgtga cttgttatta aattatagac ctagtcataa 44160
   tagttcagtt cccagtcaga atgataacct ctcttaaaac ttaggttttc ctgtgaaatt 44220
   gctaagaaaa tttttccttt agcattttaa attttcctat caaataaaag atgtcctccc 44280
   tgacatgact tttataccac agcccagctc tcagagcagc acttcctcaa gaagataggc 44340
   cccctttgaa ggtgtggcat cctagctctt tgaccaggtt ggttagcttg ttaactattg 44400
   gtgagaactt tcccttacat ctgtccttga tcccaacaca ggtttgatgt tgcaaacagg 44460
   cttatttgct ttgagacaac tgtatatgga tacgagtcct cttttggttt ctataacaca 44520
   cctaccaggt cttctcaaaa ctgtgtccag tacatacgag aactttcagt tctcaaacat 44580
   ttcagttccc catctccatc cagcccctcg ccaacttgtt ttcattcttt gtcatcacaa 44640
   gttagggaag catccaaagt ctggacctca gaatgtccct ttatctgaac tttgctcatt 44700
   ccagttcaca cagaagaact gataattaaa caaaacttat ttcccagggc ctctttcttt 44760
   agttaaactg taggccattg gtaaaattta ttgaaccagg ggttaatcca catggaataa 44820
   taatacagca tagtattttc agaaaacgga gtctctgcca agaaaggcat gaattcacta 44880
   ctttcactaa tgcttctcac ttttcacttt tgggacagaa aaatctagaa aactagacaa 44940
   cttcatcctg atcttttttc tcttcactcc catagcaatc agctgaagtt ctatgctggt 45000
   gagagcttca agaacgtagt ctcatggctt tcaggcaaat cagaacctaa aatgtctata 45060
   aatgccaaac agcagcaaat acaagacacc tcctaatatg cttggatttc tttccaagaa 45120
   tgtgcatcac agtcaagaat caagtgctca tatcctaact gagttctcca tgttagaaga 45180
   tgctcctctt cctggtgaca gtcattcttg gcaaattctt tgaatgcccc actggcctcc 45240
   tttggtccat cttggtcaga cagcaaagtt tacagaatag gaaggaaaaa gaatagggga 45300
   ctctatattg gggaatccat gtagcctaaa tcttgccact gttaccacca actagacgtt 45360
   tgcaactgtg cccagatagc aaaaaattcc tgaggacact tatcctggaa aaggtaatga 45420
   tgactatgag gcaaaatagt ctttaacatg tgtgttgcct ttgtaactta gaacagtagt 45480
   tcttgttgat agcttgtata catctgtctc ttacctcctg actcatctca ggctggtcta 45540
   cctgctgctc cttcccagga accactggca attttgagac tcaacatact cactcactac 45600
   tgctggcacc agcacacctt tatttgatat tccatgccat atctctcaat tttattttta 45660
   aggtataatg aggagaacca ccaatttcaa tctggcattt acctctggct ttgagaggta 45720
   aaaatgttag ctaattagat aagtgttcag taattaatta ccacagtgaa ttaactaggt 45780
   tgattaacat gtctgtttcc ggtgcagatt cagtcaacca gtgatcttga tcactaacag 45840
   tgccaggtaa tgccatctaa gatttatgga ccccatgtaa caactcggtc agacatttag 45900
   gcaagtaatt gtcatttgga tgaagttcag aataatcttg tggaatctat aaaagttgat 45960
   gaaagtagaa tactagttaa tgccttctgg gcagtcttaa attattctcc ccacctttca 46020
   atcttctcaa gactcgatcc tgctcttatg gtaggttgtt tctcagcaat gctttttgat 46080
   tgactgtatt gcaaaaggcc agtcacctga agagcagggg aaaaggtttc gtttttaaaa 46140
   attatgcaca caggagtgca caaggcaaac tcagacctgg ccactgggta atttgcaaac 46200
   aaaggctgtg tgccaggcac agcccatgac tcagtccagc cttgtgatca ggagggtctt 46260
   gcatcagatc tgccaaatcc atgctccatg gtgtcttgcc tgagacaaaa cacttcactt 46320
   cctaccgtct tcctttatga tgctggcact attacattct caattccatt acagttttct 46380
   atctggctta attttgtccc tgaacctcta atattagaaa aagaaagcac agtcttgatg 46440
   tacaactgga tagcatttag gcctccctga gaccatgctt ttgtgctctt tccctgcctg 46500
   ctagactagc cctgggacca gagtctcaat tcttagatgt ctctccatgg ggtatcagca 46560
   aaggttgtat tggttcacct gaggctgcag caggcacccc ttcccagaag acactgcccc 46620
   ttacacatgc acacacacac tagcctcaca gcctatggga gtaggggtgt caacgcacag 46680
   caaagtcctc acatctcctc cttctttggc gggtctcaga gtggggatgg ggtaaccaag 46740
   agccctttcc tcctgccaca tcagattccc tggctcttcc attcagccca tagcaccagc 46800
   aatggcttga gagagctcca aaaatgtcct aagctatcca ttctgaggct aagcagttta 46860
   tattttcatt tattcattca ataaatatta actgagcata caccatgctc caggaagtct 46920
   aggaagtgct gaagataaag gcatgaaaaa gacagttctt gcttcttttc ttccacaaaa 46980
   atattgacat tccatgaata aagaaaacgc aagttaccac actcctcata ctaaccttct 47040
   gtatgttaaa acacatatac taagtgtaaa ttcagcattc tctccttcgt gacaaataac 47100
   taaaactaat taaagatctt ttgatctttc cttgagtgtt tattttccaa tacataggtt 47160
   ataggtgcct caaaaccttt gtataaaagg ggagctatta agagtgtaca attttttatg 47220
   gtatagatgt gttatagttt aacattatta catatacgtg caaagaaaat gtacagtatt 47280
   ttctattagc tatccagtct ttctcagtat acgcaccctc ctggaagata gagcagagga 47340
   cagtgttttt aaacttgcca attaacagca tctctgtctt tggtttaaat agtctcattt 47400
   agatgattta ttttctttta gatacgcgtt catccaattt cttccttgtg cccactgctc 47460
   ccatctgagg tgacacgtac aaaatggaat ggtggggatg agcaaggagc aagagaaggg 47520
   ctcctggatc catgtgctca tttccttgta ttatcccttg gctctatgtg atgatgcagc 47580
   tcaggcattg tccgctgttt tctcataagg gacctgtggc tacttaggtg tagtctatgg 47640
   gtccttgaat gcgctcactg agatgcagct ggccccatct ggccctaagg gagcattatg 47700
   ctggccctcc ccctcaccat cttggatgca ggcctaagct gaatatggaa attctcctag 47760
   agaatcacaa cctttcttag gctgcctcga aggatgcagg cctctggctg cagtgttctc 47820
   aggctggcct atgcctagtt ggatagggat agcaccctcc tcctttattt tcacccccaa 47880
   gggtgggtgg tcaccgcaga ccttggcctg cctcttgcac catttctcct gccctcttac 47940
   tctcataatt aaaagaggag accctctttg tactttttct ctctgtctta tgtgaatttc 48000
   ccttatatcc tatcctgagt tctctctggt ttctagcaat ctccgtgttc aagtccagac 48060
   ctaggttttt ttgtgttaat gacagtataa acatatctaa aatctttatt tttcctcaca 48120
   ttgccttatt agggactcat aattgctatt gaaagaattc taatttccta ctctaactcc 48180
   cattccccag cagatagacg ggagtgtgca ctatgaaaaa ggccatgtat acagaaaaga 48240
   aaaaaagaga ttagtattag gtttcaaagt actatattat aattataagc aagaatgata 48300
   tccctaggac aaatgctaca accctacggt gtttgttttg ctcttttttg gagggtgttt 48360
   ccctccacaa ttctgtcaaa atgcactgct gttctaggca gggccaggca gaggggtggc 48420
   agctgaatgt ttgcacagat accttccccc gagggttggt gtagtggagg ctgacccatg 48480
   ctgaggccct ggggaggatt gcagcatccc ctaatccttg caaaggactg caagctcctg 48540
   tttccagtct caggagatgt ggttatgttg taaaagtttt aataattgtg tatgagattt 48600
   agaaatacaa aacaatgaca tattttccag gacccacaag gcattgacat ctggaatctt 48660
   tataaagttg aaatttgcct gaggaaaatt tcttgattga cgaaatttat tttttaccag 48720
   aattatatac acactctctc tatatatata tatatagaga gagaaaccag gaatttctct 48780
   ttggacaatg attaaagtta agaaaataac aagtttcaaa ttgcgtgaac atttatttat 48840
   aggcttttgt aggggtttga gtaaggagga cttgactatt gtcagccatt ccagaaaggg 48900
   tctgtctcca ggtaaatgga aacgccacat aagaagtgtc atacttatca tgcagtaagt 48960
   gattattaag cacctactat gtgctaagtt tgttaatgct cgaagatgag taatgactta 49020
   ctttatttta tttttttctg gttcatattt tttatcatgt ttttaaaact ataaaatatt 49080
   taatatatat tacaaagtat tcagaaaaat ttaacatgtt cttttctacc agacttaata 49140
   ttaatttgaa aacattaaaa ttaatccatg tacagttgaa gatttatcca atctcatttc 49200
   cttccctttc tccccagata agtaaacata accctaaatt tgggaagtat acttctggtt 49260
   caagtgtttt ataagttgaa gtatagatga taaaaaaata gttttggttc ttctgcttaa 49320
   aaattttacc ttaatactat cccactctac cttcctttac aactttgttt tcaaaaattt 49380
   aatattatat atgcaatgat atcaaattat tgttaggcat aggtctaatt catcagatta 49440
   ccatatagtg atctgttata tgacattaca gcaatttatc cattctccta ttgatggcca 49500
   cttattgttt attatattta tttatttatt tatttttgag acagagtctt gctctgtcag 49560
   ctaggctgga gtgcagtggt gcaatctcag ttcaccgcaa cctccgcttc ccaggctcaa 49620
   gcaattctcc tgcctcagcc tcctgagtag ctgggattac aggcctgtgc caccatgccc 49680
   agctaatttt tgtattttta gtagagacgg ggtttcacca tgttggccag gctggtctcg 49740
   aactcctgac ctcaggtaat ccgcctgcct tggcctccca aagtgctggg attacaggcg 49800
   tgagccacca cacctggccc actattttat tattttttaa taatggtctt tgttctcagc 49860
   ctgttttttc tggcaacatc caaatgaaaa gaactttact tacaacttcc ctggcttttc 49920
   tggtggcctt tcttatcacg agcctgactc ccacagtgtg accaaagcgt aaattcactg 49980
   ttttctaatg gctgacctta agccaacacc agagcctcag agtcaacaga aaactctgaa 50040
   cagccagcaa aggtcatcag tgcctctagt ggcctggtca acagagggcc ttaagactga 50100
   aaaacccttg acttctgcat gtacttgact tcaattttta ggatgtttct gttcttaatg 50160
   atgtttgaga aaatatgagg caccgcgtgt ctaacaaaaa ttcaagaaaa gctatttcat 50220
   tggaaataaa gcatatttta aatcaatacc agtagggaat aaaatgtaaa agcttttgtc 50280
   taagctcgaa ataaccacat tcaactcaga atagaaatct gaaggataag gaaaaataat 50340
   gaaaaaaaaa acaggaagaa tgtcagtaac aacagtggct aatagcatta ctccaggcca 50400
   ggctttggac taaacagttc gactccgtat ctcatttcat cctcacaaca cgtctgtgag 50460
   ataggtatta ttatttcccc aattttcaga tgaggaaatg gaagcctaga gaggttaaat 50520
   caaatgcctg agatcctata gtgagtaaat ggagattgga gttcagatct agatctgtgc 50580
   agccacaaaa ccatgaaaac tctttctact ctgcttctgg tacatggtgg agtccattaa 50640
   agaagacaaa gtccaaggag aatggcctca acaagagaag ggaatttgat ctgggtgttg 50700
   aaggtgaagt aggactggga tgtaaggaac atctctctgc cacattttac cctgctagtc 50760
   atcaggttgt tgatttaact catttatagg aaaagagggc acagttaaac catagcagct 50820
   gtgtgttctt tgaatgctga tgaacaagat ggccccatca aaatgatggc ccagtcctaa 50880
   agctgatttt aaagtcatct cattgccaaa ttttaaattg acccttaaaa tgtctctcac 50940
   acacacttta cgattaactc atctcaggct catcacttcc ctatgatttt acccaaatag 51000
   cagggcccaa ctcaccaccc tgtctggagc aatttctgtc tgcccctgag ctgtccctga 51060
   aatattagtt aaagggtatg agggctgaga ggctctgttt ctgctggtct cctagcctgg 51120
   cttcctcact gcctgtgact ggccattctg gtgacttcaa gccttggaga ttaaagcccg 51180
   tgttagtctt tcagcacctg atgaaagcgc ttaaccagat catgaagtac attgggaaat 51240
   cagcctccac agctggagga agctctgtga gtttgacaaa ccccacactg aagggagagg 51300
   gggtgatttt cctgggaact caaggaaggt tcagtcctaa cctctaacac acaccacaga 51360
   ttattactgc tgctgcccaa gagggctcag atgggaaaat aatgctaata cattgtggag 51420
   gtggtgaatt ctaggccccc aaagtcatga aatttcaaag ctgactgatt ctaatttagg 51480
   tttagctgag tgaaagagaa cagtatgttt tatggaactc tcttaacatt actaagtgca 51540
   atatattgag ttaaccatta attttttttc taaggaagta gaaaaccttg gttgtataat 51600
   catggtcaca ttgacatttc tgtaaatgct tcaccttagg catgtcatga cttttcaaaa 51660
   atgttattct tttgaacact ttacttggtt ttttatttaa agtaactaaa cagaaaatac 51720
   tgttgctgtg gtgattgtta ggtctttgtg actacttcat gttagttact atagttaaaa 51780
   tttttaattt taaaacattt aaacataaag gtacagatat attagatgcc tgtgtagtta 51840
   ctacccaaat ttaacaattg ttacattttg ccatatttaa cacatttttt tttaaaggaa 51900
   agatttcatt aaagccctgt caccttcctt actttagatt accttcactt tcttcctgaa 51960
   ggtaatcact gtcctaaagg tgaagagttg gtaggtatgc ttttggggta tatgttaaaa 52020
   ctcgtactta taagtatgta cttaataact acatgagcag gtttaaggtg tgtgcgtgtg 52080
   tgtatgtata tggtatatgt atatatatgg tagatatgta tatataagaa tcatatgtat 52140
   atataatata tggcatatat atataaatgt aacatatact atatatatat ttttaaaact 52200
   ggctttttta tgttgcttcc aattttttcc tgtaaacatc cttatgggta tcttcttgtg 52260
   tatgcacata agagtttctc taatgtgtgg taataatatg tttgcatgta ttcagtttta 52320
   ctagatagta ttacattgcc ctacctaata gttttaccaa gttacacttt tgtcaacagt 52380
   gtataataaa ccccatttcc ccatattgtt gccaataaat ctgatgttgt tagccttata 52440
   attcttttgt taatgtgatg actgtaaaat ggtatctctt tgctttattt catttccctg 52500
   attactagtg agattaaacg cctttatata tgtttggcag tctttcagct ttcttctcca 52560
   ttctttgccc gtattttctt ttgggtattt tatttttgtc ttactaattt gtaggagatt 52620
   tttatatatg atgaatactg atcctttgtt ggttatatat attgtccttt acttccaaac 52680
   tttccatgtc attatgtttt aggagtgtat caggtaaaca atacatcaat ggattttcaa 52740
   aaagttgtct tttatctatg tttaatacat ttacaaatac tgtgattcct gatatatttt 52800
   tactcctttc ttcttatttt ttttctaatt gtgtgtgtgt gtgtgtgtgt gtgtgtgtgt 52860
   gtgtatgttt ctttttaccg ccttcctaca ttctatagaa tgattaagtt ctctgtgctg 52920
   ggcgcaatgg ctcacgcctg taatcccagc actttgggag gctgaggtgg acagattact 52980
   tgacgtcagg agtttgagac cagcctagcc aatgtggtga aaccccattt ctactaaaaa 53040
   tacaagaatt agccagtcat gatggcgtgc acctgtagtc cctacttctg gggaggctga 53100
   ggcacgagaa tcacttgaac ccgggaggcg gaggttgcag tgagccgaga tcatgccact 53160
   gcactccagc ctgggtggca aagtgagaaa aaaaaatgat tacatgattc tttgcagacc 53220
   ccatttccct ctgttatttt ggaagttata tattttattt ctattctttc aatagttacc 53280
   cttacatttc aacaagcata cttaatacaa ttcaaagcta attaatacct atactcttct 53340
   gcataacaca agcaacttgg aacaatttaa ctctaattac tacttcacat tttggtataa 53400
   agtaacttaa tctatcttgt tttcatccct tcctcccttt tagtcttatg gttaccactt 53460
   tgttgatagt taataataat ttacattatc catgtctgcc aatgtctttg atcacaattg 53520
   cttctcagat ctccttcctt ctgggtttct ttctttctaa agtacattat ataatagttc 53580
   ttcagcatgg gctttgagct gaaaactatc tttgttttat cctcattttt gagtgactat 53640
   ttagcatggt ctagaattcc agattgccag tgattttccc tcagcactca tgggatgttt 53700
   attcattatc ttctggtacc cattgttgct gatgaaaagt ttgttttcat ctgcttgtgt 53760
   ttgagtaagt aatctgtctc ttctttctgg ttgcatctag attttctctt tatatcctga 53820
   agctttatta aaatgtgttt aggtgtgact tttttagaaa ataacttatt ctcatggctc 53880
   actgtgcttc caaattatga aaaaacctgt ctgttatcaa ttttaggaaa ttctcactat 53940
   ttcttcctga ataggttatc ttcttctggc acccctctta gatgtatgtt aggccacctc 54000
   agtttgtcct cctaatgtat taacttccct tttatacatt caatttgtct ctcggcaata 54060
   aaatgagaaa ttttctcaga tctttcttct tcacacactc tctgggtgtg cttgaccact 54120
   cttaacctat ctgtggttag ttgcagtaat gtcctcaatt atttactcca ccctgtatct 54180
   acaccctatt ccatataact atgcaatgcc ttctcaccat aaactgggta acattgactg 54240
   ctccttgacc atggactcga tcatgccatt attttgatca agaaaatgag tcagaactga 54300
   caatgtgcca gttgttcatg cctttgcact tccccattgt catactaaca tgcccagggt 54360
   agtctgctgg aggaagagaa gcatgtaaaa cggagcccag gcacctcgtc accccagtga 54420
   gggccagcct agatcatcca acagccagct gagccccaga agtgagcaag cccacgaaga 54480
   tcagcaaagt cactttgcca gcccagctga ccccaggcac atgagtagga aatgcttgct 54540
   gtgtgtcttt caggacctat ggcaatagca ttattgtggc aacagggtaa caaatatacc 54600
   atgcattgag atttttcgtt tactaaactt tttcatttgt aggtaagcta attattactt 54660
   tttcaaatta acctatattt aaaaaccgta tcctattttt tctacatatt tctgtttcat 54720
   cttttgtcta tgtaatcatt ttaaacatag atattttata atttctttca ggttgttgat 54780
   ctatctgaag ttcttgaagt gctaatcttc ccattacagc tgatttctct cattagtgtt 54840
   tcctcatttt acctataatt tcaattttgt agtccatatc tattttacca tgggaatcca 54900
   gtacacagaa atatttgaaa gcatccccag gcagcagttt tgtatttgct tctgctagag 54960
   gctacaagtg tgtccacagt ctaggaccag tttgacatta atgtcttatt ttaaggattt 55020
   tggtaccatg taggtagcat aaactgtcat tctacaaaca caagtgacgc gttacttccg 55080
   tttctcactg gagactgttt ttgtacccac gtgccacaga gacaaacttt ctttgcattc 55140
   aagacagtca ttgggatgtt agccctcttt cactgaaggt acagagtttc ccagttttgg 55200
   cttaatgtag aagtttcagg ttcaacggtc acctacaaac caggaccctg tgtatacatc 55260
   ctgcagaatc ccagcacctt ggcacggggg cctattccaa gtttgatcct ctctgggcct 55320
   cagtggcatc agctcatgag cttacattct tcatttctaa aacttggggg ttatctcttt 55380
   tattccttaa agctaaccca tgctagcctt tagacaatac tccttttatg tctgttagga 55440
   aagttgtcca tgtgggtccg tggccgtgct gctggcagat gctttgttta acaagcaaaa 55500
   ataatattgc aaaaaattag aaatgaaaat ttttttaaat ttatttttaa ttgctatggg 55560
   taaataatag atgtatatac ttagatacat ttggtttgtt tgtttgtttg ttttttgggt 55620
   tttttgggtt tttttgagac ggagtctcac tctgccgccc aggctggagt gcagtggcac 55680
   catctcggct cactgcaagc tccgcctccc gggttcacgc cattctcctg cctcagcctc 55740
   cggagtagct gcgactacag gtgcccacca ccacgcctgg ctaatttttt gtatttttag 55800
   tagagacagg gtttcaccgt gttagccagg atggtctaga tccccgacct cgtgatccgc 55860
   ccgcctcggc ctcccaaagt gctgggatta caggagtgag ccactgtgcc cggcctacat 55920
   ttgatgtttt aatacaggca cacaatgtgt aataatcaca ccagagtaat taaggtatcc 55980
   atcacctcaa gaatttattt atttgtgtta ggaacattcc aattccaccc ttttagttat 56040
   ttttaaatat ataataaatg attgttgact gtagtcactc agttacgcta tcaaatacta 56100
   gatcttgggg aaaatggagg ataggaggca ggactaattt gcagctccca ctcggatgga 56160
   cagagcagca tgtagagact aacatcatga acttttgctt caagaactac tgcagaaaca 56220
   gccaagagag tccacagacc ctttgaagga agtgaactgc tgctgcaggc cccaggatat 56280
   acccaaaaaa ctgtgagtgc ccaaaatgtg aaagggggat attctgcccc caaacatata 56340
   cccttactgg gaaacctgaa ggtccagatc ccagatcaca ggagaaggat ttgaccttac 56400
   ctggagctga gatgaattta gagagtcaag tgaaatacag gggtagaagc agcagcgaga 56460
   agagccctgt gggcactttc attctccagg gatgccattt ctgactttgt ctcacaggga 56520
   tcctttggga gggctgccag aggaactggg aaatgaccag agagaaggaa acttccagct 56580
   gaactttgta acaatttcaa ccgagtgcaa agtttcctgg acagaagttg gggtcggggg 56640
   tggttgaatc gggagtgcag acacagcaca gaaactgcag caggcaggga ggtgcaaaat 56700
   ctgaaagccc tgcttgcttt ctaaaccagg aggctggtag cctggggcaa gttctcagcc 56760
   ctgcttaccc actgcctgga aacaaactca gtgctgtcaa aggggcacgg tgggagtgag 56820
   cccagtcatt caggctgcag agaagccagg tgaggcctgt aactaacaac tttcccccac 56880
   tctcctggca acctacatga tgcacaaagg caaccataat ccccctggga acgtaactcc 56940
   attggcctga gaaccacacc cctatccccc cagcagccac tgcaagaccc cacaaggaga 57000
   ctcagagctc agacacacct aaccctgccc ccacctgatg gtctttctct acccgctctg 57060
   gtagccaaag acaaaggaca taatctcttc agagctctat ggccctgccc actccctgat 57120
   cctccctaca ctatcacagc taatgctctc ttgaaagcac caccccctgg ctggaggcca 57180
   accaacatga aactagcaca ataagcaaaa ctacaaccag ggaccctcac agagtccact 57240
   tcactcccct gccacctcca ccagagcaga tactgttatc tacagcaggg agacctgaag 57300
   acggtccaca tcacaggact ctgtgcggac actcgccagt accagcccag agtccagtag 57360
   ttgcaatggg tggctagatc ccgaagagaa attacaatca ctgcagttca gctctaggaa 57420
   gccacatccc tagaggaagg gggagagcaa cacatcaagg gagcacccca tgggacaaaa 57480
   gaatctgaac agcagccctt gagcccaaga tcttccctct gacatagtct acccatatga 57540
   gaaggaacca gaaaaaaaat tctagtaata tgacaaacaa ggttctttaa cacccctaaa 57600
   agatcacact ggctcaccag caatggatcc aaaccaagag gaaatctctg aattgccaga 57660
   aaaagaattc agaaagtcaa ctattaagct aatcaaggag gtgccagaga aaggtgaagt 57720
   caaacttaat aaaatttaaa aaatgatata tgaagggaaa aagcttcagt gaaataaata 57780
   gcataaataa aaaacaatca ctacttctgg aaataaagga cacacttaga gaaatgcaaa 57840
   atacactgga aagtctcagc aatagaatca agtaagtaga ataaagaact tcagagctcg 57900
   aagacaaggc tttcaaatta actcaatcca acaaagaaga ggaagaaaga attttaaaaa 57960
   atgaataatg cctccaagaa gtacgggatt gtgttaaatg gcagaaccta ggaataattg 58020
   gtgttcccga ggaagaagag aaatctaaaa gtttggaaaa catatttgag agaataatta 58080
   cagaacactt cccaggcctt gtaagcgatc tagactccaa aaacaagaac ctcaaagaac 58140
   atttgggaaa tttattgcaa aaagactatc acctcagcat agtcatcagg ttttctaaag 58200
   tcaagatgaa ggaaagaatc ttaagagctg tcaggcaaaa acatcaggta gcctaaaaag 58260
   gaaactctat cagattaaca gcaggtttct tagcagaaat cctacaagct agaagggact 58320
   ggagacctat ctccttaaac aaaacaatta tcagccaaga attttggatc cagagaaact 58380
   aaacttcata aatgaaggaa agatggtctt tttcaggcaa acaaaggctg agagaatttg 58440
   ccactcccaa gccagctcta taagagctgc taaaaggagc tctaaatctt gaaacaaata 58500
   ctcaaaaagc accaaaatag aacctcatta aagcataaat ctcacaggat ctatataaca 58560
   aaagcacaat gaaaaacaaa caaaaaaagg tattcaggca acaaatagcg tgatgaatag 58620
   aatagtacct cacatctcaa tactaaaatt gaatgtaaat ggtcaaaatg ctccacttaa 58680
   aagacaaaga atgggagaat ggagaattca ccaaccaagg atctgctgtc ttcaagagac 58740
   tcacctaaca cataaggact cacataaact taaggtaaag gggtggaaga agatattcca 58800
   tgcaaatgag catcaaaaat gagcaggagt agctattctt atatcagacc aaacaaactt 58860
   taaagcaaca gtagttaaaa atgccaaaga gggacattat ataatgataa aaggacttgt 58920
   taaacagaaa aatatcacaa tcctaaacat atatgcacct aacattggag tgcccaaatt 58980
   tataaaacaa ttactattag acctaagaaa taatatagat ggcaacatat taatagtggg 59040
   ggactttaat actccactaa cagcaataga cacgtcatca tgacagaaag tcaacaaaga 59100
   atggatttaa actataattt agaacaaagg gacttaacag atatttacag aacattatac 59160
   ccaacaactg cagaatatac attctattcc tcagcacatg gcgcattctt taagatagat 59220
   catatgatag gccacaaaac aagtctcaat aaatttaaga aaaccaaaat tatatcaagt 59280
   actctttcag accacagtgt aataaaattg gatatcaact caaaaaggaa ccttcaaaac 59340
   catgcaaata cctgcaaatt aaataacctg ttcctgaatg atcattgggt caacaatgaa 59400
   atcaagatgg aaattaaaaa attctttgaa ctgaaagata atagtgacac aacctatcaa 59460
   aacctctggg atacagcaaa ggtggtgata agaagaaagt tcatagcctt aaatatctac 59520
   atcaaaacat ctgaaagacc acaaatagac aatctaaggt cacacctcaa ggaactatag 59580
   aaacaagaac aaaccaaacc caaacccagc agaagaaaag aaataacaat gatcagagca 59640
   gaactaaatg aaattgaaac aaaaaaaata caaaagataa attaaacaaa aagctggttc 59700
   tttgaaaaga taaataaaat tgatagatca ctagtgagat gaaacaagaa aagaagagag 59760
   aatatccaaa taagttcaat tagaaacaaa atgggagata ctacaaccaa taccacagaa 59820
   atacaaaaga ttattcaagg ctactatgaa cacttttatg tgtataaatt agaaaacata 59880
   caggagaaac tagaaatata taaccctccc agattaaacc aggaataaat agaaactctg 59940
   aacagaacaa taataagcag tgagagataa atggtaatta aaaatgccaa gaaaagtaag 60000
   tccaggatca gatggagtca cacctgaatt ctatcagaca ttcaaagaag aattcgtgcc 60060
   aatcctgttg acactattcc acaagataga gaaaaaggga atcctcccca aatcattcaa 60120
   tgaagccagt atcaccctaa taccaaaacc aggaaaggac ataacaaaga aaactgcaga 60180
   ccaatatccc tgatgaacgt acaaagatac aaaaatcctc aataaaatac tagattaact 60240
   gaatctaaca gcatatcaaa aagataatcc agcatgacaa gtgggtttca taccagggat 60300
   gcagggatgg tttaacatat gtaagtcaat aaatgtgata caccacataa acagaattaa 60360
   aaacaaaaat cacatgatca tctcaataga tgcagaaaaa gcatttgaca aaatccagca 60420
   tccctttatg attaaaaccc tcagcaaaat cagcatacaa gggatatacc tcaatgtaat 60480
   aaaagccatc tatgacaaac ccacagccaa cattataccg ataggggaaa agctgaaagc 60540
   attctctctg agaactggca aaacacaaga atgccctctc tcaccacttc tattcaacgt 60600
   agtactagaa gtcctagcca gagaaattag acaagacaaa taaataaagg gcatccaaat 60660
   cagtaaacag gaagtcaaac tgtcactgtt tgctgatgat atgattgtat acctagaaaa 60720
   ccctaaagac tcctccaaaa agcttcctag aactgataaa tgaattcagc aaagtttcag 60780
   gatacaaaat taatgtatga aaatcagtag ctttgctata caccaacagc aaccaagctg 60840
   agaatgaaat tgaaaactca atccctgtta caatagttgc aataaaataa aatataatgt 60900
   aatacttagg aatatacata accaagaagg tgaaagacct ctacaaggaa aactgcaaaa 60960
   cactgctgaa aaaaatcata gacaaaacaa acaaatggaa acatatccca tgctcatgaa 61020
   tgggtagaat caataatgta aaaataacca actgccaaaa gcaatcttca aactcattgc 61080
   aattcccatc aaaataccac catcattctt cacagaacta gaaaaaaatt ctaaaattca 61140
   tatgtaacaa aaaaagagtc tgcatagcca aagcaagact aagcaaaaag aacaaatctg 61200
   gaggcatcac attacccaac ttcaaactat attataaggc catagtcacc aaaacagcat 61260
   ggtactagta taaaaatagg catacagacc aatggaatag aatagagaac ccagaaataa 61320
   acccaaatac ttacaccaaa ctgatcttca acaaagcaaa caaaaacata aagtggggaa 61380
   agtatatcct attcaaaaaa ttgtgcaggg ataattggca agccacatgt aggagaatga 61440
   aactggatcc tcatctctca ccttattcaa aagtcaactc gaggtgtatc aaatacttaa 61500
   atctgaaacc tgaaactgta aaaattctag aagataacat cagaaaaacc tttgtcgata 61560
   ttggcttagg caaagacttc ttgaccaaga acccgaaagc aaatgcaaca taaacaaaga 61620
   taaatagatg ggacttaatt aaatgaaaaa gcttctgcac agcaaaagaa acaatcagca 61680
   aacagacaac ctacagaatg ggagaaaatc tttgcaattt atatatccaa caaaggacta 61740
   atttcctgaa tctataaaga actcaaatca gcaagaaaaa aaaatcccat caaaaagtgg 61800
   tctaaggaca tgaatagaca attctattca agaaggtata caaatggcaa acatatgaaa 61860
   aaatgttcaa tgtcactaat tatcagggaa atagaaatca aaaccacaac acaatatgag 61920
   cttactcctg caagaatggc cataattaaa aaataaaaaa ataataaatg ttggcatgga 61980
   tgtggtgaaa agggagcact tttatactgc tagtcagaat gtaaactagt acagccacca 62040
   tggaaaacag tgtggagatt ccttaaagaa ctaaaataga gctactattt gatccagcaa 62100
   tcccactact ggatatctac tcagaggaaa agaagtcatt atatgaaaaa gatacctaca 62160
   catgcacgtt tatagcagca aaattctcaa ttgcaaaaat atggaaccag cccaaatgcc 62220
   actcaatcac catagataaa gaacttgtga gatatatata tatatataaa aaacatatat 62280
   atataaacag acataaacat atgtctatat atacgtgtat atatatatat gtatatatat 62340
   atacaccatg gaatactaag ccataaaaag gatgaaataa tggtattcag agcaacctag 62400
   atggaattgg agacaattat attttttttt aattatactt taagttctgg gatacatgca 62460
   cagaacctac aggtttgttt cataggtatg cacatgccat ggtggtttgc tgcacccatc 62520
   aactcatcat ctacattagg tatttctcct aatgctaccc ctcccctaac cccccatccc 62580
   ccaacgggcc ctggtgtgtg atgttcccct ccctgtgtcc atgtgttctc actgttcaac 62640
   tcccacttaa gagtgaaaac atgcggtgtt tggttttctg ttcttgtgtt agtttgctga 62700
   gaatgatggt ttccagcttc atccatgtcc ctgcaaagga catgaaatca tcttttttaa 62760
   tgactgcata gtgttccatg gtgtatatat gccaattttc tttatccagt ctatcattga 62820
   tggacattta ggttggttcc aagtctttgc tattgtgaac agtgccgcaa taaacatacg 62880
   tgtgcatgtg tctttatagt agaatgattt ataatccttt gggtgtatac ccagtaatgg 62940
   gattgctggg tcaaatggta tttctgattc tagatccttg aggaatcgcc acactgtctt 63000
   ccacaatggt tgaactaatt tacactccca ccaacagtgt aaaagcattc ctgtttctcc 63060
   gcatcctctc cagcatctgt tgtttcctga ctttttaata atcgccattc taactgatgt 63120
   aagatggcat ctcattgtag ttttgatttg aatttctcta atgaccagtg atgatgagct 63180
   ttctttcata tgtttgttgg ctgcatgaat gtcttctttt gagaaatgtc tgttcatatc 63240
   cttcacccac tttttgatgg ggttgttttt tccctgtaaa tttgtttaag ttccttgtag 63300
   attctggata ttagcccttt gtcagatgag tagattgcaa aaattttctc ccactctgta 63360
   ggttgcctgt tcactctggt gatagtttct tttgctgtat agaagctctt tagtttaatt 63420
   aaatcccctt tgtcaatttt ggcttttgtt gccattgctt ttggtgtttt agtcatgaaa 63480
   tcttcgccta cgctcatgtc atgaatggta ttgcttaggt tttcttctag ggtttttatg 63540
   gttttaggtc ttacgtttaa gtctttaatc catcttaagt taatttttgt ataaggtgta 63600
   aggaaggggt ccagtttcag ttttctgcat atggtggaga ccattattct aagtgaaggc 63660
   actcaggaat ggaaaaccac acattgtata ttctcattca taagtggaag ctaagctata 63720
   aggatgcaaa ggcctaagaa tcatacaatg gactttaggg acttagggaa agagtgggag 63780
   gtggtaagcg ataaaaggct acacgttggg tacaatgtat actgctctgg agataggtgc 63840
   acccaaatct cacaaatcac cattaaataa cttattcatg taaccagacc aggtataatg 63900
   gctcatgcct gtaatcccag cactttggga ggccaaggca ggcagattac ttgaggtcag 63960
   gagttcgaga ccagcctggc caacatggtg aaacccatct gtactacaaa tacaaaaatt 64020
   acccgggcat ggtaatgggc acctgtagtc ccagctactc aagaagaggc tgaagcagga 64080
   gaattgcttg aacccaggag gtggaggttg cagtgagccg agatagagcc actgcactcc 64140
   agcttggatg acagagtgag actgtctcaa aaaaaaaaaa aattattcat gtaaccaaac 64200
   accacctgtt ccccaaaaac ctattgaaat aaaaataaat ataaaaaata ctagatctta 64260
   ttaattttat ctacctatat ttttatatcc attaatcatc tttacttttc cccctcccca 64320
   ctaccccttt tagcctctgg taacatcatt ctactctcca tctccatgag ttcaattttt 64380
   ttaattttta gctccccaaa ataagtgaga acatgtgaag tttatctttc tgtgtctggc 64440
   ttattaatgt gctccagttc catccacgtt gttgcaaatg acaggatttc attcttttta 64500
   tggctgaata ttattttatg gtgtatatga ccacatttcc tttatctatt caaccactga 64560
   tcgacactta gattgcttcc aaatcttggc tattatgaac agtgctgcaa taaacatggg 64620
   agtgcagcta tctctttggt agactgattt tctttctttt ggatatatac ttcacagtga 64680
   gattgctgca tcatttggta gatctatttt tagttttttg aggaactatc atgctattct 64740
   ccataatgac tactaattta cattcccttc aatagaaaaa aatgttttaa ctgacctagt 64800
   cctcaagcaa aataagaaca tagtgcaata caaaaaatat ggtccagggc acaagctttt 64860
   ctttaaagtg accttaattt ttgttaagca ttagaaatat cctctatctt tgttaagcag 64920
   taaaacttga cataagcagt gtttggacta atggtcctgt ttttccaaaa tcatcatttc 64980
   atacaattcc tctatattct gtgatttaat ttatgagact gtatccattc tttattttcc 65040
   tggtaacact tgattcttat gtctgtaagt catttgagat gtaacaacac taaatcaaca 65100
   tttagaagag tacacaggca tgacacactt cattttttaa cattagattt tatttgaaaa 65160
   tattttaaaa taaatgcctt taaatgaata cttttaaaat actgcaacgt ggttttctaa 65220
   caaataactg aggtttctgg agatctttcc tctcctctct ccagagcctg ttcaggtgag 65280
   agaaggtcag gggatcagag agcagagatg ttccacacta attctaatcc tcccctactt 65340
   tgtcttaatc ccttcttcat tcccacacta atgagggcac agtctggtgg acaaaataca 65400
   gaggctggct ttttggtaaa agtcataatt ttgtttcaag gtagaaaaaa tatttccaac 65460
   gtttggccag ttttgatttg aactggacta gctgggaaag ataactatgg cttcaataag 65520
   ttggaaggga gtagtgcatc aagatttata aaaacagact aacaatatac caaatgatgc 65580
   attgttccgg gtataatcag tgtgtggata aatggaaagt aattgtattc agaagaaagc 65640
   cattctcttg gctgtggaag caagaaaaac agtacagcac aattctgtca caggatatag 65700
   atgctgtttt gtttcaaaat ggtagactaa caaattgcac ctacagaggc agtgtgatat 65760
   actgaaagga taattgtttt aaagccaaat ggacccatgt atcattgggc cagcaagctc 65820
   ataatcttag tgttttcttc ttgtaaaatg gaaataataa taattactat gcaggagtat 65880
   tgggaagata aaataagaaa atatatatta aatatctggt ttgtaagcac ataataaaag 65940
   ataaaattat tgttatttta ataccattta tcttaacata tactttttaa ataaaaattt 66000
   atgaatatgt aattaaagaa gcatgaaatg gtgtgtggct catggaacac aaatctctta 66060
   atcaaatgag aaagctctga aagggaaatt ccggagagtc aaggggctaa gaccagggat 66120
   atttatgtgg ctgtgtggat ctaagcaaga ctcttccccc agacagtgag acttcaaaca 66180
   cagatgatga ttacgctgaa tggcttggtt gtctctgtgc aaatagtgcc tgacagacag 66240
   gaaactgact cccatgatac acttcacaga atacagttag ctttacattt gttaacactg 66300
   attatataaa agaacaatga atgaggcagc atagaactta cctctctctc aattctgact 66360
   cactcgaact cattcaacat tactctgcca gaaacacact cattgttgga gagcagaaac 66420
   agtgacaagt cagggagaac atattggaaa aggctttttt tcagccattt gaaaaaaatg 66480
   tatgtaagag aaatgtatga gtggggaaaa taattgaatg aagtggaatg ggtttagtat 66540
   gatttaatcg ccttttaatt tttctaaatt tggctaatga tgacaggtgc tacctgtctg 66600
   tgatcttgtt ttgtcatcac aagccccctg taacgtagat atttccattt tataacaatg 66660
   ttagttcatt tgccccagat cttccaggag gtgaatagct caaggctctc caatcctaaa 66720
   ttccacaact ctttcattaa cccctgatca gccaatccaa gaagcatatc taggaccaaa 66780
   ttcttataat ttggatatcc tacagggaca tatacaagta ggctttttcc tttttttaaa 66840
   aaaaattatt tttaattttt gtgagtacat agtatgtgta tatattatgg ggtatgtgtt 66900
   agtccgttct catgctgcta tgaagaaata catgagactg ggtaatttat aaaggaaaga 66960
   tatttaattg actcacagtt ccacatggct ggggaggcct caggaaactt acaatcatgg 67020
   cagaaggcac ctcttcacat ggtgtcagga gagagaatga atgctgagcg aagggggaag 67080
   cctcttataa aaccatctga tctcatgagg actcactgtc atgaaaacag tatatgggaa 67140
   actgccccca tgattcaatt atcaccacct ggtcccactt ttgacatgtg gggattatta 67200
   caattcgagg tgagatttgg gtggagatgc agaaccacac catatcaggg tacatgagat 67260
   actttggtac agacatgcaa catgcaataa ccacatcagg gtaaacgggg tatccatccc 67320
   ctcaaacatt tctcctttgt gttacaatcc aattctactc ttttcattat tttaaaatgt 67380
   gcagtcatta tgattatatt caccctgttg tgctgtcaaa tactaggtct tctttattct 67440
   ttctaactac tttttgtacc cattaataat ccccacatcc ccactcccat ccccctgcta 67500
   ctcttcccag cctctggtaa ccatcttcta ctctctatct tcacaaatac aatcgttttc 67560
   atttgtatct cccacaaata agtgaagtgt gatgtttgtc tttctgtgcc tggcttattt 67620
   cactcaatgt aatgattgcc agttccatcc atgttgttgc aaatgacaag ctcttattct 67680
   ttttatggct aaatagtact ccattgtgtc tgtgtatcac attcattcat ccattcatct 67740
   gttgatggac acataggttg cttccaaatc ttggctgttg tgaacacagc tgcaacaaac 67800
   acaggagtac aaatctttct tcaatatact gatttctttt cttttgagaa tatacccagc 67860
   agtgggattg ctggatcata tggtgactct attttactgt tttctgagga aacaccaaac 67920
   ttctccatag tggtcgtact actttacatt ctcaccaaca gtgtacaagg gtttcctttt 67980
   ctccatatcc ttcctagcat ttgttattag ctgtcatttg aataaaagcc atttttattg 68040
   gggtgggatg atatctcatt gtagttttga tttgcatttc tctgatgatc aatgatgttg 68100
   agcatttttt catatgcctg tttgctattt gtatctcttc ttttgcgaaa tgtccattta 68160
   gatcttttgc ccatgttttc attggaatac tagatttctt cctatagagc tgttttgagc 68220
   tctttatgca ttctggttat taatcccttg tcagataggt agtttataag tattttctcc 68280
   ctcttattct gtgggttgtc tcatgcactt tgttgattgt ttcctttgtt gtgcagaagt 68340
   tttaacttga tgtgatccca tttgtccatt tttgctttgg ttgcctgtgc ttgtagttta 68400
   ttactcaaga aatttttagc caatccaatg tttcccaatg ttttcttgta gcagttttat 68460
   agttttaggt cttcgattta agtctttaat ccattttgat ttgattttta tatatggtga 68520
   gacataaggg tctagtttta ttcttctgcc tatggatatc cagttttccc agcaccttaa 68580
   agagactgtc ttgtcatcaa tgtacgttct tggcaccttt gtcgaaaatg agttcactgt 68640
   aagtgtatgg attagtttct gggttcttta ttctgttcca ttggtctatg tgtctgtttt 68700
   tatggcagta ccatgctgtt ttggttatga tagctctgta gtataatttg aagtcgggta 68760
   atgtgattcc tccagttttg ttctttttgt tcaggatagc cttggctgtt ttgagtcttt 68820
   tgtggtttca tataagttct aggattgttt ttttctattt atgtgaagaa tgtcattggt 68880
   attttgatag ggattgcttt agatagtatg gacatttaac aatattgatt cttccgatct 68940
   atgaaacgga atatttttcc attttttagt gtcctcttaa atttctttca tcaatgttta 69000
   gttttcatta tagagatctt ttacttcttt ggttaattcc taggtattta attttatgtg 69060
   tggctgttgt aaataagatt acttttttgt ttctttttca cattgttcac tgttggcata 69120
   tagaaatgct actgattttc gtatgttgat tatgtatcct gcaactttac tgaatttgtt 69180
   aatcagttct aatagtttcc ttgtggagtt tttaggtttt tccaaatata agatcatatc 69240
   atctgcaaac aagaataatt ttacttcttt cttttcaatt tggatgccct ttatatcttt 69300
   ctcttctctg atcactatag ctaggacttc tagtactatg ttgaataaca gtggtgaaag 69360
   caggcatctt tgacgtgttt cagatcttag aggaaaggct ttcagctttt ccacattcag 69420
   gattatacta gctgtgagtc tttcacatat ggcttttatt gtgttgaggt atgtttctct 69480
   tatacccagt tttttgagaa tttttatcat aaagcaatat tgaattctat taaatctgtt 69540
   ttcagcatca attgacataa tcatatgatt ttcatccttc attctgttga tatgatgtac 69600
   acattgactg atttgcgtat gttgaaccat ccttgcatcc ctatggtaaa tcccacttgg 69660
   tcatgatgaa cagtctttta actgtattgt tgaatttggt ttgctagtat tttgttgaag 69720
   atttttgatc aatagtcatc agaaatattg gtctatagtt ttctttttta gatgtgtctt 69780
   tgtctggttc tggtatgagg gtaatagtgg cctcatggaa taagtttgga agtattctct 69840
   actcttttat tttttagaat agtttgaata aagttggtat taattcttct ttaaatgttt 69900
   gataaaactc agcagtgaag ccatcgggtc ctgggctttt ctttattggg ataatttgta 69960
   ttacaacttc gatcttgttg ttattggtct gttcaggttt tggatttctt catagttcaa 70020
   tcttgttagg tggtatatgt ctaggaattt atctatttct tccagatttt ccaatttatt 70080
   gacatatagt tgctcataat agccactagt gatcctttga attccagtgg aatcagttgt 70140
   aatgtctcct ttttcatatc tgattttatt tatttgtgtc tttttttctt agatagtctt 70200
   gctaaaggtt tctcaattta tcttttcaaa aaatcttttc attttgttga tcttttttat 70260
   tattttcttc atttcatttt tatttatttc tgctctgatc tttattattt cttttcttct 70320
   aataattttg ggtttagttt gctcttgatt ttctaatttt ttaagatgca tcattaggtt 70380
   gtttatttga atattttctt cttttctgat gtaagtgctt atagctataa attctcctct 70440
   tcatactgct ttcactgtat cccataggtt ttggtatgtt gtatttctat gatcatttgt 70500
   ttcaagaaat ttttcaattt ccttcctaat ttcttcattg acccaatggt cattcaggag 70560
   catattgttt aaattccatg tatttgtgca gttgtcaaaa ttcaacttga tattgatttc 70620
   tagttctatt ccatggtggt cagagaagat gcttggtatt atttcagttt ttttaatgtt 70680
   ttaagactta ttttgtgacc taacatatgg tctatcctta tcgatatgct gaggagaaaa 70740
   atgtgtattc tgcagccatt ggctaaaatg ttctcttaaa acattcacat ttttattaca 70800
   ctaaaaatgt tctcttagaa catttacatt tatactattc cctaatataa atatctctta 70860
   ggtccatttg ttctacagta cagattaaat ccaatgtttc tttgttaatt ttctttctga 70920
   aagatctgtc caatgctgaa agtggggtgt tgaaatcttc agctattact gtattggggt 70980
   ctatctctct ccttagctca aataatattt gctttatatc tgggtacttc aattttgggt 71040
   gcatatatat ttataattgt tattttctct tgctgaactg accatttttt cattatgtaa 71100
   tgactttcct tgtgtcttct tacagtcttt ttcttaaaat ctattttgtc tcatataagt 71160
   attgccactc ctgctctttt tttggtttcc atttgcatcg aatgtctttt tccagccctt 71220
   tatttgcagt ctatgtatgt gtttataggt gaagtgcctt ttttgtaggc aacaggtcaa 71280
   ttggtctttt aaaaaaaaaa aaaaaattct ctcagtcacc ctgtcttttc attagagagt 71340
   ctggtccatt tgcaatcaat gctattattg ataaataagg acttactact gcctctttgt 71400
   tatttttctg tgtgtgtgtg tgtgtgtgtg tgtgtgtgtg tgtgtatgaa ggtcttctat 71460
   tccttctttc cttccttcct gtctttcttt gactaaaggt gattttctct ggtgtatgat 71520
   ttaattactt gctttttatt ttttgtatat ctatactatg tttttaaatt tgaagttatc 71580
   atgaagcctg taaatactgt catataaccc gttgttttaa agtgatgtca acttaacact 71640
   gattgcataa gtaaacaaat aaacaagcaa caaaaaaact aataaaaact ctacacttta 71700
   actttatccc ccagcttttt aactttttat tttcttctct ttatgtctta ttgaaatgtc 71760
   tatgtcttga aaaggtgtta tagctattat ttttcactgg ttcaccattt agtctttcta 71820
   cttaagaata gtaaacacat cacaattaca gttttataat attctgtgct tttctgtgtg 71880
   cttgctatta ccagtgagtt ttctatcttc agatgatgta ttttctttct tttctgtttg 71940
   gattttgttt gtttgttttg agacagggtc tcactctgtc accttggctg gaatgcagtg 72000
   gcacaatctt ggctcactga aacctctgca tcccagctca agtgattttc cctcctcagc 72060
   ttcccaagta gctgagacta caagtgcatg ccaccacacc cagctaattt ttaaataaaa 72120
   atttggagag acaaggtctc actgcattgc ccaagctggt ctcaagcttc caagcttaag 72180
   ctaccctccc gcactggtct cccaaaatgc tggaattaca gacgtcagcc actatacctg 72240
   gccctcaaag gatttcttct tgtttgttgg catctttctt ttcagtttga agaactccct 72300
   ttagcatttg ctgtaggaaa gatctggtgt tgataaaatc cctcagctgt tgtttgtcta 72360
   ggaaagtctt tatttctctt tcatgcttga aggatatttt cattggatat gttctagggt 72420
   aaaagtttgt ttccttcagc actttaaata tgtcatgcca ctctctcctg gcctataaag 72480
   tttctactga aaagtctgct gccagatgta ttggagctcc tttgtatgtt gtttcttttc 72540
   tcttgctgtt tttaggatcc tttctttatc cttgatcttt gggagtttga ttattaaatg 72600
   ccttgaggta gtcttccttg agttaaatct gcttggtgtt ctatcacctt tttgtatttg 72660
   aatgctgata tctttctcta cattttggaa gttctttgat attatccctt tgaataaact 72720
   ttctactcct atctttttct ctgccttctc tttaagatca atactcttag atttgccctt 72780
   ttgaggctgt tttctagatc ttgtagggat gcttcatttt tgtttattct tttttctttt 72840
   gtctcttttg actatacatt ttcaaacagc ctgtcttcaa gctcactaat tctttcttct 72900
   gcttgattaa ttatactctt aaggaactct gatgcattgt tttagcatgt cagttgcatt 72960
   tttcagctcc agagtttcag ctcaattctt tttaattagt tcaatctctt tgttaaattt 73020
   atctgatgga attctgaatt ccttctcttt gttaccttga atttctttga gcttcctcaa 73080
   aacagctatt ttgaattctc tgtctgaaag atcacatatc tctgtttttc caggattggt 73140
   ccatggtacc ttatttagtt tggtgaagtc aagttttcct ggatggtctt gatacttggg 73200
   ggtgttcatc agtgcctggg cattgaaaat ttaggtactc attgtagtct acacagtctg 73260
   ggcttgtttg tgcccatctt tcttggaaag gctttccagg tatttgaagg gacttgtgtc 73320
   ccatgatcaa taatgctgtg gttcttatag actcattgag ttaccacctt ggtgttcttg 73380
   gataaaatct agaagaattc tctgaattgc caggcagaat tcttgttctc ttccctgatt 73440
   ttctcccaaa taaatggagt ctgtctgtct gtctgtctgt ctgtctgtct gtctctttct 73500
   ctctgtgtgc tgagctgccc agagctgagg ttggagtgat gccaggagcc ctgtggccac 73560
   caccactggg acggtgctgg gttggacctg aagccagcac accactgggt attgcctaag 73620
   gcccactgta accactattt ggctactgcc tgtgttcact caaggcccta gggttctaca 73680
   gtctgcagat gggaagatag tcaggtttgt gtcttttctg ttagggcagc aagtttcccc 73740
   agacccttgg caggtacaga gatgctgtct agaagccaga gattggagta aaaaaaccta 73800
   gaaatctacc tgatgtttta ttctgctgtg gttaagctgg ccctcaaacc acaaaacaaa 73860
   gtctttcctg ttcttccctc ccttttccac tggcagagga ggctctccca gtggccacca 73920
   ccaccactgg cttgcaggga gttctgccat gccaccacca atattcacgt aaagccgaat 73980
   ggctcttcag tcagcttgtg gggaatgctg ctaggcctgt gactcatact tcagggcagt 74040
   gggctctcct ctggcccagg gcaggtccag aaaagctgtc caagaaccaa ggcctagact 74100
   cagggtcccc aagagcccac ctgatattct actctactgt agccaaatac gtacctaagg 74160
   tgtaagacaa agttcctttt acttttccct ctgcttttct caagcagaag gagtctttca 74220
   tagtagtcgc tgcagctggg aacgtgctgg gtctcacctg taaccagcat atctcacact 74280
   ctcacccaag acccactgca tactacctta gtgtcacttt tagttatttg gggctgaagg 74340
   gctctttagt cagtagatga tgaatcctgc caggacttgg ttcttctcaa ggcagtgggt 74400
   tcccttctag tccagggtgt atatagaaat gttgtctggg ggactcataa ctctgcccag 74460
   tgtcctatct tattgtggct gaactggtag tcaagataca agccaaagtc cactttcctc 74520
   ttagctttct tctccttaaa tagaaggaag tagtcacttt tttttttttt tttttttttt 74580
   tttttgagac agaatctcgc ttctttgccc aggctggaat gcaataccat gatctaggtt 74640
   cactgcaacc tccacctccc gggttcaagc aattctcctg cctcagcctc tccagtagct 74700
   gggattacag gcatgtgcca ccatgcccag ctaatatttg tatttttagt agagacaggg 74760
   tttcaccatg ttggccaggc tggtcttgta ctcctgacct taagtgatcc gcctcagcct 74820
   cccaaagtgc taggattaca ggcatgagcc accgtgctgg aaggagtcac ttttgttgct 74880
   gcgagttgca ctgcctgggg ttgtgggagg gatgatgctt gcactccctt agccacccca 74940
   gctgatgtct ccctaggtca cgtgccgccc tagtccactg gctctaatcc tagcccagca 75000
   ttaggacttg cctaggaaat gcagtatttg tgtccctgac tgccttttaa ttttacctag 75060
   tactccagag cactttagcc cttggtgatg aggcttgcag agaaactcaa gtcccaacca 75120
   ctgggatgga tgatccccct ccggctaagg ctgatccaaa tgatctctct gtgggcaggc 75180
   aatggctgag cccagctcag ctttgctctc tgccatggca gaacagcact gagttcaata 75240
   tcaagtccta cagtcactgt gctctccctc cctaaagtgc tcagattccc tctccatgcc 75300
   atgtagcagc tctggggaat agggaagagg tggcatcagt gattcaagac tgtgtcctac 75360
   cctcttcagt gcctctttca atgatataaa gttaaatcca ggtactgtga ctcctcacct 75420
   gacttttggt tcttatgaca gtgcattttt gtttgtagtt agttgttaaa attttgtgtt 75480
   cctttggggg cagtcagtgg aggcttctgt ttagccacct tgctccgccc cccataaaca 75540
   gtggcttaat taatattttt agtgaaaaat gccagcatgg cagagaggaa agaacattgg 75600
   gttaggagtc agaataccag ggttctgatc ccagctccat aattaactgg ctatgtgatc 75660
   ataaggaaac catacagctt ttcttgttct cagttatcta ttaaatgagg ttggataatt 75720
   aacactttac ttgaatcaag agacctgtac atagatagaa acacgtctct gatttctggg 75780
   tagggacagg tagagatggc ttgtctagaa tgggtagaga tgtcctatct gaatgacacc 75840
   aacataaatg gaaaagctgc tgaatgcttg ggggtagctc agtttgggtt cattcatgag 75900
   acagagtcca cacggtaatt tggacaaggg aaagtttaat ataaagagtt gttaattata 75960
   gggagagact acagtagcaa aagattggct agtaagaagt gaagagagtg ctaaataata 76020
   taaaaatagc agatacaagg agccactatc cctagagctg agctagaaca cccaagaaaa 76080
   agctccctgc actcaaggtt gagatccaga ccttgtttgg tgaggccttt ggggggtgat 76140
   taggtcagag tggagccctc atgattgaga ttaaagccct tataaaaggg accccagaga 76200
   gttctccatg tgaggataca agaagaagtc agcagtcttc ttgttggaag aaggccttta 76260
   ctaggaactg accatgctgg ctctctgatc tcaaacttcc agcctccaga acttgataaa 76320
   tttctgttgt ttataagcca tccactctat ggtattttga tgtaacagcc tgaacaagtc 76380
   aagacattgt tggagggcat ggctgtggct cacagagtga ctgagagatc actgaggtgc 76440
   catgccagtg gaacttgctg aaaatcctcg ctctagggtg tcagggagag ctgttcatag 76500
   ggacatgcct caccagagag attctgctac aaaagaacac aaagggttct aggggaagct 76560
   gacgaccacg gtgcactgta gaagccaggc actaaataag ctgtatttac tgcaagaact 76620
   agatgcttaa ggagtcaccc ctactgtagg agctgggcac tagagaagct ttgcactctg 76680
   cataagctcg acactggaga agctgcccac actgcaggag cctgccaaga gggctcactg 76740
   gaatcagaga gcaaacccct ttctcctgca atatctcttc tgcacactct actagcatag 76800
   cttaaccttg tgtcagctga caactgaaaa ctaaatggcc cagcttcatt tcatggagca 76860
   agcaaaaagg atgactttgg atctcatggg cactaaatca agaacttgca caggtggaca 76920
   tcaaaagcag tttcctaaaa gataggagtt catgaaatgt gtcttattgt accttggcaa 76980
   tcatttgttc cagaggcgac tagcctctgg acagcaaaaa gccttaaaat tttgagtttg 77040
   atcgtgcctg taatcctagc actttgggag gccgaggtgg gcggatcacg aggtcaggag 77100
   accatcctgg ctaacacagt gaaacctcgt ctctactaaa aatacaaaaa attagcctgg 77160
   cgtggtggcg ggcacctgta gtcccagcta ctggggaggc tgaggcagga gaatggcatg 77220
   aacctgggag gtggagcttg cagtgagctg agattgcgcc actgcactcc agcctgggtg 77280
   acagagtgag actccgtctc aaaaaaaaaa aaaaaaaaaa aaaaaaaaaa aaatatatat 77340
   atatatatat atatatatat atatatatat ttgaatttga atgctttaag gaaggccata 77400
   cctattctat ttatcatagt cctcagtaac tccataggat cttaagctca gcagcaaact 77460
   tcatacaaat atatgctggg cccacacaag catctgagcg tgtggtcctt aaccatttaa 77520
   ccacttcatt ttacaggtga ggaaacagca tgcttaaatg gctacttctg cccctgcccc 77580
   cgtctctgtc atagtcaaac agaacagcgg gggaggtaat acataaactt tcctttagtg 77640
   gtgatttttc tcaaacattt gctttgaatt tgggctgttt gtattgcaca gattttgctc 77700
   atttacataa aatcaagatc gttggaataa atatgttcag gtaattctct gaggcaagaa 77760
   aataaccaaa tgcttactca gacttgtttc tcctcgggtt aactagcctg atattaagta 77820
   tgccttaatg taaaatccca ttcaaaatct cctaaaacaa aaccagtgct tcacaatttg 77880
   taattagtac tataagaaca atttaattga ttctcctatt ctcccttctt ttgcattttc 77940
   agagccatta ctttccaaca gatgaggttg tagagatttc tatcacttca ccagaatggg 78000
   aaatctatgc cattttcatt aagtaactaa atgttaggcc atgtccaatt cttaaataat 78060
   ttcacactct tggcaagggg ggaaaaaaga ttattcaaag ttatttcaaa gtttttacag 78120
   aattgctaat aataataaca gtagcaatgc cttattaatg cagaatactt tatggtttat 78180
   aaatatgtat aatatgtaat ataacatgtg acataatgta gataaaatat atataatttc 78240
   atttgaaata actctttaga aattttactt gctaggcctg ttttattgca acttaaagat 78300
   gctaaggtga aaaataaatt gaaaacattt tagttcattc caaacagaaa cataacccta 78360
   attattttat ttaaaaataa aatgtacctc caatcattga taactactga aattataggt 78420
   aaataagtga gtttcaggaa ttattctggg gcacagtatt tgtaatgaaa taaaaaaatg 78480
   ggcatggtaa aaacttggaa tctggcaatc tgaacacatc gatttgtaaa tcttctctgc 78540
   ccataatggg acaccattca tcttctgaaa gttttggttt tccaatccct gatgcacgta 78600
   ggattccata ttcctttaca aattttgctt ctccctctcc ccacaaaggc agtgaagagg 78660
   aattccctat atccttcatc tcatttgtct ttctttgtct tcactcctga tagggaagaa 78720
   gtttggtttg tttctttttc tctctctctc tttttttttt tttttgatga taaatgcatt 78780
   tcactattta tttagatgtt taagctctca cactaagttt ttacaagctg atgaacactg 78840
   acaaattatt tctcccacag aactaaaacc acacatcccc agacagtata aatatatggt 78900
   tgaactaacc ttaatacaca tcaccatttt atcaattaca taataaaaca aattatcaag 78960
   tatccaaata ttaagttgca cagctcaaaa ggcatataaa aattagatgt ctgctcaatt 79020
   cattagcaga aacctgcttc ttttttttgc aagaatggtt aggaagagaa ggaaataaaa 79080
   aatcactttc aaacaaaaat aagttggtaa acaacttctg ataaatatgg aaaaaattac 79140
   aataatttta gaaattttat gattaagaat tgttttggct acaataacaa atcatctcta 79200
   cctttaaaaa atattttact aaattaaagg gcatatccca catgctctgc acaagacaaa 79260
   ggcaaaattt tgctaaaaat aatgaatagc cccctcccat tcttttctca gtccctgcct 79320
   gtgaagttgc acctcagatt gcaaacatta aatgaactgt aaggcctttc tgtctgaaga 79380
   cattaaagac acatgcttct atacaatgta gatttttttt aatggaggct tttcataaca 79440
   gcacaaagta agatttattg aaacacatta aataatcact gtaagacatg gtaaaaaaaa 79500
   aaaaagaatc aatggttaaa caaatctgat atttgctagc atgaatgtgg gagatgaaaa 79560
   catggagtga gcaggcaaat cactatgtag gccctacttt caatgtttga aatgattgaa 79620
   ggaataaact acataaggtc tagagtgttc atagttcctg ggattttagt tctgtatggt 79680
   actaaaaata cacaagtatt gtgccgggtg gcacctaatc ttcctaattt tcttatatat 79740
   tgataagatt ctggcaagca aatagattta aacaagacat aactcaatac tgcatgattt 79800
   cgggaaaggt cagttggtac aaacgacagg cacattttaa atgctaacag caagaatctt 79860
   ttgccaagtg tccaaatagg ttgactgtaa cccaaacaca gaggttgcca aatctgtcta 79920
   gcttgtggcc tggtaaaact gcctttcagt actcctatgg aagtttcatt agacttactc 79980
   aaagccaaca tgttggctaa taagtataac aaatcaggtc ctatctcaag ttccacttcc 80040
   aaggcaatag catcagggac cccagtttta ttaactgcaa cagttgtatc tatgaccact 80100
   ggagtctttg aagtgcagcc acatctttaa ggatgatact tctccaaata caaaagttaa 80160
   cttgctgtta aaagctccat actgcttttt tcttgcgaat tgtactgcat catcatcttt 80220
   cattttgcct tcaattaatg ctagggcaac aagtactgga gctctcccaa ggcctgcaat 80280
   gcaatgaaca gcagtacaac aatgaggttc tttatgaaac ttaattttca caagacttaa 80340
   ccagtcatca atctggttgg atggtggtgc accatcatca aaaggcctat cgagaacatg 80400
   ggtaccttct ttctccagaa gagtagtgtc ataagttgct ttacatactc ttatctattg 80460
   tgaaaactcc atacttctta agttcctcta cgaatttgtt taaggtcaca ttggttgggt 80520
   tgtgtgtagt aagaaatcct catgttcttg tatgtgactt ccacaggagc tggacagttc 80580
   atttgagcca tgttaattta gttaaaagac actcaatagg gttatgaaag aattttaaaa 80640
   gttgaataca gaaatgatgc aaagaaactg aagtctactt caatatactc cacttgaaat 80700
   tctcagtgct ttgagtatga agttgtaagt aacaataaat gaaatgaacc tcctaacaag 80760
   aagcgacact tcttcaatcc agtaatactg aggcaacaga aaggaagcac actgaggttt 80820
   accctatcta ggtcagaact cttgtaaaat gccctatggg tttcaattca acacttctgt 80880
   gtccaggaca atgggtgctt cttggtagag tagtaatgaa tttctacagt ttccttgttt 80940
   gcatagaggt catgctatgc ctggcagtga tctccactgc ccttcagaaa ctccataaat 81000
   atgtgaccaa gaacaccaca gaactgagga atgtgtttat gcattgaaaa tggtggccta 81060
   ctgcgtcgat ctcactggga gctgcagacc ggagctgttc ctattcggaa gcatcacgct 81120
   acctgacttc aaactatact acaaggctac agtaaccaaa acagcatggt actggtacca 81180
   aaacagatat atagaccaat ggaatagaac agaggtctca gaaatagcat tgcacatcta 81240
   caaccatctg atctttgaca aacctgacac aaaaaagcaa tggggaaaag attcctattt 81300
   aataaatggt gttgggaaaa ctggctagcc atatgcagaa aactgaaact ggaccccttc 81360
   cttacacctt atacaaaaat caactcaaga tgtatcaaag acttaaacgt aagacttagg 81420
   accataaaaa tcctagaaga aaacctgggc aataccattc aggacatagg catgagcaaa 81480
   gacttcatga ctaaaacacc aaaagcaatg gcaacaaaag ccaaaattga caaatgggat 81540
   ctaattaaac taaagagctt ctgcacagca aaagaaacta tcatcagagt aaacaggcaa 81600
   cccacagaat gggagaaaat tttgcaatct atccatctga caaagggtta atatccacaa 81660
   tctacaaaga acttaaattt acaagaaaaa aaaaaaccat caaaaagtgg gtgaaggata 81720
   tgaacagaca cttctcaaaa gaagatattc atgtggccaa caaacatatg aaaaaaatgc 81780
   tcatcatcac tggtcattag agaaatgcaa atcaaaacca caatgagata ccatctcaca 81840
   ccagttagaa tggcgatcat taaaaagtca ggaaacaaca gatgctggag aggatgtgga 81900
   gaaatagaaa cacttttaca ctgttggtgg gactgtaaac tagttcaacc attgtggaac 81960
   acagtgtggc aattcttcaa ggatctagaa ctagaaatac catttgaccc agcaatccca 82020
   ttactgggta tatactgaaa ggattacaaa tcattctact ataaagacac atgcacacgt 82080
   atgtttactg cagcactatt cacaatagca aagatttgga accaacccaa atgtccatca 82140
   ataatagact ggataaagaa aattggcaca tatacaccat ggaatactat gcagccatac 82200
   aaaagaatga gtttatgtcc tttgcaggga caaagatgaa gctggaaacc atcattctca 82260
   gcaaactatc acaagaacag aaaaccaaac accatatgtt ctcactcata agtgggagtt 82320
   gaacaatgag aacacacaga catagagagg ggaacatcac acgccaagac ctgtcggggg 82380
   gtggaggcta agggagggat aacattagga gaaataccca atgtaggtta tgggctgatg 82440
   ggtgcagcaa accaccatag cacatgtata cctatgtaac aaaactacac attctgcaca 82500
   tgtaccccag aacttaaagt ataaaaaaaa aaaagaaaag aaaatggtgg cctaatccta 82560
   cagctggtcc ccaggcagca gtggcagagg ctgcagggca ggaggcagag atggttgttg 82620
   ctgggtggcg gtggccatgt tgcaagcagc cagtgggaac accagactct accatgcagt 82680
   gagcggctcc tggtttttca gacatcaaag ttatactaca aggtataatc ctgattcttt 82740
   tacttattca aaaatatttg agtacctact ttctgtcagg cactgctctg tgtaataaga 82800
   atacagcaat gaatcaaata gaattcctgc tgttgtgtag cctatattct aaagaggcag 82860
   acagaaaatg agttcattag gtaattgcag agtttgtcag gtgataaagt gctttgggga 82920
   aaagtaaagc agggaaagag gacaggtgag tgatgtgggc ctaaatgagg tgatgaagaa 82980
   tggctctcca gaaaaggtaa ttgttcagaa aaaaaagtta aatcattcaa gaaaaggcta 83040
   cacttatatt gcaagtattc cctctacaac tgctgaaatc aggctcaaag ggatgattgt 83100
   gaaactgtgt ttgtcagctt ggggatgcag gagaataaca gaaaagaggg gcaggggagc 83160
   agattgggta cttcctaaca gtggattgag tgcttgaaac gtatcagata cccaacaaat 83220
   gtgcattgaa tctgagcata gtcaagtttc acacctctca taaattgagg taatatagca 83280
   gtcctatcaa gaacagtatt ttggaatgcc agtatgctgg atctccctta tagttcagct 83340
   ggtttctatc tctgcacctc atccattgga tagtcctctg acgggaaaat aacaagtgtt 83400
   atctcagaag aagatacagc atcctataag ggaagtccta gccacatgaa aaaggaggga 83460
   attcctgccc tcctggaccc aagggatagt gtaaaaagga gagtgctaca gaattgcctc 83520
   tttgtgtcct atccttgggt caaacattgg ggtagggtag atagtgctgg acattaaacc 83580
   tttgctagct atggaagaat ctggaaagca gggtttaata gcatgttttc cccactgtgc 83640
   ctgagaaaag atgcaaaccc ctggttaaaa accttgcacg tggatacatg tcccaggagc 83700
   actaatagag aaatggccag ggagaagacc agaaggtaga attcctggct ccccaaggcc 83760
   cattcagcat ctagaactac actgtccaat atggtagcca ctagccacac aggctcctta 83820
   aatttaaagt aaccaagctt aaatgtaatt taaaatgtgt tttcttagct gcattagcca 83880
   catttcaagt gctcaataac tatgtatggc tcaagactac tgcactgtac agcacaaata 83940
   aaaaatgttt ccgtcatcat agaaagttct attagacagc agtagtcagc aaacttttcc 84000
   gtaaagggct tacaagtaaa tatgtttggc tttgtggaca agaaagtctc tgtcacaatt 84060
   attcaatact attgctttag cataaaagca gctgtagtct gtatgtaaat aaacaagtat 84120
   ggccgtgttc caataaaact ttattttaaa aaacaggctt tggtctgagg gatgcaacat 84180
   gcctgtcgct gggatagagg agccaaaagt tcccacatgc ctcagtaaag ggacagaagc 84240
   cactgaaaga atcagtgagc tagtggatac ctgggctagg gccagattac ctgatctgtg 84300
   cacgcaaagg cgatatgggt ccaggtgcaa ctgagacaca taggccaagg gccagaccac 84360
   aaacctgcac acagcttcga gtccatcctg ggaaggagca ggaagagagg aaagaaattc 84420
   aaaagaccaa ccccaaataa tccaaatgat ctagaagaaa ctgtttaaat ggaagagaca 84480
   gtgatactgt ctgttggcag atgtaggtgg ccccgccccc tgtgccagtc ccaccactcc 84540
   ccatatccct aaccccctga aggagtagga gctcaggagg aagtttaaat ctattattta 84600
   aagaaaataa gctacagttt ccttgacttt ctaagttaca gcgaaaataa aaagtcaatg 84660
   ctgctacaca gtgttttctc aaatgtatgt aaagaccctt tagaaaagaa atctttgcac 84720
   tctttctagg agcactaatt tgggctaatt catctctaca gcccctccag ggcctccttg 84780
   tgactgaaga tatacactaa ggccttgtgt gaggggaaca ctggcatgga gggagaaaat 84840
   ctgggctcca gctgtgtgac ttatgctaaa cctctaacta ccctgggctt cagtgacctt 84900
   atctgccaca tgggaagata gatcaaatga ctcctaagtt cttatccagt tcagagataa 84960
   taattttatg attcatctga aagaaatctg actggggttg tggcccgttg ctataattaa 85020
   catagttagc ctgatcacat gtcaatggat taagccttag ctttactatt aagataaaac 85080
   tgaattagct aagtttctac cagttcactg aaaagttctg atatttaata cttggatagt 85140
   tgtaaggaca tgctatcact caaactatat tagtcatcca ggctgccata ctaaaatgcc 85200
   atagaccggg tgacttaaag aacaaacatt tatttctcac atttccgaag gccggaagtc 85260
   gaagatcagg gtgtcagcat aattggtttc tggtgagggc tctcttcctg acttgtagat 85320
   ggccaccttc tcactgtgtc ctcacatggc agggagagag tgagctctct ggtggctctt 85380
   ctttttaagg gtactaatct cattatgggg ccctaccctc atgacttcat ctaaatctaa 85440
   ttacctccca aaggtttcat ctccagatac tatctcattg gaagttaggg cttcaacata 85500
   cgaattttga gggaacacag tttagtccat agcacaaacc ttcaaattaa ggtaagatgc 85560
   tgacttcttg gaattttgca atctgctcat ggacaaaaca aaagatgtgt ctctcatcag 85620
   caagaaattt ttgagaaagg aatatcaagt tgtgtcccac ctttctgaca ttcaaagcag 85680
   aggcaggatg ggaggatgac tgtctcaatt acccactatc ataataacta acatttactt 85740
   gacagcttac cgtgcaccag acactccttg aagtgcttta catgtatcat ctcatctcac 85800
   tccacaataa cctaaaatga cagcctttat tactagacct attgtagaga tgaagacaca 85860
   ctaatacttg gaaagcctta ggagctttca tacagtgtca caagtaatat gagactgagt 85920
   ccagatataa aactcagtag actaactcca gagcctgccc tcctaatcac catgatctac 85980
   aaccttcagg ttgaacacaa gtcatgggaa gagtgcctgt gtgcatgcat gtgtgtacac 86040
   acatgcatgc acgcacacac acaaatcaga tgtggttgaa aatagcgtgc atttaatgta 86100
   ttcataatat gtgttaatta atgtattaat gcctataagt tattcaccat ctatgatgga 86160
   cttctttaac caactaagca aaatgcattc tcccaacatc ttgatgagat tagaatctca 86220
   gaaaatggga attttgatga gtggtgaaga ccaagaagat cctttgaatt ggagcccttg 86280
   aaggatgaaa ggtccagata gaacattgaa cttagaagcc atgaacagct agggtatgag 86340
   acaaaggctc aagtgctcga tgagaggaaa aggatggaaa aaaggaggaa gccatattgg 86400
   ggcaagaact atgccaagtg aggtgttcac actccagagc tctgtgtgcc agacgttgta 86460
   caaattctcc ctgcaataat ctgtagtctg tacacagcct cccagagaca aatcactttt 86520
   acccttttgc ccaatatgac caaagtcaag tcattaagac ctctttttgt gccagcaagg 86580
   tgcttaagtt tggtcattcc tggctgccaa gatatgatgt atgacttatg ttaaaatgtg 86640
   actgactgca tgtggaacaa cactcagagg aagctcagaa agttcaatag ttgagtttaa 86700
   agacaagagc ccagttcaat gacagaatgg atgtgcaacc agtcatccta gccatcaatt 86760
   taaatggcca tataatttga aaggctgaca ggcattagtg ttcagtatga agctgtctga 86820
   ggcactagct tggttacctg atggtcaagt tcattatatg gctagagctg ggcagcaagg 86880
   tctgtaagtt cggagattcc ataccatatg ttttcccatc atgcttggac ttcttgaaga 86940
   tatctatgat ctcagactct gaagtcacaa tcgactagat agttgttctt tggaagtggg 87000
   tcaggcaatg gaacatggat atctttaatc acttcttgaa gacctcctca atggctgaga 87060
   atcagtagct tagctaataa caaagccctt cgctttgacc tcctcagctg actctgggac 87120
   cttctgtgct tgtcctcttc atatcccttc actcatctct cttcacctta accacttgaa 87180
   tctttaacca ggaaatcttt gcttgacctg ggcaattccc tgcctgcctt tttcataggc 87240
   attgtgcctg caactctctt ctccctctca gcttcccaaa ccattcactt tccttattta 87300
   aacccttttc attgcccaga tcttggtgtc taatatcttt ctctaataaa aggaaccagg 87360
   gttcgttccc tcaaaacaaa tggctgattt taggactggg caggaaatat acaagcctgg 87420
   agcatcttat agtgccagaa aatatggaag tgcttgaaaa agaaattgat gggatgtatt 87480
   agtctgctag ggctgtctta aaaaaaatac cacagactgg atggcttaaa taatagaatt 87540
   ttattttctt atggttcagg aggctggaag tccaagatca aggtaatgtc agggggttgg 87600
   tttctggaga ggcctgtctt cctagcttgt aggagaccac cttctcgttg tgtcctcaca 87660
   cgtcctttcc tctgtgcatg cgcgtacaca cacacacaca cacacacaca cacacaggtt 87720
   ggggggagag agagagagag agagagagag agagagagag agcatacaag catgctccct 87780
   tgctcccttg cgtctcttct tcttcttctt cttttttttt tttttttttt tttttttttt 87840
   tttgagacgg agtctcgctc tgtcgcccag gctggcatgc agtggcacaa tctcggctca 87900
   ctgcaagctc cacctcctgg gttcatgcca ttctcctgcc tcagcctact gagtagctgg 87960
   gactacaggc gcacgccaca atacccagct aattttttgt atttttagta gagacggagt 88020
   ttcatcgtgt tagctaggat ggtctcgatc tcctgacctc gtgatccgcc cgccttggcc 88080
   tcccaaaagt gctaggatta caggagtgag ccactgcgcc caggcccttc ttattcttat 88140
   aagaagcaca ctagtcctat tggattaaag cctaccctta tcacctcatt taacctttat 88200
   tatctgctta taggccctgt ctccaaatac agtcatattg gagtttaggg cttcaacact 88260
   gtggatttca ggaggacaca attcagttca taacatgagg ttataagcca cccacttcta 88320
   caaaaatttt taatggcagc catagaaaac tactacagca tcctggaaca acaaaaaaag 88380
   aagttaaaaa acaaaaaaga atctgaatca actgtagact atacttaata ataaaaatta 88440
   ggttaaaaaa cataaagaat ctgaatcaac tatagactgt agttaataat aatgtatcac 88500
   tattggatca ttaattgaaa caaatgtacc atagtgtaat ataggatatt aataagggaa 88560
   aacttggtgt ggggtagatg agaactctgc agtagtttct caaaaggaca cacatgccaa 88620
   ctcaaagagt tttcactggc aatgctagaa taatctgagc aacaaaatag agtagtaatg 88680
   gattataatc caatgtataa aatatccatg aggtcatgct gatataaata catgattaaa 88740
   taaattaata aatgggggga aacagacaaa tcttccacac agatgaattg caaataattt 88800
   atgtagatct ttcaccctga agagcaggag cctaactctc cccactcctt aggtgcgggc 88860
   tgtgcatagc aacttccttc caaaatgcac aatgtggaaa gggagaaaaa agagtaactt 88920
   tacagtagag aaacctgaca aaacactacc tcggccaggt gaccaaggtc aacatcaaca 88980
   gtgataaatc ttgttgacac tgtgtaccct tgataagatg tgatgaaaat atcactttac 89040
   ctctgtgacc ttcctgccag taacacataa ccctagtgtt atcataagaa aagcttcaga 89100
   cacattccaa aaaacgggac atcttacaaa acacctatcc agtagtcctc aattttgttg 89160
   agtcatcaga agataaggga aatctgaaaa actgccacag ccaagcaaag cctaaagaga 89220
   catgacataa tgtggtgtct tggatgagat ctgttatggg ttgaattgca tctcctctca 89280
   gaaaaaaaga tatgttgaca ttctagctcc cagtacttca gaataccacc ttaattgaaa 89340
   ataaggtcat tgcagtgtaa ctagttaagt tgggatgcag tctagatagg gtaggtcctg 89400
   aatccagtac gactgccgtc cttctaagaa tctcacggcc atgtgaagat acatggagaa 89460
   ggccatttga cggtgaaggc agggattgga aatatgttgc tgtaagccct gggacaccaa 89520
   agattgccag cagccaccag aagctaggaa gtgaaaagga agggctctcc tacaggtttc 89580
   agagggagca tagccttcct ggcaccttgg ttttaggctt ctagcctctg taagacaata 89640
   catttctgtt atttttaagc caccagtttg tcaaaaattt ttacagcggt cataggaaat 89700
   gaatactgga tcctggaaca gaaaaaagta aataaacaaa acaaagacat ctgaatcaac 89760
   tatagactgt agtcaataat aatgtttcca ctactggatc attaattgta acaaatgtac 89820
   catggtgtaa tataagatat taataatggg gaatctcggt gtggggtaga tgggagctct 89880
   gcactcagtt ctcaattgtt taacaatgga aaactgtttt taaaataaag tatattttaa 89940
   aagaaccttt ttcttatttc agcaattgtt gaaataaggt ctcaatttct caaagctttg 90000
   attttgaaac catctgcctt tgtgtagttt atatcaaatg cattctaatt ttaaagaatc 90060
   aggaacttct ccacagaaag cgatcatcct aaaggcatca tgcctcccag tttagtcttg 90120
   tctccttcct ctcctttcct cacacgtgaa tcttttaggt cttgttaacc ttccattcat 90180
   tcactgtatg ccaggaaatg tgccagggat gtagggtaaa aagtgacctt gccatccagg 90240
   gaaggaggca gacactagaa acatcatcac acatagaaat gacaaatcac cctgtgacct 90300
   tttaagctta aatggctggc tgtgaggaca acacaatctc cgccatgagc agaatgctta 90360
   gcttagaaag aaaccaacac aggacacagc agagaggccc tgggacattg ctggaaatgc 90420
   tggagttggt tatgcctgaa ttccctaccc ttccttgaca tcatagttgc acagcctgtt 90480
   aaatacccat ttttaactta aatatcacaa ctttcaaaaa ttgtataaaa gagaagttca 90540
   tcatgttatg ggaatacttt tagagggcta tcacttagga gtgtcaagaa agacagctct 90600
   gaggatgtat atccttgagg acattatgcg aagtgaacaa gccagtcaca aaaaaaaata 90660
   ctgtattatt tcacttctgt gaggtacctg ctatgatttg aatgtcccgt gcaaaattca 90720
   ggttgaaact taatccccaa tgtaacaata ttgagaggca gggcctttaa gaggtgattg 90780
   ggtggtgagg gctctgccct tatgaatgga ataatccatt catggactaa tggattaatg 90840
   tgttatcctg agagggaaac tggtggcttt ataaaagaga cccgaaccag cacattagca 90900
   tgctcagccc ctcaccatgt gataccctgc accgccttgg gactcagcag ggagagcccc 90960
   caccagcaat aaggctctca ccagatgcga cccttcaatg ttggactttc cggcctccag 91020
   aattgtaaga aataaattcc tattcttcat aagttaccca gtttggggta ttctgttata 91080
   agcaacagaa aatgaactaa gacagtatct gagtagtcaa actagtagag acaggaagta 91140
   gaatagtggt tgtcaggggc tagggagaga ggggaatggg aagtttagtc taatgggtac 91200
   ggagtttcag ttttgcaaga ttaaaagaag ttgtggagat ggatggtggt gataattcac 91260
   aacactgtga atttatttaa tgccactgaa ttgtatatac acttaaaatg gttaaaatag 91320
   caagttttat gttttgtgta tttattgtag tttaaaacat ttttaatggg aaaaagattt 91380
   gttagctcaa catcatgtag aagtcattga ctatttatgg ggatgagaag aaccaattaa 91440
   aatactagca gcagatgttt ctggaaaagg cagtgaaaaa gctgcaaaac tagtctccca 91500
   gcaagcccct agtacacacc agattctcaa aagtcactgg ctttgggatg gttttcatgc 91560
   tttttctcag cctcactctt tcccatgtcc cactacagaa atttcagcac aatctcatat 91620
   tatcagagga aaatgacttt caatatgcaa agcccatgat tctccaaggg aacatagaga 91680
   aatacattaa ttaatttctc tcacttcctt ctttctctac acattgcaag gatttaaaaa 91740
   aaaaaaaaaa cctcaaaata accaacaaag caaaaacatg aaaaagtaaa ccaggcattt 91800
   atcaattatt tataagctat atttttccct cttgcactgt ttctttcttt ttatttattc 91860
   atttatttac ttttccttga gacagggtct cactctgtca cccaggctgg agtgcagtgg 91920
   caccatctaa gctcattgca acctccccca ggttcaaacc attctcatgc ctcagtagct 91980
   gaaactacag acgtgtgcca ctgcgcccag ctaatttttg tatttttagt agagacgggg 92040
   tttcatcatg ttgggcaggc tggtcttgaa ctcctgacct caggtaatcc acccacctcg 92100
   gtctcctttt gcactttttc aaaaatgcag acttttattc aacacagcta ctaatatttt 92160
   gctgtaagtc accacaaaat tgtgaaaatg cttgtgacag atagttacat tgtcccatca 92220
   aacctggtaa agaaaacccc ttctttggaa taacactgca ttagcttttt cactatttac 92280
   tttttccaat taagaaggcc tataatctgt ctatgcccca ttctccacat ttgaattgta 92340
   gaagataaat tcctcacact tcgagtaaac tatggtaatg tgtgagacat cctgtggcta 92400
   aaatccttca aattccctgg aaaacctaat ctatagcttc ataagattat ttataaacat 92460
   gttaacaatt ctgccaaagg tcaagtagct actgtatgga taatgattta cctagaaatc 92520
   aaagccctat ccatccttcc atccatccat ccatttgctt gttctacgaa tggttataga 92580
   atggtcctga atccagccct gtacatggta ctgagctagg cctgtacaag atacaagcaa 92640
   tatggtatgg gagttaggca ttcagacatt ggagtcagac tgcttgaaat tgaactctgg 92700
   ataaccatga tcacgagtct ttccaaattt cagtttcctt atctaatata cctgttcctt 92760
   gagaatttgg ggtgtactaa atgagaaaat gcatataaag tgcttagcac agcagccagc 92820
   acataacaga gcacccagtg tatgttaggt attattatga aagtatgtaa aacccaaccc 92880
   ttgccctcca ggttttgtta gaaaggctac atctttctta cttattcttc taaatcagga 92940
   cttcccaaaa ggctattgtt attcaccaca cacagttgac acccaagtct tctaccacct 93000
   tctttacact tggtgtgggg gcttctacac acctcctgct agaaaggtgc tccagcttat 93060
   ctaccggttc caggggcacc tcctttcttc ttttcccatc ctttccctgt aatgaggaga 93120
   tttgtgaggt gattgaggag ccctgcttta aatcagggat tggcatacta cagccctcag 93180
   gccaaatctg acccaatgcc tgtttttgta aataaagttt tattggaaca cagccatgtt 93240
   catacttgta tgtattgttt gtcgctgttt tcagactata atggcagagt tgagtcttgg 93300
   gactgagaca ttatggccca acaaacctcc aatgtttact atctgggact ttacagaaaa 93360
   catttaccag ttcctactct agataatgat gatgaagatg atgatgacga cagtgaagaa 93420
   gacaagccac ttctgccatt tattgggcaa aaggcaataa tattaattct attctacaaa 93480
   taaagacatt gacgcccaaa gatgttaagt aaccttatca aagtgacaga gctaatatat 93540
   ggcaaccaat gtcactccta agtttgttct cccaattcag tcattcaata cccatataat 93600
   gaatggctac tgttggtgtc cagggcatta gccactaggg atagaatggt aggccttact 93660
   ccaaggaata ataactgact ccttcctagg taagagtaac tctcgatagt gtccctgttt 93720
   attgaaagct taccccaagt atacttcttt ccagtttacc cacatcttga tatcgccacc 93780
   atcatacttt ctactacagc ttcatttgat agaataagtc aggtacatct tttgttcctt 93840
   aggaacattc ctcactgttt attggacctt agactcattg ttgggttcca attccttacc 93900
   tactttttcc ttcaggtgac atgttcccat tattccctta cctagagtta ccagataaaa 93960
   tacaggacac cagttgaatt tgactgtcag ataaacaaca aattttttta gtctaagtat 94020
   gtcccacata atatttggga catatgaagg gtgttctcta tttttatttg ctaattctgg 94080
   caacccctac ctaaatattt ttgtagctgg aagaattatc cactgtgggt agcagggtat 94140
   ttactgggaa ggcctcatgg gttaggacag acagcattgg atttcccagc agaggatatg 94200
   tgtgccaggc cagctgggca gcatccctcc acagaatggg aatgagagcc cctactctca 94260
   aggtctggtg aagactcagt gaaatgttgt atatgaatag cattgtgttt ggcaaagtgt 94320
   agttgttttg ttttgttgtt gttgttgttg ttgttgaggt ggagtcttgc cctgttgccc 94380
   gggctggagt gcagtggcat gaactcggct cactgcaacc tccgcctcct gggttcaagc 94440
   gattctcctg tctcagcctc ctgagtagct gggattacag gtgcccgcca ccacacccag 94500
   ctaatttttg tacttttagt agagatgggg ttttgctatg ttggccaggc tggtcttgaa 94560
   ctcctgacct cgtgatctgg ccgcctaagc ctctcgaagt gctgggatta caggcgtgag 94620
   ccaccgcccc tgaccatgta gttgttattt atcttcactc caagtaatat ttgttcattc 94680
   attttagtca tttcctctgg tagttcagat tctaaaaaac agatattcct caatataccc 94740
   tcagcacagc actgtcagat tgtgtccctc tagcctcttc tttttccagg atctcactga 94800
   aagatttaaa tgactatcaa agttaattta attatgaagt atgcaaaatg ctcctcccaa 94860
   tttataaatt ccatgggata aaaaaaggaa gtttgacaga ctgcgttgtt atgaaatgaa 94920
   agatattata cacacataca cacacacaca cacacacaca cacacacaca cacacacata 94980
   ttcctagagt gaccctcaat gatttttttt taatttttat gggtgcatag tagatatatt 95040
   tatttatata atgtttatgt gtgtgcatat atatatattt atggggtaca tgtatatata 95100
   tttctggggt acctgagatg ttttgataca ggcatgctct gtgaattagt cacatcatgg 95160
   agaatggtgt atccatgccc tcaagcaatc ctttgagtta caaacagtcc aattatactc 95220
   ttttagttat ttttaaatgt ccaattatta ttgactatag tcactctgtt atgctatcaa 95280
   acagtaggtc tttttcatcc ttcctaacta cctcagtgca tttcaatagt tcatattcag 95340
   ttacattatc tgctttcgag ttccaccctg tttcaaattc cataatcaca tttgacagca 95400
   gcaaataaaa actcattttt aaaaaattcc tggaataata gaatttttat tttctttgtg 95460
   cattcagtaa atgccagatc catcttactc ttttttcttt ttttgagaca gggtcttgct 95520
   atgttatcca cactggcctg gaacttccag gctcaaggga tcttcccgcc tcagcctcct 95580
   aagtagcttg gactacaggt gtatatcatc acacctagct agatccttct tattctaagg 95640
   tctcttttag aaactgaaat ttgatggtta taattagtgc aatttttcat ctcttacctt 95700
   attagagaat aaggataaga tagactagta catttaaaaa aaaaaaacta aataatccat 95760
   gtaaatacat tggagccaaa ctgtttactt ctcagtcttc acttagagtc tttgcttctg 95820
   tgcttttagc aaatagttgc taatgcctct cacactttct taaaaatgca gaagagaatg 95880
   atatgttacc tgtctccagg agatggtcaa tgtgggaatg tgctcatgga aaacctcagt 95940
   tcataaaggt gtctgtttat ttctcatgaa actctttagc caaacactct gctaaatttc 96000
   tgtatcagga attaagcata aaacgctcca ctttctgttg ttgcctatga ctcttaatat 96060
   tttgagaact aacaaaaggt aaataaaatg tcaggaatca agattaacca ttagaaataa 96120
   aaaatgaatc accacagtga agaaaaaaag cttaaattca aggggatttt tatttcaaat 96180
   ggtaatgggg cctattggtt caagtgaaat cacataagag ctattttgct caagtctttt 96240
   ccaagtgaaa aattgtcaac aggattaaag aaatatattt tgaattctaa tgtatgatca 96300
   taagaaacta gtgtaaaaca tttacaaaat tcttctccct aggtaatatt ccccagttca 96360
   ctttaaaatc ccaaatgaaa gctgacagac tgttctacta cagactgaga tcttttaaaa 96420
   tggtctcaga tcccaaatgc atgagtcaat taaagtgcct ggcgctcctc ctccccgcag 96480
   agtgtattag tctgattgct gctgcctgtt ctccccgact atgatttcca tgcacctgtt 96540
   gttcacagta gaggaaaaag ctcactaatg tattacccct aaaatgaaaa gaggctgaaa 96600
   aaccattctc tcctgctaat aaatgctggg ctacagtaag cagatgaaga agtttaaaaa 96660
   ggaaaacagg gaaagaacat ttaacaaaac ctgatgtcct ctggtttgta ctgcagctga 96720
   aaatcacttg gaacttaacc aatgaggtta aaggaagagg cctgtggcat gaatgggaac 96780
   ttcttttgcc acacaggtgc tgtggttttg agcatgccac tgacagatgc acacacccct 96840
   aaatctctct ccatctgtca cagagcccac catgatagat gcatgccttt gaaataaact 96900
   acccatctct catgctttgc ttttgaactc tatgtctcta tctggagaag ttagacatgg 96960
   atccaaagtc aaccattatt ttagaaccca gtgctttagt aagaatcacc tagtaaggcc 97020
   ctaagggacc aaacaggtaa caattaaatt gtgatgaggg gtgggcaagc ggaatgtctg 97080
   gaaccaccct ggggaactct aaattgtcag ggctcacacc cactgcactg ggtgcctgga 97140
   gcaagggaga tgccatagtg gagaaaggag agatggtcaa gacacattga ctcacatcac 97200
   atttttcaaa agcctgctct gctttgcagg cagaagggca ggggtagaaa ttgggccaga 97260
   gttctcctct atgtctaaac acccctctag ccctcatgtg gctttctgcc ttcctccttt 97320
   tcaagccaca caaatggtag atcccaggga agcaagaaga tggttaaaca tggaatcagg 97380
   caccttgtat agctaattta caatttttta atactcttta aacacttgtt taacttagaa 97440
   gagagactgt gcactacaaa gggcaaaagc tgagagggaa gatatgtagg gagtcctcag 97500
   tttcttttgt tgttgttgtg gtagtgttgt ttgagacata gtctcattct gtcatacagg 97560
   ctggagtgca gtagtgcaat cacagctcac tgcagcctcc atttcttggg ctcgaacaat 97620
   cctcccacct tagcctccca agtagctggg accacaggtg catggcacca tgcctgggta 97680
   attttatttt ttggtagaga tggggtgtca ctattttgct caggctcgtt tcaaactcct 97740
   gggctcaagt gatcctccca ccttgacctc ccaaagtgct gggatcacag gcgtgagcca 97800
   ccatgtccac cctcagtttc ttttgttttc ctgagcaact cagttcttct aactgagcat 97860
   gggggcccca ctcaggaaat caaaactttc atctgcccct gccccaatcc aactctcttg 97920
   tccaagtgtt ccttccagcc acacagaatg caatccatga ggagagttct tgcaaaaact 97980
   tgtgtccttt cttttctgtg tctagtactg tttagtgccc cttatagatt aggtgcttgg 98040
   ggaaatgccc agaagcctca ccccttaatc cttctctggt tgaacatctg gctatgtaat 98100
   ctcagatgcc cccagccttc attaaccctc actgaactct ctgctcagga ctgctcactg 98160
   tacatacttc tctcctcaga ttttgagctg tgacgggagc aaaaatccca caaacaaaca 98220
   aatatcctaa taaatctgag gtaagagaga ctccttctag aatttagaaa aaccaattgg 98280
   gaagacttgt aatccagcaa tttacaaaga gcctggcata tagtatatgt tcaccaagta 98340
   cttgttgaaa gaaaattaat gaatgaatga gccactgtgg caataggata aaggatgata 98400
   tgctgccttc tatctgcaac tgtaactgaa gctacattcc tttataattt ggctctatca 98460
   tgatctgttt tttaacaatt acagatactg ataatgaagt gcattttttt gacatatgac 98520
   actaaggagt ggtgctggaa aaaaagactt cctgtgcctt ctcatttcca ctggtctctt 98580
   ggtttggcct tgggtaaacc catatgatat ttctgagtag agaacaatgc ccgggtttaa 98640
   accggatgcc agctacaaaa tccttatatc agcatgctcc tcttgccact gacacataaa 98700
   ctgggaaaaa ggcaggttta aactcagctg atggtagacg ttcaagtaaa ccagtagcca 98760
   cctctcgttc ccaacacccc tgggggcatc atgtggcttt gccatcagct aatcagcctg 98820
   tgctcatgct aaagttttca tcacctaaaa tgggctttcc agttggttcc ccttaaatga 98880
   acctcaatac ccccgctgtg tgaacatgtg gagttcctga taaagatgtt tatgaggtag 98940
   gaaatgcacc acaggacacc ctgaatttgc ttttgggtgg aatgaaactt agaacaaatg 99000
   caacatgaga tacacattat aagctgactt cgactattaa ccaaaactcc ctcagatgca 99060
   catagaaaga acaatgatag ctgatttctc tatttataac ccagagagac tgcaaaattc 99120
   tgaactatct tctgaatctt caagcatggt gtcgttttag ttgatctctt ttaatatatt 99180
   taaattcttt ggaaaatcat tctattctat aatggcaaca tcttattaac tataatatct 99240
   gagtagttat aaaaataaaa ttctgcatga gaaagaatgg actgattccc tggttcccta 99300
   tataaggaaa agagatgaaa cttacagaac tccctaagct tatttctggt agtagaaaaa 99360
   ctcaatcaca attacataag tggagaggtt tgtaggaatt acataagtga agaggtttat 99420
   aagaattaca taagtggaga ggtttaaagg gaagcaacgt gttgtcaaga cagaacattt 99480
   tcaatatctt gacaaggcat ttttcccagt tctcctttgg tcataacgtt aaccataggc 99540
   aaactagtga agaattgact attaatttat ctacctttag atgcttcctt ggaagttctt 99600
   ttgtgtgtag ctccgtctcc ctatttccta acccaaaatt caaatctcaa tccatacaac 99660
   ctggcacaga aaaacaactt ggggcaagca gattctctgg ctgggggagt ccttgaaata 99720
   ataaataatg agcaagccag atgcttaaga gtaaaaagtg aagttgtagg ttatgattta 99780
   aagagcaatt gagaagttgg ctgggatgtc atgatggatc atattcaagt gagggggagt 99840
   ggaaactatg agcaaacata cactctggcg atgaggttat acatttattt aaactacaat 99900
   tccactatgc agacaaaata tagcttcttt atttgattta gaacagcctt aatcttgtta 99960
   atgagcccta cccactggat aacaaaagga acaaaccaaa ataacatgct ttagtctagt 100020
   tcttagtttg aagcaataat tttgacattc ttaggcatta ctcaattttt tagaatctta 100080
   tttcttttcc ttctctgtca atgtgatgtc tggggctata gggcagattg gagcttatat 100140
   gatcttgtgg gctttgaggg gaaagaggcc caaatgccat ttaatattct ttgtctcctc 100200
   tagtctccac agccatgacc ttgaacagct gctcatggct ggccccactg gtacttgcag 100260
   ctacaattgg cggctggtac cacttgcgat ctagcctctc gtagcttggg tggtggctga 100320
   tggccttccc tggctcatgt ggcctcaccg cactttactg atgctaataa cccttcctaa 100380
   agggttgtgc ctcccatttg tctgtcacag catcaaaaca cgcttactga gaaatagaac 100440
   tccccaaact ctgtttcctc tacattgaat ctacatcact ggagcaccaa aagccaaccc 100500
   aaaaagtaaa acatcaggaa gtttatctca ccacatggca cctccaggtg gacaacttga 100560
   tgaagtcgtc ccagaggtgc agagggcaac atgggaagtg actgcctcct agagtcccag 100620
   gcatggtatg gagcgaaatg ctcatgccac ttcagtaccc caaattacct ggatgttcct 100680
   aaagctgccc actgcccttg aagtttggcc ttgagatggt gttgggagat aagaggaagt 100740
   tagtctttcc atctctgcct ctctcctttt tcccaccgtg cttcaggcta gaaagggtgg 100800
   ggttttcctt gtcttcgcct gcatcttacc atcctttcac catcactagt taagccttgt 100860
   gactaagcca tattagatag aaagggtatc atctctatac ctcaggagga attctatggt 100920
   ctgattcatc caagttagtt cctgaaattc taaaagggat tttgaaaatt cctcctgtca 100980
   tctaagcctg gttttcaagc ccattatttt ctcaagggac aagggggttt catggcttaa 101040
   cctacaaaga atatgatttg gaagggaaaa gaaaatacat tcaggaaaaa ggtggaaagg 101100
   tggaaaattg caaagaatag gatttgaggg ggaaagaaaa tacattcatt taacattcat 101160
   ttaatatttg caaagtattc attcattacc tatcaagaat ggaaaatata tttataaagg 101220
   gtagggaatt tgtcagaagt gagaggactg ctgttcagac acatggagcg gagttaaggc 101280
   tgtgattggt agggactaaa atgaaacagc aaacatctta ctagagccgt ttaggttaca 101340
   gaggactctc tggttaccgt ctctctggag cctccttttg ttagtttatt tggcttttcc 101400
   tgtgttccca cgcttgtgta caaatctcta attacttgat ttaacttaag tgcatttctg 101460
   ttccttaaaa ccaaaatacc tgatcgaaca taagcattaa tttaagactt ttatattcca 101520
   caatgtgtat acattgtgca cacgagtgtc actaaatctc atttcctgtt ccctgtaaat 101580
   gtcgtaatag tctcagattc cggctgccac actcccaggt caccaccatg tcaccctagc 101640
   tggctgacct gactctcttt gcggttatcc tgccatcctt gcccctttag gctccctgaa 101700
   actccactgt cactacatcc tctcctgccc cgagacttac acattgctct tcaccctcct 101760
   cccagtcatt gtaagttgga tttgcctcag tgcagttctc gggttctgcc catttaaaat 101820
   aatccaattt cccactatgg tcaacttgcc tgtactctag gcaggcctac atcaaaactg 101880
   gcctgtaaag gggaacaccc tgtctgtgct tgctctctca ttttctgcag ttcctcaatg 101940
   atttttgccc ccatctaccg ctagtctaaa tctaacccat ttttccaagc ttcctccaat 102000
   ccaacttatt ttctttactc tgagctgtta gttactctag gccatgctac tttagagctt 102060
   aattatatct catctttgga taataggtat ctaaagctaa tttttgttga gcacctttta 102120
   tgggccaagt gccttcccag gatagctcat ttgatctgca ctccacccct gtgaggtggg 102180
   cacaatgatg tacatactgg aaatgaggaa atggagcctc ctggtgacta agtaactttc 102240
   ctaaagtcat acaggtgatg ataagtggat ggaactaaaa ttcacaccat gatatcagat 102300
   tccagagact aaacttgtcg ttaatgctgg caaaccgccc ccagagaatc actgcttcca 102360
   atttcaagtg tcttgatctt atctccccaa ccagatggtc gtaactatgc tggcagcaat 102420
   atgggcaaga tcatgaattc tgtgttgtct gcatcctcct aagcaagcac atacaagacc 102480
   cttgaatgat tatctcccct gactggcctc tcccattgaa tcaccattat ctcccatata 102540
   gagcaacatg gtctttacat ttgaggctta ttcagattct gttaagtttg agtgctgcac 102600
   ttcctctctt tcctcccatg catactctgt gagcccctgg atggctaagg tgctgaccgc 102660
   caaaggtagg aggcatctgg atgctttccc agagactcta cttctctctc tctgcccatc 102720
   tgccaggctt gacttctgat gtcatgggac tctggtcaac ccctgatgcc cttttgcaat 102780
   gtcgggattg cacaatgttc ctaaatctac cttccagaca aaccaatccc aaaggagttc 102840
   atgagcagga ttatagcctg cagaaggtgt tcccaggcca gctcccctga ggctgggctt 102900
   tttgtacagt gttgaaagga gacccctaag atttctcatt gaaatgctcc ccagccctgt 102960
   gatttattta gagggaaacc agctgcccca gtgagagcag agaaatgtct agtttgtgaa 103020
   cagattttaa acattagagc ttatgccttc aaagggtggt ttcccaaggg gatctctctt 103080
   cctgctccta aatgaaaagc tacaagcatt tagggctacc ttaagatgtg gtgaaatgag 103140
   ggtttgagac tgtcaacatt tggctttgct ctttgcggag ggtgctgggg agcttcacag 103200
   gcccaggcct ggcaggaaaa ggctcggcag gggcctcttt gttgtgagag gatttggggt 103260
   ggaaatcatt cgtttcctcc aaaggcctcc tccccaccaa cagtgtggcg cctcccttcc 103320
   tctccagcat cctcgccggc ctctccagtc tctctgcttc ccctctgcca cccagtgtcc 103380
   tccttgggag gaagaagaga acaaagtaca ttcaaaaagg ccttcctctc tcttcctata 103440
   aggtcacccc aaaaaagtct ccccaatatc ttagagtgca agtttcttgt tataccaggg 103500
   tttggctgca gaaactagaa gtaattccat cagtgatgac atgacaagat catttaagga 103560
   tccccttaaa agccctgctg tttgtctaaa tttctgccca cagctgtctg tttaagaaac 103620
   ctcacagaaa caccatgaaa tcctgtttgt tctcaaccct taatcagtgc taatccaaca 103680
   ggatgggaag gtggagcttg ttttaagatg ggggaagggc ggcatctgtg atcatgacac 103740
   acatcagaca acttcccctt ctagacatgt caggggatgt tatggcttat gggagatgct 103800
   caggaaaaaa agcgaggggg gttgttcttc cctggcctgc ttttatcttt aattgtcaac 103860
   actcctggcc tgcagaggat tgaaaagaaa aaactgtaca ggcctaaatc agtcagagta 103920
   gcagggaagg ggtgagatgc tccacactca ggatttaagt ctcttcctgc aagtggggaa 103980
   cttttttcta tgcctggcgc acctgtgcct gcttcaaaat ggtcctggac ctggcccctg 104040
   gtctgcaaag aatgaagtga ggcagcatta cttgacccct aattcactga agaaatgtat 104100
   gtatatccaa ggaagtgtga ttcctggaaa tccagattta taaaggtaag attaagtcag 104160
   ggattagata ggctttttga tttccttggg aaacatgaaa ggatgcctta aaatggtgtc 104220
   tcccaaccaa gggcatttga aatatgatct gattttaaga atttggtccc caatcaaatt 104280
   cttggcaatt gaatcatatt cagtgtgggg gaggtgggag agggaactca tttactcatc 104340
   aactacatct atgcaattcc aacatcggat gggaaagcaa aaattctcag gacagaaaaa 104400
   gattcactaa ttaattgacc acttatgtaa ttagttctca tttaatcata agtttccact 104460
   tatgctgttg tctaccctca cactggcatc aagatctttg ttggtcactc ccatgaggtc 104520
   attaatacaa tgcaaatcaa atgcaaattg attgcaacaa agtgaaattg catatcctac 104580
   aaaagatgta ggctttcatc caacttggat gaggacataa tttgagcttc aaaagagagt 104640
   gatctgaata tcagagaatt aaaagagact ctcggggaaa tgagtgaagt cctttaatat 104700
   ttttgcagaa atgaccctct catgactgta gcaaaagtca atgtaaagta aaggagatct 104760
   taaaattttg ccagcaaagt taagccaaaa aatcaacatt taattcattt cctgattcta 104820
   aaattagcaa ttgattttaa ctgtaacatt ctggtaaata taaaataaat actaaatgat 104880
   tttatacctt tcatttttaa aaataaaatt caatcagtac tttaaaaata ccatttattg 104940
   taaaaaaaat gcttcttgtt tcaagtgatt tctctttgaa tttttaaagt atcagctatc 105000
   cttggatatt gaggaccatc tgtggttctt tattatttca catatattat ttcatttact 105060
   gctcacagcc accctgttag gctaagcagc tattagcacc atttttacaa gtaagaaaat 105120
   tgaggccttg ggaggtgatt tgaacagtat tacaaagtta gtaaatatta gcttcaatta 105180
   cataggcaat gcaattattc ttattttgta aaggagaaaa ttctgtctct agcaggttca 105240
   atgactaacc cttgggcgca tggctagtaa tattagttac attccaaccc agatcttctg 105300
   tccctaaatc aaatcttatc ctactatagc caccaaattt atttacaaat aaaaatagcc 105360
   aagcatgatg taaccaatgt tttgagagct aaagatgtca aagggaacct ctagtctatt 105420
   cagaaagcag ggttctgttg caacctccat tgagaggaag ggaggggtaa tcactattag 105480
   attaaagtgt atgtttaatt cattttagat tgagttaaat gagcacttca gtcatgagta 105540
   cagcaggcta tattcttctt cagtgaccca tttgccaaaa gtaccacagc aattaaacac 105600
   tgagcaaata catgcatagg taagataaaa atagcactca gatgttgaaa acctaaattt 105660
   atccagcaca tttaagccta gcatagcaag gcatatgtgg tatctgtttt tattaatgga 105720
   tttttatttc tggcttcttt ttccctttca gctttggtaa tgctagatgt atgaacaaaa 105780
   atctagatat ctatccagaa attgtcactt tagtactcat cagttgatac aaacaatata 105840
   actgaggcca ttataatatt aagtgaattt tcaacaaata gagttagaaa tccaattatc 105900
   tttccataga aagcttttgt gtggagtcat tgttaaataa aggagggaag ccagtgggag 105960
   agttagaaca gcaatttctc ctccctgcaa aaggacgttg gagaagttca tcctgcacat 106020
   acattaatcc attcataatg gcctgcccac tgcagtcagc ccacaaagat catttttaac 106080
   ttccttttca cttctggtta ctggaggctt ccttttggac acagagcagc aaagcaggag 106140
   ccgacaactt gggcaaaaaa ctaaaagatg aaacacacag ccctgcacac aagcaaatgg 106200
   cttaacacta agttccagat ggaaggcaca gcacccctcc cctggggcct cagtctaggg 106260
   ccttcagagc ttactggaaa tgagagaaaa cagaaattcc ttaccttggt tccaatttga 106320
   ctagactttg gtcggcctta gattagtgcc tctcaagctt taataggtgt gtgaaccaag 106380
   ttagtatctg ggttaaaatg cagattctga ttaacagatt tggagtgggg tctaagactc 106440
   tacatttcca gaaagatggc aggtgattcc catgctatta gtccatgaac tttgagtaga 106500
   aagaatcaaa actaacctaa gccttagttg ggacactgag tcttatgctt gactctcggc 106560
   aatggccatt aaaattatat tcaacaggga gcttaaagac cctctggcct ttgacaatta 106620
   ctaactatgt aaccttgagc aagttgctta acttttctgt accttgattt tcataaaagg 106680
   ttggttataa ggattaaatg aaataatgct tactacagtg cctgatactt agcatttgct 106740
   ttgtaaatac aactgatttc actgcttcat tttacagatg tacagaaaag tcattctgag 106800
   gtgaagaatc ttgctcaatg tgacacagct tgctagtgcc agtggttgca caggaacaca 106860
   atctcctgac ttttagttga aagccctttc taccacctgt gggtctgctg cttagttaga 106920
   gcccattact aattaagaaa gctgacctag ggtacaccca ggcccccttc acttccccag 106980
   cctcagaaac ccacagagca actatggctt acgtcacgca cccaaatatt tttgctatag 107040
   ccggtcctga caacacaagc cttctgcctg ccattaatta tgaagattac ccagataatt 107100
   gatttaattg ggaacaggga gtatctcagt gcaaccagcc aaagtggatt agatctcctc 107160
   agtgcggtgt gtagatccct gcagctctca aggggaaagc atttccctca tggggaaaat 107220
   tatggatgcc ccgatggaac ctattgtcta ctctctgcat aacactagct ttggtgacct 107280
   ggtctctggg gataaaatta aaattagtgc caggcacata caaccctttc ttcttccctg 107340
   ccatctttat agaaaccctc aatagagttc taatgcccac atttgacaat gatgtctttt 107400
   gggaggtcag gaagcagaga gagtaaaaaa aattctctcc caatgtggtt ttacaaatgg 107460
   gcattagcca caggctgttc cagctcaagg gtgaagagta gcacaaactc ttttctttct 107520
   ctgcaataaa aagaatgaaa ttacttcttt ctatagcagt gctaagatat gaataactga 107580
   actggaaaaa aatattagct gtctgtctac tgttgctgca taatacaaaa tatatatcat 107640
   tccagcatct gcccagtgaa aaattacaag aacactagca aacatgtgct acaaagtatc 107700
   tttgatttgc ttgtcctcat tattgatgca atcaaaatgg ggttatcaat ctggttcttt 107760
   gaaatcaact attttagtat ccatttattc taaagacaca acacttttaa caaatcagcc 107820
   tcacatcact ttaacctctg tggaatattt tcttttctgt ttctccatgt atgaaagcca 107880
   tttaaaacat ctggtagact gaaagcccag gaaagagaag ccacctgaat aaaatacaaa 107940
   aagcagtgtt attgatacaa atactaaatt atgtgtgctg agattcttaa acttgggaaa 108000
   ctgaaaataa gatggttctg gagactgatg atggaatata gagcctttgt cttttaggcg 108060
   actgttgaca ttctaatcag agcagaaatg ggaaaacaat gcagcagaaa tgcttctacc 108120
   cattgaggat acattctgcc ttagtttctt tctaagggcc catgaacctg ccaccataaa 108180
   tcaaaaccat ctaagatgtt gctttacatg ctttggcaac attcactatt tgatgggatc 108240
   agatatttcc tcattccctg ttatggattg aattgtatct ccccccaccc cccaaaaaat 108300
   gcaaaaatat taacccccag tacctcagaa ttgaacttat ttggaaatag ggtcgttgca 108360
   gatgtaatta aattaagatg aggtcatact tgagtagggt ggatccttta tgctatgtga 108420
   ctggtgtcct tataaaagag atggtcatgt gaagacaggc acacacacag aacaccatgg 108480
   aagatggacc agagattgga gtgatgcctc tacaagccaa ggagtccctg tgactgctgg 108540
   ccatgaccag aagctaaaga gaggcatgga acagactctc cctcggaggc ctcagaagga 108600
   attcaccctg ctgacacttg gattttggac ttctaggctt tagaacaatc agagaataca 108660
   tttctgtgat tttaagctat ccagtttgtg ctactttatt acaatagccc taggaaacta 108720
   atatatcccc ccaaaaggat tttttctttt tttctttttt ttgagacagt ctccagcctg 108780
   ttgcccaggc tggagtgcaa tggcacaatc tcggctcact gcaacctccg cctcccaggt 108840
   tcacgcgatt ctcctgcctc agcctcccga gcacctggga ttacagatgc ccgccaccac 108900
   acccagctaa tttttttgta tttttagtag agatggggtt tcactatgtt ggccagactg 108960
   gtctcaaact cctgacctcg ttattcaccc accttagcct cccaaagtgc tgggattaca 109020
   ggtgtgagcc accacaccca gccaaggatt gttacatttc tatcctttat gtcaagtcag 109080
   agcctggtta gccctctgag ataggaatga atctgagatt ccaaaaaata aatgaacttg 109140
   aagataggaa actttcccag ccccatacag actttctact gagccagtgt tttgttggag 109200
   atgtgtcact ttcttttaag gaacatacag aagaccttgg ggtctccgtg tctgtgtgtc 109260
   tggtaccctt cagtgtgaag caaaacacat ttggaaatgt gaaattttga ctgtgccccc 109320
   tttcaactgt tcaccattct ttgatttatt taatgaatat ttatcttctg cccacaggga 109380
   cagataactg acatgttgag aacttaaaag taactggtct tatcttcacc tgcctttcca 109440
   taaacattta tattcatagc atcagtgtca caatctatca ttgattgatt ttgaagttgt 109500
   gtgtttctca gagccttact tacaccagct tatgaacgaa cagataagag ctagcaaaat 109560
   ttctgctcag agtctgtaca taaaatggca ccactggcta tagtttatac tggaaaatag 109620
   ctttcatctt tcatggcaca caataggccc acactaatgg cctggtgatg aatgctgatg 109680
   ctgagagctc tgtattgagg tcaattacaa gaccacacct ggactccaca ggaactgtgc 109740
   tgggaggcat caatgtctcc accaagcgct cgggggtaca gtcgtagtga aggagctgag 109800
   tatttgccca ccctgctttt caggttcaag tgaaaagact ttgaaattat accttaaaaa 109860
   aaagaaactt gtgaaacata aaatataact ttaataacag aatgtgtgac actatgaatc 109920
   ttggagaatg gcttaagaaa ctcagcaagc atttttttct agcccttctc taatgataat 109980
   gtatctaatt ttacagatta aagaagtaaa gttgagtatt ttgtttttta aatcatcact 110040
   gctactttcc aagcttgtga gcttgagttc ttttaggatc tacattcacg acccttctac 110100
   tgcccaagag acttacatgt cagaggagtg acaacatctc caggagcttg gtaaattttg 110160
   gccaactatt tgacacaatc cttgtccctt ggaaaacact aatgtatgtt taacgtgaaa 110220
   atcaacttcc tagaaatccc ctattccagt agcttgtaag gatgacagct gtttaacaga 110280
   ggaaaactgg tttcaaccct tgtgtatttg aacaactgct tacaatgtaa aataagaaca 110340
   tctctggacc agtcaatccc gtccctgcag aattatgtgc tggtttttat acagtgctaa 110400
   acagtttggc agatctaatg tcatcaatgt atgaattagt ttcacatctc tggctgtaag 110460
   aaactgataa cacccataaa gcaattccca ggtagactct gattggccag ctcaggttac 110520
   aagcccatgc cgcagtagta gggtatggtg cccaattaat aacccatcaa gattctgtaa 110580
   aatggaggag gagttccttt gattgtatcc agcacaatag gaatgggata atatgctgat 110640
   caatgacaga ctatcagagc ccacttcaat attctgatcc ttggccgggc gcggtggctc 110700
   atgcctgtaa ttccagcact ttgggaggcc aagcaggtgg attatgaggt caggagttcg 110760
   agaccagcct gaccaacatg gtgaaacccc gtatccacta aaaatacaaa aattagctgg 110820
   gcgtggtggt gcatgcctgt aatcccagct actcaggagc tgaggcagga gaatcgcttg 110880
   aacccaggag gcggaggttg cagtgagctg aaatcgcacc actgcactcc agcctgggcg 110940
   acagagcaag actccatctc agaaaaaaaa aaaaaaagat tctgatcctc ttaaactgag 111000
   gtaccccatc ttatatctct acgtatcctc tgtagtttct agcactgtac ctgaaaatgc 111060
   aatcttttaa taaattctga taaaaagaag aggatgtagc aaggtaaaga gctcttttcc 111120
   acacctctgc ttcacatctt caagaacccc atttgatgcc aggccagaaa tggggctcta 111180
   atgcaagcta cagtggctct ggggcttctg atgtgcactt tatttggagg agggccaact 111240
   taattcagaa aagagttctc caggctggac tgacagtgac ctttccacag tgacctcagt 111300
   ttaaggcctc attcaatttg aaggagaatc ttaaggctag ttcgaacaag ctgcccctga 111360
   gataaagcca gaaatggttc atgctctgca gcagaggcat agacataatg aactgctgac 111420
   tggtgccaaa caatctcttc cttttcattc actatgcgta attagtccag atttatagga 111480
   cagacttcct agaatcctca ggcacacaca cacgacaaat ctctggaagg aaatggactt 111540
   caattttccc ttcccaagct cccctcttgg aagctctgtc actcaaggcc ctctgtctgt 111600
   cacatctcct ctgagatcct tgaaggctga gctggtttgg aaaagagcac actccattat 111660
   caagccaggc tccttacaca tcttagaact tactgacacc tgctggacgg tttccttctc 111720
   tctgaaaagc atctgagcag aagcctttgc cctatgtgtg aggtcgaagg agccaggacc 111780
   ttggcttctg cactggaaga ggaacagaga gctttccctg ccctgcggag tgctgtgtct 111840
   gctcagtgaa aacgtacctc ctccccacag agagcagact tgagagtccc caggcagctt 111900
   caagaacaag caccctgatg gtaaaaatgc atgcaggaag gaacaccatg aagagcagtt 111960
   cagaggtgga aggttggaat tcatgatcat ggtcaaactc ctagattctt atggcttttc 112020
   ctcctggaga gactgaacat acagctagct tgaaaagtat ccacttccaa gtgtggagcc 112080
   ttacactgtt ctctgggttg ccagagccag gagatgctgc ttgaccgggt ctgtgttgcc 112140
   ccagagcaaa tcctgaagga agtaaaggtg acccttggta ctgtccactc tcaaaacatc 112200
   tggttgtctt tggctgcctt ctccaactcc taatgttttc agggcaatgc actcttctca 112260
   acaggttgga ataagcaaat aaactaattt cattgcatcc ttgtgaaact gcatgtttta 112320
   agagaacctt ggggtgagat ttgtgctaaa gtccagtgaa tctctcctaa tggtagcatt 112380
   ttaaacctga ggataggcta ataacagaaa ctacttggaa aagtgggctt gtggaacccc 112440
   tttaattata acgtgttgga cagctgcctg caggtagatc ttgcatagaa ctagttagtt 112500
   attgctgcta tcttgccaag gaattgccct acatggagac caactgctgg accgcccatt 112560
   ccccagcctc ttctccttct tcccaggaaa gaggaggagt actcactggc tggctggctg 112620
   gctggctccc ctcaagctcc ctggaagatc ccttctggag aagctattct tcagcattcc 112680
   ctcctggaac cacccatccc atctgcagtc ataagagcac ccctgagtct catctcagtt 112740
   ttcctcccta agagcccagc taaatgcaga aagcaggaga gtaggtgagt gtgagagcat 112800
   ctgtaagcct ttttgccgcc agggctctag tgtggcagag agacctccct gggagtggag 112860
   ctataccctt ccctatacag gaacacagcc tgcccaggcc aacctcagaa actaaacacc 112920
   tttatttcaa gaaagaaaca ccatgctcaa aggtcctcca cctttggggc ccccctccca 112980
   tcttcagtat gcttcaggcc agaatttagg tagagctctg aagtttgagt tctaatagct 113040
   gggtgttgcc tacatttggt gttctaaatg acccctggcc tcctaggggt aattatacag 113100
   cgggatttga gggtttaagg atgggtaggc caatactggt aaataccaga aggaatgtac 113160
   aatttccccc taaaagcctg ttagggccag agcctgcaga gatgaaagag gtcaggaaaa 113220
   tgccatgata ttacttgctt cctaccttaa catttggcct ctctgtgtcc ctgcacaaag 113280
   aaacctgtgc tgcctgtcag atatgaaaag cattgctatt tagcaactca aaaaccatta 113340
   gaggatttac ttcaataacc tggaactcat ggatttccag aatccagcct ttgtagtgct 113400
   ctctaatttt atgatctgta tcctcacaaa tcctcctctg tcccagacat actgtgttcc 113460
   agcacagtct tttgttcaca atgctccccg tagccccaaa tgcagccctg agtcttcctc 113520
   tccagtgatc tcagtcccag ccatgtttca gcacctcccc catgtcccac ctcctaatga 113580
   agccctcctc tgccactgcc ctcaccaccc acagctctta atatttcccc tgtcctatgt 113640
   catacacttc cttaggttgt tgcttatatt ttcatgtagg cagcctttgt tctggaagac 113700
   cacaggcccc tcagaggtaa gctctttgtc atatacctgt ttgactcccc agttcaaacc 113760
   cagaactttt caccacaact tagcccatgg agggggtccc ctacatggca tccctgaaag 113820
   atgtttcccc agtctcgcca tgaacatttc tggtgacagg atgtgcatga cttcatcctg 113880
   acccactcca atattgaata gctataagtt ttattaagtt cttatttcta ctaagccaaa 113940
   atcagcaata tctgctccca atgctgcatt acaagcagct gctagctctc aatttttgtc 114000
   aggattctct tctccagagc agagattccc caattctttt gatcttcatc tatcatagtt 114060
   tacagaccct gtaccattcc ggcagctctc tcctgggaaa tttctggttt ccagagtctc 114120
   tcatgaaagt tgccacccag aggggaacac aatattcagc tgtatgaaga tgtgtgctct 114180
   gaatgatgaa catactcgag ggaggtatct tggtgataat ggtgatgatc atgatcacga 114240
   tcaaattcta accacctttt aaaacacgag gaaattgcta aagaaagaac gctagtattt 114300
   tacccatcca agggattcca gttttgaaga aaaccaaaaa tgtaggaaat gcagtcacct 114360
   ggattgtggc agacattgaa tctactttct caatgttatt tcatagcctt ctgatggtag 114420
   agttgtggtt tgagtgagtt atacaaagaa gttcatgtat ctgattaaac agctttatgt 114480
   gttaccaact ggaaagataa ctatggcctt ccaaaccgac actggctgcc tctgcagatc 114540
   cttgttagaa tagagctggc tcggaacctc agcggtcctg gagatgttcc aacagctcag 114600
   gtttcccaga tctctgcact cctcacccat ccctaagcct cctccagggc ctcaaaatct 114660
   cggaagtccc ttcaaagagc caaattaaga gaatgaaatc atcaaatgta tgaagaaaac 114720
   caccaggaag aaacaatttc cttttctcca agtccctcaa cataccaagc aatgccttac 114780
   gtaggtttag ggcttgggag gccagattat gctttagttc ttgagggcct tttatgtagc 114840
   cacaggacct tacgtgagtg aattctcctt cctggccctc actttacttc ctactccaaa 114900
   atctatgaac ctaaagtcta caactaatga aggaggaaag cctgataaga aatcaaatga 114960
   ttcccctaat cactccagcc cacactgatc ttttgttttc ttttaattct gaggcattta 115020
   ttttctgtac catttattta attttaatat tttgctgcct tcttattttt attctcataa 115080
   ataaaaggat agtaagctct aggaaaaaaa aaataacaca actcatctaa tgcccttgga 115140
   aagttgaggg aactgactgg cctaaagttg caaagctgat tagtacaaga gtacagataa 115200
   ccatcccagc tagatgctaa gctcccaaga ggtcagaaga gaacaggttg acacatcctt 115260
   ctttttagat ccaaccagca gctcactttt aatagatgtg cctcaggtca ccttaatagc 115320
   aaactaggtt aggtcttggg tctatgactg aaggcttctt agagaagtgg gatgaaaagg 115380
   aactgcccct gggacattta gttatcctgg ctgtcatcta ctaagctgta ggatcttctc 115440
   atattcataa tctcttacct taaatgtggt ggtattttga aagacagtag ctgggcttag 115500
   acatcagccc aagcaaggca tagctcacag tctcaatgcc ctaccctgtc acctggagtc 115560
   ctgatgaagt ttctgcccaa acagaaagtt ggggcaccct ttattctcca agccttaact 115620
   tcaggagtct gttgccacaa acttacacag aacagaaatc tatatggtga ttataacata 115680
   tgctcaaata taagatggcc acaaatacaa agtgatttcc cataagaaca gccaaaggaa 115740
   aagtttttgg ccaatttgaa tatataaact ttcagggata tggatgaaat attcaattgt 115800
   ctactcataa taatttgtta attaaattac ttgttgatat taaaaatcat gtactaccta 115860
   aaataatgct taatttagaa tttcttgcca ccctcatgtt tcagaataat tttgtacatt 115920
   ctcaaaatat attttccatg ttcgttgtct ctttcatcat aatcgccaat tttttatctt 115980
   ttaccaactt gtccagagca cattgtttga catacagcac tttttgaaat cgtgtgagat 116040
   gatgtctgta aaatttcttt caaaccataa actttcctaa cagtaggggt tcccattcat 116100
   tctataagtt gacaatacaa ctacttccct ttttgctatt ttgtgttttc aaaacccaaa 116160
   tttgaaagtt tgaaggacta ccctaaggaa taattacgaa atctgtgtca aattgccatg 116220
   cctgctttct aaaacgcatt tttgtaagat gttctctata aaactgtaga actagcagtg 116280
   attgaatcat ttcagggaat gtatctttcc taaatagtgt tttaaaaaaa aaaaaaaaaa 116340
   gaaacgagat gacctagaat atgagaaact tcaagaagtc ttgaataata cctctattta 116400
   tatctgtgtc tatttttatg tctatgtctc cctccctttg tgaaataact ttgaaggaaa 116460
   aactttgcct tataggtaag gagtgcccag tttatacctg ttttctggag taattattaa 116520
   tagtgcattc ttttatgctc aaaagtgtcc cagtttggac aatatattca gttgccactc 116580
   tactgatggc aaggaatatg ctatatatta taaatctgta gccttgtcaa ggcattcacc 116640
   atattggcca ttcagtaagc ttcctataaa gctaatatct gcatggcagc cagatggaac 116700
   ttgaaggcat ggtgcttgga aattaaagca atgtaactaa ttatctaaag tgatattaca 116760
   atgtgtaaga tactgaaaat ttgactatca gtcaacacag catctgcttc aaagaaaaca 116820
   tttttttaaa gcatctttat tttttagaaa tctaattatg aggaactcag ttacgaagtg 116880
   ctgctttgat cttagaacag tacatttagt ttattattag tttaaactag atattttgtt 116940
   taatatctct aaatatttgg agataaattg tccttattaa cttactttac cattgaacca 117000
   ttcagactta caatgatgtc ttgtttaagg caaaatttat ttccaaagga ctgtcttgaa 117060
   agtgagtgca ttatgtggaa tgaggggcga gtgaacagat agactggctg caaagagctc 117120
   tgggctgacg aagctctgat cagaaatcca agccttgggc aagcctgcca gccttcctca 117180
   ctttctcatg tgttgtggga ggggcctggt gggagataat ttgaatcata ggagtggttt 117240
   cccctatact gttcttatgg tagtgaataa gtctcatgaa atctgatgct tttatcaggg 117300
   ggtctctgct tttgcatctt cctcattttc tcttgctgcc gccatgtaag aagtgccttt 117360
   tgcctcctgt catgattccg aggcctctcc agccatgtgg aactgtaagt ccaattaaac 117420
   ctctttttct tcccagtttt gggtatgtct ttatcagcag catgaaaaca gactaattca 117480
   gtaagttggt accagaaatg gggtgttgct gaaaagatac ctgaaaatgt ggaagcaact 117540
   ttggaactgg gtaacatgca gaggttggaa cagattgggg ggctcagaag aagacaggaa 117600
   aatatgggaa agtttggaac ttcctagaga cttgttgaat ggctttgacc aaaagcctat 117660
   tagtgatatg gacaataagg tccaggctga ggtggtctca gatggtgatg aggagcttgt 117720
   tgggaactgg agcaaaggtg actcttgtta tgttttagca aagagattgg cagcattttg 117780
   accctgccct agagatttgt ggaactttga acttgagaga gatgatttag ggtatctggt 117840
   ggaagaaact tctaagcagc aaagcattca agagatgact tgagtgttaa aggcattcag 117900
   ttttataagg gaagcagagc ataaaagttt ggaaaatctg cagcctgaca atgtgataga 117960
   aaaagaaaaa cccattttct aagaagatat tcaagcaagc tgcagaaatt tgcataagta 118020
   agaggagcca aatgttaatc tccaagaaaa tggagaaaat gtctccaggg catgtcagag 118080
   atcttcacaa cagcccctcc caacacaggc ccagaggcct aggagaaaat ggtttcgtgg 118140
   gcctggccca ggatcccagt gctgtgtgca gcccagggac ttggtgccct gcatcccagt 118200
   cactccagcc gtggctgaaa gaagccaatg tagagctcag gccatgactt tagaggatac 118260
   aagccccaaa ccttgggagc ttccacatgg tgttgagcct gcaagtgcac agaagtcaag 118320
   aattggggtt tggaaacctc cgcctagatt tcagatgtat agaaatgcct ggatacccag 118380
   gcagaagttt gctgcagggg tggggcactc atggagaatt actgctaggt cagtgcagaa 118440
   gggaaatgtg gggtcagatc ccccacacag agcccctact ggggtaccgt ctagtggagc 118500
   tatgaaaaga gggccaccat cctccagact ccagaatggt agatccacca acagcttgca 118560
   ccatttgcct ggaaaagcca cagacactca atgccagccc atgaaagcag tcaggaggga 118620
   ggctgtaccc tacaaagcca taggggtgga gctgcccagg atcatagaaa cttacctctt 118680
   gcatcagtgt gacctggatg tgagacctgg agtcaaaaga gatcattttg gcgctttaaa 118740
   atttgactgc cccgctggat ttcggacttg catgggtcct gtaacccctt tgttttgtcc 118800
   aattgcttcc atttggaatg gctgtattta cccaatacct gtacccccat tgtatctagg 118860
   aagtaaccag cttgcttttg attttacagg ctcataagtg gaagggactt gccttgtctc 118920
   agatgagact ttggactgtg gacttttcag ttaatgctga aatgagttaa gactttaggg 118980
   gactgttggg aaggcaatga ttggttttga aatgtgagga catgagattt ggaggagccg 119040
   gggtggaatg ataaaggttt ggctgtgtcc ccacccaaat ctcatcttga attgtactcc 119100
   catgattcac aagtgttgtg agagggaccc ggtgcgagat aatttgaatc atgggggcag 119160
   tttcccccat actgttctcg tagtagcgaa taagtctcaa gagatctgat agttttatca 119220
   ggggtttccg cttttgcatc ttcctcactt cttcttgctg tcactatgta agaagtgcct 119280
   ttcccctccc accatgattc tgaggtctct ccggccatgt ggaaatgtaa gtccaattaa 119340
   acctcttttt cttcccagtt ttgggtatgt ctttatcagc ggcatgaaaa tggactaata 119400
   cagactgctt gcctgttata ttttatatta aacatgacct ggtcatctca tggcagtcag 119460
   caggtttgaa ctctttctgt gtggccaact ttgggaagcc actctcctgg agccacaggt 119520
   aatgtcatgg aaaaagagtc ttgccaaggc ggtgacttat gaagtatccc ctctcctcaa 119580
   ccatatatgc atctcctcca tctaccaagc agcacagaga gggttcccat gagatatagt 119640
   cctagagaga cgctcgttct tacattggga acatgagtcc tgctctctgt atatgctgtc 119700
   atccaatttt gcctgcagtg agctggcctg atcagagaac aaccaaagga cagctgtccc 119760
   tcagaacaat caggaccttg agacctttac ttgggcccca cactgtccta ctttctgctt 119820
   gagagctgaa ggcatatttc tagaaagaag gcagcctaag caccaagggt aatgctctgg 119880
   gaattttcag cccccgatgc tgctctgcga ggataagaga agaggcagaa agagaaacct 119940
   cttagaatcc tctcccacac tctccaaata tctaagaaac caggccgcct caggtctctt 120000
   gtcgacaaga gctttccctt cccaccattt cctaacctca caaagagcac gacgggagat 120060
   acaatgaaga aggctggaga ggaaggcctc agctatggag gctgctgtac ttcatcacca 120120
   cctcttatgt cctggtggcc ttttgcctcc ctcgattatg tcctctttct ttccggggct 120180
   gcttcttcca gacgaagctg aaatgagagg gtaggatgtg aggccaactt tcccatgtgc 120240
   ccttggaaca cgaggatgtg atgtttatcc ctccagaccc ccttctactg acctctctac 120300
   gcacattgtt ctggaattca gatgcgtcag aggatgtctt ccgtgtgagg ctctggccag 120360
   gagttgctct ctgaactaat tttacacaga tgtgtacaca tatggcccaa agcccactaa 120420
   gtgaccagca gccttgtccc cagcattctc tgctggtctg atgccattgc tatattttcc 120480
   aacctatccc tgtggaatgg tgaccgtggc ctcaccataa accccaacta ctggctgctc 120540
   tgtccttgag gtcacaggct ggcagtgacc ttgccagtgg ctcggcctcc cggaggcttg 120600
   aggcagggct tcttggcttg cagttgcact gtggtgagtc ctcaaggcct cctgtaccct 120660
   gctcttagcc tgtttggctt ccctcccctg tagctgctcc tccctgcagg cctgctggcc 120720
   tccacactcc aggacagtta ggataacatc taaagcacgt ggagttgctg ggaagaaagg 120780
   ctcaggtctg catcagccaa gccacagctc tgctgtcatt tttggtccac aatggccctg 120840
   gggttattgc atttatccag agcatttcca cacccacacc cttccctcca ccctgacctt 120900
   tttatatttt ctgatctttt tttttttttt ttttgagagg gagtcttgct ctgtcaccca 120960
   ggctggagtg caaaggcacg atctcagctc actgcagcct ccacctcccg ggttcaagcg 121020
   attctcctgc ctcagcctcc caagtagctg ggattacagg caccccgcca tcatgcctgg 121080
   ctaattttta tatttttgta gagacggggt ttcaccatgt tgtccaggct ggtcttgaac 121140
   tcctgacctc aggtgattca cccgccttgg cctcccaaaa tgctgggatt acaggtgtga 121200
   gccactgcac ctggccctat tttctgatct tgattatgca ccaataaaaa gaaaaattta 121260
   ccagatgata attttttttt attttttatg accagattgc acctgctact tttccaaaaa 121320
   gccaactttt cagaatgctt taaaaaacac tccctttaag tgacacattt cattttgcaa 121380
   agactatggc tttttctcaa acagattttt atcttttttc tggggtctga tgtggaaacc 121440
   tcactaaatt cctccatcta gatcatgtca gtatttgatt aaaatgtgag tttgaaggct 121500
   ctcctgtgaa gccatttctt cttgcatcct tttaaaatta acactcacgt taaaaactga 121560
   tgacatttaa cattgaaaat aagctaaacc tgatggtaac tctctgagct cttcaaattg 121620
   acgccgaagg tggccacctt gtggctgttg tggaaactgc cacttagacc tgatttttcc 121680
   caggcgttgg ggccctactg ctaattaaat taaatgactc ttaagattat cttttcccat 121740
   gcagtttaca tcccaatcct gctcctctac aggtcaactt gtagtgatat cctggcagtt 121800
   tgggggcaag aaagctattc taaatggcct cagtgggtta gttgaaagaa accttttggt 121860
   ccttaaacta aggagaagag ataacctccc aggaagccca ggtctttgtc ttcctcaaac 121920
   cttatatgct ctaagtccca tattttaaaa aatatgctcc aggggatccc tatatccgca 121980
   ttcctgggta agatctttaa aatgcaggtt gctggggtcc ctcctcaaga cctaagaatt 122040
   ggaatgtcta agtcctggga tttgcatttt aaacaaattt ctcaggtgaa tttttaatgc 122100
   acattcaagt tctaacaatg ctacatttta gtctactaag gaaagaaact ggagacctaa 122160
   catatgaaat gcttttgacg caggcaatct ccccttggcc ctgggaggga ggggatccag 122220
   agctgaagag agctcaagag accaatgcag gagttctcaa aagcccctga caacccaaat 122280
   gtttgctgct tgaacatagg aggctccacc acagtgacct ggcaacttcg gtcgaaccct 122340
   ctaaacaaga agccatgggc caccagaaga gtggctgctt gtgtatggtg tctccctctg 122400
   gcctgcatag gaagcagcaa aaggcagtta gaaggagagg cacctctgct ggctactcca 122460
   tcacccccac ccaactccca ccgtgtccac cccaccccaa agctccatgt cccaactcga 122520
   tgtgaaaggc aagagaccag cttccaggcc aggctctcct gctgaccagc tttgtgaact 122580
   tggacaggtc gctggcttca ggtgttattg ctgtaaatgg aagtagccag ataaccatca 122640
   cttagcttct ttccaattct aacattctgt gatctcattt tgagctttca gagggagagg 122700
   aggctcttta tgagaagtga tcatgggagt ccagattcca agcttagcat taagcctttg 122760
   ctgtttgtag caattaatta ttatggagtg tcactgagaa ttgagatcct ctgccaatac 122820
   aaaaacaata aacttttgca atgtcaattc tctacattgg gggtaaagca gcactggact 122880
   gaaagccttc caaatgcccc cccccaactg acagcaaccc catgacctca tcacaccctc 122940
   ccctcctcat gcactattct ccaaagactt taacacaccc catcctcaca cacgcccttc 123000
   ccacacccac ccccacctcc caatcaggcc tcctgtctct gaacccactt cctgctctag 123060
   cattcatctt gttgggtgct gagatacctg taagaatgtc taggtggaga cttcacccag 123120
   atcatccttc ttatcctttc aagatcattg cccccaggtg ccactgctct gagactccac 123180
   cattccctat gcatactcag ctcagttctt aacctgtgac attataatca tctgctttta 123240
   tgcctgtatc cctaaatgga agatctgtgg ctgccacata ctgaatattc aataaatgtt 123300
   tccttgaatg aatgacagta ggaatgaaag aatgagtgga taaataagga attaagaaga 123360
   tgcaagctga ctatcgtaca gaacctctat acctattctt tgcacaagtc tggatgagga 123420
   aagaagaacc tggtccagac tgtcttgact ctggagcatt ttggggagaa gatgggaatg 123480
   gagggctgtg cctgcagtag aagtagcatg gtaggttatc aaagcacagg tcagtgaaat 123540
   caactattgt gtggtgtcaa tttagtgggt tcagaccaac atttttaaat gaagtataac 123600
   ataatagaat aaattagaaa acatgtctct tagtaagttc cagtacagct ttatggaact 123660
   tttttttttt tttttttgga gacaagtctc actctgttcc ctgggctgga gtgcagtggt 123720
   gtgatcttga ctcactgcaa cgtccgcctc ccgggttcaa gcaattctcc tgcctcagct 123780
   tcctgagtag ctgggattac aggcgcccgc cactacgccc agctaatttt ttgtattttt 123840
   agtagagaca gggtttcacc atgttggcca ggctggtctc gaactcctga cgtcgtgatt 123900
   cgcccgcctc ggcctcccaa agtgctggga ttacaggcat gagccaccgc acatggccaa 123960
   gacttttatt atatagacat gtgcaaaata tacttcttta ctacacacag tggtcaaaaa 124020
   atgtagaagc cactcatcta ggaaatactt cctaatactt tactaaatac ttcattgact 124080
   caatttgaaa aaaaaaaaaa ctgctgactt tatggtatgg cttccattta ttggaatgtg 124140
   gggatccatt tgtcaggaaa tgtatatgga ttccatgatg tttttgagag agctatatat 124200
   tttctaatta cttgaaatga taatgatctg atctcatcta atttcaatta tgttcagaat 124260
   aggtatcttt catagagata aataatttaa gaaaattttc ctaaaatagg gaccttccct 124320
   ctgttttgtc ttttatgtct ccccatgtag agcctgtcta tgatgatagt ataatactag 124380
   ctctctctct ttcctaccaa ggtgaaaaag ccattttcta agagttagta gaaaagaagt 124440
   gataaaagca aattaaaaat cttaaattcc aatatcaaac tttgataaat ctatctaaat 124500
   atctccatgg gaaatcatag tgacaggtga gataataaaa ttaaaatctc aaaggaaggc 124560
   aaatatcaac tagctttgtt aagaacatga atccaagcac ataggataca atttcttctc 124620
   tgctgtgttt tgttctcctc cgatgaacag acagccatgt gttaggcttt ccctggtttg 124680
   gccataacac tgtccccagt ctcgtctgtt gacaccatat gggattgtca cgcttctttg 124740
   tttatgaaga gtctcctgga atcttaaaat taagtttcag tatcagacca gcatccaaaa 124800
   ccaatccaga tacatactta gactggcttg gtgtttaaaa agacttcatg ttaacagtta 124860
   aaagtcagaa catttctttt aaaaattagg gttcttgact tctcttggaa aggttaaaat 124920
   atttagaagt gcaagaccca tattactgca tggagactat tgctcttaac ctaggtccag 124980
   gggacccctg tgttttgata cagtttcctc tgtaatccta tgtattttat tttctttatt 125040
   taaaagcgtc atcttgagaa gggatccatg ggcttcacta gactgccaga ggggttcatg 125100
   gcccaataaa ggctgagacc atcctagagt agagccaagc agcagctgtc ccctcgatcc 125160
   ctgttctcat cattctttac tttctctgaa gccatgcatc tgttttcatt ttccattgca 125220
   ctaacaattt tcctggagtc aagaaatatt ttcctgtaat tatgtctcta tcaaaaattg 125280
   gaaaaagaaa aatgaattga gggttttgga attcttacac taagctgctc cacttattta 125340
   tgccagctca ctggcctctg gtccacaggg attgtgttta acatccttga tcaccagtga 125400
   agcacagcca ccaaccagtc agaatggttg tcatctgtgc acgaacaatg ctggtgcatg 125460
   ccctgtgaac ctcagccctg tgcctggagg atttccatcc ctgcttcagc aggcttctct 125520
   agctctctgg gtgtcattcc atgttgcatc tgtccttctt cctggaatat cagggagtgt 125580
   gagcagtggg ctatttggag tgtgcttggg agcatcctgg ggaactagga gaagatcaat 125640
   ttctcctttt tggcaaagga gcagagatgc cagctccacc ctgccgtcca gtctctggac 125700
   ttgaggggaa atggagctga tcatcatcat gtccgcactt gtcctgcata gtgcagttaa 125760
   cagagctgtc tccctgggcc ctcagctgag agatcctcat accaaacctt ctgaagaaag 125820
   agctggaact aacgccactt tgcaaaggag gaccatggac agtcgaagaa caggagcagt 125880
   gaagtcagaa ggagaacaca gacctgcatt ttagtccact caaacacttc gaaaacactc 125940
   gctcctcaag ggaagtgact tttggggatt tccacagcaa gtgcttaata aggctttttt 126000
   taaaaaaaac aaaaaccaaa caggccttgt ttaatgcatt aatattttaa cagagcaagt 126060
   ctgggccttt caagcaaatc tagtcgctat tcaacagtca ccttagcctt tatgctaggt 126120
   ataaagagac agattttgga ttttgtatgg aagatgggct ttttgtgaca gtaaaatgtt 126180
   ttattttatt tttacaaatt ataatacaat aaaatcgact ttttcttttg gtgtataaaa 126240
   ttttaaccca tgtgtaaatt tacgtaacca ccttcaggat acagatctat cactctaaaa 126300
   aactcctgca tgctagttct ttgtcttaaa aaatatatat actgatgtac atgtaaaatg 126360
   gaagctacaa ggatatttgt aaaataaagt gtacatttta tccatatgaa atcaagatag 126420
   aatttctctc tctctctctg tctttcttgt gtgtgtgtgt gtgtgtgtgt gtgtgtgtgt 126480
   gtgtgtgtgt gtgtgtttta aaggattgta tttccttcca ctttggagga tataaattct 126540
   tgagaaataa cctatacagg ctatcattaa agagaatttt caataattct ttaaagcaat 126600
   atgatggttt gaccattttc agattgtgac ggaaggcata agaggaagca agatgaacca 126660
   ctggggtggt gcctaaacat agcggcccca ggagtgaggc ttctgaacat tttccccacc 126720
   ccaacccaca aggtcttctg cgcctatact actccccgct ccgtcctcca gcaatggatc 126780
   tgtatatctc tccaggtttg tcctctcttc tctctcctgc ctcctttcat atcccaccaa 126840
   cttgcctctt ttttaccttc tgtggagaca aaagtgactc catcttggat gctaatccac 126900
   catcttgact ttccatcagc cccagttcta ggaaggcctc ctgattcctc ctttatttac 126960
   tgaccctagt gcaagaacat gtgctcctga ttttagatca aagcaatctt gatgttttca 127020
   cacaaattat aggctatgac acatatagca ttcttgcttg ttctggatgg tttaattgtc 127080
   ttgctagagt atgtataccc tttccttatg gtggataaga cttgggtttg tggagtaacc 127140
   atgtggagat ctacctatct tggggccacg caagaccatg cttctgtcta taagttccct 127200
   caataaatta cccaatacaa acaaagtgga tttgtctacc acctccttta gtttcttggc 127260
   tccttcgaca tctgggagct gttaagcata tatggccgtt ttcacagagc tcctaggaaa 127320
   aggagatata aagatccaag aagagtggga aagagaaggt gttctgagag acaggaatgt 127380
   aagagtgtat tgacaaaagg gaaaagatat cctttttctg tcccactaaa agtttaccta 127440
   gtttggactc ttcctgttca cctaacatgg cctaaagtaa caggtagctg tcctctctcc 127500
   ctacctgctt tccaaataga gagattatgc agcccccttc ccccactgcc acctgggaaa 127560
   cagccatgca cctgccttcc agccttgcag cctgctcttc tgcacccact tggtcgaccc 127620
   tgcccttggg cccctccctt agaagaagga tgggagctga gagaaaaggg aggaggagag 127680
   agagaagaga agggaggaac aatgggtaca ggtaaaaagg aagctaatgc tcacttgcat 127740
   tcagtgaatt cctaataaga aaaaaagaca cagtgccagg ttcactgccc tctataaata 127800
   ttggaacaga gaatattagc ataatccaaa ccgtcttaat aagacttctg aattctgtaa 127860
   acaagtgcac ccttactaga tatttttgca agtagctatt tcttgggata tcacaggaag 127920
   cggagctttt atttagtcac taaaactagt gtcctttgga taatgcttac tatgacacca 127980
   catacgagag gctgtttagt aaatgatttt cattggttat gatatcatct agttattcta 128040
   tgtgtcttca gtgggaaaga gaagaaatgc ctaagtcagt ggttttctca tttttaaaac 128100
   attaattatc attacatcca aatgaggctt cacatacagt gttaaggtaa agaccaaact 128160
   gtattcattg tgtatgcata tatatatgac atatatttaa tagactacaa ctatacaact 128220
   attttcaaag tatataagct taaaaagaag aaacaaagaa taatagaaaa tgaattgttt 128280
   gtgcttcatt gaaaaatgta caagagtttg atataatcga agaattgcaa cccagtgaaa 128340
   ttttggccat aagtaaaaag cacttgaaca tcttaattag ttttggagtc aaatttggtt 128400
   ccccctatat gggaatgaac tcatcccaca ggcccacatc agcttacaga gttgtatgcc 128460
   caaaaacgaa taactacata aaccagatac tgagagtatg aacttttccc agtttcaaca 128520
   ctcagctatg caaagatttg actttatgtt tctataatac ctagctttaa cttttgaatc 128580
   cttgtttcaa cttggtagca gaaatgttaa gtgtgcccat ttgaggtaaa acttagttac 128640
   ttctagagtt aaaatctgta agttttgggt aaatagccat ttcaagatgc atccctgctc 128700
   ctgcttgctg ctgcttcctt ccaggagaga tataagtact aagatgactt ctgtattgcc 128760
   cagaaaggta aatttatggc ttaaatatgt tgttctatta ttcacaaggt acctctgggt 128820
   cacctttgct ataaaagtgg agactatttt taaaataaag ttaggacaca ctccataaaa 128880
   gttttacaac tcacttaaaa tcatagaaga gctgtatatt tcaagatgaa gatttaagct 128940
   ctggttattt aacagggtct taagaggcag gaattgctgt gtcaactaat gttgtgcact 129000
   atatttcatt actgccttta tgttttttta aatacctcaa gaataaagtt tatttaaaat 129060
   gtcacacttt gcaaactgca cattatatcc atagaatatg ctagtaccac tcccattcct 129120
   tttatttatt acagtagatt agttatggac cctctgctat agtatcttgg cttattaaga 129180
   tgctgcatgc taaaaagcat agatggccct gtaatgagca gttacaaaat caagtcacca 129240
   attcttcctt ggctttcttt tttgttcaga gggaaaaatt gaggatttga cctgtgcctg 129300
   aaacattaac cacctgattc tgcttgtggg gtggggacag aaaagggccc tctcaacact 129360
   taattattga gaaatcctcc gtatctctcc aggattatag aaaaggatta acaatcatca 129420
   aggttgcttg aacaaatctc aagttcaaat tccagaggaa gacgggatgg tctagggtga 129480
   tcaaaaccta gacccagggc tgggtgcagt ggctgacacc tgtaatacag cactttagat 129540
   gccgaggcag gcagatcact tgaggtcagg agttcaaaac cagcctggtc aacatggtga 129600
   agccctgtct ctactaaaaa tacaaaaatt agctgggttt ggtggcgggt gtctataatc 129660
   ccagccactt gggaggctga ggcaggagaa tcgcttgaac ccaggaggtg gaggttgcag 129720
   tgagctgaga tagcaccatt gcactccagc ctgggcaaca tgagcgaaat tctgtctcaa 129780
   aaaaaaaaat catctaattc acaagaattt tttttgctaa tataagtcat ccataaacct 129840
   ctatgaaagg tgagcccttt atgagacaac ttgatcccta taaagtattc aataaacaca 129900
   atgggcacta aaagacagtc ttaatgaaat gggacaattt agaaatattt tcttaattga 129960
   catcaagaaa aagtaattta aaaaaaaaat ctgttagttt tttttttttt aaacaagtgt 130020
   tagtgaggat gtggagaaac tggagccctt gtgcactgtt ggtggcatta caaaatgggg 130080
   ctactgccat ggaaaatagt gtggtggttc tccaaaaaat taaaaataga attaccatat 130140
   gacccagcaa tttcacttct gggtatatgc gttagtcctt tttcatacca ctataaagaa 130200
   ctacctgaga ctgggtaatt tatgaacaaa agaggtttaa ttgactcaca gttccacagg 130260
   ctgtacagga agcatggctg ggaggcctca ggacacttac aatcatggtg gaagttgaag 130320
   gggaagcagg cacatcttac catggtggag caggagagag agacagagag caaaggggta 130380
   agtgccacac actttcaaac aaccaaatct tgtgagagct cactatcaca agaacagcaa 130440
   gggggaagtc tgccccatga ttcagtcacc tcccgccagg cccctcccct gacacgtgga 130500
   gattacaatt caagatgaga tttggtggga acacagagca aaaccacatc cgtatataac 130560
   tagagtctca aagagatatt tgcacatcca tgttcacagc agcactattc acaatagcta 130620
   ggaaatagaa gcaatctaga tatccaacaa cagatgaata gataaacaaa atgtggcata 130680
   tacatacagt ggattattat tcagcctaaa aaaggaagga aattatgtca catgctgtaa 130740
   catggttgaa ccttgaggat atcatgttag gtgaaataag ccagtcacaa aaaaagatca 130800
   atactgtatg attccactta tatgaggtat ctgaagtagt taaattcata gaaacagaaa 130860
   gtagagtggt ggctgccaag agctgggaaa gggggagtgg gaagttgttg tttaatgggt 130920
   atagagtttt agttgtgcaa gataaaaagt tctggagatc tgtttaacaa gaatgtgaat 130980
   atacttaaca ctactaaact gtatacttta aaatggttaa gatggtaaat ttgacattgt 131040
   gtgtttttgt ttaattataa ttttaaatct gttagtttct ctaatgatca taatgaagat 131100
   taattaaatt gtgctcaaaa gatgtattat gggataggaa tacaggtaat tttattctct 131160
   ttattgttgc tggaccaagt tttctgcttc ctacatatca gaatggctag gcattgcaaa 131220
   ataatctatc accatagaat ctaaggattc ttttggaata taagcatcat tttcaaaagt 131280
   aatttgtgat tcaagagttg cttagaacca aatttcccta ggaaactgaa tgatttgggt 131340
   gttttgttat ggggtggagg gagaagatgt ggtggaagtt ggcatgatct ctatttgggt 131400
   ggggctcttt ttgtattttt gatagagaat agctgtatta atcagtagtg tattatagaa 131460
   ggcatatgtc ttatgtttca ttgcttggtt ttatgttgtg cagcagcaat ttgctaacca 131520
   cagtcctgaa gaaatatagc cacgcagcaa aagaagcatt tattttcaga aacacatttc 131580
   ttttctgaca ttacagggga gtgggaggga aggtccagat taactgtact ttttaataag 131640
   agatggagtg ctattggaaa actattaagg aaattgtggc tttagctcag attcaaggaa 131700
   gcccataaga agaatttact accttccagc ttaatactgc agcagggcca gttctacaga 131760
   cccactgctc cctgaatgat tctggagagt atttgcgagc tggggttgtt ctaggagagg 131820
   caatcagccg ctgatgagct cccctgctta actttcctaa ttgattccac cagacctcct 131880
   agtagactag ttgctaaccc tgccccatcc caactctggc ctggctcttt ctgatggcag 131940
   tcttcagaga ccatttgcca gtttggagtt aggccttgga gctgcagaac aggcttgtaa 132000
   agcagatgcg tagtaaatgc tgcagccaaa tgtacagcac attttcctaa gtggaaatcc 132060
   tgtgttacag acagaacatt acttaacagt aggtgggcag ttcaatccta aacagctcag 132120
   ttcttcccca tcagagaccc tgcagaagca ggcttctgcc acagctgtgg gaggaagaac 132180
   agttcttgta actgttagga gcagacagtg gctaagagaa acagtctctt gggaagtccc 132240
   tgtctgtgat tttcctacgt aaacacaaga aaacaaaacc cctgggcttg tcgttcacac 132300
   aagcaagccg cacaaagctt tctttccttc atctgcatgt gctagtgtgc tgttcttcct 132360
   ttaggtttta ctgctgctta aaggtgtttt tgtccagata tgtaaaggtg cctcgccctt 132420
   ctccttctca ggaaaaataa ctatttattg agtgcctgtt gccacgcagg aactctgtat 132480
   ccatcatctc acctcttcct catcaaacac atctcttccg agtgagttgc caaattacac 132540
   tgctggcttc ccaacctgag gttttgcttc tgtttacatt ttaccagagc aagagagaaa 132600
   aagtcacaaa tgatgtgctg tcttctcatc ttactaatcc cctcttcccc aagcctttcc 132660
   acatctagaa ttaaatacct gggccttggc agccaggcca acccctaaca ttggtaaagc 132720
   ccaggcaaga gcacaaatgg atgcccatat gcctaaagct gtaagagtta taatgcccct 132780
   ttaatgcatc ctcatgtcca cgttggatat gtgcattaaa attcatgaaa aagaattatt 132840
   ggccaggtgt gatggctcat gcctgtaatg ccagcacttt gggaggccga agtcagagga 132900
   tcacttaaag ccaggagttt gagaccagcc tgtgcaacat agtgagactc catctccaca 132960
   aaaaataaaa ctttttaaaa aaattagcca gttttggtgg caatacctgt ggttttagct 133020
   actcgggaga ctgagatggg aggattgctt gaggccagga gctacgattg caccactgca 133080
   ctccggcctg ggcaacagca agaatctatt tcaaaaaaaa aaataataat aacaatagct 133140
   taaattcgaa acaatctaaa tattcaacaa tggggcattg atgaatttta ttataatatt 133200
   tctccatata gtagggtatg agatcatcac ttaaaatttt tagtaataag ttattttgag 133260
   tttaagtgaa taaagattat taaagtgtct ttgtaattat tgttttgttt aaagtatatg 133320
   tacacataaa aacatgatcc aacaaaatag ttaccatctc cttgtagtga cttcataaat 133380
   tatattttct tctatgtatt agtattttct aaaaattggt ataatgaggg aaataaataa 133440
   atttataata aaaatatttg tatgctttaa taattctgtt tccaggaatc tcttttaagg 133500
   atcaaagatg tggttaaata tcacaagata atttatcaca atgttattca taattttaaa 133560
   attatatcag ctaggcacgg tggctcacac ctgtaatccc agcactttgg caggccgagg 133620
   tgggtggatc acttgaggtc aggagtttga gaccagcctg gccaatgtgg tgaaaacccg 133680
   tctctactaa aaatacaaac atcagccagg tgtggtggca cgtgcctgta atcgcagcta 133740
   ctcgggaagc tgaggcagga gaatcacttg aatccggtag gcagaggttg cagcgagccg 133800
   agatcgtttc actgtactcc agcctgggca actggagacc ccctctcaaa aaaaaaaaaa 133860
   aaaaaaaaaa aaggtagaaa caattcagaa agattattga acctttgaaa cttcctcatg 133920
   cttattctgt tcttaaaatt ttctcttttc ctcagagtga ttagcaatca aactcttttt 133980
   taaaaggctt acatgtactg gtctatgcgg gcagagagta cagggaggct cagagagtga 134040
   gcatgcaaac actaaaggca ggaccgcaga ggagaagata cccaaaggat aaaaaagatg 134100
   ctcactgagt caagtagatg aaaaccagca ttcttggtgt aagctctaag caagagggga 134160
   gaccatatac ggccatagcc agggagtgga aaaacccaga attttagaag gaaaggaacc 134220
   ttgtggtttc ttcagctcca ggaaatagca aacacacccc tatccccacc ccactaccat 134280
   ttacacctac cctaggatct tctattcaat tagaaaatct ttcatttatc gagcaaatat 134340
   ttgttgagtg atgtgccagg catagacagg agaggaaagg aggaagtctg tttgtttcaa 134400
   ttagccaagt tggtttattc atctcttcct tctttaaaca aatggctcct tccctccctc 134460
   cctccctcca gacaagtgtt ctgtgtgaaa acttgatctt ttcacatgac cctacatcct 134520
   cctccacagt gattggttcc cttcagtggt ggacaactta cccaagctga gcctagctcc 134580
   cttgaccatt gcaattgatc aaagggtggt catgtggccc aaaacaagcc aatcagactt 134640
   cttccctagc tttttttcaa aactttatgg gaaaggaggg ctccttctct ccagttagag 134700
   agccatgagc ccagatgtga gcctggaaac tgtaaagagc cagaaaagaa gcagagggga 134760
   aaaagacaat gagcaaaaca ctcagagacg caggagggag aaagagtcct gctggcattc 134820
   agaaccctgt gtcaggtctt tctgaatcac ccatttcctc tgtccttctg gctgtctggt 134880
   tacctgagtc attaccttcg cattcttctg aagctatttc ctattgtatt tctgccgtgt 134940
   gaaaccaaag ggtgttcccc agcacaaggg tttggaattg cagcgctctg tatgagctct 135000
   ggtaagcctc cttggcctag tgaagcagac aggtgccaga tcagaagctt tattaagata 135060
   atgagggagg aggccgggca ctgtggctca cacctgtaat cccaacactt tgggaggtcg 135120
   aggtgggtgg atcatttgag tcaggagttc gagaccagcc tggccaacat ggagaaaccc 135180
   cgtctctact aaaaatacaa aaatcagctg ggcatggagg cgtgcacctg tagtcccagc 135240
   tactagggag gctaaggcac cagaatcact tgaacccagg aggcagagat tgcagtgagc 135300
   ccagattgtg ccactgcact ccagcctggg tgacagagcg agactctgtc tcaaaaaaaa 135360
   aaaaaaaaaa aagaaaaaga gggagctaat gatgctctca ttggagggaa ctcattgtat 135420
   taccttttac ctattaatat aactgtgcct tcacttaaca ttaatgcaaa caatccttgt 135480
   tgaatacctg ccctgtattt ccctcaccag tttttggcct tttaaattga attcatgcat 135540
   acccgttctt ctttcctgct taatttgatt cagcctacta ggaaaggtaa aagggaggat 135600
   tccacttatg cccgtaactg tgaaaatgca acatatcttt agtggaaaat ttacaaacaa 135660
   ccacaagatg gggctgttat gcaaggaagt aaaagttaat ggttggcggg aagaggatgc 135720
   aacagtttcc agcatccagg ataggatatt taaatctttc ctcactagaa aataaggaaa 135780
   ggaaaatatt ttaagaacat atattcataa aatcaaagca ctacttataa caaattcagg 135840
   gattactctt tttaacaatc tagtgttcct ttttgggaaa gtgatacata attattatag 135900
   tagatatttt ccgcctatat caaggttgat tgcttgtctt tcttccgggt tttcttcatc 135960
   ctcattcttc tgtcgctttc gatgaaaagc cataacataa aaccaaaata catgcattaa 136020
   tttattaatt tatttacaga cttctatgtc ctctgtattc aatttcaaag acaaagaaat 136080
   agcatttgat atatttgatt aacatactcg tttcctggtg tcttccacct caatctaaat 136140
   taaatcactt tgacctcatt tttcccccct tgggtttgca gtttgaagat ctggaaaatt 136200
   cttgattaat agcaataatg taaggtagcc acgtttttag aaaatccaaa agaagcaaga 136260
   ggaaagtaac cctgcatagc cctttgcctt acgagagact acagcatttg ctggggcaag 136320
   atgaccacag aaggcagccc gcaggagaat gcccattcac cctggagctg tgcaaagcaa 136380
   gaagtctgca ggtagatgca ggggaagcca caaagaggaa gcattcctgt tccttctagg 136440
   gcacagtcca gtctgcgttg cgctaactca aaactgggag ccttctgttg agaaatggga 136500
   aacctaaaaa tagacctgca gaaagtactt tgaccttggc ttgaaagtca gcccagtgct 136560
   atgacaagaa aagccttttc catgaagatt atgttcttaa aagccatatt tcatctgcac 136620
   acagactccc agtaaaacca cactcagaaa atgtgcgtct tggctatcct tcaactgtaa 136680
   aatcattatg tttggctctg gttattctgg acattagatg tctatgtaag ggtttctttc 136740
   tgaaattcct tgtacaaaat gttaggggtt ataaactgga ttaatagcga catttaagca 136800
   aatctgctat tactgtgtgt atccaatacc ttatgatttt tggaaagaat tcaaggacaa 136860
   agcagaaaaa ctgttgtatt caagtaattc agaatgtaca tgctttagaa ttaactacaa 136920
   taaactaata acttttcctg aaaaaatcta gccagaaagc caagagaata tatttcatta 136980
   aagctttagt aattaaatcc aaaattaaag ggtactgtcc tataatttct tgcatgtgtt 137040
   agaattataa tatttaaaat ttgcaatatc aaagcatttc aggagaccat catatgtagt 137100
   ctgcatgtat ttcaaggata acccttaaag ggtgataaaa agctattaaa gaggggagaa 137160
   aaagcacttt tatatactta ggtaactttc taaggaacag ctgccaccaa ttaaacaaca 137220
   aatacctttt aaagactctt gggggttcaa tgttctttca gtggactgaa aaatctggga 137280
   aaatggactt cattagcaac tacaaaatta ttgaaaggat atttgaaatg gcatttaata 137340
   cctgcaaaga ctgaaacaat cagtagttta aaatttcgcc ataaagattg gtaaataaca 137400
   ctgattacag gtcttattca aacagaaaat aatgaatatg taatgaaaag agaataatga 137460
   tccaaattgc ttttaatctt ggagttatgc ctattgattg gcctggaaat aatagtgcta 137520
   atcttacagt aggaaagtca agtgctttag aaagtcatta gctttaggga aacgggatac 137580
   ccatttcatt gtgccagtac agtattttat tgagtaaagc ctcaaacaac tgtgtgttta 137640
   agaaaaattc caaggttagc ctaagtcaaa taacattcct ctccactctg ctctgtaact 137700
   ggtaaaagca atttaggtac tctttctaat tcatctacct aaacacctgc tatattcctg 137760
   caacacaaaa taatcaaata cagtaaataa ttcagcattc caaaaggtaa ttattgatct 137820
   cacactggct tatttctaat aagctgaaga agtaataacc ttctcaaaat agtaaattta 137880
   cattttagac tggcattcct gagacactct ggcaaagttt catgaacgaa tatatttata 137940
   agcaaatata cattaaaaac acacaaagtc tttttaaaag tcataatttc tcatctctag 138000
   gaaaagcctc aaaggattct gaatttgaaa tatctttact tcccattaac tttccatgta 138060
   tccaattaca agttaaattt acattctttt caatccattt ttgagacctt aaatgtattc 138120
   ttagatcctg agcttgaaaa ttgattgctt ttattttaat ttaaatgtta acacagaagc 138180
   atctggttaa tgaaaataga gatacatgaa aagccaggca cggtgactca ctcctgtaat 138240
   cccagcactt tgggaggcct gtaatcccag cactttggga gtccaaggca ggcggatcac 138300
   gaggtcagga gttcaagacc agctgaccaa catgttgaaa ccctgtctct agccagatgt 138360
   ggtggtgcgt gcctgtaatc ccagctactc aggaggctga ggcaggagaa ttgcttgaac 138420
   ctgggaagtg gaggttgcag tgatcactcc actgcactcc agtctgagcg acagagcaag 138480
   actccgtctt aaaaaaaaaa aaaaaagaga gagagagaaa tacgtgaaaa acccacgctc 138540
   ctcttagcaa atgtgcatgg aacaaggaac tggccggtgt taaaagttgc caggcagggc 138600
   agcagagagc acttcacaat gacatggtta gatgaggccc tcaagacaag ctggttgcca 138660
   gggaacactc agggcacctg agaagtgggc aagatcaatt tgttatgggg aagtcagctg 138720
   aatgcagagc tgaaaagaga aagtgcaagc cagagggaac aagaaaagac tgaagtcaga 138780
   gtggaaagag cttgggcttg gaagtcacga aaacctgact tctgaccctt atcaaatgtg 138840
   tcacctctgg gaaaggaaac acctttgtct ctccgacttg gtgtttttca cttgtcaact 138900
   gagtataatg atagcaccgc ctcgcagaat tattataggg ttataagatg atgcatatca 138960
   agcacttagc acactgccta tgagtacaca gaaagggctg agcaaaggat atgagtatta 139020
   ctactgagtg ctgctgccag agaggctggc aaagggaagg tgaatgccag tctcagagaa 139080
   ggatacaaat gggtacctca cagagtcaaa gtcagggagg caaaggaaac aaggcaaagg 139140
   gaactaggat ggtgtggaat ctcctcacta ggctttgcat tgaaccaagg gccttctggt 139200
   acacaaaatc ttgcacactg aaatatgccc cttggaatgt tcacctttcc tgaccctgtc 139260
   ttaaagatac cttgctctaa atccagtcat ttgtaacagc tgctctggaa agcaaatctg 139320
   gtgtcatttc agatctgaca agtgcctttg agtaagagca ggaaggagaa gctcattcca 139380
   ttgaaaggct tcctcgagta tgcctaggaa gacgcccact ggagatggtt tgttagcttg 139440
   tttgccaaaa caagtgagtc ttggaagttg cttttatttt tttaattcaa gtgaatggtc 139500
   cctttatttt ttaaaatgtg aggtaagtat ctctcccttg tttctggaga agatggtgaa 139560
   gtttccatgc atctgagtgc agacactgca gtctggaaga tgttgagtgt tctgcttcat 139620
   attgggaatt atggactttt tttttttttt tttttttttg agacggagtc tcgctctttc 139680
   acccaggctg gagtgcagcg gtacgatctc ggctcattgc aagagccgag gctccgcctc 139740
   ccttgttcac gccattctcc tgcctcagcc tccagagtag ctgggactac aggcgcccac 139800
   caccacgccc ggctaatttt ttatattctt agtaaagact aaataacacg gggtttcacc 139860
   gtgttagcca ggatggtctt gatctcctga cctcgtgatc tgcccgcctc gtcctcccaa 139920
   agtgctggga ttacaggcgt gagccaccgt gcccagccat tatggacatt cttaaccaca 139980
   ttgtgctctt gaagaattaa ataactttat tagccataaa atggtgtttg taggctgaac 140040
   tctcctctac ttactgccct ttaggactaa cagcccagac ttgggtaaag gggaatgcgc 140100
   tttggggtgt tttgctgatc actgtagctg cgcagtgtaa tgagaccatt ccctaggtga 140160
   gtgtgaggag gggatgaaca cgcctcagga tagagaagga gagaagctta tttgcttcaa 140220
   ggggactgat ggttgggcac aggactgagg cttgcggatg cctagaagtc ctgtgtgtgg 140280
   agtgtgagct gctcctggcc atggagaggc cttcgggggc tgggccagga ccctgaagtt 140340
   tgtttagggt cagctgtaag ccggaagagc ccccactggt ccctgcagtg tcaagcactc 140400
   aacttgattc cattggcact tagtaaagtt tttattaaat atcatgttac ttttcatttt 140460
   tggtaaatgg cagagccata taaattcacc aaaatgttca aactgaaaac ttccccgttc 140520
   cttcccattc ttccactgaa accatcaaca agtactatca attctgcctc caaagagcct 140580
   taagctcaag ctcgtgcttt gtttgcctct cctgtttccc accctgattc tgattctgtt 140640
   gttttccacc tgggtgacaa caggatcctc ttcctctcct ggctttctct tttcatcttc 140700
   catcccattc tttaccagca acctgagaat cccctttaca atataaatga agtcaagtcg 140760
   ttctcctgct taaacatttc agtttcccag tattctcagg ataaagatcc aaatgcttag 140820
   catggcttgc aaggtcaagt ctgggcttgg tgagctttgg agaactcagt gagagccctg 140880
   gcctctggaa aaagctgcct ggaaaacaca attatagtca gtgcaaagtg atcactcaaa 140940
   gtgtgggctg cagggtaggt gacaagagac cataacagat ttcttgagat tccttctaga 141000
   agggaaccca gtagaagaac aggctcctca ttcctgttgg tgagaggttg tgacagggca 141060
   gtaaaggaaa catgactaga ggtgtaggca ctggttgata agatttaggg atatatcagt 141120
   gacacaccca acctagggta ctgtaactgt aaatcaaaac aatcttcttt tagtcttaaa 141180
   aacaagaacc aaaaattgta caatttaaca tgtaataatg gcttctattt atcaagcatc 141240
   tgcaaagggg ctggctttat gccaggcatt ttacatattt gattacaaaa accttataag 141300
   ataattatta tctccatttt tgatatatgg gaaccaaggc tcaaaaggtt actaaaagtc 141360
   aaatgaataa taaggcacaa actttaaaaa aagttaccaa attgcatttc tttatgaagg 141420
   aaagggacag atctaatgtt aatttgttat taactgagga agcacagaaa gttgggcgtt 141480
   tacttgtttc ttatccatct cccccgacta ggaggagaga gcttcatgag gggagagcct 141540
   gttttgttca cagctgtctc ccgagcacac tgaatagtga ctggtcgcag cagacctgta 141600
   ggggtttgtt cacagactga agcaatgaag tttgacagct gctttcatta ctcagtgatt 141660
   ttgattcaga gctttacaaa agtaggaaaa attagtgtga atatccattt ttccatagat 141720
   taggtgttgg attttttttt ttcttattct ctccactacg tactgctatt aaaatacaac 141780
   taagccttat tcaccgggag gtttccatta gcaacaagaa ttaattaaat tatgagtcat 141840
   cagcagatga catactaagg cagatcatcc cccgacgatg attgtttttt atttttctaa 141900
   ttataaaagt aatatagtaa agaacactag caagatttac taattttaaa aagtatgtac 141960
   agttatgaaa atatttctaa gatatatttt tagtgaaaag aataagatgt agaatagtgt 142020
   tcattgtaag cttttctccc atgggataat aataatagtt attgagagct tactgggtgc 142080
   caggcattat attatatcca ctgcatgaat tatctcattt aatccttcaa taacaccatg 142140
   ttgttggcac tataacatta ttagtactat actccatttg aaatagtact attgctctca 142200
   tattattttt cttgttttta ttttgaaaca atccagcact tagagaaatg ttgcaacatg 142260
   caaaatacac atacacacac acaagcatat acatgtgtac attttacatc tgtatttact 142320
   tctatatacc catatattaa aagccatgag gttgtactaa tatctacaat tccattccta 142380
   ggccacagag ttgtagtttt ctctcttttt tttttttttt tattttttga gaaacagtct 142440
   cactctgttg cccaggctgg agtgcagtgg tgtgatctca gctcattaca acctctgcct 142500
   cccaagttca agtgattctc ctgcctcagc ctcccacata gctgggatta caagcacaca 142560
   ccaccacacc tggctaattt ttgtattttt agtaaagatg gggtttcacc attttggcca 142620
   ggctggtctc gaactcctga cctcaggtga tccacccacc ttggcctcct aaagtgtttg 142680
   gattacaggc atgagccacc ctgcccagct ggctctcttt ctatatgtat atttattctc 142740
   ccaaataatg tgaaggctgg ctccaattgt cttcaattta tttattcatc gaatcactct 142800
   cctgttgcct ccactgtccc tatccctggc aggaatgttc ctcctcccac tggtctccag 142860
   cactctgcat cggaccctct caccccctac aacctccaag accttgtgcc ccatcatgct 142920
   ctgacacctc caccaacaca aaagtccttc tttctctgtg tcaggacctc cctgagcccc 142980
   ctaagttgtg acaccccacc tacccatccc tgacaaagac agctcccttg cccatcaggg 143040
   atagccatga ggaggtggct ccatgggttc tagtattttg agactgcatc aaacttaata 143100
   tgttagcatc tttggtagcc cattcttaac ctggaccagc agttagacct aaagcataga 143160
   tggccattta gctagtcatt tgcatgagaa ttaaacatga gagatgaaag aaaagaaaaa 143220
   ggaaaaaaaa aaattgtacc aggaaggaag atatttcatg agctgaatcc caagcagcaa 143280
   agtcaccact aatgtcacaa cggtgttgcc agctaataat ggggctgctt cttaactgtc 143340
   aggaaatgca aacagcacaa cattaaaatg gttctatttt tttccatcct tacaatagca 143400
   acacagcaat ggagtgctga tctctgaaag acaaaggctg tgaaaataaa catgcactta 143460
   agccaggggg cacttgggtt gtgatttttc tttcaagata tttggtaaca tatcccaaat 143520
   cctcaaacca ccccagggat cttcccttca atacggtctg aatcacctac ctgtctcaaa 143580
   tggcccagca aagctcttgc tctgtgcagg caggcctgcc agatagctgc ctttttcttt 143640
   tcctcttacc ttggctaagt ggcttccaag gtgtttcgga aaggaaaatg taggattcaa 143700
   aataaagagc agagagttgg gccaggcctg cattgtcctc taactgtgat aatgggtagc 143760
   tgctcggccc ctggttcatc ttccacagca aaaactggac caaggcctgc tttttctctc 143820
   taatggtggg attgcctctg gcttcgagtt gcacgtttat ctttggtact catcactttg 143880
   attttgttat tacttcaccc tcccctttgt cttcactttt atctgtctgt atatttccta 143940
   acaacctgtt tttacctcat ctccctcttt tattattacc atttttattc ccctgccctg 144000
   catccttggc ttcccctgta agcactttga aaaatgaggt gaattttata ttggtaaaag 144060
   atgctgaaag gtgaatctgt gaagcccccg cccccgtagc ctccttccat atgagtacct 144120
   tctttccatg taaggtaaca gctctctctc ttcttttttt tttttttttt ttttttttgg 144180
   tgctcattct ccccaggcaa ccagaaaagc tgtccgcttc aaggaatggc ttgatctaag 144240
   atccaatcat gggatacaac taaagaacaa aacctcctgt taccaggttg caaatgtcca 144300
   ggtatttgca agcagtgttt tgctttctta aataccagac tatggctagc aatcttcccc 144360
   aacttcaggt tatagaaatt cagagctccc tatctcaaga ccaggaaaat ccagagcaga 144420
   cacatgtttt gtgaatatga ccaggaataa gttatttcat cttgggaaaa aaataagacc 144480
   aagatctagg gcgtgcttga tgtttcccag ttgttcttcc agtccaccct attcccttca 144540
   tcactcttct ctgtgcccca agtaggaggc tgacctattg ggctgtataa acaggctctt 144600
   ttgcactata gcttcttatt ggattcaccc catggggagc attggagatc agaaagaggg 144660
   tagaatagtg agagcagaag gccagggtat ttattcacca gctccctctc cttgggccac 144720
   tacaggctgg ctgaatatcc ctgctcctgt caggtgaccc tctctctcca gcaccaacat 144780
   cattctgtcc tttgtcccat caggccttct actagcccag ggtactgcac tactccttgt 144840
   gatttcccta taacctgcta atacctttat aaataatctc cttaataaac tctactcaaa 144900
   ttacccaatt tgagtggacc atttgtttcc tgctgctggt ttgatgctta caaaagatct 144960
   ctgcttaggc aaggcagaaa caggtgcatt tgagaaatgg caatggcaaa tgatcgggaa 145020
   cccagttacc tggaaactga ggcccatggt aggaattcaa gcaactcttg gcacagagaa 145080
   acaaaggaga aacttcttta ctgaggatta gcaatacaga gaagtgaatg cagttactga 145140
   caactggagg tactatgctc agatttaata ataacacatt taccatgcac ttatgataag 145200
   ccaagtacct aataaatacc cggtcatatt ttagaatttt ttatccactt tactgatgag 145260
   gaaactgggt gaattgatca ttgctgtttt gtagacccaa attccctcct tcagcatctt 145320
   ctagtaataa aactactctc tcctgcaagg agctcatttc acaggtttta actagtgcta 145380
   acccaggcct tgcctgtcag aacacacctc ctcccatgca caatgcttgg ctcagtaatg 145440
   gacatgtgac tctaacaggc caataaattt ctccgagatt tgatacttgg acctagggaa 145500
   agactagaag gatgatgata gcagccacca ttgcttcagg ccacctttcc ctctgtttgg 145560
   aggaagccat ctccagcaag aaatcattta gcatcagcaa agagaacagg agagagagaa 145620
   aagggataga tggaggaaga gcaacagtga gcacaaaaga gatgacctca tttgctactg 145680
   cagccagtct cttgagtgag ttttatcctt cagttttcca gtattatgag acaatgaata 145740
   ctttattttt acttaatata agttggatct ctcatttata tttccaaaaa accctgtaag 145800
   tagattaagg ctgccttgca gagattcttc agctgggcag tgatcagccc atgcgtgtga 145860
   gaaaactacc aaaagcagga aaaagagcca ctcagaagga gcaggtagag caatctccag 145920
   agcttacaga gatccagagg caggagaatc acttgaaccc aggagaatca cttgaaccca 145980
   ggagacagag gttgcagtga gccgagatcg cgccattgca ctctagcctg ggggacaaga 146040
   ccaagactct gtctcaaaaa aaaaaaaatt tgaaaggcac aaaatgaaga gaaaaacata 146100
   ttatcttttc taccattctt acattaatac ttattttgaa aagttttaat aacttttgac 146160
   atttctccct ctctctctcc ttccctcctt ccctttttat cctaatccct ctagtgcaat 146220
   ctccatctct tttgatgttt ttagtgtact tctcttattc tctctcacac tcactgattc 146280
   tttttgggta agtgtgtgtg tgatttgaat agcatcccaa aataaaaata gatgtaaaat 146340
   ttttcttaac ttttattcca tttacttttt agactttttt gttaaaaact aagacaaaaa 146400
   catgcaggcc tacatagagt caggattatc aagatgtcag taggcaatag aaatttgtca 146460
   gctttattaa aattttatgg aactactgtc atatatgtaa ctcatcattg gccaaaacgt 146520
   tattatgtgg tgaatgacta tagcagtcat ggttattaaa acaggtttat aactatagtc 146580
   tatatgttta agaaagtaga ggaaaccata agtgtgttaa gaaaagacat agatgatgac 146640
   ataaaagaga cccaaatagg ctgggtgagg tgtctcacac ttgtaatccc agcactttgg 146700
   gaagccaagg cgagtggatc acttgaggtc aggagttcag gaccagcctg gtcaacatag 146760
   tgaaaccctg tctctactaa aatacaaaaa ttagctgggt gtggtggcag gcacctgtaa 146820
   tcccagctac ttgggaggct gaggcagaag aatcgcttga gcccaggatg tggaggttgc 146880
   ggtgagctaa gatcacacca ttgcactcca gcctgggcaa cagagtgaga ctccatctca 146940
   acaacaataa caacaacaac aacaacaaca aaaagaccca aatcaaactt ctagagttga 147000
   aaaatacaat atgtgagatg aaaaataaac tagatgggat tgaaagtaga ttagacacta 147060
   caaatgagga catgtcaata gaaactactc aaaatgaaac acacagataa aaaatgactg 147120
   aaaaagatca acagaatttc agtgagcagt gggacaacct cgtgcagcct actctgaatg 147180
   taaatgtggt ctccaagatg agagagaaga gaggacagga acaaagtttg aagaaatatg 147240
   gctgaaattt ccccagattt gttgaagaac ataaacatat agatccaaga aactcaataa 147300
   actttattta tttttatttt attttatttt atttttgcga cagagtcttg ctctgttacc 147360
   caggcagtgc agcggtgcaa tctcggctca ctgcaacctc cacctcctgg gttcaagtga 147420
   ttctcctgcc taggcttccc tgagtagctg ggattacagg catgcgccac cacacccagc 147480
   taatttttgt atttttagta gagatgggat ttcaccatgt tgtccaagct gatatcgaac 147540
   tcctaatctc aagtgatcca gccacctggg cctctcaaaa tgctgggatt acaggcatga 147600
   gccgctgtgc ctggcctcaa taaatgttaa tgaaaagaaa gatgaagaca cacatcaagg 147660
   cacatcataa tcaaatcagg gagcccatct aagtgggcaa catcccccac agtcatttct 147720
   gaccttcgaa gtagagtaat cacagagcat ttcaaggatt cagacgtgcc ctgcactaaa 147780
   gtatttctga atatcttagt gaagggaaag tcttcgaggt ttctggtgga gcattaggtg 147840
   ggaggatggg caccaggttg cacatgataa atccagttca accttatgtc ctaagcctgt 147900
   ggattccctt ctccatagca tgccaaggaa gctctggcat ggcatcccca ttaaatgtag 147960
   actaccattg agtccaagtt ttagtcaatc caccaagaaa gatgttaaaa ccacctccag 148020
   ttgcatgagc taatacatta aatccagaat ctctactaag tacacccata tccatgattt 148080
   aacccaatct aatattgtat cttcttatga aaaagaccct tagaacatac tctcacacat 148140
   gttccccagg tttctgttgc tatatattta ttagcaaagt cttgaaatgt ttggggcatg 148200
   caagttaatt tttcccaggt cattgtgtgt acttatccac ctacaacata ctgagatttg 148260
   gacctgtcca aacgtttagt ggtaagaggg ggtgattgta gtgcatctta aggaaaatga 148320
   acatcccctt tcaaagcatc ttccccagat gaaggttttc ctagcaatga tctaaatgcc 148380
   tcatcacaca cccaatctct cagaaatggc tgggctattt caaagctgga atagttcact 148440
   gaagactctg ttatggtgcc aactgggaga caacggctta aaaggatgat attctgtctt 148500
   aacaggatgt ggtacatgct ttgaattaga gaccatggtg gcatcttccc cataatcaaa 148560
   atatatgtgt ctgggaatta aggcatggaa gtgaaggagc aatctgtcct caaaagtaaa 148620
   ttcaaaattt tacctctata aacagaagaa tttgctaaca atttaaaaat agctatttcc 148680
   agtttctagt cccactttct ccttattact accaggcaac aagtttccaa aaaaatatta 148740
   acatccaatt ttagctacct aaaaatgtga agacctttac agatgttcaa tggtatatta 148800
   taaaatggta aacttataat aaagaaatga gcaaaggttc ccaaaatgag cagtagaatg 148860
   actggcagta aaataaaaat aaaaaaaaat aggaggtcca aacacaaaat attatgtagt 148920
   gtcctaacta tattttactg attttttgat aagctcccta gttcatcctc ttatttgatc 148980
   taataatcag acaagcagtt ccctgatgtt acaggcaaat gaattaccct cagagtttcc 149040
   aaagaaacgt ttgagaaaac aatagggaag aactgcagca atctgctact gcagtgatgt 149100
   gaagacacac tgttgaggtt aaaagtcaaa ggtcaagttg gtgttattct cttggtcctt 149160
   ctgcagttga ttaaacaata ttacttgaga tgaaagcaat cagtgaaaag aattatgatt 149220
   actttattag cattactgaa taaatgtctt ttcagaatcc aagttatcac tcaaaaacat 149280
   gtcatgttgc cctaaaatta cttattcaat ttctggccag agaggatttc tttataagaa 149340
   agactttcct attaaacttc tcattgaagt ttaatcaata tgcttcatcg atatgtgtcc 149400
   cctaaagcaa tcctaggatt tagaaaagga tctaagtatg tgggcttcta tgagtaatat 149460
   ctgagtctga gcttactcaa aaggttggtt ttcaggctgt ttcttctcta attctagaaa 149520
   gctctagaag gccatagtaa atcctcacag aatacaacta aagcataaaa ataactttca 149580
   aagatttata attagccgga ataattttct ttgggatcta attcttattt acaagtcatt 149640
   ttagagaccc caaattaggc aacaacattg caacatgact aacactgact tcagattgga 149700
   aaaaagccat acatttattc aatagggagt aaacaagttc tagggaaata tgtgagagaa 149760
   taaattaata atatcattta gcaagaaaaa agagctcaat aaatattata gcctagctta 149820
   aaaaagaaaa aaaagtatgt ttaggcaata gttgtatctc ctcactaaag gtatggtaag 149880
   ctaagatata gccaaagcta tagtaaacca agaaaagctc ttctctccag tggagagagg 149940
   caatgttgcc tctacaatca tgtggtcaca gtgtgtatgg tagagtggca aggcatcttc 150000
   tgttcccctt agtccaaagt atgcaccttg gttgataagc agctagaagg agaaaaccaa 150060
   atagatttag atctgaactt ttaaaaacag gtttatgcaa acaggcattc ccacattgag 150120
   ctgggactat atactggtaa aaccaatctt gaagacaatt tagcaatata tatcaagggt 150180
   cttatgagtg tatatgtcct ttgaatagga tatttcactt tagcttaaga aaataatcag 150240
   atttttttac aacaatggac ttcatagaaa aaaaaacaca ggaaatcatt taaatgacca 150300
   aaaaaactat tatagctttt tttcaaaacc attaatgctc agtataagta ggaaaaaatc 150360
   cagattacaa aaaattatgc ccaccatttt ctattttgta agtctgtgta tgtgtgtgta 150420
   tgtgcctgtg aaaaaaatgc agaaagactt agaataattt ttctggggtc tttctttttg 150480
   tattttcaat ttttttctat ttttttttaa aaattatgta ttaaagggat cgttagagtg 150540
   gtcaactacc ttaaagagta ttatgaattc atggtttaaa aaggtgattg aaaataaggc 150600
   accagtgtag ggctgttcac atagcatgtt tttctaaaat aacctcttcc atcgtctgta 150660
   gtagctacct gaatttcagt gggcctagct ctaaaaaagt tgagaaattg taccaatatg 150720
   tgttgcatca tcccttaatt atttgtgcca gttgatggaa gctgaaatgt ggaaaacatc 150780
   aaccacataa ttcaaaatta atttgtactg agacttggaa tatgaccaga cacatgtgct 150840
   tagacatgaa tgtatttctc ctcttccctc actaaccttc aggccttgtc ctcaccactt 150900
   cttggtctgg gtccaactca cagcctgggg ccagcatggc agcactggtg aagtccctag 150960
   gaataaaaaa aatgtttgag aaaagagtgg tgacaatgac tgggctctga attgtgaaca 151020
   tttacacgtg taaatgcaga aacacccata tacgatcatc cacatattaa ttttccacct 151080
   agactcatta cttttccata actctgatga aaaggggagt tttatttgtg tagaatcttt 151140
   ccgatttggt gatagtggtg actggaataa tagtaagaaa agatatcttg gcaggactgg 151200
   ctacatcatt taagggaccc aatgcaaaat gaaaatgcag gctccttgtt ctaaaatgat 151260
   taagaatttt aatactgaga cagcagagca tgaaaccaag ctcagagccc ttctaaattc 151320
   aggtctctgt gtgactgtac tggtcacggg cccatgaagc cagtcctgag ttttagagtc 151380
   aggtagatat gggatcaaat tctcgatttt ttcatttata agctgagtat cttggctaaa 151440
   ttatttaaac tttcaaagtt tcagttatgc aatcttaaaa tgggagtgat gtgagtgtgg 151500
   cattattctc aagattgctg tgaggattaa aagagagagc attctattag gtgcatacca 151560
   tagtgcctgg ctcagggaga ctgttcatct tctctatgtt gtatactgca gttagagttt 151620
   gatatagttt gaatatttgt cccctccaaa tctcatactg aaatttgatc cccaatgttg 151680
   gaggtggggc ctagagagag gtgttggggt catgaggatg aatccctcac aaatggcttg 151740
   gtgccattct tgcgggattg agtgagttct cactcttagt tcctgttaga tctgttaaag 151800
   agtctgacac ctccctcccc acccctcttc ctctctctct cattctctct ctctgttctt 151860
   tccctttacc atgtgatgcc tgctctcctc tgccttccac catgagtgga agctccatga 151920
   agccctcacc agaagcagac gctggtgtca tggtcataca gcctgcagag ccatgagcca 151980
   gataaacctc ttacttttac aaattaccca gactcaggta ttcttttata gcaacatgaa 152040
   acagtctaag aaagagttca tattctaaag tgcaaacttg aaagtttgat atggtttggc 152100
   tctgtgtccc catccaaatc tcatgttgaa ttgtgaccct atgtgttgga ggtagggcct 152160
   ggtgggaggt gattggatca taggggtggt ttctgatggt ttggcaccat ccccctagta 152220
   ctgtctcatg atagagttct caaaagatct ggttgtttga aagtgtgtag cacttccccc 152280
   tttgctctct ctccatctcc tgctccgcca tgatgaatat atgcttggtt ctgcttcatc 152340
   ttccgccata attttaagtt tcctgaggcc ccgcagccat gctgcctata cagcctgcag 152400
   aactatgagt atattaaatc tcttttcttc ataaattagc cagtctcagg tagttcttta 152460
   taacagtgta agaacagact taatagaggg tttctccctg atttaagatt atttttggtt 152520
   atccattatc tacgaaacaa aatgaaaatg cacattatga catataagga ccttaactat 152580
   tcaggtttta tcaatctctc taaaacttgc cacctaccac tctcacatat gttcaaagta 152640
   taatttgtat ttattttcac ttttccttaa gaaccagtaa atataatagg catagatgac 152700
   aaataataaa ttagcatgtg ccaggcactg tgctaatcat tctgtgggag ggaatactca 152760
   ttctccactc ctgctgtttc ctcccatgag agagagctgc atgagctgta acattcctgg 152820
   atacttccac ctcctcattt atcctcccct ggcaaggata agttgagaga atctgggtat 152880
   ttgtattcca gcctctttct cttttggtag caactctcac agggagcatg attttaggca 152940
   agctgtaagc atagtggcaa agaccttttc ccggtggaac gacccattgt ccaggactac 153000
   ctgataccag ttggtgagcc tgtctatttc tggtattcag cgtcaggaag cctaagttct 153060
   ccagtgtcag ctttatcaat gataatggtc tcctgcctgc gttactcttg tctgtttcta 153120
   aattggttca gaacttgggt ggggaaaatg ggtattattt actgaggata cgtaagtatc 153180
   ccaacaaagt ctacattcat tgtctccttt atacctcaga gctaccatat gaggtaagca 153240
   tatctatcat ccaattttta tagataagga aagtaaggat tagcaatcaa gtaagtggca 153300
   taatcaagat ctaaacagtt ctatctgatg ccacagatta tgcccttaca ttttcccatt 153360
   ttgtttttgc atgaaggttt aataataaac tgataagaac attcaaaatt tgagttcatg 153420
   ttgaaaaaaa aaaagctaac acagtattat atgcagtgta cttatattct atctggcaga 153480
   aaagttagcc aagctaaact taaattcagc acatgctgaa ttttatttta gggagaacaa 153540
   gctgcaatca taataaagaa ctgtatttac agctaacagc aatacccacg tttctctata 153600
   ggtcttacac tacaatctga aattgcattt catgatttac aaggtctgag acataaacag 153660
   taaaaggtat gtttctggga attttctcat taacagccac aaaacaagca attttatgtg 153720
   gaattgcttt gttcaccttt tctgaaatat gtgcaagatc tctctttgat gttgaatcca 153780
   ttctgatttg aacatctcaa tatttttgat gttccatgaa ctcctccaca ccccacctat 153840
   ctccgggaag ctcagacctc cctacctact tggtgaaaag aaggaaattt agaacatttg 153900
   agctcgcagg ctgcctactt aagactaccc gaagaatgag ctctgcttac ccctgttcct 153960
   tcgtaggtac atgaatgagt cgtcaaaaat tcactaatag ttgattaatg cagttgtgac 154020
   agtatgaaaa acaacaaaag gagtgcagtg ctgaagaaat tctagatctt gcagatttaa 154080
   tgcatccact tatatccata actcaatccc ttttcatatg acactagtta ttgttgttac 154140
   tgtcttccat aaccattcct accacgactc cagtccaggc cataccccct tcttcttgtc 154200
   acaagaacat caacactctg cagtcttttc ttctttatca tgtcttcatc cctccatact 154260
   ttgctcacca agatgtgttt ttccatctgt catatttgtc taatcctgtt gatttaatgc 154320
   actatatttc tactaagaaa acagtacata caaaatactc ccaagcatct aactactttt 154380
   aaaaatatag atctttctaa attacccata tgtattgcct gacctggcat ctctctctat 154440
   tttcatacag tgctcagcac tcctgattaa ttgtctcttt aatattttat ccaaaattgc 154500
   actgggaaac caagaaaaca ggacaaagga atgtcatttg tactttttgt aatcttcatt 154560
   atttaccaaa tgtatttttc ttataaccac ctttgtagta cttcaagata tgcctaacag 154620
   gcactgttag ctcttttaca ggcctgtgga taatggaaag tatggagaat cctggcttgg 154680
   aaaggcaata aataaggtta ttttgacact actctctatt ggaggaattg tgattgtttt 154740
   tgattttgtt tctgttttga gagaagaagc ttgcctaatg gcaatccata agacattctg 154800
   gagaacagtt atagattcaa aatgtgagtt ttagtctact tgaatgtagc accttgttaa 154860
   caaaaaggct tcacttttat tgcctcactg ataattcaca taacctgctg acatagtgct 154920
   atggtctgaa tgtgtgtata cccccaaaat ttgtatactg aaacttagtt ctcaaggtga 154980
   tggtgttaag aggtggggcc tttgggaggt gattaaatca tgagggctct gccctcaaga 155040
   atgatgttag gtaccctctt aaaaggcctt gagtaagtga agtttggctc tcttgttctt 155100
   ccacctccca ccccgtgata ttgcagattt cacaacaaca aggcaccatc ttgggagcag 155160
   agagcagctc tccaaacatt aatgctagca ccttgatctt gggcttctca gcttccataa 155220
   ttgtaagaaa ccaatttctt ttctttataa gttacccagc ctaaggcatt ttgttatggc 155280
   agcacaaaca gactaagaca tatagagttc atggtggcca ctttttaaaa aaatgtacaa 155340
   agaacttgag gcactgagag gcaaatgact ggctcaagaa catgcaaagc atgatcaagt 155400
   aattttgagg tatacatttt taattcaaac tcccttggta atctgatggt gaaaagaatt 155460
   ttcaattttc aaatttccta gactacatgc aggagagaac tcaggagcat ggcaccaact 155520
   tattttgtct tatgtagaag gatatctgac ttgaggttga gaattccaca tcagaatcta 155580
   taggcctttt cccctgccca gtagagttta gttgcctacc ttcaatctct gccaacagca 155640
   atcctctacc cctctggttt aagcaggctg ttctaaatag ttgccggtgg ttaccgtggt 155700
   aacagacgct taggtttcag cttcgaatgc cccttttaat gaggcaattt caagaatatc 155760
   tgtgataaat taccagacca cagacttcac tgcagggagt tttgctttgg aaaatctaat 155820
   aaaaagacag cagaaagaat gagcagtaaa tgcaacagag aaaatggggc cagatttgga 155880
   ggggcaaatg gaagagatgc cagatctgac taccaaaagc caaatcagaa tcagttcaaa 155940
   ccagagcttt aaattataaa cggtccctga agggcttcct gacatgctga gccaagattc 156000
   cttgggagga ggaatggtaa agtgagaggt ggtgagggca agagttgagt atgtcctgca 156060
   gagggctgtg tattttaaag cttccatttt ctattttatc attaattgta gtggctaaat 156120
   cagaaacggt ctccttttgt ttctccattt ctccatgtat gagaccaaga gacattgcca 156180
   cctggagagg acataatgaa ctaaaatgtg gactttcgcc taagtgaatt tcacagcaac 156240
   ctaggttatc cacatttccc aaagacatac ctgacttgat aatggtccag gattctgtta 156300
   ttaatctctt atccattgta cccattaaaa atttcacaga agggtaaacg tacttaaaca 156360
   cattgctaca ctattgtgtg gaaagaaaca tctcctaatt acccaaagga agacatgtca 156420
   ttcatccttg ttatgattga atataaaaac tatgcctcca ctatataaaa tagcttcagt 156480
   ttatacttgg ttgaaggcaa gagggtagcc taaaagagcc ttcacttaca ctgtaagtgc 156540
   cttgccaggc tccctaggtc ccttcaggaa catcttactc tcgctgctca cggaaagcac 156600
   cagggaacag atgcattaca gtttgtgaca ggagaacagc ctgtggattc cgcctactcc 156660
   ctccagtcca tctgggaggt gggtgagaca gggagcccac agagaggggg gctctcctgg 156720
   gaatccctca gggtaccatg gctattggtg ctttattttc agtagatggc actgtgtcta 156780
   ttgagataag caaagggaga ttctagatgg gaaatttaat tgtttctaga gaaagcacaa 156840
   ggcatgtgag aatgcacggt attttatttt tgccttagtt acatgataac gcaaatacta 156900
   ttcagtattc aggttacatt ctctttggag cccatggaat tttatgattt attattctgg 156960
   ctccatctta ggcctgtggt gaagcccagg gccatcctat acagtatata cttggaataa 157020
   gaataaatca tgctgaatgt tcacctctgt tacccaacac atacagcagc acctatactc 157080
   catctgtgcc ctggatcccc acctgtgcac tggctctctc ccttttcccc agacaggaaa 157140
   ctctgcctgg aacccaggca gttcctgcct ggaacccagg agtttttaca ccccaagaat 157200
   gagacaacca gaacagtttc ttgcacactg gtggtcccat ggagcagaca ttctcctttg 157260
   tccgtccatc ctttttcttt tctaatagac atccagttat ttttttcctc tcttctgagt 157320
   atttccagga gagtaaactc tagcaccaga agatgaatca attttttttt aattatgctt 157380
   taagtctggg atacatgtgc agaacgtgca ggtttgttac ataggtatac acgtgccatg 157440
   gtggtttgct gcactcatca acccatcatc tacattaggt atttctccta atgctatccc 157500
   tcccctagcc ccccaccccc aacaggccct ggtatgtgat gttcccctcc ctgtgtccct 157560
   gtgttctcat gaatcaattt tattttaagc catatgtagt cttttaaatg ggaggagaga 157620
   acctcaagta tgtttcatag aaaatgaact gtagacacag gatggaagat ggaggacata 157680
   tgggccaaac actaggtgat gcacttaata taaaactctt ttattcctta tgataatctt 157740
   gagatagata ttttgctacc tgtcttacag atgagcaaat ggaagttcag taagtttaaa 157800
   taatctacct aaaatcatcc tcaatattga ctgggcatat aatgagattc taaacttttg 157860
   agtagtttgc tagaatactt ctggaaaggg acttgcatgg agaaacttac ttctgtccat 157920
   tgacattatc ttctgaatat gacccctgga actgcagcag tcatcttgag acaattatgg 157980
   aaggacacct aagagaacaa gataaaagtc caaagacaac agagcagaat gatagaaaga 158040
   acctggaacc ttgatgatac cttttgagct gctgatttaa tcaaccttca aacttcagat 158100
   gtatgtttta gccactttga gatatgtctt ctattactca tagcctgaaa taccctaaac 158160
   aatactacct aaatactcta gccatagatg actagaaaat gtattagtgc ctgagccata 158220
   ggcagccatc cataggatga ctgctgagga ggtaggatat gaaagttcac tcatccaggc 158280
   atccaaattg gataatatct aatgaaacca atcagatcct ttctaggggc atctgaataa 158340
   gacagcattt tccctgtctc ctcaggaacc ttgctccatc aattaaccca tctctcctgt 158400
   gtcttcaatg ttttttcctt aaactaacac tttttttttc ttttcttgag acagagtctc 158460
   actttgtcac ccaggctgga gtgcaatggt gcagtcatgg ctcactgcag cctcgacctc 158520
   ctcttgggct cacatgatcc tcccacctta atctcctaaa tagctggaac tacaggcatg 158580
   taccaccatg cctggctaag tttttgactt ttttttgtac agatgagttc tcactgtgtt 158640
   tcctaggctg gtcttgaact cctggcctca agctttcctc ctgcctcagc ctctcaaagt 158700
   gctgggatta taagcatgag gcaccaccta actaacccct tcttaaatac cgtttaagtt 158760
   tttcctatta aaaacaaaca aaacatcctt tgtcaaatac agaacttctt ccaataccac 158820
   tcaaaatagc tcctctcttt cacagtccac ttcttaaaat agatgtctcc agtttctatc 158880
   cttcaagtca ctcttcaacc cattgcaatt tacacctgca gttccccaac aggacagctg 158940
   ctcttatgga ctttacaaaa tagtaagaaa ataggttgtc aattgctgag taacacacta 159000
   cctcaaaact tagtcattta aaacaacaga caccactgat tatctgccat gattctgtgg 159060
   gttgacaagg ctcagttaga caattcctct gttctgcaca gaactgcagt cacctggagg 159120
   cttgactgag ctggaacatc cactatggct cattgacatg gctggcagat ggtgtttgct 159180
   tttggttggg aactcagcta gaagttagtc cttctcctcc tggcctctct acatgtgggc 159240
   ttctctcaac atgatggctg aatttcaaga ggaacaattt caaccttgtg aaaatagaag 159300
   ctgctcagcc tcagaaatta aagaacctca cttctgccac attggtcaat acaagtccaa 159360
   tccagcccag attcaacaga aagcaaatag acttcacttc ttgatagaga gaacagcaaa 159420
   taatttacag ctatctttaa tgtaccacag ttattaatga atttctttat tgttaaatcc 159480
   aatgaaaaca ttgtagtcct tattttctgt ggcaccttga ttctggaaat actttcttcc 159540
   ttcaactttc atgacaacac actctcctgc ttgtccaggc acttctaggg cttctccttc 159600
   tctttttggg gaagaatctt ttcctcaaag tcaaatatta ctgttccttg gaattccatc 159660
   cttcttcttc ttctattatc actatattca ctcttactgg gcattctaat aaacttacta 159720
   ggcttccatt actatctaca tactaatcac tcaaatctat actatactca cagttcatat 159780
   tgctcacttt agtttcaaat ctagatatcg cactctttat tgatcatctc tagtctccat 159840
   ttgaatatcc aacaagtatc acaaattcaa aaggtctaaa actgagcttg ttgcattttc 159900
   tcccaaagct cctatttcaa tgttatctgt ttctatgaat agcaccatat cccttcagtt 159960
   acatgatgca gaaagctaaa catttttcct cttctatttt cttcaaccct acattcagtc 160020
   accatagcta gttaatatta tatctttaac atttttcaaa tttgtcgtca gcctctgttc 160080
   tctaacactt cagtattgaa ctccagttta gactactatc atcttttatc tgtttactcc 160140
   agcatcttgt atcagtcttc ttacctccac actggcctcc tctttaatcc atttttcatg 160200
   ctgtcagagt gaagttttta atggtaaaat cggattacat tgtgctcaat tcagcaacac 160260
   atataccaaa attagaacaa ttcagagaag actagtatgg cccttgtgca aggatgactt 160320
   aagatttgca aagcatttca tattttttct gtatccaagg gcacacaatg tactggagat 160380
   gatcagtaaa gagtaggata tctaaatttg gaattatgta ttataatttg catgacaata 160440
   acaacaaaaa atgaggggaa aggggatggg ctatatggga acaaagttgt gtatattatt 160500
   gtaattaagt tggcattaac ttgaaataga ttgttataaa ttatgatgtt gtaattctaa 160560
   cagcaacctg tgatgattaa ttttatatgt caccttgact ggcctaagga atgggaatgc 160620
   ccagatagct ggtaaagaat tatttatggg tgtgtctgtg aaggtgtttc ttgacaaggt 160680
   tagtatttga atcaacagac tgagcaaaga agacccaccc tcatccatgt gggtggacat 160740
   aatctaatct gttgaaggca tgaatcaaat aaaaaggcag aggaagggtt aattttctct 160800
   ctcttcttga attgggacat tcggcttctc ctgccctcag atattgaaac tactggttct 160860
   tgggcctttg gacttcagga cttccaatgc ctccacaact caagttctca ggaaatagag 160920
   ttccaccagt gtacacccta caccaggatt aaagttttca ggactgggac ttatgtcatc 160980
   aggtagtctt gttctcaggc ctttgaactt aaattatacc actgactttc ctggtttccc 161040
   aggaaatatg cagacagtat atcattggac ttcttggcct ccatagttcc atgagtcaat 161100
   ccctataata aatctcctcc tacatatctc tatatagccc attgtttctg tttctctgga 161160
   gaaccctggc taacatacac ccactaagaa aacaattcaa aaatagcaca acagaacact 161220
   aggtagagtt ctaaggtttt ttactctagt tcttggtgcc tggatggccc cccgacccac 161280
   acaagacctg ggggaatttg ctgccctgaa gagaaggaca taggcctggc tggttttgcc 161340
   agctaatgat tgtagagccc tagagcattg agtgaacata agtggtagcc agggagtggt 161400
   tacagcaggc cttgggccag acccagtgct gttctcgctt taggtctgac ccagcacagt 161460
   cctaagggtg gtagccacag gggtgcttgt gtcactccat ccccagctcc aggtggctca 161520
   gaacagagaa agagagactc tctttgtttg ggagaacgta aaggaagaga agatgagtct 161580
   ctgcctagta atacagagaa ttcttccaga tcttgtccaa gaacatcaag gtagtacctc 161640
   tatgagtctg caagacccag agcattgctg cctcctaaag cagatacagc ttagatcaca 161700
   aaaccctagt cctttcaaat agctggaaag cctccccaag aaagatgggt atgaacaagc 161760
   ccagactgtg aagactacag taaataccta actcttcaat acccagactc agaagatcta 161820
   caagtattaa gatgatccag gaaaacatga tctcaccaaa agaagcgaat aaggcacgtg 161880
   ggaccaatcc tggagaaaca gagatatgtg actgttcaga cagaattcca aatagctata 161940
   atgaagaaac tcagagaaat tcaagataac acagagaagg aattcagaat tctatcagat 162000
   aaatctaaca aagagattga aataattaaa aagaataaat agaaataatg gaatgaaaaa 162060
   tgcaattgac atactgaaga atgcatcaga gtcttttaat acagaattga tcaagcagaa 162120
   taaagaatta gtgaggctga aggtaggcta tttgaaaata catagtcaga agagacaaaa 162180
   gaaaaaagaa taaaaaacaa tgaagcatac ctacggaatc tagaaaatag cttcaaaagg 162240
   gaaaagctga gagttattgg tctgaaagag gaggtaaaga aagagatagg gatagaaaat 162300
   ttagtcaaag ggataataac agaactttcc aaacctagaa agatatcaat atccaagtat 162360
   aagaaagtta tagaacacca agcagattta atccaaagaa gactacctca aggcatttaa 162420
   taatcaaact cccaaaggtc aaagatgaag aaaggatcct aaaagcagca agagaaaaga 162480
   aacaaataac gtacaatgaa gctctagtac atctgacagg agacttttca gtgtaaaacc 162540
   ttacaggcta gaagagagtg gcatgacata tttaaagtgc tgaagagaac aaattcttac 162600
   cctagaatag tatatcccac aaaattgccc ttcaaacatg aaggagaaat aaagactttc 162660
   ccagacaaag aaaagccaag tgatttcatc aacaccagac ctgtcctaca agaaatgcta 162720
   aagggagtac ttcattcaga aagaaaagga tgttaatgag caagaagaaa tcatctaaag 162780
   tacaaaactc actggtgata gtcagtacac agaaaaacac agattattat aacactgtaa 162840
   ctgtggtgtg taaactactt ttatcttaag tagaaagata aacagtgaac caatcaaaaa 162900
   taataactac aacgttttaa gacatagtac agtaagatat aaatagcaac aataaaaagt 162960
   taaaaagcag ggggacgaag ttaagacata gtgtattagt tttcttttgg cttgtttgtt 163020
   tatgcaaaca gtgtcaagtt gttatcagtt taaaataatg ggttataaga tagtatttgc 163080
   aagcctcatg gtaacctcaa attgaaaaac atacagtaag tacacaaaaa ataaaaagca 163140
   agaaatgaaa tcatatcacc agagaaaata atcttcacta aaaggaagac aagaaagaaa 163200
   ggaggaagag aagaccacaa aacaacctga aaacagataa caaaatggca ggaataagtc 163260
   cttacttatc aataataaca ctgaatgtaa atgaattaaa ctctccaatt aaaagaaata 163320
   gagtaaatga atggattaaa aataaataaa taaaactagg cccattaatc tgttgcctac 163380
   aagaaatgca cttcacctac aaacacaaac attgaatgca aataaagggc tggaaaaaat 163440
   atatttcacg ccaatggaaa ccaaataaga gcaaaagtag ctatacttat atcagacaaa 163500
   atagatttca agacaaaaac tataagagac aaagaaggtc aagatataat gataaaggca 163560
   tgaaatcagc aagagaatat aataatttga aacatatgtg cacccaacac tggagcacac 163620
   agaaatataa agcaaatatt atcagagcta gagagagaga gagaatccaa tacaatagta 163680
   actggagatt tcaccacccc actttcagca ctgaacagat cttccagaga gaaaatcaac 163740
   aaagaaacat tgggccatgg cccaaagaaa cattgtacta tataccaaat ggatctagta 163800
   gacatttaca gaacatttca tccaatggct gcagaataca cattcttttc ctcagtacat 163860
   ggattattct cagagataaa ctatgtgtta ggtcactata tgttaggtca caaaacaagt 163920
   cttaaaatat tcaaaataat tgaaataata tcaagcatcc tctctgacca caacagaatc 163980
   atactagaaa tcaataaaaa gggcggggcg cggtggctca cgcctgtaat cccagcactt 164040
   tgggaggcca acgcaggtgg atcacgaggt caggagatca agaccatcct ggctaacaca 164100
   gtgaaacccc gtctctacta aaaataaaaa aaattagcca ggcgtcgtgg cgggcgcctg 164160
   tagtcccagc tactctggag gctgaggcag tagaatggca agaacccggg aggcggagct 164220
   tgcagtgagc ggagatcacg ccactgcact ccagcctggg caacagagca agactccatc 164280
   tcaaaagtaa taataataat aataataaga agaagaattt tggaaactat acaaatactt 164340
   gtttagtatt tgtatgtgca ttagtccatt ttcatgctgc tgataaagac agacccaaga 164400
   ctggtcgatt tacaaaagaa agaaatttaa tgggccaggc acagtggctc atgcctgtaa 164460
   tcccagcact ttgggaggcc aaggtgggcg gttcacaagt tcaggagatc aagaccatcc 164520
   tggtcaacat ggtgaaactc tgtctctact aaaaatacaa aaattagttg ggtgtggcag 164580
   cgcgtgcctg taatcccagc tactcgggag gctgagaaag gagaatctct tgaacccagg 164640
   aggcggaagt tgcagtgagc tgagatcatg ccactgcact ccagcctggt gacagagcta 164700
   gactccatct caaaaaaaaa aacaaagaaa agggtttaat ggacttacag ttccacatgg 164760
   ctagggaggc cttgcaatca tagtggaggg caaggaggag caagtcatgt cttacatgga 164820
   tggcagcagg caaaaagaga gaagttgtgt acagaaactc ccccttataa aaccatcgga 164880
   tctcatgaga cttattcact atcatgagaa cagcatggga aataactgcc ccatgattca 164940
   attacctccc atggggtccc tctcacaaca catgggaatt caaggtaaga tttgggtggc 165000
   agcacagcca aaccatatca ttccacccca gcccctccca aatctcatgt cctcacattt 165060
   caaaaccaat catgccttcc ctatagtccc ccaaagtctt aactcatttc agcattaact 165120
   caaaagtcca tagtctaaag cctcatctga gacaaggcaa ggtcttccac ctatgagcct 165180
   gtaaaatcaa aacaagctag ttacttccta gatacagtag ggtacaggca ttgggtaaat 165240
   acacctattc caaatggaag aaattggcca acactaaggg gctacaggcc ccatgcaagt 165300
   ctgaaatcca gcagggcagt caaatcttaa agtttcaaaa tgatctcctt tgaatccatg 165360
   tctctcatcc aggtcatgct gatgcaagag gtgggtttcc atggtcttgg gcagctctgc 165420
   tcctgtggct ttgcagggta cagcatctct cccagctgct ttcatgggct ggtgttgatt 165480
   gtctgtgggt tttccaggta tatggtgcaa gctgtcagtg gatctaccat tctggagtct 165540
   ggaggatggt ggcccttttc tcacagttcc actaggtggt gcccaggtag ggactctgtg 165600
   tgggagctct gagcccacat ttcccttctg cactgcccta gcagaggttc tccatgaggg 165660
   cctcacccct tcagcaaact tcttcacccc ttcagcaaac ttcttctagg tttcctctga 165720
   aacctagatg gaggttccca aaacccaatt cttgacttct gtgcactcat agactcaata 165780
   ccatgtggaa gctgccaagg cttgaggctt gcaccctctg aaaccacggc ccaatctcta 165840
   tttcagccat ggctggagtg gctcggatgc agggcaccaa gtccctagac cgcacacagc 165900
   atgggaccct gggcttggcc cacaaaacca tttcttcctc ctaggcctcc aggcctgtaa 165960
   tggaaggggc tgctgcaaaa gtctctgaga tgccctggag acattttccc cattgtcttg 166020
   gggattaaca ttgggatcct tgttacttac gcaaatttct gcaggcatct tgaatttctc 166080
   cttagaaaat gagtttttct tttctattgc attgtcaggc tgcaaatttt ctgaactttt 166140
   atgctctgtt tcccatttaa aactgaatgc ctttgacagc acccaagtca cctcttgaat 166200
   gctttgctgc ttagaaattt cttctgccag ataccttaaa tcatctctct caagttcaaa 166260
   gatccacaaa tctctagggc aggggcaaaa tgccaccagt ctctttgcta aaacataaca 166320
   agagtcacct ttgctccagt tccaaacaag tttctcatct ccatctaaga ccacctcagc 166380
   ctagatttca ttgttcatat cattatctga attttggaca aagccattca acaagtctct 166440
   agggagttcc aaacttttcc acattttcct gttttcttct gagccctcca aatttttcca 166500
   acctctgtct gttacccagt tccaaagttg cttccccatt tttgggtatc atttcagcag 166560
   caccttactc tactggtacc aatttaatgt gttagttcgt tttcatgctg ctgataaaga 166620
   catacccaag actggccaat ttacaaaaga aagagattta ttggacttac agttccacgt 166680
   ggattgggag gcttcacaat catcatggaa ggcaaggagg agcaagtccc atcttacacg 166740
   gatggcagca ggcaaacaga gaaagcttgt gcagggaaac tcccccttat aaaaccatca 166800
   gatctcatga tacttattca ctatcatgag aacagcacaa gaaagacctg cccccatgat 166860
   tcaattactt cccatggggt ccctcccaca acatgtggga attcaagatg agatttgggt 166920
   ggggacacag ccaaacaaga tcaacgtgga aattaaacaa tatgcttctg aatgaccagt 166980
   aggtcaatga aataattaca aaggaaattt aaaaatttct tgcaacacat gctaatggaa 167040
   acacaacata ccaaaaccta tgggatacag caaaagcagc actaaaaagg aaagtttata 167100
   gctataaatg cccacattaa aaaaagaaaa acatcaaata aacaacctaa caaagcatct 167160
   taaagaacta gaaaatcaag agcaaaccaa atccaaaatt aacagaagaa ataaaaaaga 167220
   tcagagcaga aataaataaa attacaatga agaaaacaat acaaaagatc aacgaaacaa 167280
   aaagctggtt ttttttaagt taaacgaaat ggacaagcct ttagccagat tagctcagaa 167340
   aaaaaaagac acaaataaat aaaatcagag atgaaaaagg agacacaaca actgattctg 167400
   cagctattca aaggatcata agtcactact gtgaacaaca ataagccaat aaattggaaa 167460
   atctagaaga aatgggcaaa tttctagaca catacaacct acaaagatta aaccatgaag 167520
   aaatccaaaa catgaacaga tcaataacaa tgagatggaa gccataatca aaagtctttc 167580
   agtaaagtaa agctcggaac ctgatggctt caccactgaa ttctaccaaa catttaagga 167640
   agaactaata ccaatcttac tcaaactatc tgaaaaatca aggagaagtg actacttcca 167700
   aactcattct acaaggctat tattgcccca ataccaacac caaagatacc tcaagaaaag 167760
   aaaactacag gtccatatct ctgatgaata ctgatgcaca aatcttcaac taaatacata 167820
   tttaacaata cattaaaaag atgattcatc atgaccaagt gggatttatc ccagggatgc 167880
   aatcatggtt ccaacatatg caaaataatc aatgtgatac attatatcaa cagtatgaag 167940
   gacaaaaacc atacaatcat ttccatcaat gctgaaaacc catttgacaa aattcagcat 168000
   ctcttcatga aagaaaaccc tcaaaaaact gggtacagaa ggaacatacg tcaacctaat 168060
   aaaagccata tacaaaagat ccatagcaga tgtcatacta actggggggt gagggggtac 168120
   tgaaagcttt tcctctaata tctggaacat gacaaggatg cccactatca ccactgttat 168180
   tcaacgtaat actggaagtc ctagatagag cagctagaca agagaaaaag aaaagggcat 168240
   ccaaattgga aaggaagaaa tcaaattatc cttgttggca gattatgtga tcttatattt 168300
   ggaaaaacct aaaggctcca ccaaaaaact gttagaacgt ataatcaaat tcagtaaagt 168360
   tgcagggtgc aatatcaaca tacaaatatc aatagcattt ttatatgcca acagtgaaca 168420
   aatcgaaaaa gaaattttaa aaagtaatct catttacttt agctgcaaat aaaattaaat 168480
   gcctagaaat taatcaaaga agtgaaagat ctttataata aaaactataa aacactgatg 168540
   aaaaaaattg aagaggacac cacaaattgg aaagatattt catgttcatg gattggaaga 168600
   atcaatattg ttaaaatgtc catactaccc aaagcaatct atagattcaa tgcaatccct 168660
   atcaaaatgc caatgacatt attcacagaa atagaaaaaa aaatcctaaa atttatatgg 168720
   aaccacaaaa gacccagaat agccaaagct atcctaaaca aaaagaacaa aactggagga 168780
   agcacattat ctggcatcaa attatacttc acgactatag taaccaaaac atcatggtac 168840
   tagaataaaa acagacacac agaccaatgg gatagaatag agaacccaga aacaaatcca 168900
   cacacctaca gtgagctcat gttcgacaaa ggtaccaaga agatacacta gggaaaagac 168960
   agtctcttcc ataaatgatg ctgggaaaac tggacattca tatgcagaag aaagagacta 169020
   gacccctatc tctcatcata tacaaaaatc aaatccaaat ggattaaaga cttaaagacc 169080
   tcaaactatg aaactactac aagaaaacat tggggaaact tccaggacat tggactgcac 169140
   taagatttct tgagtaatac cccacaagca caggcaacca aagcaaaaaa gcataaatgg 169200
   gatcacatca agttaaaaag cttttgtata gcaaaggaaa caacccaaaa aggaaagagg 169260
   caacccacag aatgggaaaa attattcaca aactacctat ctaacaaggg attaataaca 169320
   agtatatata agtagctcaa ccaactctgt caaaaaaaaa aaaaatctgg ccaggcatgg 169380
   tgggacacac ctgcaatccc agcactttgg gaggccaagg cgggtggatc accagaggtc 169440
   aggagttaga gaccagcctg gccaacatgg tgaaacccca tctctactaa aaatacaaaa 169500
   attagccggg cgtgatagca catgtctgta atcccagcta ctcgggaggc tgaggcagga 169560
   gaatcgcttg aacccaggag gtggaggtcg cagtgagccg agatcgcacc attgcactcc 169620
   agcctgggtg acaaaaaaaa aaaaaaaaag taaaaaaaac taataatcca attttaaatg 169680
   ggcaaaagat ttgaaaagac atttctcaaa agaagagata caaatggcaa acggacctat 169740
   gaaagatgct caatgtagtg atcatcagag gaagggacaa atcaaaacta caatgagatg 169800
   tcatctcacc ccacttaaaa tggcctcaaa aacaggcaat aacaaatgct ggctaggatg 169860
   tggagaaaag ggaacctttg tacactgttg atggaatgta aattagtaca gccactgtgg 169920
   agaacagttt ggaggtttct caaaaaatga aaaatagagt taccatatga ttcagcaatc 169980
   ccaccactga gtatataccc aaaagaaagg aaatgagtgt gtcgaaggga tatctacact 170040
   cccatgtttg ttgcagctct gttaacaata gtcaagattt gaaagcattt aagtgtctat 170100
   caacagataa tggataaaga aaatgtgatt ctcatacaca gtgtagtact attcaaccat 170160
   aaaaaagaat aagatccctg tgcaacaaca tggatggaac cagaattcat tatgttaagt 170220
   gaaataagcc aggcacagaa atataaacat cacgtggtct catttatttg tgggacctaa 170280
   aaataaagac aattgaaccc attgagatag agagtagaag gatggttacc agaggttggg 170340
   aaggttagtg ggggatagga ggaggtgaga tccttaatag gtatcaaaaa gttaaaatga 170400
   ataaataagg cctagtattt gactgcacaa cagggtggct accattaata taatttaatt 170460
   gtgcattttc tattaagaat agcaaagaca tgaacacaac ctaaatgccc atcaacgata 170520
   gactggataa agaaaatatg gtacatatac accatgtaat actatgcagc cacaaagaaa 170580
   aggatgagat catgtccttt gcagggacat ggatggagtt gaaagccatt atcctcagca 170640
   aactgatgca ggaacagaaa accaaacacc acatgttctc acttataagt aggagctaaa 170700
   tgatgagaac acagggacat attgggggga aaacacacat tggggcctgt tggagcaggg 170760
   gtgaaggcag gcagagcctc aggaaaaaca gctaatgcat gttgggctta atacctaggt 170820
   gatgggatga tccatgcagc aaaccaccac agcacacatt tacctatgtg aaaaaaccct 170880
   gaacatctgg cacatgtacc tctgaactta aaatgaaagt tgaagaaaaa aattaaataa 170940
   tttaattgtg cattttaaaa taactaaaag aatacaactg gattgtttgt aacacaaagg 171000
   ataaatgctt gagagtgtga atactccatt ttccatgatg tgattattac acatagcatg 171060
   actctatcaa aacatctcat gtaacccatg aatatataca gctgctgtgt acccacagaa 171120
   attgaaaatt aaaaattatt ttaaaaaaga aaataaaaat aaatacagta aaagaaacaa 171180
   tgaaggatta aaacaataca ttagaaaata tatttataac acaaaagtag gtattaataa 171240
   aggaaggatt gaaaaagaca tgaaacatct aaaaaacagg tagaaaaatg gcaaatgaga 171300
   acattacatt attagcaatt tcattaaatg taaactggtt gaacattcaa attaaaaata 171360
   agagattcag ccaggtgcag tggctcacac ctgtaatccc agcactttgg gaggttgaga 171420
   tgagtggatc acctgaggtc aggagttcaa gaccagtctg gccaacatgg tgaaattccg 171480
   tctctattaa aaatacaaaa aaaaaaaaaa aaattagcca ggtgtggtgg caggtgcctg 171540
   tagtcccaac tacttgggag gctgaggcag gagaattgct tgaacccagg aggcagaggt 171600
   tgcagtgagc cgagattgtg ccattgcact ccagactggg tgacaagagt gaaactatgt 171660
   ctcaaaaaaa gaaaaaaaag gggttagcag atttaaaagc aagattcaac tatatgctgt 171720
   ctatgagaga ctttagattt aaagacacaa agaggttgaa aataaaagta tgaaaaaaga 171780
   tgtaccagtc aaactgtaac taagagagac ctagaatgac tatattatta aagtagactt 171840
   taagacaaaa aattgttact aaagactaag aaggacatct tacaatgata aaagagttaa 171900
   tcagccaggc atggtgtctc atgcctgtaa tcccagcact ttgggaggcc aagccaggtg 171960
   gatcacttga ggtcaggagt tcgagaccag cctggccaac atggagaaac cctgtctcta 172020
   ctaaaaatac caaaattagc caggcatggt ggcatgcacc tgtaatccca gctactaaag 172080
   aggctgaggc aagagaatca cttgaacccg ggaggtggag gttgcagtga gctgagatcg 172140
   taccactgta ctatatagcc tgaacaacaa agtgagactc tgtctcaaaa aaaaaaagag 172200
   ttaattgatc aaaaacatgt aagaattata aacatatatg caccaaacaa ccccaaattc 172260
   taacagaatt gaaactgaca gaattgaagg agaaaatagt caattcaaca gtagttggag 172320
   attaattaac ccactttcaa tggtggttag aatagctaga cagaatgtct tccagaaaat 172380
   ggaagacttg aacaaggtat agtccagttt ccagagatct aaagaacctc catttattaa 172440
   taaaataata cacattcttc tcaagcacac aagtaacatt tgccaggata aaacatatgt 172500
   tacaccataa agcaagtctt aataaagtat aaaagactgt aatggtacaa attatactat 172560
   gtgaccacag tgaaatgaaa ttagaaatta ataacatgaa aatttggaaa tttcacaaga 172620
   aaattgagca cagttgtaaa taattaaagg tcaaaaagta aattacaaaa gaaatttgaa 172680
   aatgccttga gataaatgaa aataaaaaca cagtacacca gatcttagga gatgcagtaa 172740
   aaatagtgcc tagagggaaa tgtataatta gaaatgccta ttttttttta aaaaaagata 172800
   gatctcaaat caaaaccaaa actttatctt aaagagctaa aaaaaaaaag caaatccgaa 172860
   gcaaacagaa gaaaagaaat aataaatatt acagagaaaa taaaggaaat agaaaataga 172920
   aaaatcatag agaaaatctg taaaaccaaa agttggttct ttgaaaagac caacaaaatt 172980
   gacaaaactt tagctaatct caccaggcaa ggaaaaaaaa aaacaaacct cagccgggcg 173040
   tggtggctca tgtctgaaat cccagtactt tggaaggctg aggcaggtgg actacctgag 173100
   gtcaggagtt cgagaccagc ctggccaaca tggtgaaacc tcatctctac taaaaataca 173160
   aaaattagcc aggtgtggtg gcacgcacct gtaatcctag ctacttggga ggctgaggca 173220
   ggagaatcac ttgaacccag gaagcaaggt tgcagtgagc tgagattgca gcactgcact 173280
   ccagcctggg tgacagagct gacagagcaa gactctgtat ccaaaaaaaa aaaaaagaaa 173340
   gaaagaaaag aaaaaacaga ctaaaattac taacatcaga aatgaaaaat gggacactat 173400
   taataacttt acaaaacaaa aaatattata aggtgttttt gactgaatgt tccccctcac 173460
   tgtctctaaa tccacatgct gaagccctag ctctcaatat gatgatattt aaagatgggg 173520
   ttagggtaag gtaaataggg ttaggttagg tcatgagctt gggccctcat catgggatta 173580
   gtggacttag aaaaagaagg agagatgaga tcatggcaga caggcaggac tagattgaag 173640
   ctcccacttg gacagacaca acagcctgtg gaggcttgta ctgtgaactt ttgctccagt 173700
   atgactgtag aaatatatta ggaaagccaa gagaacccat atatcttccg aaagaagtgg 173760
   attgttcctg caagactctg gagacacccc aaatactgtg agtgcccaaa ctctgcaagt 173820
   gggaaaggaa gattgttcat ccatgaacat acatccccac tggggaacct gaaggtctag 173880
   attatgggag aagattctga ccttacctgg agctgagtca atttagagag ctgagtgaaa 173940
   tacagaggta gaggaagcag aaggaaaatc tctgtgggct tgctgggtcc cttagcaaga 174000
   catttctgcc tcacctcaca ggcatccttg aggagagcta ccagagtcac tgggaaaagg 174060
   ccacagggag aagaaaagca ccagctgaac tttgtaacaa tttgaaccaa tttagaagcc 174120
   tcctggccag aaactggggg agggtgtgaa tctggtgtgc atactccaca ggtgggagag 174180
   gaaggaaagc cctagttatt ttcacagctg ggaggtgggt agcctggggc aagttctcag 174240
   tcctgctggc ccactgcctg gaaacagact tgttgctgtt gggggtggga gcatggtgga 174300
   agtgagatca gccctttggg ttgggtggga gctgggtgag gcctgtgact gacagctttc 174360
   ccccacttcc ctgacaacct gcatgacaca gcagaggcag ccataatcct cctggaaaca 174420
   aaactccatt gacctaggaa cctgaacctc acccccaccc ccaacaggag ccgcagcaag 174480
   acttacccaa gtagagtctg agctcagaca cacctagtct tgcccccacc taatggttct 174540
   tccctaccca ccctggtaac tgaagacaaa ggacataaac tcatgggagt tctaaggcct 174600
   ctcccaccac ctgttcctcc acatactacc acagctgatg ctctcttgaa agcaccacct 174660
   cccagcagga ggccaaccaa cacaaaaata gtgcaataat caaccaaagc taaggaccct 174720
   cacagggtcc atttgatacc cctgccacct ccaccagagc aggtgccagt atccatggct 174780
   gagaaaccca cagattgttc acatcatagg actctgtgca gacaaccccc agaacctgtc 174840
   tactagagca agtctactag cccagagcct ggtaggcttg ctggatggct agaagagaga 174900
   taacaattac tatagctcag ctctcaggaa gccacatccc taggaaaagg gggacagtac 174960
   tacaacaaga aaacaccccg tgggacaaaa ggacctgaac aacagccttg agcctgagat 175020
   gttcctgctg acaaagccca aatgagaagg aacccaaaaa ccaactcttg taatatgaca 175080
   aaaaaaggtt ttttaacacc ccccaaaaat catactagct ctccagcaat tgatccaaac 175140
   caagaaaaaa tccctgatct acctgaaaaa gaattcagaa ggaggttagt tattaagcta 175200
   atcagggagg caccagagaa aggtgaagcc caatgtaagg aaatccaaaa aatgatacaa 175260
   gtgaagggag aaatattcaa tgaaaaagat agcataaata aaaatcaatc aaaacttcag 175320
   gaaacaatgg acacacttat gcaaatgcaa aatgctctga aaagtctcag caatagaatt 175380
   gaacaagcag aataaagaac ttcagagctt aaagacaagg ttttcaaatt aacccaatct 175440
   agcaaagaca aagaaaaaag aataagaaaa tatgaacaaa gcttccaaga agtctgggat 175500
   tatattaaat aaccaaatct aagcatcttc agcgttcctg aggaagaaga taaatctaaa 175560
   ggcttggaaa gtgtatttgg gggaaaaatc aaggaaaact tttctggcct tgctagagaa 175620
   ctagacatta tacaagaagc tcaaagaaca tctgggaaac tcatcacaaa aagatcattg 175680
   cccaggcaca ttgtcatcaa cttatctgaa gttaagatga aggaaaaaat attaaaagct 175740
   gtgaggcaaa aacaccaggt aacctataaa ggaaaaccta tcagattaat agcagatttc 175800
   tcagagaaag tctacaagct agaagggatt ggggccctat cttcagcctt cacaaacaaa 175860
   caattatcag ccaagaattc tgtacccagt gaaactaaac tttgtagatt aaggaaaaat 175920
   acagtctttt tcagacaaac aaatggtaga aatcactact accaagccag cactacaaga 175980
   actgctaaaa ggagctctaa attttgaaac aaatcctggc aacacatcaa aacagaacct 176040
   ctttaaagca taaatctcac aagacctaca agacaaaaat acaatttaaa aaaaaaaaaa 176100
   caacccaaga tatacaggaa acaaatagca taatgaatgg aatgatagct cacatctcag 176160
   tcaaacgttc aatgtaaatg gcctaaatgc tccactgaaa agatacagaa ttgcagaatg 176220
   gataagaatt caccaaccat ctgctgcctt caagagacac acctaacaca aaaggactca 176280
   cgtaaactta aggaaaggaa tggaaaaaga cattgcatgc aaatggacac caaaagtaag 176340
   caggggtagc tgttattata tcagacaaaa caaacattaa cgcaacagca gttgaaaaaa 176400
   caaagagggt cattatataa tgataaaagg tcttgtccaa caggaaaata tcacaatcct 176460
   aaatatatat gcacctaaca ctggagttcc caaatttgta aaagaattat taatagaagt 176520
   aagaaataag atagacagaa acacaataat agtgggggcc tttaatactc cactgacagc 176580
   actagacagg tcatcaagac agaaagtcaa caaaaaacaa taaatttaaa caataccctg 176640
   gaacaaaggg acttaactga tatacacaga acattctacc caacaaccac agaatataca 176700
   ttctattcaa cagtgcatgg aactttctcc aaaagaccat atgataggct gcaaaacgag 176760
   cctcaataaa ggtaagacaa ttgaaattga attcaagtat tctctcaggc cacagtggaa 176820
   tacaactgga aatcaactcc aaaaggaacc ttcaaaacca tgcaaataca tggaaattaa 176880
   ccttctcctg aatgatcact gagtcaaaaa tgaaatcaag atgaaaattt aaaaattctt 176940
   ctaactgaat gacaatagtg acacaaccaa tcaaaacctc tggaatacag caaaggcagt 177000
   gctaacagga aagttcatag ccctaaatgc ctacatcaaa aaggttggaa gagcacagac 177060
   aatctaaagt catacctcaa ggaactagag aagcaagaac aaactaaacc caaacccagc 177120
   agaagaaagg aaatcaccaa gatcagagca gaactaaatg aaattgaaac aaacaaaaaa 177180
   atacaaaaga taaacaaaac aaaaagctgc ttctttgaaa agataaataa acttgataaa 177240
   ccattagaaa gattaaccaa gaggagagaa aatccaaata agcacaatta gaaacgaaat 177300
   gggagctatt acaactgaca ccacagaaat acaaaagatc atcaaggcta ctatgaacac 177360
   ctttatgtgc ataaattaga aaacctagaa gagatggata aagtcctgga atgatacaac 177420
   cttcctagct ttaatcagat agaattaggc actctgaaca gaccagtaac aagtagagag 177480
   actgaaatgg taatagcaac aattaccaac aacaaaaaaa gtcaaggacc aaacgaattc 177540
   acagcagaat tctaagagac gttcaaagaa gaattggtac caatcctatt catgctattc 177600
   cacaagacag agaaagaggg gaccctcccc tcaaatcatt ctatgaagcc agcataaccc 177660
   taataccaaa accaggaaaa gacataacag aaaaagaaaa tgagaggtga cagtgtgctg 177720
   gcagtgctca cagccctcac ttgctctggg cgcctcctct gcctgggctc ccactttggc 177780
   ggcacttgag gagcccttca gcctcccgct gcactgtggg agcccctttc tgggctggcc 177840
   aaggctggag ctggctccct caacttgcgg ggaggtgtgg agggagaggc gcgtgtggga 177900
   actggggctg cgcccggtgc ttgtgggcca gcgcgagttc caggtgggca tggactcagc 177960
   ggaccccgca ctcggagccg ccggccggcc ccaccggccc ccagcagtga ggggcttagc 178020
   acctggccag cagctgctgt gctcaatttc tggccaggcc ttagctgcct tcccacggga 178080
   cagggctcag gacctgcagc ccgacatgcc tgagcctccc ctcccactcc gtgggctcct 178140
   gtgccgcctg agcctccccg aggagtgctg ccccctgctc caagagccca gtcccatcca 178200
   ccacccaagg gctgaggagt gtgggcgcat ggctcgggac tggcaggtag ctccacctgc 178260
   aggctccgtg ggggatccac tgggtgaagc catctgggct cctgagtctg gtggggacgt 178320
   ggagaacctt tatgtctagc taagggattg taaatacacc agtcggcact ctgtatctag 178380
   ctactctggt ggggtcttgc agaacctttg tgtccacact ctgtatctgg tggggacatg 178440
   aagaaccttt gtgtctaact cagggattgt aaatgcacca atcagtgccc tgtcaaaaca 178500
   gaccactcgg ctctaccaat cagcaggatg tgggtggggc cagataagag aataaaagca 178560
   ggctgcccga gccagcagtg gcaacccgct cgggtcccct tccacagagt ggcaacttgc 178620
   ttgggtcctc ttccacacag tggaagcttt gttctttcgc tctttgcaat aaatcttgct 178680
   actgctaact ctttgggtcc acactgcctt tatgagctgt aacactcacg gcgaaggtct 178740
   gcggcttaac tcctaaagcc agcgagcccg cgagcccaca gggaggaagg aacaactcca 178800
   gacgcccgcc ttaagagctg taacactcac cgggagggtc tgcagcttca ctcctgagct 178860
   agcgagacca cgaacccacc agaaggaaga aactcggaac acatccgaac atcagaagga 178920
   acaaactcca gacatgccgc cttaaagaac tgtaacactc actgcaaggg tacttggctt 178980
   cattcttgaa gtcagtgaga ccaagatccc accaattccg gacacaaaaa ctacagacca 179040
   atatctctga tgaacataga tgttaaaata cttaacaaag tactacctaa ccaagtcaaa 179100
   caacatatca aaaagataat ccaccatgat caagtgggtt tcataccagg gatgcggcag 179160
   tggtttaaca tatgcaagtc aataaatgtg atacaccact taaacagaat caaaaacaaa 179220
   aatcacacga tcatctcaat agatgcagaa aaagcattca acgcaatcca gcatcccttt 179280
   atgattaaaa ctctcagcaa aatcagcata caagggacat acctcaatgt aataaaagct 179340
   gtctatgaca agcccacagc caaaataata ctgaataaaa aaaagtggaa agcattccct 179400
   ctgagaactg gaataagaca aggatgccca ctctcaccac tccccttcaa cataggactg 179460
   gaaatcctag ccagagcaat cagacaaaag agagaaataa aggacataca aattggtaaa 179520
   aaggaagtca aactgttgct gtttgctgat gatatgatcc tttacctaga aaaccataaa 179580
   gactcctcaa gaaagctcta gaagtgataa aagaattcag caaagtttct ggatacaaaa 179640
   tcaatgtatg caaatcaata gctcttctat acaccaacag caaccaagct gaaaattaaa 179700
   ttaagaactc gaccacggcc aggcgtggtg gctcacacct gtaatcccag cactttggga 179760
   ggccgaggtg ggcagatcac gaaatcagga gttcaagacc agcctggcca acatagtgaa 179820
   acctcgtctc tactaaaaat acaaaaaaat tagctgggca tggtggcatg tgcatgtagt 179880
   cccagctact caggaggctg aggcaggaga atcacttgaa cctgggaggg gggggttgca 179940
   gtgagccgag attgtgccac tgtactccag cttgggcaat agagtgagat ttcgtctcaa 180000
   aacaacaaca acaacaacaa caaaactcaa ccccttttac aatagctgca aaaaaataaa 180060
   ataaaatgct taggaatata cctaaccaag gaggtgaaag acctctacaa gcaaaatggc 180120
   aaaacactgc tgaaatatat catagatgac acaaacaaat ggaaacacat ttcatgctca 180180
   tggataggta gaatcaatat tgtgaaatgg ccaggtgcag tggctcaggc ctgtaatccc 180240
   cgaactttga gaggctgaga tgggtggcta acttgaggcc aggagttcaa gaccagcctg 180300
   gccaacatgg caaaccccct ctttactaaa aatataaaaa ttagtcagga gtggtagtgc 180360
   acacctgtaa ttccagctac tgaggaggct gaggcatgag aatcacttga acccagggtt 180420
   caagtgttgc agtgagccaa gatcatgtga ctgcactcca gcccagatga cagagtgaga 180480
   ctgtgtctca aaaaaaaaaa aaattgtgaa aatgaccatg ctgccaaaat caatctacaa 180540
   attcaatgca attcccataa aaataccatc gttcttcaca gaattagaaa aaacaattat 180600
   aaaattcgta tgcaaccaaa taagagcctc catagccaaa gcaagactaa gcaaaaagaa 180660
   catatctaga ggcatcacat tacctgattt caaactatag tataagacca tagtcaccaa 180720
   aacagcgtgg tactggcata aaaataggaa catagaccaa tggaacagaa tagagaaccc 180780
   agaaatgaag ccaactgatc tttgacaaag caaacaaaaa tataaagtgg ggaaaggaca 180840
   caccctattc aacaaatggt gctgggaaga ttggcaagcc acacgtagga gaatgaaact 180900
   ggctccttat ctctcacctt atacaaaaat caactcaaga tggatcaagg acttaaatct 180960
   aagacataaa actataaaaa ttctagaaga taactttgga aaaacccttt tagacattgg 181020
   cttaggcaag gatttcatga ccaaaaaccc aaaagcaaat gtaacaaaac aaagacatat 181080
   agctgggact taataaacta aagagttttt gcatggcaaa aggaacagtc agcagagtaa 181140
   acagacaacc cacagagtgg gagaaaatct ctgcaatctg tacatatgac aaaagactaa 181200
   tacccaggat ctacaacaaa ttagcaagga aaaaaacaaa caatcccatc aaaaattggg 181260
   ctattctcaa aagaaaatat acaaaaggtc aacaaacata tgaaaaaatg ctcaacgtca 181320
   ctaatgatca gggaaatgga aatcaaaacc acaatgtgat accataatgg tattacatta 181380
   atggccataa tcaaaaaatc aaaaaataat agatgttggc atggatgcag tgaacaggga 181440
   acacttctac actgctggtg ggaatgtaaa ctagtacaac cactatggaa aacagtgtgg 181500
   agatccctta aagaactaaa agtagaacta ccatttgatc cagcaatctc actattgggt 181560
   atctacccag agaaaaagaa gtcatcatat gaaaaatata cttgcacccc cgtgttttta 181620
   gttgcataat ttgcaagtaa aatcatggaa ccaacccaaa tgcccatcaa ttaacatgtg 181680
   gataaataaa ctggtataag gctgggcata gtggctcacg cctgaaatcc cagcactttg 181740
   ggggtgccga ggcagatgga tcatttgagg tcaggagttc aagaccagct tgccgaacat 181800
   ggtgaaaccc tgtttctact aaaaataaat acaaaaaaat gagccaggca tggtggtgca 181860
   catatgtaat cgcagctact caggaggctg aggcatgaga atcacttgaa cccaggaggt 181920
   ggaggttaca gtgagatgtg attgtgccac ttcactccag cctgggtgac agaatgagac 181980
   cctgtctaaa aaaaaaaaaa aaaaagatgt gaaactggtg tgtgtgtgtg aatatatata 182040
   tatatgatgg aatactactc agccataaaa aggaatgaat taatggcact cacagtgacc 182100
   tggatgagat tggagactat tattctaagt gaggtaactc aggagtggga aactaaacat 182160
   cgtatgttct cactcatatg tgggagttaa gctgtgagga tgccaagaca taagaaggac 182220
   aaaatggatt ttggatactc agggggaaaa ggtgggaagg ggatgaggga taaaagacca 182280
   caaattgtgt acagtgtata ctgctcaggt gatgagtgca ccaaaatctc aaaaattacc 182340
   accaaagaac ttactcatgt aacgaaacat cagctgttcc ccaataacct acggaaataa 182400
   aaaataaaat aaaaaagagg gagagatctc atctctctct tctttctagt gctctctctt 182460
   tacatgcatg caccaaagaa aagctttgtg aggacatatc aagatagtgg ctgtctgtaa 182520
   gtcaggaagc aagccctcaa gaggaagtaa atcagctagg accttgatct tggacttccc 182580
   aaagtccaga actgtaagaa atgaatttct gttgtttaag tcacacagtc tgtgatattt 182640
   tgttacggca acccaagcca actaagacat taggcatact atgaacaatt ataagccaac 182700
   aaagtagata acttagatga aatagacaaa ttcctagaaa catacaaaca accaaatcta 182760
   attgaaaaag gaatagaaca tctgaaaaga tcaataacaa gagtctgaat tagtaataaa 182820
   aaaacttttg aaaaggccca ggactataca gatttactgg tgaattctac caaacgtttg 182880
   aagaagaatt actactgatt cttcataagc ttttgcaaat aataaaagag gagggaacag 182940
   tttctaactc aatatttgat gtcagtactt ccctgatata aaagcaagac aaacacatga 183000
   caagaaaaga aaactacaga gcgatatctt tataaatatg gaagcaacat tgctcaacca 183060
   aatactggca aactgaattc aaaaaaacat ataaaaacat tttagaccac tcagcatccc 183120
   cttcccttca ctgtctcttt cccttgttcc ctctcttccc cctcccagag caccttcccc 183180
   gtgaggctgc agtagtggca atggggttca actgggtccc ggtgaacagg agcgggggct 183240
   ggcagacatg gcggccaaca tgtaccaggt aggagatttt gtctactttg agaattcctc 183300
   cagcaatcca taaataatca ggatagaaaa tctcgacaag actgcaagtg gcaacatgga 183360
   agcaaaagta gtatgctttt acagattaca tgatattttt aacacactga taatgcttgt 183420
   ggagaagcat gctaaagaaa ttgaggaaga atctgaaaca atagttgacc cttacttgac 183480
   caatactcag aaacatcaat caaaacataa gttactttct ttgttacccc aatatgaatc 183540
   tttgccttca acacatatta ggggaaaatg cagtgttggc ttatgaatga gacagaatca 183600
   gtattgtcat atcttcataa aaaggatact ttcttctact tattgatcat gacccctcat 183660
   cgaaaacact attagctgat gaaggtgaat tcagaatggg acctagatat caagtagatg 183720
   tcccagaaat gctcatagaa ggagaatcag atgagaggga gcagtgaaaa tggaaagtta 183780
   aagtttgggg atccaaatag cccacttaaa gattgatcaa ttaaccagtt tttaattgta 183840
   gcaagtgcta ttgggacact ccctagacct ctcgattgca gcagttcttt gaggcagcct 183900
   agtttgcata tgagtgctgc tgcagcttcc caagacatca ccttctttca tgctatcaat 183960
   acattgcgta gacatagcta tgatttgagc agtgcaatta gtgtctcagt accacttgca 184020
   gaagctgttt tatgcagaga taaattggaa gaatggtcag cctcttaagc tagcttattc 184080
   gaagaggtac tggaaaaaat atggcaaaga cttcaacgac atatgccaaa actttttccc 184140
   ttggaaatca ttgactagca tcactgaatg ttatttcttg tggagaacta ctgacagata 184200
   tgtgcaacag aaatggttaa aagcagcaga aactgagagt aagctgaaac aagtatatat 184260
   cccaatttac aggaaaccaa atcccaacca aatatctacc agcaatggca agcctggtgc 184320
   tatgagtgaa accatgaggt tgacattcct gcctcagaat ccccttgtag tgcaagcctg 184380
   ggagggctgc tgtgctacac agtctaacca gtagtattct tggggcccac ctaatatgca 184440
   gtgcaatttg ttggctttat tgagaaaaat atggagacct gaaaatgccc acccagtcag 184500
   aagaaaagtt atctcctagc ccaattacag aggactctca tgttagaaat gctgccaggc 184560
   cattcaaggg atgccactcc aaaacactgt cagtacaaag tctgcagtga aaactcacca 184620
   agctttcttc cttcatactg tgtttttcac aaaatttgct ctccatttat tcaaaagtat 184680
   cctctggctg aagcaggcag caagatggcc acttgtagct attaatttat gctgccatta 184740
   gggcagaatg taagcttttt tttttttttt tttttagaca gggcttcact gtatcaccca 184800
   ggctggagtg cagtggcatg atcatggatc actacaaact tcctctccca ggttcaagtg 184860
   atttttctgc ctcagcctcc tgagtagctg gaattacagg tggctgctgc cacgcctggc 184920
   taatttttgt atttttagta gagacagggt ttcaccatgt tggccaggct ggtcttgaac 184980
   tcctgacctc aggtcatcct cccacctcgg cctcccaaag tgctgagatt acaagtgtga 185040
   gccattatgc ccagccactg ctttaaaatt cttaatctta tatgttgtgc ttctgaccat 185100
   ttcctctcgc tccttttttt gtttgtttgt aataaatgta agttattgtg tacaaccttt 185160
   tatttggttt attttttaac attatttttg tctgttgcta tttgtatcat gccaatctga 185220
   aaaaaatcaa agcattaaaa acttctgtta tcttatcagg gaatactgct cagcaaaaga 185280
   ttggtgggag gtgatcctat cccatggggt tttatgatgg aattgcctac aaagcccata 185340
   agcactttta cctactaggt agtgcaacaa gataacccct aaggatggtt gatatagtat 185400
   ggctgtgtcc cacccaaatc tcatcttgaa ttgtaattct gataatccct atgtgtcatg 185460
   ggagggacca ggtgggaggt aattgaatca tgggggtggt tacctccatc ctgttctcat 185520
   gatagtgagt gagttctcat gagatctgat agttttataa gggttttttc tccctttcac 185580
   tctgcacttc tccttgctgc caccatgtga agaagggtgg atttgcttcc ccttccacca 185640
   tgattgtaag tttcgtgagg cctccctagt cctgctgaac tgtgactcaa ctaaacctct 185700
   ttccagccag gtgcagtggc tcacgcctgt aatccaagca ctttgtgggg ctgaggcgcg 185760
   tggatcacct caggtcggaa gtttgagacc agcctgacca acatggagaa acctcatctc 185820
   tactaaaaat acaaaataat tagccgaacg tggtggggca cgcctgtaat cccagcaact 185880
   caggaggctg aggcaggagt attgtttgaa cccaggaggc agaggttgtg gtgagccgag 185940
   attgcaccat tgcactccag tctgggcaac aagagcgaaa ctgacattaa aaaaacaaaa 186000
   caaaacaaaa caaaaccctc tttacttcat aaattaccca gtctcaggtg tgtctttatc 186060
   agcagtgtga gaacagacta atacagtaaa ttgataccag gaatggagct gttcgggtaa 186120
   acatacccca aaatgtggaa gtgactttgg aactggataa caggcagagg ttggaagagt 186180
   ttggaagagt tagaagacaa cagaaagata tgggaaagtt tggaacttcc tagaaacttg 186240
   ttgaatggct ttgaacaaaa tgctgatagt gatatggaca ataaagtcca ggctgaggtg 186300
   gtctcagatg acgatgagga acttgttggg aactgaagta aaggtcactt gctatgcaaa 186360
   gagactgatg acattttgcc cctgccctag agatctgtga aactttgaac ttgagagaaa 186420
   tgatttagta tatctggcag aagaaatttt taagtgccaa agcattcaag agagagtaga 186480
   acataaaagt ttggaaaatt tgcagcctga tgatgcgata gtaaagaaaa acccattttc 186540
   tagggagaaa ttcaagccca ctgcagaaat ttgcataagt aacaaggagc caaatgttaa 186600
   tcaccaagac gatggagaaa atgcctacag ggcatgtcag agaccttcag aacatcccct 186660
   ttcatcacag gcccagaggc ctaggaggga aaaatgactt catgggctgg gcccatggcc 186720
   accctgctct atgcagcctc tggacatggt gcccagcatc ccggctgctt cagctccagc 186780
   catggctaaa agggaccaat gtacaactca ggctgttgct tcagaaggtg caagcccttg 186840
   gtggctccca caaggtgttg ggcctgtagg tgcacagaag tcaagaactg aaatttggga 186900
   accttcacct agatttcaga ggatatatgg aaacatctgg atgtccagag agaagtttgc 186960
   tgcaggggtg gaaccctcat ggagaacctc tgttagggca gtgtggaagg gaaatgtggg 187020
   ttcagagccc caacacagag ctcccactgg ggcactgcct cgtagagctg tgagaagagg 187080
   gccactgtct taccccagaa tggtagatcc accaacagct taccccatgt acctggaaaa 187140
   gtcacagaca cccaatgcca gcccatgaaa gcagctagga gggaggctgt accctgcaaa 187200
   gccacaaagg tggcactgct caaggccata ggagcccacc tcttgtatca gcatgacctg 187260
   gatgtaagac atgaagtcaa aggagatcat tttgtaactt taaggtttaa tgattgccct 187320
   gctgggtttc agacttggat ggggcctata acccctgtgt tttggccaat tttttcccca 187380
   ttggaatgag tgtatttgct caatgcctgt acccccagtg taactagaaa gtaactaact 187440
   tgcttttgat tttacaggct cataggcaga agggtcttgc cttgtctcag ataaaacttt 187500
   gaacctggac ttttgagtta atgctggaat gaattaagat tttgggggac tgttgggaag 187560
   gcaggattat gttttgaaat gtgagaacat gggatttgga aggggccagg gcataatgct 187620
   gtggtttggc tgtgtcccca cccaaatctc attttgaatt gtagttacca taatccccat 187680
   gtgtcatagg agggacccag ttggaggtaa ttgaatgttg ttcacatgac agtgagtgag 187740
   ttctcatgga tctgatggtt ttaaatgggg ctttccccca ctttgctctg cacctctcct 187800
   tgctgctgcc acgtgaagaa ggacatgttt gtttcccttt ccatcatgac tgtaagtttc 187860
   cttaggcctc cctagccctg aagaactgtg agtcaattaa acctctttcc tttataaatt 187920
   acccagtctc aagtatgtct ttattagcag tgtgagaatg gaataatacg atgatgttat 187980
   aatgaaactc cataattgag acagtgaggt ccattcatat gtagattaat taggtctaaa 188040
   taaaatttgc ccagcctggc aaacacagtg gaaccccacc tctaccaaaa aatacaaaaa 188100
   ttagccaagt gtggtggcac atgcctatag tctcagctcc ttgggaggct gagacatgag 188160
   aattgcttga gcccaagagg tggaggttgc ggtgggccaa gattacacta ctgcactcct 188220
   gggcaacaga gcgagaccct ctcataaata aataaataaa taaaatttgc caatttacac 188280
   ttaaaaaagg attttatacc ataaccaaac gagatttatc acaggaatgc aaggttggct 188340
   caacataaag aaatcaatca ataggctata ccatattaat aaagaaaaat cccatatgat 188400
   catctcaatt aatgcaaaaa atgcatttga caaaatgcaa caccatgtca tgatttaaaa 188460
   aaaaaaaaaa cacttggcaa atggggagta aaagggaact taacctgata aagagaatct 188520
   atgaaaaccc cagaactcat ataatattta atgatgaaag actgacagtt ttactcctaa 188580
   aatcagaaaa aagacaatgg tcttccagtc tgatattact ttatgattat aaagtcatgt 188640
   gtgttgtaat ttaaaatact ttctataaag tctagatgtg gtggcacatg cctgtaatcc 188700
   cagcactttg ggagattgaa gtgggaggat tgcttgaggc caagagtttg agaccagcct 188760
   gggtaacata gtgagaccct gtctctacaa aaaattaaaa tattagctgg gtgttgtggc 188820
   atgtacctgt agtcctacct acttaagagg ctgaggcagg aggattgaga ggatcaattg 188880
   agctcaggag tttgaggttg cagtgagcta tgattgcacc actacatcca gcctaggtaa 188940
   taaagtgaaa tattgtatct aaacaacaac aacaacactt cttaaaggat atgcatggtg 189000
   taatccaatt ttcattcttt aacaagtata tgtgatgcac agaaatgagt ttagaaggct 189060
   gtataccaag atattggcaa tggtaattgg gatggtggca gtgttgatga tgtatatagc 189120
   ttatatatat tttttaacaa caactttatt gagatgtaac tcagatgcca tacaatccac 189180
   ccatatttaa agtatacaat ccagtggttt ttggcacagt tatgcaagca taaccactat 189240
   ctaattccat aatattttat caccctaaaa agaaaccttc tactcattag aagtcactcc 189300
   ccacttcctt ctcttccagc ccccaacaac cactaaccat ctttctgttt gtatgagttt 189360
   acccattctg gatacatgct atgtaaatgg tatcatataa tatgtatttt tttgcatgtc 189420
   tggcttcttt catgtgtcat catgttttca aggtccatcc atatcgtagc atgtatcagt 189480
   actttattcc tttttacagc taaacaatat tccattgtat ggatatacta tattttgtgt 189540
   atgcttttat cagttgatgg acatttgggt tgtttctact tcttgactat tattattaac 189600
   ggtgctatga tcattcatgt acaagctttt gtgtagacat atattttcac ttatcttgga 189660
   tatatagtag gtatgtgttt aacatcttaa gatatatagt aggtatgtgt ttaacatgtt 189720
   aagaaattgc aaagcagttt ccaaaatgat tgcgtatttt atatttctat cagcaatata 189780
   tgaaatgtat gaaagtccta gttgctctat atcctctcca atatttggtt tgattagtct 189840
   tttaaatttt aacccttcta atggatgtgt catagtatct cattgtgctt ttaatttgca 189900
   tttgcctgat gattaatgat gttgagtgtc ttttcatggc cttattggct attagcgtat 189960
   cttctttttt aaagtgtctt gccaagactt ttgttcctgg ttaaaattgg gttttctttt 190020
   tgatacctat ttgtagaagt ttttacatat tctgaacaca catcttcatg aatattttct 190080
   atagtttgtg gcttgccatt tcatattcca actttgagtc aaggtctact attagactcc 190140
   aaacactggg cagacctcaa gctttgtctt ctgttctcca ccccacttcc tgtgaaacca 190200
   ttataaatgc aatgtcaaga tcactacagt ttagcatatg ttcttaaggc aaaagcctta 190260
   gcacttttct cacctcttag gaggttccat gttcactcta tttttagttt ctgggagcct 190320
   ttactttctt ggcatttcag taatatgttt gaaagtttat ttgtttatat atattttttt 190380
   tttctttttt tttttttttt tttgagatgg agtctcactc tgtcacctag gctggaatgc 190440
   agtggcgcaa tctcagctca ctgcaacctc cacttcccag gttcaagcga ttctcctgcc 190500
   tcagcctcct gagtagctgg ggttacagcc acacaccacc acgcctgact aatttttgta 190560
   tttttagtag agacggggtt tcactatgtt ggtcaggctc gtctcgaact cccgacctcg 190620
   tgattcacct gcctcagact cccaaagtgc tgggattaca ggcatgagcc acggtgcctg 190680
   gcttgtttat attttatatg gcagcagtat tactttgagg gagaggatga tagcttccct 190740
   tgcagctagc actgatcatg tgacatagtt ctggccaata agagaagagg aagtctgtgg 190800
   gcaatgatcc tttcctcttc cttccacttt tctgcttcga atatagatgt gagaaccaga 190860
   gatgaagcac ccatctttaa ttcattagga agtaacacat ttgctaagaa tggctaaata 190920
   gaaataaaac agaaaccata aacaatgatg acatcatgag ctgtgggacc aatcctgggt 190980
   tgcctaactc caaacttctt agtgaaaagt tatatactct aatttgtcta aactactgtt 191040
   ggttgggttt tctgatactt acagtcagaa attaaagtta tttttgagat ttaatattta 191100
   tataatcatt catccatcta atcaataagc atgtattaaa tacatatcaa gtgccagaca 191160
   ttgtttataa tagagtttct cattaggggt ggtattacta ttagttagca ttttaaacat 191220
   ttgtgagaaa gtttttgtag tgttacaact ggaagcaact aacatttagt ggattggtac 191280
   tgggaataca atatttcttt ttttacatct tatttttttt ttttttttga gacagagttt 191340
   tgctcttgtt gcccaggctg aagtgcgatg gtgcaatcct ggctcactgt agccactgcc 191400
   tcccgggttc aagcgattct cctgccccag cctctcggat agctgagatt acaggcatgc 191460
   accaccacac ccggctaatt ttgtattttt agtagagaca gggtttctcc atgttggtca 191520
   ggctggtctt gaactcccga cctcaggtga tctgcccacc tcagcctccc aaagtgctag 191580
   gattacaggc ataagccact gcgcccagcc aatacagaat ttcttaaaat gcataagact 191640
   gtcccatgca gtaagaattg ccctacatct cgcatacttt tcaaagtcca ccaaagacat 191700
   atctctagct gaaaaaggtg tttttaatga tctaaaatta gaacttaatt cattttgcaa 191760
   ataaatacca agtagtttct tcatgtgttt aatagattta aattttccag aaattttttt 191820
   taaagagaca gggccttgct ctattgccca ggctggagtg cagtggcatg atcatagctc 191880
   actgcatcct caaactccta gattcaagca gtcctccaac ctcaacctca gtctcctgag 191940
   tagctaggac tacaggggta gtcctaccat gtagcatggt agtagtaact accatgccca 192000
   gcttttgctt tttttttttt ttttttttta gagatggggg tctcccaatg ttgcccaggc 192060
   tggtcttgaa ctttggcatc aagtgatcct ccttccttgg cctcctaaag tgctggtatt 192120
   acagacatga gccaccaagc ccagcttcca ggaatatttt gttagaaata taccataagg 192180
   tattcaacat ttaggaaaat cacataatca ctcgcaatgc tgcatatgat tttaaagttt 192240
   ccaatataac acacctatat cagactacat gtatagctgt ctcatttaca tttattctat 192300
   atattagtgt aagtaagcat ctaagttatt ttgtctagaa gtcaaaacta gatatttacc 192360
   ttttgaaata catactatat tataaacaac tttgctttta ttactccttt acataatcag 192420
   gtgttatgtt gcttttttaa attatgttca cttgtaagtt atatagtcta tttagttaat 192480
   gacagaattg taagcaggtc ttataaaata tctataatag gaagggagtt gcgcctggat 192540
   ggagttaaga atcattggtt ttaatggtaa ttccttatct ttaaaaaatt gttggccagg 192600
   cgcagtagct catgcctata atcccagcac tttggggggc caaggtggga ggatcacttg 192660
   agcccgagtt caagacaagc ctggacaaca tggcaaaact ccatctctac taaagataca 192720
   aaaaattagc caggcatgtt ggcgtacccc tgtagtccca gctacctggg aggatgagga 192780
   gagaggatca cctgagcctg ggaggttgag gccgcagtga gctgtgatta taacactgta 192840
   ctccagtctg ggtgacagag tgagaccttg tctcaaagaa aaaaaaattg ttacatagta 192900
   tttaatactt ttcattcaag atgacaagaa aatgcctaaa agcaaagaaa atccctggtt 192960
   gcttggaaaa cctgaccccc agctagaatt ccctgcctat agatttttaa tttctgagta 193020
   cactttatgg ctctttgact cagattgcat tttactccgg tgggcagcag aagttactgc 193080
   cactctgcca gctcctggcg actactgggg agggttgtga agtcacctca ttccagcagc 193140
   aaatccatat ggctctgcag caatttgatt cttgcctcct cagaggaaaa aatttggcta 193200
   aggggcataa ggcagagtga gagacagggg caagttttag agcaagagtg aaagtttatt 193260
   taaagtttta gtacaggaat gaaaggaaga aaagtacact tggaagaggg cccagcagac 193320
   cacttgagag atccaagtgc cccgtttgtc ccttgacttg gggttttcta cattggcacg 193380
   gttctggggt tggcatttct tcttccttga tttttccctt ggagtgggca gtccatatgt 193440
   gtgatggccc gccagcactt gggaggggct gcatgtgcag tgtgtttact gaagttgtgt 193500
   gcatgctcac ctggggcatt ttttccttac cagttgagcg ttcctagagg aaggtcatat 193560
   accagttaaa ctctgccatt ttgcctctta gtgtgcatgt ttgagcccat ttgcccaact 193620
   cctgaaattc tatcggaaag ctactgatca ccagcttcgg gtattttctg tctattggga 193680
   ggctgtcttt tcgtggcgcc agctgcaacc aattattttg gagagacagt ttaacaacaa 193740
   cctgaccata acctgatggt cacctgacat tcctggtggg ggccctctcc tgccctgctc 193800
   gtgtctgcct aactaccact ctaaaaacct aagaacttgg ctgtatttct cttgaagttt 193860
   gacaccattc ctctgaagag gaaaacacaa ttaaggagga cttactgact tacagatgga 193920
   gaatgaatga aaggagaaat gcaacttgtt taagatcatg caaaaagcag tgagtgttca 193980
   gaggtaggaa ccaaatctaa ttttccaatt cctgaagcta aattaggctt aacccattag 194040
   tgtatgtttt atatttatgt atttatttac ttatttttga gacagagtct cactctatca 194100
   cccaggctgg agtgcagtgt cacaatctcg gctcactgca acctctgact cctgggttca 194160
   agcaattttc ctgcttcagc ctttctgtgt attttttata tttttgtatt attagtagag 194220
   acggggtttc atatgttggc caggctggtt ggaactcctg acctcaggtg atccacatgc 194280
   cttggcctcc caaagtgctg agattacaag tatgagccac catgcccagc ctgttttcta 194340
   ttaaaaaaaa aaaaaaaaag acatgagaat atctgtgtat ggtcagaaag acctctctcc 194400
   tcttgaagtt taataactta aaaaaagtgg cttactacag gcaacaatag gaaaaataga 194460
   taaatggata aatgggatta catcaaacca aaaagcttct gtataacaaa gaaacaatca 194520
   aaagggtgaa gaaacaacct acagaatggg agaaaatatt tgcaaatcat acaaagaggt 194580
   taatgtccaa aatatataaa gaacttgtac acctcaatag taagaaaaca aataacccaa 194640
   ttttatttta ttttattttt attttttgct gttccttgca gagctaattc ttaggcagtg 194700
   ggcccagggt cagccccaat tttaaaatgg accaaaaacc tgaataaaca tttttgcaaa 194760
   aaagacatac aaatggccaa caggtatatg aaaaaatgct taatatcact aatcattaga 194820
   aaaatgcaaa ttaaaatcac aatgatctat cacctcatgc ctgttagaat ggctataata 194880
   gaaaacacaa gtgttggtga ggatgtggag aaaagagaac gcttgtacac tgttggtgga 194940
   aatgtaaatt agtgcagcca ttatagaaaa gaatatggag gttcctcaaa acaattaaaa 195000
   atagaactac catatgatcc cgcaatccca cttctgggta tatatccaaa ggatatgaaa 195060
   ttaatatgtc aaaaagatat ctgcttgccc atgtttattg cagcattgtt cacaataacc 195120
   aagatataga atcaacctaa gtgtccatca atggatgaat aaagaaaatg tgtatctata 195180
   caatggaatg ctattcagtc ttaaaaaaga agggaatctt gtcatgtgac aacatggata 195240
   aacgtggagg acattatgtc aagtgaaata tgccaggcac ggaaagacaa tctgaaaaag 195300
   tcaaactcat agaagcagac agtagaatta gaatggtggt tattaggggc tcatggttcc 195360
   ccgttggtcg ggaaggtcat gacataaaat agcaggattg gagatataga tgctagtcaa 195420
   gaaaagtcaa gaaatataaa ttttgagtta actatcagaa ataccttcag gagatgtatg 195480
   ttgcggtgac tattgttaat aacatattga atatttgaaa gtagctaaga gagttggttt 195540
   taagtgttct cagcacaaaa aaataagtga agtaatgcat atgctaatta gcttgattta 195600
   gccattccac aatgtatgca tatatcaaaa catcatgttg tacaccatac atatatacaa 195660
   tttttagtca tcagttaaaa aagggaaaga aaaggtggct cacatttctc ccattgaatc 195720
   ttcttttttt ctaagtgcag tcatcccttc gtatcctcag gggatttgtt ccaggacccc 195780
   cgaggatacc aaaatccatg gaagctcaag tccctgatat aaagtggtgt agtatttgct 195840
   tattatctga gcacatcctc ctatacactt taaatcatct ctagattact tataatgcca 195900
   acacaatgtg aatgctatgt gaagcattgt tgtattgcat tcttttgaat tggcattctt 195960
   ttttatcatt gtcttgttat gtttaattat ttttttccaa atattttcta actgagactg 196020
   gttaaatcca tggatgggga accacagaca caacaaagga ccaactataa tttcacttgt 196080
   ataagttcct tatatgtgag gagagtaaag gaaagaaaat ttcaaaggtc acagaattat 196140
   taattgacaa ggtagaagct cagcttcctt aagttctagc caagcttctt tgatatagta 196200
   aagtatttac aaaggagtca atgttcaact cttattaata ttatgggttt aaaatgtttt 196260
   cattatctaa ttatacttgg tctcctctga ttttgttacc actccccact catgcaacgt 196320
   catgctctct tactggatac ctgatggttt cttccctttt atactgggat ctttccattt 196380
   ggctctacca gacaaatttc atttaagcaa attattagaa tcagacgaga aattttaaag 196440
   gcaatgtgac acgttttgcc taagacttgg gcaaatgtct gaaactctct atactccagt 196500
   tcattttagc ttcatatctg tgattgtttg ctctttataa atactcacaa cagggttata 196560
   atatcccagg tattaactga gcctgtaaaa ctgcctgtga ttttaggtgg aggataaagg 196620
   gaaagagaca gagctgattc taccggggat ccaacagagc tttgatgata tggactgttt 196680
   cctgttttgc ctgtgaagat tgtccagagc taaagtaaga aaacatgtgg agaatatgga 196740
   gataaaatat gagacaaaag gtagaaaatg gacaaaagga aaataatata tgtattatac 196800
   attttataat taagagtgta aaggattagc tggaggtagt ggtgtgggcc ggtagcctca 196860
   gctacttggc aggctgaggt gggagaatga tttataagcc caagagatgg agactgcagt 196920
   gagctgtgat tatgccactg cactttagcc tgggcagcag agtaggacct tgtctctaaa 196980
   ttaaattaaa gaatgtaaag gggagccagg cacagtaaca cacatctgta atctcaacta 197040
   cttgggaggc tgaggtggga ggattacttg agaccagcag tttgaggtca gcctggacaa 197100
   catagtgaga ccccatctct acataaaaga aaaagaaaga atgtgaaggg gaatgctcag 197160
   gtggtattgc tatgaactac actgtcacta tatcaaacag atcaatttta gctgtaaatg 197220
   acagagacct taaaaaatgg tgacttaacc attaggaaag ttcttcctca acagacagca 197280
   atctggaggt aggtgatccc cagttgggtg gggagctcat gacatagcaa gggcccacac 197340
   ttttcccacc tgcttagagg accatcctct gcatgtcgac caagtttccc attttgatca 197400
   taaggtggct gctggagctc ccagcacaaa attctcacac atcagctcct atcaatacct 197460
   aagcaggaag gaagtggata tgggtaaaga gagacttttc tttttctatg cctttctctt 197520
   aactgagagg acagtctttt tctaaactac tcaacagaac ttagtagcat gcccctagac 197580
   caacacaaag agaaatggga tggcagtgtt gtaaaccaat cctaattcat gtcttaggat 197640
   tagacatact actataagag agaaggtaga ctggccaatg ggtaggcaag catcagaatc 197700
   agtcacacaa atacacacac acacacaaac acagaatcaa tcaactatca gaattaataa 197760
   gggatttggg cgaggttgac agacgcaaga ctatactaaa aaacggattg cctttagcct 197820
   caataacaaa ataagagtat tttgtaaatt atgccattca tttttgcaac aaaaataaag 197880
   tatcttaaat taacccagat attttgttaa cattagccaa ttgaccaaga atacacaaga 197940
   catctatgga ggaaatttta agacttttat aaagggcaca gatatgaatt tagtacatga 198000
   aaagacttta tagtctcaaa cataatattt taaatatcta attacactaa cgtaaaaatc 198060
   aattaatata tgtaaagcag ttgattaaaa ttccagttag atttcgtgag gaaatttata 198120
   ctctagaatt tatatgccaa ctaatttcct agactaagtt gccattttaa atgtagactc 198180
   ctactttgta atattaaaac ataaacagta gtaataagga gcaggatggt gtttatttga 198240
   ataaggaatt gaaaagggaa gcagaatttc tattcataga ataatggatt tactatagtg 198300
   ttaagcttta ggcaattatg ggagctggca gaagagtcta tataagcttt tgcatctgca 198360
   tctagtgttg acctcaagcg tttataggtc agacagacta gcagatggaa aggaaagcta 198420
   gctgtggctg agaagatggg caaactggaa ccagtgaaga caaactggag ctggaactgg 198480
   aacccacaag acagagcaga accagtgtgt gtctcttgcc atttccaaca tcagtgctat 198540
   gagtgatcag tagaaggtgg gacctttcac catggaactg tccacatact tggggcagaa 198600
   attggagaat ctggtggaga tttgacagga gctggaggag ctggatgtct cagttgattc 198660
   aataagataa gccagcagat cagtaacaat gcatgcaaga tgttgctata catggcactt 198720
   tctgaggacc ttcagagcag atagatgata gatagataga tagatagata gatagataga 198780
   tagatagata gatagataga cagatagaca gatagataga cagatagctg ccccttcact 198840
   taccactttt caagtatcac acaaaatgcc tcctgtggcc aaaacaaact gaggaccata 198900
   aggaaaaaat aattacagga aatgtaattc cagtaaaact aatctgacac agttcaaagc 198960
   tatcacaata ttgtattgtt ggtttggttc aagaacaaac agacaaaaaa cagaagtgag 199020
   tgccagggac atgtttatac agaaacttaa tagacactaa aggaaaacta gctatatgca 199080
   cacaaagact ggatctttat acaacataat aaaaaatgga ctccagatag atttaagaat 199140
   aaatatgctg taatcccagc actttgggag gccgaggtgg gtagatcaca aggtcaggag 199200
   ttcaagatca gcctggccaa gatggtgaga ccccatctct actaaaaata caaaaaatta 199260
   gctgggcatg gtggcgggcg cctgtagtcc cagctactcg ggaggctgag ggagagaatt 199320
   gcttgaatcc gggaagcgga ggttgcagtg agccaagatc gcaccactgc actccagcct 199380
   gggtgacaga gcgaaactcc gtctcaaata aataaataaa tgaatgaata aataaataaa 199440
   agaataaata tgaatgttat aactataaat ttataaaata aaatatagaa catttttgtg 199500
   acttaggagt agggaagggt tacttataca aaacttcaga agcacaagct ataacaaaaa 199560
   gaaaaaaagt aaagtaattt gattacacct aaattgaggc tttctgttga acagagaata 199620
   ctttggacaa gattgtgtag ttaggcctgg gtgtggtggt tgacacctat aatcccagca 199680
   ctttggaatg ctgaggcagg aggatcactt gaggccagga atttgagacc agtttgggca 199740
   acatagtgag actccatctc tattaaattg aaaaataaat attttttaaa ataaaaattt 199800
   ttgaatgtac aaataaaatc acagttgagc aaaggaatac aaaaaaacac tcaagtgtat 199860
   tagctggatg cacacatatt tattcccgcc cccattgtgt aaaagcagcc ctactgcctc 199920
   ctaaagaaca gggtccgtga tccctgccaa gatgataact tctcttcgtg tctgccagtc 199980
   cctggacaca agaagcatga aatgcctggt ggtggttgtt gtctacattt gctggatctc 200040
   ctagcaaaac cgtgcctcct ttggtaacca ggaccctaac ccttcagagc ctagggttgt 200100
   ggggagagaa agttcaagtc tccactgagt ctttaggagt gatggtaagt ggggtcactc 200160
   ctgcttctat tccttagttc ttggacccct gatttttctt actggagata cagtgtcatg 200220
   taaaaatgtt ttgattcaat cttctgggag atggaagcca atctatcctg atgcccagta 200280
   gctggagctc tttctggaga atgaaacccc tttttggtaa agtattgtct ctaagctagc 200340
   atttcaactg tgtcttcggc atgacatttc aatctcccag actgccagct tctgagtggt 200400
   gtggtatgtg atatgaccag tggatccaat tgtcataggc tcactcctat acctcctttg 200460
   cagtaaagtg aactccctgc tctaacagtg tcaggaaagg atcctttcct aaggagaaaa 200520
   catttcacaa attcttggat ggtgatggta gctaaggctg tgcatgcagg aaaagtaaac 200580
   ccacttccaa aacgtgtgtt tattcttatt agaacaaacc tctggctttt ccaagttcta 200640
   aggggcttgt agtagttaac ttaccactaa attgtctcct tgagaaacta tatatcagat 200700
   gctcagcatc agtgtctgcc cctggcaagt tgaacatttg atgccagcaa cagctaaatt 200760
   caccttggta agtgggaact catactatct ggcccataca taacatctgt ctctcctacc 200820
   atgcccattt catcatcaac ccattgtgcc agcactgggg tagctgatga cagaaactgg 200880
   tgtagtaatg ctgtctactt ggctgtttag atcaatctcc atggttaacg ctctttggtg 200940
   gatatgtgat acaaagactt tctcacttca ttcctactcc catttgttca accacctcct 201000
   tatctcattc cttcaatctt actctttcat gggctcctgc ccatgagtct ggatttattg 201060
   ttacatcaag ccgctactcc ttttacacaa agtggatcaa caggtgaatc attccaagct 201120
   acctattaga aagattgtcc ttgtccctca ctatagtctt ttaaaacctc ttcggaatga 201180
   gactgcagtt caattgtttc catttttaag aaacacatac taagctcttc catttctaaa 201240
   tcaagctttg gattttggtt cttccattag ctggtcatga gagattccca atatgatcat 201300
   agtgaggtga aggagaggtc ttagtatgca gtggtgaaat tcatgggggt ctgggctaca 201360
   tgcttatgct gcttgttttt tgctagtcct tcttatgctt ggagctcatg cttatcttta 201420
   gataactgct gtggcgctta agcaacctta tgatttggag attctgtgtg aatcaagctc 201480
   atgttgggca tttcttggag gcatgaccat tggtgtctcc tggtcaggca tttttatgaa 201540
   ttctatgaat gccgggcagt atcccaggag ttttgttttg gttttttcaa gtatataatt 201600
   ctctttgctc cagaatctca gaagctggca ctgttattct cctgctgggg cttactgtag 201660
   ccccattgcc actttgtatc ttttcttgct actggtattt ctaataccac agtatctgca 201720
   gagtcttgtc gtccaagtag tggagctgct agtcctccac cttggatttg gtacagaaat 201780
   cttttctgca ctaggacctt cttaaagatg ccaatatttc tttctttttc tttttttttt 201840
   tttttttttt gacagggtct tgctctgttg cctaggctag agtgcaatgg tgggatcaca 201900
   gctcagtgca gcctcaactg cccaggctca agtgattctt ctacctcagc ctcctgagta 201960
   gctgggacta caggctcatg ccaccacacc agctgatttt ttttattttt tgtacagatg 202020
   aggttttgca aggttgctca ggctaaagat ggcaatattt cttatcagtt ggtgcatggg 202080
   ctgaagtatt ccctagtgcg gaataagcta tctccagaat tgagggcaac gtattatgca 202140
   tagaggtttc ttccttgtag tgaaaggtgc aagatgtaat ggtttgtctt ttactttgaa 202200
   atatgtgccc tgatatatta atacttttga ctacaggact cctaaaattt tgcttacatg 202260
   tcagcccctg gttctctata ggctttatct catatcctat ggagcacatg tatcttacta 202320
   agtcctctaa tgcgctagcc attttttatt cattagctcc aattaacata atatcattga 202380
   tgtagtaggt cattgtgaag ttttatagga tgcccagatg gtccatatct ctctggactg 202440
   tattattaca gaaagcagaa gaattagcat agctttggga caaaaccata aatatgtctt 202500
   tttacccata ccatgagaat gcaaactctt tctgatcaga atataaaaga atgtttttgc 202560
   caagtcagtg gctatgtacc atgtacctga aacccttttc atctcctcta gcaaaggtat 202620
   taattatatc tagtcctgtg gctgaaatta gggaaacaac ttatttaagt ttgcagtaaa 202680
   ctgacacttt ccaggagcaa tctggtttct tgtggagcca aattggcaaa ttaaatggaa 202740
   atatgagagc atctattatc tctgcatcct caaaagtagt actaatttct gccatctacc 202800
   aacctggggt ggttatattg tttttggtcc tttgatgata tcttagttca tttgtgctgc 202860
   tacaacaaaa tgccacatat tgggtaatat ttaaacaaca gaaatttatt ttcttgcagc 202920
   tccagaggct aggaagtcta ggattaaggt gacggaaagt ttgatatctg tcaagggcct 202980
   gttccctgtt tccaggctag cactttgttg ctgcgtcctc atatggtgga agagatggaa 203040
   gggaataaac ctgctccctc aaagcctttt ataagtccta atcctatcca ggatggctct 203100
   gccctcataa tttaatcacc tccaaaaggc cctccatctg gcagatcact tgaggttagg 203160
   agttcaagac cagcctggac aacatgtcga aaactcatct ctactaaaac tacaaaaatt 203220
   agccaggcat ggtggcacat gcctgtaatc ccaactactt gggaggctga ggcaggagaa 203280
   ttgcttgaac ccaggaggtg gaggttgtag tgagctgaga tcacaccaca gcactccagc 203340
   ctgggcaaca gagcaatttg ctgtctcaag aaacaaaaca aaacaaaaca cctccctctt 203400
   aatactatca cattaaccat taagtttcaa cacatgaatt ttgggggaca catttagacc 203460
   gtatcagatg ggattgagta gaagatgggg tatttttaga ggcttccact tggcatttcc 203520
   cactatgaga tcccttactc tacaggccaa agacccaaag ttagggttgt gccaagtact 203580
   acatagggca atcccaatta tacttttgtg aaccagagga atgacagcta gttgggaaaa 203640
   caaacccaat aggcctgcta taagcaagac tttggtcata acactgtaag gcctcattct 203700
   aacagggaga ccatgatgtt actttgggtc tctggatttt gatgtcacct cagacctttg 203760
   cctcccacaa tgtccaaaat atttgggata cctgagtaag tgggcatttg tccctttgga 203820
   gaaagactag caggtattat taacacgtat atttggctgg gcacagtagc tcacacctgt 203880
   aatcccagca ctttgggagg ccgaggtggg tggatcacct gaggtcagaa gttcgagacc 203940
   agcctgacca acatgaataa accccatctc tactaaaaat acaaaattag ctggccgtgg 204000
   tggctcatgc ctctaatccc agctactcag gaggctgagg caggagaatc gcttgaacct 204060
   gggaggcgga ggtgagccga gagatgtcgc cactgcactc cagcctgggc aacaagagca 204120
   aagctccatc tcaaaaacca aagaaacaaa cacacaaaaa aatgtatatt tggcatgagg 204180
   ttagggtcta tccttctgga agcctggccc ttttttcagt caatgggttc tgtagctgaa 204240
   aacgaattta gatccaggag ctaaattgct ctcaatttag ttgtcaccca tattcaattt 204300
   cttctgatag tacaggccga gttatactct tactgtcaat atattttgcc cctatggata 204360
   ctgtattcca accatctcct ttactctctg acggtcaggc ccttttggtt ataaccctat 204420
   gagtctgcta ctcatttaga taattatgtc aatgtggctt ctggcagttc ctaccataac 204480
   acaccctcta ttgttttgga atcctaacat ccccactgct tttattgagt ctactctgta 204540
   atggcctctc ctaatattaa ccctgtctaa taaagaaatc ccagcactaa acattttagt 204600
   gatgctggta cccctctcac ctgtgcatta ctatggcctt gctaatggtg tgcacaatag 204660
   tccctctcat ggtacataat catctggtga gtctttcagc attacataga tatttaatgc 204720
   ctatttcagc atgcccattt ccttaagctt ttaatccttt cctccactgc ctaccatagc 204780
   aattctggca tttcaacttt agtatggttt gttattttct gcatgcttct gggatctata 204840
   ctagtagtga gttgctaccg tctcctcaga tccttgacag agtattgaat cttgtatttt 204900
   tggagagtgc cccaaataaa taaagtctct cctatccaat atattgttcc agattgctta 204960
   atggtgcacc ctcagaatcc agtgccaata atgccaacca atacattctg tttaggtctt 205020
   gcaattcctt tgggtagagc ttcttttcat ccttatcaag cccagcaaat ctgaagctgt 205080
   attacgttgg aacttaaccc tggttataga tctagtggcc aggaagggaa atgggagcaa 205140
   atcaggaggc aaatcatgtg cctacaggaa aagccctctc cattgtcttc aagcaagaag 205200
   gatgtgctaa ctcttggaga gggatggtca cttctgcaga ccaggagaat tcaaagaagc 205260
   ctaggggttc aaaatcttaa atgacattca cccaaatctc cccagctcaa gggacagtgt 205320
   cctgtttttc ctaatcttgg ccctaacctt agcataacag aattgcttta attctgcatt 205380
   tcctttcttg ccattattat tactaattaa gaaaaaagcc ctctctgtct gtatgtgtac 205440
   aagtgtacac acatgcatgt acatccaatg aagtgaatat gaatttaaag aaatctctag 205500
   aagaaaatat caaactttta atactggtta aatacatgaa ggagagtaca atgaaaagct 205560
   ttggagaaag aaatttgcac aattttctat acattcttcc atgtctaaat attttttgca 205620
   aatatttgta taacatttta aagtattagg aaaagtaaaa ggatcaatca acgtagggga 205680
   agagaactgg aagagggaag aaaactttag ccatgtcctt tattctctcc taagaagttt 205740
   gacctcactt cacccaaccc aagaggagag aatgaatggg tgatgggcat tgagagcttg 205800
   tgcgatgagt catttccttg cccctggaga aggtgggaat gaggagaagg gggcttactg 205860
   tccgtagtca gccatactct ttcattcctc cttcagaaag ggcccaaggg cttggggatt 205920
   gcagctctga tccagggacc atgggaacca catgagaggc atatgaacct gggtacattg 205980
   cagaaggatg attcaactag ggacagtcat gagtgacacc acaggaggag gtgtatctgt 206040
   gcagaaacag ctcttcacag ctttttggaa catcggcatg gtaggaaagg gcaaaataat 206100
   ctatataaat aaagttaagt ttctgtctgt taaaacaatt gattagacag attttcaata 206160
   catttgacaa aggtatttag aatcacatat tggttttctg tagctactat agtaacaaat 206220
   taccacagac ctggtggttt caaacaacag gaatttattc tctcacagtt ctggaggtca 206280
   gaattccaaa attggattca taaggcagaa atcagggtgt tggcaggaac atgctccccc 206340
   taaaggctct aggggccttg actcttccag tttctggtgg ctggcagtat tccttgactt 206400
   gtggccatgt caccccaatt ctcctatgac ttcacatcat ctctctgtgt agataaaact 206460
   tccctctccc tccctcctat aaaggccctt gagattccat ttaaggtcca ccgtaaatgt 206520
   aatttggata atctccccat cccaagatcc ttaacttaat caaatctata gaaactcttt 206580
   atccttatca attaacactt acaggtccca gaggttgcaa cttgacatgc ttgggagcca 206640
   ttcagcctac tgcagatgga ctgaattaaa acatgcaaca cacatgaagt taatgttggg 206700
   aattctggtt tttcaaagag cagtctgctt ctagagaacc caaaattgag aaacaatagc 206760
   agtttcgtgt atctgtcaat cagaagagag ctatggtatg tattaagaca ccaattagag 206820
   aaaataattg gtggcttcaa agtcgagctc tgcctcccca actcaaccta ttgagtagaa 206880
   tttctgcatt tgattcttac cctgactaaa ggcataagag tcgacactga tgtgtaggct 206940
   tcagttcaca ggaagtctgc ttctcacatg agcaactccc tatagctgac ttctcagtaa 207000
   taagtaggga tttatttgtg taaccatgta gaatccaagt aggaagtcaa attgtagtaa 207060
   taatactagc taacgtttct tgagcattta ttatatccct tgtaatgtgc taattgcctc 207120
   tcatgtattg tcctatttaa tttcaaagca aacttttgag gtagcttgac tcactaaggc 207180
   tctttgatta tatccaccac aagcaaacaa gattaattgg aaggcctctg ggctggctca 207240
   caaatcagga gagctgcaaa aacagggttg gtaaggacag aaataacagc agttcaagtg 207300
   tgaagaactc agttcttggt gcttttctca gttatcattg gataactatc tagatgacct 207360
   ttcacctctg tgggtttcca ctcagagttt gaattccaag cagacttaat ctcaccctgg 207420
   tgggacagag ccacaatcat tggctttgcc tgggacaaat gcccaccctg ggccagggga 207480
   gcaggttcag acatgaaaaa agagtgagga gtcgggtgca gtggctcaaa cctgtagtcc 207540
   taacactttg ggaggccgag gcgggtggat cacctgaggt caggggtttg agaccagcct 207600
   ggccaacatg ccgaaacccc gtctttacta aaaatacaaa aattagctgg gtgtggtggc 207660
   acatgcctgt aatcccagct acttggcagg ctgagacagg agaatcgctg gaacctggaa 207720
   ggcagaggtt gcagtcagcc tagatcatgc cactgcactc cagtctgggt gacagagcga 207780
   gactctgact caaaaaaaaa aaaaaaagta tgtgtctcaa acactatttt gcaagcgtga 207840
   aagaaggaag agatagaatt gtgacgttaa gttccccaag tcactaagca agctgatagt 207900
   gaaactataa tatatctttc agtctgtgac ctttggaact ctctgataag atgcagacta 207960
   ctttagccat gtagtagaca gctgatgttt ttgctaggcc agtactgtgg attaaattgt 208020
   gtcctcccaa aattcacatg ttgaaaccct aaccccccaa tgtgatggta tatggagata 208080
   aggcccttgg gagataattc ggtttagata aggtcatcag agaagagtcc tcttgatggg 208140
   attaatgcct ttataaaaag aagaaaaagt taccagagcg ctctctctgc cacatgagga 208200
   cacagcaaga tgacagcttt ctgcaagcca agagtgtgtc ctcaccagaa ctcatccatg 208260
   ccagcaccct gatcttggtc ttccaacctc cagaactgtg aaaagggtct ctgttattta 208320
   aatcaccagt ctatggtatt tgttatagtt ggtgcccaag ccaactaaga caaccatgcc 208380
   atccatgcca gcaccctgat cttggtcttc caacctccag aactgtgaaa agggtctctg 208440
   ttatttaaat caccagtcta tggtgtttgt tatagttggt gcccaagcca actaagacaa 208500
   ccatgccatc catgccagca ccctgatctt ggtcttccaa cctccagaac tgtgaaaagg 208560
   gtctctgtta tttaaatcac cagtctatgg tgtttgttat agttggtgcc caagccaact 208620
   aagacaacca gcatccgtac cccttatttt ggaagcagct ttccaattct aggaagtatt 208680
   tttttttttt tagacagagt ctcactttgt cacccaggct ggagtgcaca gtggcatgat 208740
   cttggctcat tggaaccttc gcctcccagg ttcaagtgat tctcctgcct cagcctctcc 208800
   agtagctgag attacaggtg cctgccaccg tgcccagcta atttttgtat tcttagtaaa 208860
   gacggggttt caccattttg gccaggctgg tgtgaaactc ctgatctcag gtgatccacc 208920
   catctaggca gtcttaatag gaaagtcaag gcccggtgcg gtggctcaca cctgtaatcc 208980
   cagcactttg ggaggccgag gcaggcagat cacgaggtca ggaattagag accaacttgg 209040
   ccaatatagt gaaaccctat ctctactaaa aatacaaaaa ttagccgggc atgatggtgc 209100
   gcgcctgtaa tcccagctac tcaggtggcc gaggcaggag aatggcgtga acccgggagg 209160
   cggagcttgc agcgagccaa ggtcgcgcca ctgcactcca gcctgggtga cagagtgaga 209220
   ctccgtctca aaaaaaaaaa aaaagtcaag cacagggccc tctaccaaac caagccaatc 209280
   agactctcca gggagaatga cagagagact ctaggtgggt gaaatggggt tattctcatg 209340
   gcatgaccta aggaaagaag gtatgtatat ttctgactaa gattcggtag cttcctgctc 209400
   ctctttcttc ccaagacctg gctcatcagt gtttccttta attctgtaag ctataaagtg 209460
   tccttccaat aaattccttt ttccatctag gtgagtcaga gcttctttca gttttttgca 209520
   gaactgattc agcaatggta gctgggagtg agcactgcaa gcaacagaac tgctgtggaa 209580
   atagcagaat taggtgcaag gcaatgagaa ttc 209613
<210> 8
   <211> 2236
   <212> DNA
   <213> Homo sapiens
<400> 8
   gggacttcac cagtgctgcc atgctggccc caggctgtga gttggaccca gaccaagaag 60
   tggtgaggac aaggcctgaa gatgtgcctg cttcccattc aacttccacc atgattgtaa 120
   gtgtcctgag gcctcccagc catgcttcct gtacagcctg tggaactgtg acttttcaca 180
   tagtagagag aatggcttca gttatacctg cctccaatag gtccatgaga tctgacagga 240
   atacatatgt tggaaaaagg tttgttcacg ttaaaaatcc ttacttggat ttgatggatg 300
   aagacattct ctatcacttg gatttgggaa caaaaacaca caacctacca gcaatgtttg 360
   gagatgtaaa gtttgtctgt gtcggtggga gccccaacag aatgaaagca tttgcactgt 420
   ttatgcacaa ggagctcggg tttgaggaag ctgaagaaga cataaaagac atctgtgctg 480
   ggacagacag atactgtatg tacaaaaccg ggcctgtgct cgccatcagt cacggcatgg 540
   gcatcccctc catttctatt atgcttcatg aactcatcaa attactccac catgcacggt 600
   gctgcgatgt caccattatt agaatcggta catcaggggg aatagggatt gcaccaggga 660
   ctgttgtaat aacggatata gctgtagact ccttctttaa gccccggttt gaacaggtca 720
   ttttggacaa cattgtcacc cgaagtactg aactggacaa agaactgtct gaagaactgt 780
   tcaactgtag caaagaaatc cccaacttcc caaccctcgt tggacataca atgtgtacct 840
   atgattttta tgaaggccaa ggccgactag atggagcact gtgctccttt tccagagaaa 900
   aaaagttaga ctacttgaag agagcattta aagctggtgt caggaatatt gaaatggaat 960
   ctacagtgtt tgcagctatg tgtggactct gtggtctaaa agctgctgtg gtctgtgtga 1020
   cacttctcga cagactcgac tgtgatcaga tcaacttgcc tcatgatgtc ctggtggagt 1080
   accagcaacg gcctcagctc ctaatctcca acttcatcag acggcggctt ggactttgtg 1140
   actagacgtc ctaactgggc agcccaaccc tcccctgcaa gtttgtagct caagttgtaa 1200
   tgtgaaagtc atattttatt tgtggcattt ttatatagtt ctcatccaca tgctaaatgg 1260
   aaagacttta tgaaatcctt ctctcttaaa aaggaattta ttgtaaaaga atactcacac 1320
   taaattaaat tcaaatttca ttttagaata agttaactaa atcagtctaa taattaaaat 1380
   tttaaaactc ctggattatg acatttggag tttcatatgc agcattaatt aagctcacat 1440
   caaaatagac cagccatttg cacatagtta ccagtcactc tgtttctgaa accaatccag 1500
   aaattcatgt tagaacattg tcaggcactt ctaggtattg gacaagtgac agaaactgat 1560
   catccatatt caaaatattt acagaaaaaa gtgtcagtga aatgtaggtc tatatggaaa 1620
   agttaccatt aggaaaatat ggccatactg cttttttacc cctgcactat ttcagtgcac 1680
   cacagaatag gattcgagat ttacatatgc ctgtttgtga ttttatctca gaatataaca   1740
   actctggatt acattttcct ctcaactgaa aaccctcagt aatgcaaaga aaggccttcc   1800
   tttttcttca gaatcacaag tgactaaatt gccttctagc cattttttct tgtaaaaaaa   1860
   gtgttttgag gaggctgtat tttttattcc atttcaatat taaccaaata ggctgagaaa   1920
   ttcatagcaa ttgaattaag acttgagttc aagatgttaa aggaatcctt ttgtatgact   1980
   tgtccagacc ggcctattgc cctacccgtt tgattctctg ggaggcatgg tgctcctcct   2040
   ggctgcacat gctctctgtg aactgatcgc atcttgggtt cttctcaatt tgacatactt   2100
   gggagtcgga atgactggat gggggatggg tagaggatga ctggcacact tgtatagtat   2160
   gtaatgttgt gtacatatta tttatatggt tgtggctcaa ataaagttta attccaacac   2220
   aaaaaaaaaa aaaaaa                                                   2236
<210> 9
   <211> 2236
   <212> DNA
   <213> Homo sapiens
<400> 9
   gggacttcac cagtgctgcc atgctggccc caggctgtga gttggaccca gaccaagaag     60
   tggtgaggac aaggcctgaa gatgtgcctg cttcccattc aacttccacc atgattgtaa    120
   gtgtcctgag gcctcccagc catgcttcct gtacagcctg tggaactgtg acttttcaca    180
   tagtagagag aatggcttca gttatacctg cctccaatag gtccatgaga tctgacagga    240
   atacatatgt tggaaaaagg tttgttcacg ttaaaaatcc ttacttggat ttgatggatg    300
   aagacattct ctatcacttg gatttgggaa caaaaacaca caacctacca gcaatgtttg    360
   gagatgtaaa gtttgtctgt gtcggtggga gccccaacag aatgaaagca tttgcactgt    420
   ttatgcacaa ggagctcggg tttgaggaag ctgaagaaga cataaaagac atctgtgctg    480
   ggacagacag atactgtatg tacaaaaccg ggcctgtgct cgccatcagt cacggcatgg    540
   gcatcccctc catttctatt atgcttcatg aactcatcaa attactccac catgcacggt    600
   gctgcgatgt caccattatt agaatcggta catcaggggg aatagggatt gcaccaggga    660
   ctgttgtaat aacggatata gctgtagact ccttctttaa gccccggttt gaacaggtca    720
   ttttggacaa cattgtcacc cgaagtactg aactggacaa agaactgtct gaagaactgt    780
   tcaactgtag caaagaaatc cccaacttcc caaccctcgt tggacataca atgtgtacct    840
   atgattttta tgaaggccaa ggccgactag atggagcact gtgctccttt tccagagaaa    900
   aaaagttaga ctacttgaag agagcattta aagctggtgt caggaatatt gaaatggaat 960
   ctacagtgtt tgcagctatg tgtggactct gtggtctaaa agctgctgtg gtctgtgtga 1020
   cacttctcga cagactcgac tgtgatcaga tcaacttgcc tcatgatgtc ctggtggagt 1080
   accagcaacg gcctcagctc ctaatctcca acttcatcag acggcggctt ggactttgtg 1140
   actagacgtc ctaactgggc agcccaaccc tcccctgcaa gtttgtagct caagttgtaa 1200
   tgtgaaagtc atattttatt tgtggcattt ttatatagtt ctcatccaca tgctaaatgg 1260
   aaagacttta tgaaatcctt ctctcttaaa aaggaattta ttgtaaaaga atactcacac 1320
   taaattaaat tcaaatttca ttttagaata agttaactaa atcagtctaa taattaaaat 1380
   tttaaaactc ctggattatg acatttggag tttcatatgc agcattaatt aagctcacat 1440
   caaaatagac cagccatttg cacatagtta ccagtcactc tgtttctgaa accaatccag 1500
   aaattcatgt tagaacattg tcaggcactt ctaggtattg gacaagtgac agaaactgat 1560
   catccatatt caaaatattt acagaaaaaa gtgtcagtga aatgtaggtc tatatggaaa 1620
   agttaccatt aggaaaatat ggccatactg cttttttacc cctgcactat ttcagtgcac 1680
   cacagaatag gattcgagat ttacatatgc ctgtttgtga ttttatctca gaatataaca 1740
   actctggatt acattttcct ctcaactgaa aaccctcagt aatgcaaaga aaggccttcc 1800
   tttttcttca gaatcacaag tgactaaatt gccttctagc cattttttct tgtaaaaaaa 1860
   gtgttttgag gaggctgtat tttttattcc atttcaatat taaccaaata ggctgagaaa 1920
   ttcatagcaa ttgaattaag acttgagttc aagatgttaa aggaatcctt ttgtatgact 1980
   tgtccagacc ggcctattgc cctacccgtt tgattctctg ggaggcatgg tgctcctcct 2040
   ggctgcacat gctctctgtg aactgatcgc atcttgggtt cttctcaatt tgacatactt 2100
   gggagtcgga atgactggat gggggatggg tagaggatga ctggcacact tgtatagtat 2160
   gtaatgttgt gtacatatta tttatatggt tgtggctcaa ataaagttta attccaacac 2220
   aaaaaaaaaa aaaaaa 2236
<210> 10
   <211> 310
   <212> PRT
   <213> Homo sapiens
<400> 10
   Met Ala Ala Thr Gly Ala Asn Ala Glu Lys Ala Glu Ser His Asn Asp
   1 5 10 15
   Cys Pro Val Arg Leu Leu Asn Pro Asn Ile Ala Lys Met Lys Glu Asp
   20 25 30
   Ile Leu Tyr His Phe Asn Leu Thr Thr Ser Arg His Asn Phe Pro Ala
   35 40 45
   Leu Phe Gly Asp Val Lys Phe Val Cys Val Gly Gly Ser Pro Ser Arg
   50 55 60
   Met Lys Ala Phe Ile Arg Cys Val Gly Ala Glu Leu Gly Leu Asp Cys
   65 70 75 80
   Pro Gly Arg Asp Tyr Pro Asn Ile Cys Ala Gly Thr Asp Arg Tyr Ala
   85 90 95
   Met Tyr Lys Val Gly Pro Val Leu Ser Val Ser His Gly Met Gly Ile
   100 105 110
   Pro Ser Ile Ser Ile Met Leu His Glu Leu Ile Lys Leu Leu Tyr Tyr
   115 120 125
   Ala Arg Cys Ser Asn Val Thr Ile Ile Arg Ile Gly Thr Ser Gly Gly
   130 135 140
   Ile Gly Leu Glu Pro Gly Thr Val Val Ile Thr Glu Gln Ala Val Asp
   145 150 155 160
   Thr Cys Phe Lys Ala Glu Phe Glu Gln Ile Val Leu Gly Lys Arg Val
   165 170 175
   Ile Arg Lys Thr Asp Leu Asn Lys Lys Leu Val Gln Glu Leu Leu Leu
   180 185 190
   Cys Ser Ala Glu Leu Ser Glu Phe Thr Thr Val Val Gly Asn Thr Met
   195 200 205
   Cys Thr Leu Asp Phe Tyr Glu Gly Gln Gly Arg Leu Asp Gly Ala Leu
   210 215 220
   Cys Ser Tyr Thr Glu Lys Asp Lys Gln Ala Tyr Leu Glu Ala Ala Tyr
   225 230 235 240
   Ala Ala Gly Val Arg Asn Ile Glu Met Glu Ser Ser Val Phe Ala Ala
   245 250 255
   Met Cys Ser Ala Cys Gly Leu Gln Ala Ala Val Val Cys Val Thr Leu
   260 265 270
   Leu Asn Arg Leu Glu Gly Asp Gln Ile Ser Ser Pro Arg Asn Val Leu
   275 280 285
   Ser Glu Tyr Gln Gln Arg Pro Gln Arg Leu Val Ser Tyr Phe Ile Lys
   290 295 300
   Lys Lys Leu Ser Lys Ala
   305 310
<210> 11
   <211> 317
   <212> PRT
   <213> Homo sapiens
<400> 11
   Met Ala Ser Val Ile Pro Ala Ser Asn Arg Ser Met Arg Ser Asp Arg
   1 5 10 15
   Asn Thr Tyr Val Gly Lys Arg Phe Val His Val Lys Asn Pro Tyr Leu
   20 25 30
   Asp Leu Met Asp Glu Asp Ile Leu Tyr His Leu Asp Leu Gly Thr Lys
   35 40 45
   Thr His Asn Leu Pro Ala Met Phe Gly Asp Val Lys Phe Val Cys Val
   50 55 60
   Gly Gly Ser Pro Asn Arg Met Lys Ala Phe Ala Leu Phe Met His Lys
   65 70 75 80
   Glu Leu Gly Phe Glu Glu Ala Glu Glu Asp Ile Lys Asp Ile Cys Ala
   85 90 95
   Gly Thr Asp Arg Tyr Cys Met Tyr Lys Thr Gly Pro Val Leu Ala Ile
   100 105 110
   Ser His Gly Met Gly Ile Pro Ser Ile Ser Ile Met Leu His Glu Leu
   115 120 125
   Ile Lys Leu Leu His His Ala Arg Cys Cys Asp Val Thr Ile Ile Arg
   130 135 140
   Ile Gly Thr Ser Gly Gly Ile Gly Ile Ala Pro Gly Thr Val Val Ile
   145 150 155 160
   Thr Asp Ile Ala Val Asp Ser Phe Phe Lys Pro Arg Phe Glu Gln Val
   165 170 175
   Ile Leu Asp Asn Ile Val Thr Arg Ser Thr Glu Leu Asp Lys Glu Leu
   180 185 190
   Ser Glu Glu Leu Phe Asn Cys Ser Lys Glu Ile Pro Asn Phe Pro Thr
   195 200 205
   Leu Val Gly His Thr Met Cys Thr Tyr Asp Phe Tyr Glu Gly Gln Gly
   210 215 220
   Arg Leu Asp Gly Ala Leu Cys Ser Phe Ser Arg Glu Lys Lys Leu Asp
   225 230 235 240
   Tyr Leu Lys Arg Ala Phe Lys Ala Gly Val Arg Asn Ile Glu Met Glu
   245 250 255
   Ser Thr Val Phe Ala Ala Met Cys Gly Leu Cys Gly Leu Lys Ala Ala
   260 265 270
   Val Val Cys Val Thr Leu Leu Asp Arg Leu Asp Cys Asp Gln Ile Asn
   275 280 285
   Leu Pro His Asp Val Leu Val Glu Tyr Gln Gln Arg Pro Gln Leu Leu
   290 295 300
   Ile Ser Asn Phe Ile Arg Arg Arg Leu Gly Leu Cys Asp
   305 310 315
   1

## Description

### BACKGROUND OF THE INVENTION

The *Drosophila Melanogaster* Armadillo/beta-catenin protein is implicated in multiple cellular functions. The protein functions in cell signaling via the Wingless (Wg)/Wnt signaling pathway. It also functions as a cell adhesion protein at the cell membrane in a complex with E-cadherin and alpha-catenin (Cox et al. (1996) J. Cell Biol. 134: 133-148; Godt and Tepass (1998) Nature 395: 387-391; White et al. (1998) J Cell biol. 140:183-195). These two roles of beta -catenin can be separated from each other (Orsulic and Peifer (1996) J. Cell Biol. 134: 1283-1300; Sanson et al. (1996) Nature 383: 627-630).

In Wingless cell signaling, beta -catenin levels are tightly regulated by a complex containing APC, Axin, and GSK3 beta /SGG/ZW3 (Peifer et al. (1994) Development 120: 369-380).

The Wingless/ beta -catenin signaling pathway is frequently mutated in human cancers, particularly those of the colon. Mutations in the tumor suppressor gene APC, as well as point mutations in beta -catenin itself lead to the stabilization of the beta -catenin protein and inappropriate activation of this pathway.

Uridine phosphorylase is the key enzyme of pyrimidine salvage in mammalian hosts and many other organisms. In the presence of orthophosphate, uridine phosphorylase catalyzes the reversible phosphorolysis of uridine to free bases and ribose-1-phosphate or deoxyribose-1-phosphate. The enzyme has an important role in the metabolism of pyrimidine analogs used in cancer chemotherapy. Uridine phosphorylase 1 (UPP1) was identified from a human colorectal tumor cell line cDNA library (Watanabe and Uchida (1995) Biochem. Biophys. Res. Commun.). Uridine phosphorylase 2 (UPP2) is another uridine phosphorylase with broad substrate specificity (Johansson M (2003) Biochem Biophys Res Commun. 307:41-6).

The ability to manipulate the genomes of model organisms such as *Drosophila* provides a powerful means to analyze biochemical processes that, due to significant evolutionary conservation, have direct relevance to more complex vertebrate organisms. Due to a high level of gene and pathway conservation, the strong similarity of cellular processes, and the functional conservation of genes between these model organisms and mammals, identification of the involvement of novel genes in particular pathways and their functions in such model organisms can directly contribute to the understanding of the correlative pathways and methods of modulating them in mammals (see, for example, Mechler BM et al., 1985 EMBO J 4:1551-1557; Gateff E. 1982 Adv. Cancer Res. 37: 33-74; Watson KL., et al., 1994 J Cell Sci. 18: 19-33; Miklos GL, and Rubin GM. 1996 Cell 86:521-529; Wassarman DA, et al., 1995 Curr Opin Gen Dev 5: 44-50; and Booth DR. 1999 Cancer Metastasis Rev. 18: 261-284). For example, a genetic screen can be carried out in an invertebrate model organism having underexpression (e.g. knockout) or overexpression of a gene (referred to as a "genetic entry point") that yields a visible phenotype. Additional genes are mutated in a random or targeted manner. When a gene mutation changes the original phenotype caused by the mutation in the genetic entry point, the gene is identified as a "modifier" involved in the same or overlapping pathway as the genetic entry point. When the genetic entry point is an ortholog of a human gene implicated in a disease pathway, such as beta catenin, modifier genes can be identified that may be attractive candidate targets for novel therapeutics.

WO 03/052068 A2 identifies human MBCAT genes as modulators of the beta-catenin pathway. Methods for identifying modulators of beta-catenin, comprising screening for agents that modulate the activity of MBCAT are also disclosed.

US 6,066,500 discloses antisense compounds, compositions and methods for modulating the expression of beta-catenin. The compositions comprise antisense compounds, particularly antisense oligonucleotides, targeted to nucleic acids encoding beta-catenin. Methods of using these compounds for modulation of beta catenin expression and for the treatment of diseases associated with beta catenin are also disclosed.

US 2002 015 943 A1 relates to the discovery that truncations in the tumour suppressor APC cause high levels of nuclear beta-catenin to accumulate by trapping it within the nucleus.

Ashour et al., (Cancer Research Vol 55 (1 March 1995) pages 1092 -1098) demonstrated that 5-(benzyloxybenzyl)barbituric acid (BBBA), a specific inhibitor of UP, enhances the anti-tumour activity of 5-fluoro-2'-deoxyuridine (5-FU) both *in vitro* an *in vivo.*

Findenig et al., (Oncology Research 8:5 (1996) pages 189-196) describes the effect of combinations of 5-FU and AZT for the treatment of human colon tumour cells.

Liu et al. (Cancer Research Vol 58 (1998) pages 5418 to 5424) investigated the expression, characterisation and detection of human UP and variant UP activity in selected human tumours. Studies suggested that 5-benzylscyclouridine (BAU), an inhibitor of UP can be used to modulate 5-FU therapy.

Monga et al., (Gastroenterology vol 124 (2003) pages 202 to 216) discusses beta-catenin antisense studies in embryonic liver cultures and its role in proliferation, apoptosis and lineage specification. It was shown that the PMO antisense to beta-catenin effectively inhabited synthesis of beta catenin protein.

WO 01/60 985 discusses human uridine phosphorylase nucleic acids and proteins encoded thereby.

Mengping Liu, et al. (Cancer Research 58 (1998), 5418-5424) discusses the expression, characterization, and detection of human uridine phosphorylase and identification of variant uridine phosphorolytic activity in selected human tumors.

### SUMMARY OF THE INVENTION

We have discovered genes that modify the beta catenin pathway in *Drosophilia,* and identified their human orthologs, hereinafter referred to as Uridine Phosphorylase (UP). The invention provides methods for utilizing these beta catenin modifier genes and polypeptides to identify UP-modulating agents that are candidate therapeutic agents that can be used in the treatment of disorders associated with defective or impaired beta catenin function and/or UP function. Preferred UP-modulating agents specifically bind to UP polypeptides and restore beta catenin function. Other preferred UP-modulating agents are nucleic acid modulators such as antisense oligomers and RNAi that repress UP gene expression or product activity by, for example, binding to and inhibiting the respective nucleic acid (i.e. DNA or mRNA).

UP modulating agents may be evaluated by any convenient *in vitro* or *in vivo* assay for molecular interaction with a UP polypeptide or nucleic acid. In one embodiment, candidate UP modulating agents are tested with an in vitro assay system comprising a UP polypeptide or nucleic acid. Agents that produce a change in the activity of the assay system relative to controls are identified as candidate beta catenin modulating agents. The assay system may be cell-based or cell-free. UP-modulating agents include UP related proteins (e.g. dominant negative mutants, and biotherapeutics); UP -specific antibodies; UP -specific antisense oligomers and other nucleic acid modulators; and chemical agents that specifically bind to or interact with UP or compete with UP binding partner (e.g. by binding to a UP binding partner). In one specific embodiment, a small molecule modulator is identified using a uridine phosphorylase assay. In specific embodiments, the screening assay system is selected from a binding assay, an apoptosis assay, a cell proliferation assay, an angiogenesis assay, and a hypoxic induction assay.

In another embodiment, candidate beta catenin pathway modulating agents are further tested using a second in vitro assay system that detects changes in the beta catenin pathway, such as angiogenic, apoptotic, or cell proliferation changes produced by the originally identified candidate agent or an agent derived from the original agent.

The invention further provides in vitro methods for modulating the UP function and/or the beta catenin pathway in a mammalian cell by contacting the mammalian cell with an agent that specifically binds a UP polypeptide or nucleic acid. Moreover, the invention provides certain agents that specifically modulate the expression and/or phosphorylase activity of UP for use in the treatment of specific cancers relating to a defect in beta catenin function. The agent is a UP-specific antisense oligomer, a UP-specific double stranded RNA, or an UP-specific antibody and may be for the treament of a mammalian animal predetermined to have a pathology associated with the beta catenin pathway.

### DETAILED DESCRIPTION OF THE INVENTION

In a screen to identify enhancers and suppressors of the Wg signaling pathway, we generated activated beta -catenin models in *Drosophila* based on human tumor data (Polakis (2000) Genes and Development 14: 1837-1851). We identified modifiers of the Wg pathway and identified their orthologs. The CG6330 gene was identified as a modifier of the beta catenin pathway, followed by identification of its vertebrate orthologs. Accordingly, vertebrate orthologs of these modifiers, and preferably the human orthologs, UP genes (i.e., nucleic acids and polypeptides) are attractive drug targets for the treatment of pathologies associated with a defective beta catenin signaling pathway, such as cancer.

In vitro methods of assessing UP function are provided herein. Modulation of the UP or their respective binding partners is useful for understanding the association of the beta catenin pathway and its members in normal and disease conditions and for developing diagnostics and therapeutic modalities for beta catenin related pathologies. UP-modulating agents that act by inhibiting or enhancing UP expression, directly or indirectly, for example, by affecting a UP function such as enzymatic (e.g., catalytic) or binding activity, can be identified using methods provided herein. UP modulating agents are useful in diagnosis, therapy and pharmaceutical development.

### Nucleic acids and polypeptides of the invention

Sequences related to UP nucleic acids and polypeptides that can be used in the invention are disclosed in Genbank (referenced by Genbank identifier (GI) number) as GI#s 31742506 (SEQ ID NO: 1), 12655106 (SEQ ID NO:2), 28422563 (SEQ ID NO:3), 34782821 (SEQ ID NO:4), 23272324 (SEQ ID NO:5), 27597095 (SEQ ID NO:6), 4156143 (SEQ ID NO:7), 34222223 (SEQ ID NO:8), and 34191337 (SEQ ID NO:9) for nucleic acid, and GI#s 4507839 (SEQ ID NO:10) and 27597096 (SEQ ID NO:11) for polypeptides.

The term "UP polypeptide" refers to a full-length UP protein or a functionally active fragment or derivative thereof. A "functionally active" UP fragment or derivative exhibits one or more functional activities associated with a full-length, wild-type UP protein, such as antigenic or immunogenic activity, enzymatic activity, ability to bind natural cellular substrates, etc. The functional activity of UP proteins, derivatives and fragments can be assayed by various methods known to one skilled in the art (Current Protocols in Protein Science (1998) Coligan et al., eds., John Wiley & Sons, Inc., Somerset, New Jersey) and as further discussed below. In one embodiment, a functionally active UP polypeptide is a UP derivative capable of rescuing defective endogenous UP activity, such as in cell based or animal assays; the rescuing derivative may be from the same or a different species. For purposes herein, functionally active fragments also include those fragments that comprise one or more structural domains of a UP, such as a binding domain. Protein domains can be identified using the PFAM program (Bateman A., et al., Nucleic Acids Res, 1999, 27:260-2). For example, the phosphorylase domain (PFAM 01048) of UP from GI#s 4507839 and 27597096 (SEQ ID NO: 10 and 11, respectively) is located respectively at approximately amino acid residues 54 to 303 and 60-308. Methods for obtaining UP polypeptides are also further described below. In some embodiments, preferred fragments are functionally active, domain-containing fragments comprising at least 25 contiguous amino acids, preferably at least 50, more preferably 75, and most preferably at least 100 contiguous amino acids of a UP. In further preferred embodiments, the fragment comprises the entire functionally active domain.

The term "UP nucleic acid" refers to a DNA or RNA molecule that encodes a UP polypeptide. Preferably, the UP polypeptide or nucleic acid or fragment thereof is from a human, but can also be an ortholog, or derivative thereof with at least 70% sequence identity, preferably at least 80%, more preferably 85%, still more preferably 90%, and most preferably at least 95% sequence identity with human UP. Methods of identifying orthlogs are known in the art. Normally, orthologs in different species retain the same function, due to presence of one or more protein motifs and/or 3-dimensional structures. Orthologs are generally identified by sequence homology analysis, such as BLAST analysis, usually using protein bait sequences. Sequences are assigned as a potential ortholog if the best hit sequence from the forward BLAST result retrieves the original query sequence in the reverse BLAST (Huynen MA and Bork P, Proc Natl Acad Sci (1998) 95:5849-5856; Huynen MA et al., Genome Research (2000) 10:1204-1210). Programs for multiple sequence alignment, such as CLUSTAL (Thompson JD et al, 1994, Nucleic Acids Res 22:4673-4680) may be used to highlight conserved regions and/or residues of orthologous proteins and to generate phylogenetic trees. In a phylogenetic tree representing multiple homologous sequences from diverse species (e.g., retrieved through BLAST analysis), orthologous sequences from two species generally appear closest on the tree with respect to all other sequences from these two species. Structural threading or other analysis of protein folding (e.g., using software by ProCeryon, Biosciences, Salzburg, Austria) may also identify potential orthologs. In evolution, when a gene duplication event follows speciation, a single gene in one species, such as *Drosophila,* may correspond to multiple genes (paralogs) in another, such as human. As used herein, the term "orthologs" encompasses paralogs. As used herein, "percent (%) sequence identity" with respect to a subject sequence, or a specified portion of a subject sequence, is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program WU-BLAST-2.0a19 (Altschul et al., J. Mol. Biol. (1997) 215:403-410) with all the search parameters set to default values. The HSP S and HSP S2 parameters are dynamic values and are established by the program itself depending upon the composition of the particular sequence and composition of the particular database against which the sequence of interest is being searched. A % identity value is determined by the number of matching identical nucleotides or amino acids divided by the sequence length for which the percent identity is being reported. "Percent (%) amino acid sequence similarity" is determined by doing the same calculation as for determining % amino acid sequence identity, but including conservative amino acid substitutions in addition to identical amino acids in the computation.

A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine and glycine.

Alternatively, an alignment for nucleic acid sequences is provided by the local homology algorithm of Smith and Waterman (Smith and Waterman, 1981, Advances in Applied Mathematics 2:482-489; database: European Bioinformatics Institute; Smith and Waterman, 1981, J. of Molec.Biol., 147:195-197; Nicholas et al., 1998, "A Tutorial on Searching Sequence Databases and Sequence Scoring Methods" (www.psc.edu) and references cited therein.; W.R. Pearson, 1991, Genomics 11:635-650). This algorithm can be applied to amino acid sequences by using the scoring matrix developed by Dayhoff (Dayhoff: Atlas of Protein Sequences and Structure, M. O. Dayhoff ed., 5 suppl. 3:353-358, National Biomedical Research Foundation, Washington, D.C., USA), and normalized by Gribskov (Gribskov 1986 Nucl. Acids Res. 14(6):6745-6763). The Smith-Waterman algorithm may be employed where default parameters are used for scoring (for example, gap open penalty of 12, gap extension penalty of two). From the data generated, the "Match" value reflects "sequence identity."

Derivative nucleic acid molecules of the subject nucleic acid molecules include sequences that hybridize to the nucleic acid sequence of a UP. The stringency of hybridization can be controlled by temperature, ionic strength, pH, and the presence of denaturing agents such as formamide during hybridization and washing. Conditions routinely used are set out in readily available procedure texts (*e.g*., Current Protocol in Molecular Biology, Vol. 1, Chap. 2.10, John Wiley & Sons, Publishers (1994); Sambrook et al., Molecular Cloning, Cold Spring Harbor (1989)). In some embodiments, a nucleic acid molecule of the invention is capable of hybridizing to a nucleic acid molecule containing the nucleotide sequence of a UP under high stringency hybridization conditions that are: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate ant 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1h in a solution containing 0.1X SSC and 0.1% SDS (sodium dodecyl sulfate).

In other embodiments, moderately stringent hybridization conditions are used that are: pretreatment of filters containing nucleic acid for 6 h at 40° C in a solution containing 35% formamide, 5X SSC, 50 mM Tris-HCl (pH7.5), 5mM EDTA, 0.1% PVP, 0.1% Ficoll, 1% BSA, and 500 µg/ml denatured salmon sperm DNA; hybridization for 18-20h at 40° C in a solution containing 35% formamide, 5X SSC, 50 mM Tris-HCl (pH7.5), 5mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1% SDS.

Alternatively, low stringency conditions can be used that are: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

### Isolation, Production, Expression, and Mis-expression of UP Nucleic Acids and Polypeptides

UP nucleic acids and polypeptides are useful for identifying and testing agents that modulate UP function and for other applications related to the involvement of UP in the beta catenin pathway. UP nucleic acids and derivatives and orthologs thereof may be obtained using any available method. For instance, techniques for isolating cDNA or genomic DNA sequences of interest by screening DNA libraries or by using polymerase chain reaction (PCR) are well known in the art. In general, the particular use for the protein will dictate the particulars of expression, production, and purification methods. For instance, production of proteins for use in screening for modulating agents may require methods that preserve specific biological activities of these proteins, whereas production of proteins for antibody generation may require structural integrity of particular epitopes. Expression of proteins to be purified for screening or antibody production may require the addition of specific tags (*e.g*., generation of fusion proteins). Overexpression of a UP protein for assays used to assess UP function, such as involvement in cell cycle regulation or hypoxic response, may require expression in eukaryotic cell lines capable of these cellular activities. Techniques for the expression, production, and purification of proteins are well known in the art; any suitable means therefore may be used (e.g., Higgins SJ and Hames BD (eds.) Protein Expression: A Practical Approach, Oxford University Press Inc., New York 1999; Stanbury PF et al., Principles of Fermentation Technology, 2nd edition, Elsevier Science, New York, 1995; Doonan S (ed.) Protein Purification Protocols, Humana Press, New Jersey, 1996; Coligan JE et al, Current Protocols in Protein Science (eds.), 1999, John Wiley & Sons, New York). In particular embodiments, recombinant UP is expressed in a cell line known to have defective beta catenin function. The recombinant cells are used in cell-based screening assay systems of the invention, as described further below.

The nucleotide sequence encoding a UP polypeptide can be inserted into any appropriate expression vector. The necessary transcriptional and translational signals, including promoter/enhancer element, can derive from the native UP gene and/or its flanking regions or can be heterologous. A variety of host-vector expression systems may be utilized, such as mammalian cell systems infected with virus (*e.g*. vaccinia virus, adenovirus, *etc*.); insect cell systems infected with virus (*e.g.* baculovirus); microorganisms such as yeast containing yeast vectors, or bacteria transformed with bacteriophage, plasmid, or cosmid DNA. An isolated host cell strain that modulates the expression of, modifies, and/or specifically processes the gene product may be used.

To detect expression of the UP gene product, the expression vector can comprise a promoter operably linked to a UP gene nucleic acid, one or more origins of replication, and, one or more selectable markers (*e.g.* thymidine kinase activity, resistance to antibiotics, *etc*.). Alternatively, recombinant expression vectors can be identified by assaying for the expression of the UP gene product based on the physical or functional properties of the UP protein in *in vitro* assay systems (*e.g*. immunoassays).

The UP protein, fragment, or derivative may be optionally expressed as a fusion, or chimeric protein product (i.e. it is joined via a peptide bond to a heterologous protein sequence of a different protein), for example to facilitate purification or detection. A chimeric product can be made by ligating the appropriate nucleic acid sequences encoding the desired amino acid sequences to each other using standard methods and expressing the chimeric product. A chimeric product may also be made by protein synthetic techniques, *e.g.* by use of a peptide synthesizer (Hunkapiller et al., Nature (1984) 310:105-111).

Once a recombinant cell that expresses the UP gene sequence is identified, the gene product can be isolated and purified using standard methods (*e.g*. ion exchange, affinity, and gel exclusion chromatography; centrifugation; differential solubility; electrophoresis). Alternatively, native UP proteins can be purified from natural sources, by standard methods (*e.g*. immunoaffinity purification). Once a protein is obtained, it may be quantified and its activity measured by appropriate methods, such as immunoassay, bioassay, or other measurements of physical properties, such as crystallography.

The methods of this invention may also use cells that have been engineered for altered expression (mis-expression) of UP or other genes associated with the beta catenin pathway. As used herein, mis-expression encompasses ectopic expression, overexpression, under-expression, and non-expression (*e.g.* by gene knock-out or blocking expression that would otherwise normally occur).

### Modulating Agents

The invention provides methods to identify agents that interact with and/or modulate the function of UP and/or the beta catenin pathway. Modulating agents identified by the methods are also described herein. Such agents are useful in a variety of diagnostic and therapeutic applications associated with the beta catenin pathway, as well as in further analysis of the UP protein and its contribution to the beta catenin pathway. Accordingly, the invention also provides methods for modulating the beta catenin pathway comprising the step of specifically modulating UP activity by administering a UP-interacting or -modulating agent.

As used herein, an "UP-modulating agent" is any agent that modulates UP function, for example, an agent that interacts with UP to inhibit or enhance UP activity or otherwise affect normal UP function. UP function can be affected at any level, including transcription, protein expression, protein localization, and cellular or extra-cellular activity. In a preferred embodiment, the UP - modulating agent specifically modulates the function of the UP. The phrases "specific modulating agent", "specifically modulates", etc., are used herein to refer to modulating agents that directly bind to the UP polypeptide or nucleic acid, and preferably inhibit, enhance, or otherwise alter, the function of the UP. These phrases also encompass modulating agents that alter the interaction of the UP with a binding partner, substrate, or cofactor (e.g. by binding to a binding partner of a UP, or to a protein/binding partner complex, and altering UP function). The UP-modulating agent may be a modulator of the beta catenin pathway (e.g. it restores and/or upregulates beta catenin function) and thus is also a beta catenin-modulating agent.

UP-modulating agents include small molecule compounds; UP-interacting proteins, including antibodies and other biotherapeutics; and nucleic acid modulators such as antisense and RNA inhibitors. The modulating agents may be formulated in pharmaceutical compositions, for example, as compositions that may comprise other active ingredients, as in combination therapy, and/or suitable carriers or excipients. Techniques for formulation and administration of the compounds may be found in "Remington's Pharmaceutical Sciences" Mack Publishing Co., Easton, PA, 19th edition.

### Small molecule modulators

Small molecules are often preferred to modulate function of proteins with enzymatic function, and/or containing protein interaction domains. Chemical agents, referred to in the art as "small molecule" compounds are typically organic, non-peptide molecules, having a molecular weight up to 10,000, preferably up to 5,000, more preferably up to 1,000, and most preferably up to 500 daltons. This class of modulators includes chemically synthesized molecules, for instance, compounds from combinatorial chemical libraries. Synthetic compounds may be rationally designed or identified based on known or inferred properties of the UP protein or may be identified by screening compound libraries. Alternative appropriate modulators of this class are natural products, particularly secondary metabolites from organisms such as plants or fungi, which can also be identified by screening compound libraries for UP-modulating activity. Methods for generating and obtaining compounds are well known in the art (Schreiber SL, Science (2000) 151: 1964-1969; Radmann J and Gunther J, Science (2000) 151:1947-1948).

Small molecule modulators identified from screening assays, as described below, can be used as lead compounds from which candidate clinical compounds may be designed, optimized, and synthesized. Such clinical compounds may have utility in treating pathologies associated with the beta catenin pathway. The activity of candidate small molecule modulating agents may be improved several-fold through iterative secondary functional validation, as further described below, structure determination, and candidate modulator modification and testing. Additionally, candidate clinical compounds are generated with specific regard to clinical and pharmacological properties. For example, the reagents may be derivatized and re-screened using *in vitro* and *in vivo* assays to optimize activity and minimize toxicity for pharmaceutical development.

### Protein Modulators

Specific UP-interacting proteins are useful in a variety of diagnostic and therapeutic applications related to the beta catenin pathway and related disorders, as well as in validation assays for other UP-modulating agents. UP-interacting proteins may affect normal UP function, including transcription, protein expression, protein localization, and cellular or extra-cellular activity. UP-interacting proteins are useful in detecting and providing information about the function of UP proteins, as is relevant to beta catenin related disorders, such as cancer (e.g., for diagnostic means).

An UP-interacting protein may be endogenous, i.e. one that naturally interacts genetically or biochemically with a UP, such as a member of the UP pathway that modulates UP expression, localization, and/or activity. UP-modulators include dominant negative forms of UP-interacting proteins and of UP proteins themselves. Yeast two-hybrid and variant screens offer preferred methods for identifying endogenous UP-interacting proteins (Finley, R. L. et al. (1996) in DNA Cloning-Expression Systems: A Practical Approach, eds. Glover D. & Hames B. D (Oxford University Press, Oxford, England), pp. 169-203; Fashema SF et al., Gene (2000) 250:1-14; Drees BL Curr Opin Chem Biol (1999) 3:64-70; Vidal M and Legrain P Nucleic Acids Res (1999) 27:919-29; and U.S. Pat. No. 5,928,868). Mass spectrometry is an alternative preferred method for the elucidation of protein complexes (reviewed in, e.g., Pandley A and Mann M, Nature (2000) 405:837-846; Yates JR 3rd, Trends Genet (2000) 16:5-8).

An UP-interacting protein may be an exogenous protein, such as a UP-specific antibody or a T-cell antigen receptor (see, e.g., Harlow and Lane (1988) Antibodies, A Laboratory Manual, Cold Spring Harbor Laboratory; Harlow and Lane (1999) Using antibodies: a laboratory manual. Cold Spring Harbor, NY: Cold Spring Harbor Laboratory Press). UP antibodies are further discussed below.

A UP-interacting protein may specifically bind a UP protein. Alternatively, a UP-modulating agent may bind a UP substrate, binding partner, or cofactor.

### Antibodies

In one embodiment, the protein modulator is a UP specific antibody agonist or antagonist. The antibodies have therapeutic and diagnostic utilities, and can be used in screening assays to identify UP modulators. The antibodies can also be used in dissecting the portions of the UP pathway responsible for various cellular responses and in the general processing and maturation of the UP.

Antibodies that specifically bind UP polypeptides can be generated using known methods. Preferably the antibody is specific to a mammalian ortholog of UP polypeptide, and more preferably, to human UP. Antibodies may be polyclonal, monoclonal (mAbs), humanized or chimeric antibodies, single chain antibodies, Fab fragments, F(ab').sub.2 fragments, fragments produced by a FAb expression library, anti-idiotypic (anti-Id) antibodies, and epitope-binding fragments of any of the above. Epitopes of UP which are particularly antigenic can be selected, for example, by routine screening of UP polypeptides for antigenicity or by applying a theoretical method for selecting antigenic regions of a protein (Hopp and Wood (1981), Proc. Nati. Acad. Sci. U.S.A. 78:3824-28; Hopp and Wood, (1983) Mol. Immunol. 20:483-89; Sutcliffe et al., (1983) Science 219:660-66) to the amino acid sequence of a UP. Monoclonal antibodies with affinities of 10⁸ M^{~1} preferably 10⁹ M^{~1} to 10¹⁰ M^{~1}, or stronger can be made by standard procedures as described (Harlow and Lane, *supra;* Goding (1986) Monoclonal Antibodies: Principles and Practice (2d ed) Academic Press, New York; and U.S. Pat. Nos. 4,381,292; 4,451,570; and 4,618,577). Antibodies may be generated against crude cell extracts of UP or substantially purified fragments thereof. If UP fragments are used, they preferably comprise at least 10, and more preferably, at least 20 contiguous amino acids of a UP protein. In a particular embodiment, UP-specific antigens and/or immunogens are coupled to carrier proteins that stimulate the immune response. For example, the subject polypeptides are covalently coupled to the keyhole limpet hemocyanin (KLH) carrier, and the conjugate is emulsified in Freund's complete adjuvant, which enhances the immune response. An appropriate immune system such as a laboratory rabbit or mouse is immunized according to conventional protocols.

The presence of UP-specific antibodies is assayed by an appropriate assay such as a solid phase enzyme-linked immunosorbant assay (ELISA) using immobilized corresponding UP polypeptides. Other assays, such as radioimmunoassays or fluorescent assays might also be used.

Chimeric antibodies specific to UP polypeptides can be made that contain different portions from different animal species. For instance, a human immunoglobulin constant region may be linked to a variable region of a murine mAb, such that the antibody derives its biological activity from the human antibody, and its binding specificity from the murine fragment. Chimeric antibodies are produced by splicing together genes that encode the appropriate regions from each species (Morrison et al., Proc. Natl. Acad. Sci. (1984) 81:6851-6855; Neuberger et al., Nature (1984) 312:604-608; Takeda et al., Nature (1985) 31:452-454). Humanized antibodies, which are a form of chimeric antibodies, can be generated by grafting complementary-determining regions (CDRs) (Carlos, T. M., J. M. Harlan. 1994. Blood 84:2068-2101) of mouse antibodies into a background of human framework regions and constant regions by recombinant DNA technology (Riechmann LM, et al., 1988 Nature 323: 323-327). Humanized antibodies contain ~10% murine sequences and ~90% human sequences, and thus further reduce or eliminate immunogenicity, while retaining the antibody specificities (Co MS, and Queen C. 1991 Nature 351: 501-501; Morrison SL. 1992 Ann. Rev. Immun. 10:239-265). Humanized antibodies and methods of their production are well-known in the art (U.S. Pat. Nos. 5,530,101, 5,585,089, 5,693,762, and 6,180,370).

UP-specific single chain antibodies which are recombinant, single chain polypeptides formed by linking the heavy and light chain fragments of the Fv regions via an amino acid bridge, can be produced by methods known in the art (U.S. Pat. No. 4,946,778; Bird, Science (1988) 242:423-426; Huston et al., Proc. Natl. Acad. Sci. USA (1988) 85:5879-5883; and Ward et al., Nature (1989) 334:544-546).

Other suitable techniques for antibody production involve in vitro exposure of lymphocytes to the antigenic polypeptides or alternatively to selection of libraries of antibodies in phage or similar vectors (Huse et al., Science (1989) 246:1275-1281). As used herein, T-cell antigen receptors are included within the scope of antibody modulators (Harlow and Lane, 1988, *supra*).

The polypeptides and antibodies of the present invention may be used with or without modification. Frequently, antibodies will be labeled by joining, either covalently or non-covalently, a substance that provides for a detectable signal, or that is toxic to cells that express the targeted protein (Menard S, et al., Int J. Biol Markers (1989) 4:131-134). A wide variety of labels and conjugation techniques are known and are reported extensively in both the scientific and patent literature. Suitable labels include radionuclides, enzymes, substrates, cofactors, inhibitors, fluorescent moieties, fluorescent emitting lanthanide metals, chemiluminescent moieties, bioluminescent moieties, magnetic particles, and the like (U.S. Pat. Nos. 3,817,837; 3,850,752; 3,939,350; 3,996,345; 4,277,437; 4,275,149; and 4,366,241). Also, recombinant immunoglobulins may be produced (U.S. Pat. No. 4,816,567). Antibodies to cytoplasmic polypeptides may be delivered and reach their targets by conjugation with membrane-penetrating toxin proteins (U.S. Pat. No. 6,086,900).

If used therapeutically in a patient, the antibodies of the subject invention may be typically administered parenterally, when possible at the target site, or intravenously. The therapeutically effective dose and dosage regimen is determined by clinical studies. Typically, the amount of antibody to be administered is in the range of about 0.1 mg/kg -to about 10 mg/kg of patient weight. For parenteral administration, the antibodies are formulated in a unit dosage injectable form (e.g., solution, suspension, emulsion) in association with a pharmaceutically acceptable vehicle. Such vehicles are inherently nontoxic and non-therapeutic. Examples are water, saline, Ringer's solution, dextrose solution, and 5% human serum albumin. Nonaqueous vehicles such as fixed oils, ethyl oleate, or liposome carriers may also be used. The vehicle may contain minor amounts of additives, such as buffers and preservatives, which enhance isotonicity and chemical stability or otherwise enhance therapeutic potential. The antibodies' concentrations in such vehicles are typically in the range of about 1 mg/ml to about10 mg/ml. Immunotherapeutic methods are further described in the literature (US Pat. No. 5,859,206; WO0073469).

### Nucleic Acid Modulators

Other preferred UP-modulating agents comprise nucleic acid molecules, such as antisense oligomers or double stranded RNA (dsRNA), which generally inhibit UP activity. Preferred nucleic acid modulators interfere with the function of the UP nucleic acid such as DNA replication, transcription, translocation of the UP RNA to the site of protein translation, translation of protein from the UP RNA, splicing of the UP RNA to yield one or more mRNA species, or catalytic activity which may be engaged in or facilitated by the UP RNA.

In one embodiment, the antisense oligomer is an oligonucleotide that is sufficiently complementary to a UP mRNA to bind to and prevent translation, preferably by binding to the 5' untranslated region. UP-specific antisense oligonucleotides, preferably range from at least 6 to about 200 nucleotides. In some embodiments the oligonucleotide is preferably at least 10, 15, or 20 nucleotides in length. In other embodiments, the oligonucleotide is preferably less than 50, 40, or 30 nucleotides in length. The oligonucleotide can be DNA or RNA or a chimeric mixture or derivatives or modified versions thereof, single-stranded or double-stranded. The oligonucleotide can be modified at the base moiety, sugar moiety, or phosphate backbone. The oligonucleotide may include other appending groups such as peptides, agents that facilitate transport across the cell membrane, hybridization-triggered cleavage agents, and intercalating agents.

In another embodiment, the antisense oligomer is a phosphothioate morpholino oligomer (PMO). PMOs are assembled from four different morpholino subunits, each of which contain one of four genetic bases (A, C, G, or T) linked to a six-membered morpholine ring. Polymers of these subunits are joined by non-ionic phosphodiamidate intersubunit linkages. Details of how to make and use PMOs and other antisense oligomers are well known in the art (e.g. see WO99/18193; Probst JC, Antisense Oligodeoxynucleotide and Ribozyme Design, Methods. (2000) 22(3):271-281; Summerton J, and Weller D. 1997 Antisense Nucleic Acid Drug Dev. :7:187-95; US Pat. No. 5,235,033; and US Pat No. 5,378,841).

Alternative preferred UP nucleic acid modulators are double-stranded RNA species mediating RNA interference (RNAi). RNAi is the process of sequence-specific, post-transcriptional gene silencing in animals and plants, initiated by double-stranded RNA (dsRNA) that is homologous in sequence to the silenced gene. Methods relating to the use of RNAi to silence genes in *C*. *elegans, Drosophila,* plants, and humans are known in the art (Fire A, et al., 1998 Nature 391:806-811; Fire, A. Trends Genet. 15, 358-363 (1999); Sharp, P. A. RNA interference 2001. Genes Dev. 15, 485-490 (2001); Hammond, S. M., et al., Nature Rev. Genet. 2, 110-1119 (2001); Tuschl, T. Chem. Biochem. 2, 239-245 (2001); Hamilton, A. et al., Science 286, 950-952 (1999); Hammond, S. M., et al., Nature 404,293-296 (2000); Zamore, P. D., et al., Cell 101, 25-33 (2000); Bernstein, E., et al., Nature 409, 363-366 (2001); Elbashir, S. M., et al., Genes Dev. 15, 188-200 (2001); WO0129058; WO9932619; Elbashir SM, et al., 2001 Nature 411:494-498).

Nucleic acid modulators are commonly used as research reagents, diagnostics, and therapeutics. For example, antisense oligonucleotides, which are able to inhibit gene expression with exquisite specificity, are often used to elucidate the function of particular genes (see, for example, U.S. Pat. No. 6,165,790). Nucleic acid modulators are also used, for example, to distinguish between functions of various members of a biological pathway. For example, antisense oligomers have been employed as therapeutic moieties in the treatment of disease states in animals and man and have been demonstrated in numerous clinical trials to be safe and effective (Milligan JF, et al, Current Concepts in Antisense Drug Design, J Med Chem. (1993) 36:1923-1937; Tonkinson JL et al., Antisense Oligodeoxynucleotides as Clinical Therapeutic Agents, Cancer Invest. (1996) 14:54-65). Accordingly, in one aspect of the invention, a UP-specific nucleic acid modulator is used in an assay to further elucidate the role of the UP in the beta catenin pathway, and/or its relationship to other members of the pathway. In another aspect of the invention, a UP-specific antisense oligomer is used as a therapeutic agent for treatment of beta catenin-related disease states.

### Assay Systems

The invention provides assay systems and screening methods for identifying specific modulators of UP activity. As used herein, an "assay system" encompasses all the components required for performing and analyzing results of an assay that detects and/or measures a particular event. In general, primary assays are used to identify or confirm a modulator's specific biochemical or molecular effect with respect to the UP nucleic acid or protein. In general, secondary assays further assess the activity of a UP modulating agent identified by a primary assay and may confirm that the modulating agent affects UP in a manner relevant to the beta catenin pathway. In some cases, UP modulators will be directly tested in a secondary assay.

In a preferred embodiment, the screening method comprises contacting a suitable assay system comprising a UP polypeptide or nucleic acid with a candidate agent under conditions whereby, but for the presence of the agent, the system provides a reference activity (e.g. phosphorylase activity), which is based on the particular molecular event the screening method detects. A statistically significant difference between the agent-biased activity and the reference activity indicates that the candidate agent modulates UP activity, and hence the beta catenin pathway. The UP polypeptide or nucleic acid used in the assay may comprise any of the nucleic acids or polypeptides described above.

### Primary Assays

The type of modulator tested generally determines the type of primary assay.

### Primary assays for small molecule modulators

For small molecule modulators, screening assays are used to identify candidate modulators. Screening assays may be cell-based or may use a cell-free system that recreates or retains the relevant biochemical reaction of the target protein (reviewed in Sittampalam GS et al., Curr Opin Chem Biol (1997) 1:384-91 and accompanying references). As used herein the term "cell-based" refers to assays using live cells, dead cells, or a particular cellular fraction, such as a membrane, endoplasmic reticulum, or mitochondrial fraction. The term "cell free" encompasses assays using substantially purified protein (either endogenous or recombinantly produced), partially purified or crude cellular extracts. Screening assays may detect a variety of molecular events, including protein-DNA interactions, protein-protein interactions (*e.g.*, receptor-ligand binding), transcriptional activity (*e.g.,* using a reporter gene), enzymatic activity (*e.g.*, via a property of the substrate), activity of second messengers, immunogenicty and changes in cellular morphology or other cellular characteristics. Appropriate screening assays may use a wide range of detection methods including fluorescent, radioactive, colorimetric, spectrophotometric, and amperometric methods, to provide a read-out for the particular molecular event detected.

Cell-based screening assays usually require systems for recombinant expression of UP and any auxiliary proteins demanded by the particular assay. Appropriate methods for generating recombinant proteins produce sufficient quantities of proteins that retain their relevant biological activities and are of sufficient purity to optimize activity and assure assay reproducibility. Yeast two-hybrid and variant screens, and mass spectrometry provide preferred methods for determining protein-protein interactions and elucidation of protein complexes. In certain applications, when UP-interacting proteins are used in screens to identify small molecule modulators, the binding specificity of the interacting protein to the UP protein may be assayed by various known methods such as substrate processing (e.g. ability of the candidate UP-specific binding agents to function as negative effectors in UP-expressing cells), binding equilibrium constants (usually at least about 10⁷ M^{~1}, preferably at least about 10⁸ M⁻¹, more preferably at least about 10⁹ M^{~1}), and immunogenicity (e.g. ability to elicit UP specific antibody in a heterologous host such as a mouse, rat, goat or rabbit). For enzymes and receptors, binding may be assayed by, respectively, substrate and ligand processing.

The screening assay may measure a candidate agent's ability to specifically bind to or modulate activity of a UP polypeptide, a fusion protein thereof, or to cells or membranes bearing the polypeptide or fusion protein. The UP polypeptide can be full length or a fragment thereof that retains functional UP activity. The UP polypeptide may be fused to another polypeptide, such as a peptide tag for detection or anchoring, or to another tag. The UP polypeptide is preferably human UP, or is an ortholog or derivative thereof as described above. In a preferred embodiment, the screening assay detects candidate agent-based modulation of UP interaction with a binding target, such as an endogenous or exogenous protein or other substrate that has UP -specific binding activity, and can be used to assess normal UP gene function.

Suitable assay formats that may be adapted to screen for UP modulators are known in the art. Preferred screening assays are high throughput or ultra high throughput and thus provide automated, cost-effective means of screening compound libraries for lead compounds (Fernandes PB, Curr Opin Chem Biol (1998) 2:597-603; Sundberg SA, Curr Opin Biotechnol 2000, 11:47-53). In one preferred embodiment, screening assays uses fluorescence technologies, including fluorescence polarization, time-resolved fluorescence, and fluorescence resonance energy transfer. These systems offer means to monitor protein-protein or DNA-protein interactions in which the intensity of the signal emitted from dye-labeled molecules depends upon their interactions with partner molecules (*e.g*., Selvin PR, Nat Struct Biol (2000) 7:730-4; Fernandes PB, *supra;* Hertzberg RP and Pope AJ, Curr Opin Chem Biol (2000) 4:445-451).

A variety of suitable assay systems may be used to identify candidate UP and beta catenin pathway modulators (e.g. U.S. Pat. Nos. 5,550,019 and 6,133,437 (apoptosis assays); and U.S. Pat. Nos. 5,976,782, 6,225,118 and 6,444,434 (angiogenesis assays), among others). Specific preferred assays are described in more detail below.

Assays for uridine phosphorylase enzyme activity measure uridine conversion to uracil, using various methods such as TLC chromatography, which are described in the art (Liu M-P, et al (1998)Cancer Research 58:5418-5424).

Apoptosis assays. Apoptosis or programmed cell death is a suicide program is activated within the cell, leading to fragmentation of DNA, shrinkage of the cytoplasm, membrane changes and cell death. Apoptosis is mediated by proteolytic enzymes of the caspase family. Many of the altering parameters of a cell are measurable during apoptosis. Assays for apoptosis may be performed by terminal deoxynucleotidyl transferase-mediated digoxigenin-11-dUTP nick end labeling (TUNEL) assay. The TUNEL assay is used to measure nuclear DNA fragmentation characteristic of apoptosis (Lazebnik et al., 1994, Nature 371, 346), by following the incorporation of fluorescein-dUTP (Yonehara et al., 1989, J. Exp. Med. 169, 1747). Apoptosis may further be assayed by acridine orange staining of tissue culture cells (Lucas, R., et al., 1998, Blood 15:4730-41). Other cell-based apoptosis assays include the caspase-3/7 assay and the cell death nucleosome ELISA assay. The caspase 3/7 assay is based on the activation of the caspase cleavage activity as part of a cascade of events that occur during programmed cell death in many apoptotic pathways. In the caspase 3/7 assay (commercially available Apo-ONE^{™} Homogeneous Caspase-3/7 assay from Promega, cat# 67790), lysis buffer and caspase substrate are mixed and added to cells. The caspase substrate becomes fluorescent when cleaved by active caspase 3/7. The nucleosome ELISA assay is a general cell death assay known to those skilled in the art, and available commercially (Roche, Cat# 1774425). This assay is a quantitative sandwich-enzyme-immunoassay which uses monoclonal antibodies directed against DNA and histones respectively, thus specifically determining amount of mono- and oligonucleosomes in the cytoplasmic fraction of cell lysates. Mono and oligonucleosomes are enriched in the cytoplasm during apoptosis due to the fact that DNA fragmentation occurs several hours before the plasma membrane breaks down, allowing for accumalation in the cytoplasm. Nucleosomes are not present in the cytoplasmic fraction of cells that are not undergoing apoptosis. The Phospho-histone H2B assay is another apoptosis assay, based on phosphorylation of histone H2B as a result of apoptosis. Fluorescent dyes that are associated with phosphohistone H2B may be used to measure the increase of phosphohistone H2B as a result of apoptosis. Apoptosis assays that simultaneously measure multiple parameters associated with apoptosis have also been developed. In such assays, various cellular parameters that can be associated with antibodies or fluorescent dyes, and that mark various stages of apoptosis are labeled, and the results are measured using instruments such as Cellomics™ ArrayScan^{®} HCS System. The measurable parameters and their markers include anti-active caspase-3 antibody which marks intermediate stage apoptosis, anti-PARP-p85 antibody (cleaved PARP) which marks late stage apoptosis, Hoechst labels which label the nucleus and are used to measure nuclear swelling as a measure of early apoptosis and nuclear condensation as a measure of late apoptosis, and TOTO-3 fluorescent dye which labels DNA of dead cells with high cell membrane permeability.

An apoptosis assay system may comprise a cell that expresses a UP, and that optionally has defective beta catenin function (e.g. beta catenin is over-expressed or under-expressed relative to wild-type cells). A test agent can be added to the apoptosis assay system and changes in induction of apoptosis relative to controls where no test agent is added, identify candidate beta catenin modulating agents. In some embodiments of the invention, an apoptosis assay may be used as a secondary assay to test a candidate beta catenin modulating agents that is initially identified using a cell-free assay system. An apoptosis assay may also be used to test whether UP function plays a direct role in apoptosis. For example, an apoptosis assay may be performed on cells that over- or under-express UP relative to wild type cells. Differences in apoptotic response compared to wild type cells suggests that the UP plays a direct role in the apoptotic response. Apoptosis assays are described further in US Pat. No. 6,133,437.

**Cell proliferation and cell cycle assays.** Cell proliferation may be assayed via bromodeoxyuridine (BRDU) incorporation. This assay identifies a cell population undergoing DNA synthesis by incorporation of BRDU into newly-synthesized DNA. Newly-synthesized DNA may then be detected using an anti-BRDU antibody (Hoshino et al., 1986, Int. J. Cancer 38, 369; Campana et al., 1988, J. Immunol. Meth. 107, 79), or by other means.

Cell proliferation is also assayed via phospho-histone H3 staining, which identifies a cell population undergoing mitosis by phosphorylation of histone H3. Phosphorylation of histone H3 at serine 10 is detected using an antibody specfic to the phosphorylated form of the serine 10 residue of histone H3. (Chadlee, D.N. 1995, J. Biol. Chem 270:20098-105). Cell Proliferation may also be examined using [³H]-thymidine incorporation (Chen, J., 1996, Oncogene 13:1395-403; Jeoung, J., 1995, J. Biol. Chem. 270:18367-73). This assay allows for quantitative characterization of S-phase DNA syntheses. In this assay, cells synthesizing DNA will incorporate [³H]-thymidine into newly synthesized DNA. Incorporation can then be measured by standard techniques such as by counting of radioisotope in a scintillation counter (e.g., Beckman LS 3800 Liquid Scintillation Counter). Another proliferation assay uses the dye Alamar Blue (available from Biosource International), which fluoresces when reduced in living cells and provides an indirect measurement of cell number (Voytik-Harbin SL et al., 1998, In Vitro Cell Dev Biol Anim 34:239-46). Yet another proliferation assay, the MTS assay, is based on in vitro cytotoxicity assessment of industrial chemicals, and uses the soluble tetrazolium salt, MTS. MTS assays are commercially available, for example, the Promega CellTiter 96^{®} AQueous Non-Radioactive Cell Proliferation Assay (Cat.# G5421).

Cell proliferation may also be assayed by colony formation in soft agar, or clonogenic survival assay (Sambrook et al., Molecular Cloning, Cold Spring Harbor (1989)). For example, cells transformed with UP are seeded in soft agar plates, and colonies are measured and counted after two weeks incubation.

Cell proliferation may also be assayed by measuring ATP levels as indicator of metabolically active cells. Such assays are commercially available, for example Cell Titer-Glo™, which is a luminescent homogeneous assay available from Promega.

Involvement of a gene in the cell cycle may be assayed by flow cytometry (Gray JW et al. (1986) Int J Radiat Biol Relat Stud Phys Chem Med 49:237-55). Cells transfected with a UP may be stained with propidium iodide and evaluated in a flow cytometer (available from Becton Dickinson), which indicates accumulation of cells in different stages of the cell cycle.

Accordingly, a cell proliferation or cell cycle assay system may comprise a cell that expresses a UP, and that optionally has defective beta catenin function (e.g. beta catenin is over-expressed or under-expressed relative to wild-type cells). A test agent can be added to the assay system and changes in cell proliferation or cell cycle relative to controls where no test agent is added, identify candidate beta catenin modulating agents. In some embodiments of the invention, the cell proliferation or cell cycle assay may be used as a secondary assay to test a candidate beta catenin modulating agents that is initially identified using another assay system such as a cell-free assay system. A cell proliferation assay may also be used to test whether UP function plays a direct role in cell proliferation or cell cycle. For example, a cell proliferation or cell cycle assay may be performed on cells that over- or under-express UP relative to wild type cells. Differences in proliferation or cell cycle compared to wild type cells suggests that the UP plays a direct role in cell proliferation or cell cycle.

**Angiogenesis**. Angiogenesis may be assayed using various human endothelial cell systems, such as umbilical vein, coronary artery, or dermal cells. Suitable assays include Alamar Blue based assays (available from Biosource International) to measure proliferation; migration assays using fluorescent molecules, such as the use of Becton Dickinson Falcon HTS FluoroBlock cell culture inserts to measure migration of cells through membranes in presence or absence of angiogenesis enhancer or suppressors; and tubule formation assays based on the formation of tubular structures by endothelial cells on Matrigel® (Becton Dickinson). Accordingly, an angiogenesis assay system may comprise a cell that expresses a UP, and that optionally has defective beta catenin function (e.g. beta catenin is over-expressed or under-expressed relative to wild-type cells). A test agent can be added to the angiogenesis assay system and changes in angiogenesis relative to controls where no test agent is added, identify candidate beta catenin modulating agents. In some embodiments of the invention, the angiogenesis assay may be used as a secondary assay to test a candidate beta catenin modulating agents that is initially identified using another assay system. An angiogenesis assay may also be used to test whether UP function plays a direct role in cell proliferation. For example, an angiogenesis assay may be performed on cells that over- or under-express UP relative to wild type cells. Differences in angiogenesis compared to wild type cells suggests that the UP plays a direct role in angiogenesis. U.S. Pat. Nos. 5,976,782, 6,225,118 and 6,444,434, among others, describe various angiogenesis assays.

**Hypoxic induction.** The alpha subunit of the transcription factor, hypoxia inducible factor-1 (HIF-1), is upregulated in tumor cells following exposure to hypoxia in vitro. Under hypoxic conditions, HIF-1 stimulates the expression of genes known to be important in tumour cell survival, such as those encoding glyolytic enzymes and VEGF. Induction of such genes by hypoxic conditions may be assayed by growing cells transfected with UP in hypoxic conditions (such as with 0.1% 02, 5% CO2, and balance N2, generated in a Napco 7001 incubator (Precision Scientific)) and normoxic conditions, followed by assessment of gene activity or expression by Taqman®. For example, a hypoxic induction assay system may comprise a cell that expresses a UP, and that optionally has defective beta catenin function (e.g. beta catenin is over-expressed or under-expressed relative to wild-type cells). A test agent can be added to the hypoxic induction assay system and changes in hypoxic response relative to controls where no test agent is added, identify candidate beta catenin modulating agents. In some embodiments of the invention, the hypoxic induction assay may be used as a secondary assay to test a candidate beta catenin modulating agents that is initially identified using another assay system. A hypoxic induction assay may also be used to test whether UP function plays a direct role in the hypoxic response. For example, a hypoxic induction assay may be performed on cells that over- or under-express UP relative to wild type cells. Differences in hypoxic response compared to wild type cells suggests that the UP plays a direct role in hypoxic induction.

**Cell adhesion.** Cell adhesion assays measure adhesion of cells to purified adhesion proteins, or adhesion of cells to each other, in presence or absence of candidate modulating agents. Cell-protein adhesion assays measure the ability of agents to modulate the adhesion of cells to purified proteins. For example, recombinant proteins are produced, diluted to 2.5g/mL in PBS, and used to coat the wells of a microtiter plate. The wells used for negative control are not coated. Coated wells are then washed, blocked with 1% BSA, and washed again. Compounds are diluted to 2x final test concentration and added to the blocked, coated wells. Cells are then added to the wells, and the unbound cells are washed off. Retained cells are labeled directly on the plate by adding a membrane-permeable fluorescent dye, such as calcein-AM, and the signal is quantified in a fluorescent microplate reader.

Cell-cell adhesion assays measure the ability of agents to modulate binding of cell adhesion proteins with their native ligands. These assays use cells that naturally or recombinantly express the adhesion protein of choice. In an exemplary assay, cells expressing the cell adhesion protein are plated in wells of a multiwell plate. Cells expressing the ligand are labeled with a membrane-permeable fluorescent dye, such as BCECF , and allowed to adhere to the monolayers in the presence of candidate agents. Unbound cells are washed off, and bound cells are detected using a fluorescence plate reader.

High-throughput cell adhesion assays have also been described. In one such assay, small molecule ligands and peptides are bound to the surface of microscope slides using a microarray spotter, intact cells are then contacted with the slides, and unbound cells are washed off. In this assay, not only the binding specificity of the peptides and modulators against cell lines are determined, but also the functional cell signaling of attached cells using immunofluorescence techniques in situ on the microchip is measured (Falsey JR et al., Bioconjug Chem. 2001 May-Jun;12(3):346-53).

**Tubulogenesis.** Tubulogenesis assays monitor the ability of cultured cells, generally endothelial cells, to form tubular structures on a matrix substrate, which generally simulates the environment of the extracellular matrix. Exemplary substrates include Matrigel^{™} (Becton Dickinson), an extract of basement membrane proteins containing laminin, collagen IV, and heparin sulfate proteoglycan, which is liquid at 4° C and forms a solid gel at 37°C. Other suitable matrices comprise extracellular components such as collagen, fibronectin, and/or fibrin. Cells are stimulated with a pro-angiogenic stimulant, and their ability to form tubules is detected by imaging. Tubules can generally be detected after an overnight incubation with stimuli, but longer or shorter time frames may also be used. Tube formation assays are well known in the art (e.g., Jones MK et al., 1999, Nature Medicine 5:1418-1423). These assays have traditionally involved stimulation with serum or with the growth factors FGF or VEGF. Serum represents an undefined source of growth factors. In a preferred embodiment, the assay is performed with cells cultured in serum free medium, in order to control which process or pathway a candidate agent modulates. Moreover, we have found that different target genes respond differently to stimulation with different pro-angiogenic agents, including inflammatory angiogenic factors such as TNF-alpa. Thus, in a further preferred embodiment, a tubulogenesis assay system comprises testing a UP's response to a variety of factors, such as FGF, VEGF, phorbol myristate acetate (PMA), TNF-alpha, ephrin, etc.

**Cell Migration.** An invasion/migration assay (also called a migration assay) tests the ability of cells to overcome a physical barrier and to migrate towards pro-angiogenic signals. Migration assays are known in the art (e.g., Paik JH et al., 2001, J Biol Chem 276:11830-11837). In a typical experimental set-up, cultured endothelial cells are seeded onto a matrix-coated porous lamina, with pore sizes generally smaller than typical cell size. The matrix generally simulates the environment of the extracellular matrix, as described above. The lamina is typically a membrane, such as the transwell polycarbonate membrane (Corning Costar Corporation, Cambridge, MA), and is generally part of an upper chamber that is in fluid contact with a lower chamber containing pro-angiogenic stimuli. Migration is generally assayed after an overnight incubation with stimuli, but longer or shorter time frames may also be used. Migration is assessed as the number of cells that crossed the lamina, and may be detected by staining cells with hemotoxylin solution (VWR Scientific, South San Francisco, CA), or by any other method for determining cell number. In another exemplary set up, cells are fluorescently labeled and migration is detected using fluorescent readings, for instance using the Falcon HTS FluoroBlok (Becton Dickinson). While some migration is observed in the absence of stimulus, migration is greatly increased in response to pro-angiogenic factors. As described above, a preferred assay system for migration/invasion assays comprises testing a UP's response to a variety of pro-angiogenic factors, including tumor angiogenic and inflammatory angiogenic agents, and culturing the cells in serum free medium.

**Sprouting assay.** A sprouting assay is a three-dimensional *in vitro* angiogenesis assay that uses a cell-number defined spheroid aggregation of endothelial cells ("spheroid"), embedded in a collagen gel-based matrix. The spheroid can serve as a starting point for the sprouting of capillary-like structures by invasion into the extracellular matrix (termed "cell sprouting") and the subsequent formation of complex anastomosing networks (Korff and Augustin, 1999, J Cell Sci 112:3249-58). In an exemplary experimental set-up, spheroids are prepared by pipetting 400 human umbilical vein endothelial cells into individual wells of a nonadhesive 96-well plates to allow overnight spheroidal aggregation (Korff and Augustin: J Cell Biol 143: 1341-52, 1998). Spheroids are harvested and seeded in 900µl of methocel-collagen solution and pipetted into individual wells of a 24 well plate to allow collagen gel polymerization. Test agents are added after 30 min by pipetting 100 µl of 10-fold concentrated working dilution of the test substances on top of the gel. Plates are incubated at 37°C for 24h. Dishes are fixed at the end of the experimental incubation period by addition of paraformaldehyde. Sprouting intensity of endothelial cells can be quantitated by an automated image analysis system to determine the cumulative sprout length per spheroid.

### Primary assays for antibody modulators

For antibody modulators, appropriate primary assays test is a binding assay that tests the antibody's affinity to and specificity for the UP protein. Methods for testing antibody affinity and specificity are well known in the art (Harlow and Lane, 1988, 1999, *supra).* The enzyme-linked immunosorbant assay (ELISA) is a preferred method for detecting UP-specific antibodies; others include FACS assays, radioimmunoassays, and fluorescent assays.

In some cases, screening assays described for small molecule modulators may also be used to test antibody modulators.

### Primary assays for nucleic acid modulators

For nucleic acid modulators, primary assays may test the ability of the nucleic acid modulator to inhibit or enhance UP gene expression, preferably mRNA expression. In general, expression analysis comprises comparing UP expression in like populations of cells (*e.g*., two pools of cells that endogenously or recombinantly express UP) in the presence and absence of the nucleic acid modulator. Methods for analyzing mRNA and protein expression are well known in the art. For instance, Northern blotting, slot blotting, ribonuclease protection, quantitative RT-PCR (*e.g*., using the TaqMan®, PE Applied Biosystems), or microarray analysis may be used to confirm that UP mRNA expression is reduced in cells treated with the nucleic acid modulator (*e.g.*, Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm DH and Guiseppi-Elie, A Curr Opin Biotechnol 2001, 12:41-47). Protein expression may also be monitored. Proteins are most commonly detected with specific antibodies or antisera directed against either the UP protein or specific peptides. A variety of means including Western blotting, ELISA, or in situ detection, are available (Harlow E and Lane D, 1988 and 1999, *supra*).

In some cases, screening assays described for small molecule modulators, particularly in assay systems that involve UP mRNA expression, may also be used to test nucleic acid modulators.

### Secondary Assays

Secondary assays may be used to further assess the activity of UP-modulating agent identified by any of the above methods to confirm that the modulating agent affects UP in a manner relevant to the beta catenin pathway. As used herein, UP-modulating agents encompass candidate clinical compounds or other agents derived from previously identified modulating agent. Secondary assays can also be used to test the activity of a modulating agent on a particular genetic or biochemical pathway or to test the specificity of the modulating agent's interaction with UP.

Secondary assays generally compare like populations of cells or animals (*e.g*., two pools of cells or animals that endogenously or recombinantly express UP) in the presence and absence of the candidate modulator. In general, such assays test whether treatment of cells or animals with a candidate UP-modulating agent results in changes in the beta catenin pathway in comparison to untreated (or mock- or placebo-treated) cells or animals. Certain assays use "sensitized genetic backgrounds", which, as used herein, describe cells or animals engineered for altered expression of genes in the beta catenin or interacting pathways.

### Cell-based assays

Cell based assays may detect endogenous beta catenin pathway activity or may rely on recombinant expression of beta catenin pathway components. Any of the aforementioned assays may be used in this cell-based format. Candidate modulators are typically added to the cell media but may also be injected into cells or delivered by any other efficacious means.

### Animal Assays

A variety of non-human animal models of normal or defective beta catenin pathway may be used to test candidate UP modulators. Models for defective beta catenin pathway typically use genetically modified animals that have been engineered to mis-express (*e.g*., over-express or lack expression in) genes involved in the beta catenin pathway. Assays generally require systemic delivery of the candidate modulators, such as by oral administration, injection, etc.

Beta catenin pathway activity can be assessed by monitoring neovascularization and angiogenesis. Animal models with defective and normal beta catenin are used to test the candidate modulator's affect on UP in Matrigel® assays. Matrigel® is an extract of basement membrane proteins, and is composed primarily of laminin, collagen IV, and heparin sulfate proteoglycan. It is provided as a sterile liquid at 4°C, but rapidly forms a solid gel at 37°C. Liquid Matrigel® is mixed with various angiogenic agents, such as bFGF and VEGF, or with human tumor cells which overexpress the UP. The mixture is then injected subcutaneously(SC) into female athymic nude mice (Taconic, Germantown, NY) to support an intense vascular response. Mice with Matrigel® pellets may be dosed via oral (PO), intraperitoneal (IP), or intravenous (IV) routes with the candidate modulator. Mice are euthanized 5 - 12 days post-injection, and the Matrigel® pellet is harvested for hemoglobin analysis (Sigma plasma hemoglobin kit). Hemoglobin content of the gel is found to correlate the degree of neovascularization in the gel.

Alternatively, the effect of the candidate modulator on UP is assessed via tumorigenicity assays. Tumor xenograft assays are known in the art (see, e.g., Ogawa K et al., 2000, Oncogene 19:6043-6052). Xenografts are typically implanted SC into female athymic mice, 6-7 week old, as single cell suspensions either from a pre-existing tumor or from *in vitro* culture. The tumors which express the UP endogenously are injected in the flank, 1 x 10⁵ to 1 x 10⁷ cells per mouse in a volume of 100 µL using a 27gauge needle. Mice are then ear tagged and tumors are measured twice weekly. Candidate modulator treatment is initiated on the day the mean tumor weight reaches 100 mg. Candidate modulator is delivered IV, SC, IP, or PO by bolus administration. Depending upon the pharmacokinetics of each unique candidate modulator, dosing can be performed multiple times per day. The tumor weight is assessed by measuring perpendicular diameters with a caliper and calculated by multiplying the measurements of diameters in two dimensions. At the end of the experiment, the excised tumors maybe utilized for biomarker identification or further analyses. For immunohistochemistry staining, xenograft tumors are fixed in 4% paraformaldehyde, 0.1M phosphate, pH 7.2, for 6 hours at 4°C, immersed in 30% sucrose in PBS, and rapidly frozen in isopentane cooled with liquid nitrogen.

Alternatively, tumorogenicity is monitored using a hollow fiber assay, which is described in U.S. Pat No. US 5,698,413. Briefly, the method comprises implanting into a laboratory animal a biocompatible, semi-permeable encapsulation device containing target cells, treating the laboratory animal with a candidate modulating agent, and evaluating the target cells for reaction to the candidate modulator. Implanted cells are generally human cells from a pre-existing tumor or a tumor cell line. After an appropriate period of time, generally around six days, the implanted samples are harvested for evaluation of the candidate modulator. Tumorogenicity and modulator efficacy may be evaluated by assaying the quantity of viable cells present in the macrocapsule, which can be determined by tests known in the art, for example, MTT dye conversion assay, neutral red dye uptake, trypan blue staining, viable cell counts, the number of colonies formed in soft agar, the capacity of the cells to recover and replicate in vitro etc.

A tumorogenicity assay may use a transgenic animal, usually a mouse, carrying a dominant oncogene or tumor suppressor gene knockout under the control of tissue specific regulatory sequences; these assays are generally referred to as transgenic tumor assays. In a preferred application, tumor development in the transgenic model is well characterized or is controlled. In an exemplary model, the "RIP1-Tag2" transgene, comprising the SV40 large T-antigen oncogene under control of the insulin gene regulatory regions is expressed in pancreatic beta cells and results in islet cell carcinomas (Hanahan D, 1985, Nature 315:115-122; Parangi S et al, 1996, Proc Natl Acad Sci USA 93: 2002-2007; Bergers G et al, 1999, Science 284:808-812). An "angiogenic switch," occurs at approximately five weeks, as normally quiescent capillaries in a subset of hyperproliferative islets become angiogenic. The RIP1-TAG2 mice die by age 14 weeks. Candidate modulators may be administered at a variety of stages, including just prior to the angiogenic switch (e.g., for a model of tumor prevention), during the growth of small tumors (e.g., for a model of intervention), or during the growth of large and/or invasive tumors (e.g., for a model of regression). Tumorogenicity and modulator efficacy can be evaluating life-span extension and/or tumor characteristics, including number of tumors, tumor size, tumor morphology, vessel density, apoptotic index, etc.

### Diagnostic and therapeutic uses

Specific UP-modulating agents are useful in a variety of diagnostic and therapeutic applications where disease or disease prognosis is related to defects in the beta catenin pathway, such as angiogenic, apoptotic, or cell proliferation disorders. Accordingly, the invention also provides methods for modulating the beta catenin pathway in a cell, preferably a cell pre-determined to have defective or impaired beta catenin function (e.g. due to overexpression, underexpression, or misexpression of beta catenin, or due to gene mutations), comprising the step of administering an agent to the cell that specifically modulates UP activity. Preferably, the modulating agent produces a detectable phenotypic change in the cell indicating that the beta catenin function is restored. The phrase "function is restored", and equivalents, as used herein, means that the desired phenotype is achieved, or is brought closer to normal compared to untreated cells. For example, with restored beta catenin function, cell proliferation and/or progression through cell cycle may normalize, or be brought closer to normal relative to untreated cells. The invention also provides certain agents as defined in the claims for use in treating disorders or disease associated with impaired beta catenin function by administering a therapeutically effective amount of a UP -modulating agent that modulates the beta catenin pathway. The invention further provides methods for modulating UP function in a cell, preferably a cell pre-determined to have defective or impaired UP function, by administering a UP -modulating agent.

The discovery that UP is implicated in beta catenin pathway provides for a variety of methods that can be employed for the diagnostic and prognostic evaluation of diseases and disorders involving defects in the beta catenin pathway and for the identification of subjects having a predisposition to such diseases and disorders.

Various expression analysis methods can be used to diagnose whether UP expression occurs in a particular sample, including Northern blotting, slot blotting, ribonuclease protection, quantitative RT-PCR, and microarray analysis. (*e.g*., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm and Guiseppi-Elie, Curr Opin Biotechnol 2001, 12:41-47). Tissues having a disease or disorder implicating defective beta catenin signaling that express a UP, are identified as amenable to treatment with a UP modulating agent. In a preferred application, the beta catenin defective tissue overexpresses a UP relative to normal tissue. For example, a Northern blot analysis of mRNA from tumor and normal cell lines, or from tumor and matching normal tissue samples from the same patient, using full or partial UP cDNA sequences as probes, can determine whether particular tumors express or overexpress UP. Alternatively, the TaqMan® is used for quantitative RT-PCR analysis of UP expression in cell lines, normal tissues and tumor samples (PE Applied Biosystems).

Various other diagnostic methods may be performed, for example, utilizing reagents such as the UP oligonucleotides, and antibodies directed against a UP, as described above for: (1) the detection of the presence of UP gene mutations, or the detection of either over- or under-expression of UP mRNA relative to the non-disorder state; (2) the detection of either an over- or an under-abundance of UP gene product relative to the non-disorder state; and (3) the detection of perturbations or abnormalities in the signal transduction pathway mediated by UP.

Thus, in a specific embodiment, the invention is drawn to an in vitro method for diagnosing a disease or disorder in a patient that is associated with alterations in UP expression, the method comprising: a) contacting a biological sample from the patient with a probe for UP expression; b) comparing results from step (a) with a control; and c) determining whether step (b) indicates a likelihood of the disease or disorder, wherein said disease or disorder is an angiogenic, apoptotic or cell proliferation disease or disorder associated with a defect in the beta catenin pathway.
Preferably, the disease is cancer, most preferably a cancer as shown in Table 1 as having > 25% expression level. The probe may be either DNA or protein, including an antibody.

### EXAMPLES

The following experimental section and examples are offered by way of illustration and not by way of limitation.

### I. Drosophila beta catenin screen

Two dominant loss of function screens were carried out in *Drosophila* to identify genes that interact with the Wg cell signaling molecule, beta-catenin (Riggleman et al. (1990) Cell 63:549-560; Peifer et al. (1991) Development 111:1029-1043). Late stage activation of the pathway in the developing *Drosophila* eye leads to apoptosis (Freeman and Bienz (2001) EMBO reports 2: 157-162), whereas early stage activation leads to an overgrowth phenotype. We discovered that ectopic expression of the activated protein in the wing results in changes of cell fate into ectopic bristles and wing veins.

Each transgene was carried in a separate fly stock:
Stocks and genotypes were as follows:
   eye overgrowth transgene: isow; P{3.5 eyeless-Gal4}; P{arm(S56F)-pExp-UAS)}/TM6b;
   eye apoptosis transgene: y w; P{arm(S56F)-pExp-GMR}/CyO; and
   wing transgene: P{arm(ΔN)-pExp-VgMQ}/FM7c

In the first dominant loss of function screen, females of each of these three transgenes were crossed to a collection of males containing genomic deficiencies. Resulting progeny containing the transgene and the deficiency were then scored for the effect of the deficiency on the eye apoptosis, eye overgrowth, and wing phenotypes, i.e., whether the deficiency enhanced, suppressed, or had no effect on their respective phenotypes. All data was recorded and all modifiers were retested with a repeat of the original cross. Modifying deficiencies of the phenotypes were then prioritized according to how they modified each of the three phenotypes.

Transposons contained within the prioritized deficiencies were then screened as described. Females of each of the three transgenes were crossed to a collection of 4 types of transposons (3 piggyBac-based and 1 P-element-based). The resulting progeny containing the transgene and the transposon were scored for the effect of the transposon on their respective phenotypes. All data was recorded and all modifiers were retested with a repeat of the original cross. Modifiers of the phenotypes were identified as either members of the Wg pathway, components of apoptotic related pathways, components of cell cycle related pathways, or cell adhesion related proteins.

In the second dominant loss of function screen, females of the eye overgrowth transgene were crossed to males from a collection of 3 types of piggyBac-based transposons. The resulting progeny containing the transgene and the transposon were scored for the effect of the transposon on the eye overgrowth phenotype. All data was recorded and all modifiers were retested with a repeat of the original cross. Modifiers of the phenotypes were identified as either members of the Wg pathway, components of cell cycle related pathways, or cell adhesion related proteins. CG6330 was identified as a suppressor from the assay. Orthologs of CG6330 are referred to herein as UP.

BLAST analysis (Altschul et al., *supra)* was employed to identify orthologs of Drosophila CG6330. For example, representative sequences from UP, GI# 4507839 (SEQ ID NO:10), and GI#27597096 (SEQ ID NO: 11) share 51% and 53% amino acid identity, respectively, with the *Drosophila* CG6330.

Various domains, signals, and functional subunits in proteins were analyzed using the PSORT (Nakai K., and Horton P., Trends Biochem Sci, 1999, 24:34-6; Kenta Nakai, Protein sorting signals and prediction of subcellular localization, Adv. Protein Chem. 54, 277-344 (2000)), PFAM (Bateman A., et al., Nucleic Acids Res, 1999, 27:260-2), SMART (Ponting CP, et al., SMART: identification and annotation of domains from signaling and extracellular protein sequences. Nucleic Acids Res. 1999 Jan 1;27(1):229-32), TM-HMM (Erik L.L. Sonnhammer, Gunnar von Heijne, and Anders Krogh: A hidden Markov model for predicting transmembrane helices in protein sequences. In Proc. of Sixth Int. Conf. on Intelligent Systems for Molecular Biology, p 175-182 Ed J. Glasgow, T. Littlejohn, F. Major, R. Lathrop, D. Sankoff, and C. Sensen Menlo Park, CA: AAAI Press, 1998), and clust (Remm M, and Sonnhammer E. Classification of transmembrane protein families in the Caenorhabditis elegans genome and identification of human orthologs. Genome Res. 2000 Nov; 10(11): 1679-89) programs. For example, the phosphorylase domain (PFAM 01048) of UP from GI#s 4507839 and 27597096 (SEQ ID NO: 10 and 11, respectively) is located respectively at approximately amino acid residues 54 to 303 and 60-308.

### II. High-Throughput In Vitro Fluorescence Polarization Assay

Fluorescently-labeled UP peptide/substrate are added to each well of a 96-well microtiter plate, along with a test agent in a test buffer (10 mM HEPES, 10 mM NaCl, 6 mM magnesium chloride, pH 7.6). Changes in fluorescence polarization, determined by using a Fluorolite FPM-2 Fluorescence Polarization Microtiter System (Dynatech Laboratories, Inc), relative to control values indicates the test compound is a candidate modifier of UP activity.

### III. High-Throughput In Vitro Binding Assay.

³³P-labeled UP peptide is added in an assay buffer (100 mM KCl, 20 mM HEPES pH 7.6, 1 mM MgCl₂, 1% glycerol, 0.5% NP-40, 50 mM beta-mercaptoethanol, 1 mg/ml BSA, cocktail of protease inhibitors) along with a test agent to the wells of a Neutralite-avidin coated assay plate and incubated at 25°C for 1 hour. Biotinylated substrate is then added to each well and incubated for 1 hour. Reactions are stopped by washing with PBS, and counted in a scintillation counter. Test agents that cause a difference in activity relative to control without test agent are identified as candidate beta catenin modulating agents.

### IV. Immunoprecipitations and Immunoblotting

For coprecipitation of transfected proteins, 3 x 10⁶ appropriate recombinant cells containing the UP proteins are plated on 10-cm dishes and transfected on the following day with expression constructs. The total amount of DNA is kept constant in each transfection by adding empty vector. After 24 h, cells are collected, washed once with phosphate-buffered saline and lysed for 20 min on ice in 1 ml of lysis buffer containing 50 mM Hepes, pH 7.9,250 mM NaCl, 20 mM -glycerophosphate, 1 mM sodium orthovanadate, 5 mM p-nitrophenyl phosphate, 2 mM dithiothreitol, protease inhibitors (complete, Roche Molecular Biochemicals), and 1% Nonidet P-40. Cellular debris is removed by centrifugation twice at 15,000 x g for 15 min. The cell lysate is incubated with 25 µl of M2 beads (Sigma) for 2 h at 4 °C with gentle rocking.

After extensive washing with lysis buffer, proteins bound to the beads are solubilized by boiling in SDS sample buffer, fractionated by SDS-polyacrylamide gel electrophoresis, transferred to polyvinylidene difluoride membrane and blotted with the indicated antibodies. The reactive bands are visualized with horseradish peroxidase coupled to the appropriate secondary antibodies and the enhanced chemiluminescence (ECL) Western blotting detection system (Amersham Pharmacia Biotech).

### V. Expression analysis

All cell lines used in the following experiments are NCI (National Cancer Institute) lines, and are available from ATCC (American Type Culture Collection, Manassas, VA 20110-2209). Normal and tumor tissues were obtained from Impath, UC Davis, Clontech, Stratagene, Ardais, Genome Collaborative, and Ambion.

TaqMan^{®} analysis was used to assess expression levels of the disclosed genes in various samples.

RNA was extracted from each tissue sample using Qiagen (Valencia, CA) RNeasy kits, following manufacturer's protocols, to a final concentration of 50ng/µl. Single stranded cDNA was then synthesized by reverse transcribing the RNA samples using random hexamers and 500ng of total RNA per reaction, following protocol 4304965 of Applied Biosystems (Foster City, CA).

Primers for expression analysis using TaqMan® assay (Applied Biosystems, Foster City, CA) were prepared according to the TaqMan® protocols, and the following criteria: a) primer pairs were designed to span introns to eliminate genomic contamination, and b) each primer pair produced only one product. Expression analysis was performed using a 7900HT instrument.

TaqMan® reactions were carried out following manufacturer's protocols, in 25 µl total volume for 96-well plates and 10 µl total volume for 384-well plates, using 300nM primer and 250 nM probe, and approximately 25ng of cDNA. The standard curve for result analysis was prepared using a universal pool of human cDNA samples, which is a mixture of cDNAs from a wide variety of tissues so that the chance that a target will be present in appreciable amounts is good. The raw data were normalized using 18S rRNA (universally expressed in all tissues and cells).

For each expression analysis, tumor tissue samples were compared with matched normal tissues from the same patient. A gene was considered overexpressed in a tumor when the level of expression of the gene was 2 fold or higher in the tumor compared with its matched normal sample. In cases where normal tissue was not available, a universal pool of cDNA samples was used instead. In these cases, a gene was considered overexpressed in a tumor sample when the difference of expression levels between a tumor sample and the average of all normal samples from the same tissue type was greater than 2 times the standard deviation of all normal samples (i.e., Tumor - average(all normal samples) > 2 x STDEV(all normal samples) ).

Results are shown in Table 1. Number of pairs of tumor samples and matched normal tissue from the same patient are shown for each tumor type. Percentage of the samples with at least two-fold overexpression for each tumor type is provided. A modulator identified by an assay described herein can be further validated for therapeutic effect by administration to a tumor in which the gene is overexpressed. A decrease in tumor growth confirms therapeutic utility of the modulator. Prior to treating a patient with the modulator, the likelihood that the patient will respond to treatment can be diagnosed by obtaining a tumor sample from the patient, and assaying for expression of the gene targeted by the modulator. The expression data for the gene(s) can also be used as a diagnostic marker for disease progression. The assay can be performed by expression analysis as described above, by antibody directed to the gene target, or by any other available detection method.

**Table 1**

| **UP Seq ID** **NO** | **1** | **8** |
|---|---|---|
| **Breast** | 22% | 15% |
| **# of Pairs** | 36 | 13 |
| **Colon** | 15% | 40% |
| **# of Pairs** | 40 | 5 |
| **Head And Neck** | 38% | 25% |
| **# of Pairs** | 13 | 4 |
| **Kidney** | 24% | 0% |
| **# of Pairs** | 21 | 20 |
| **Liver** | 11% | 33% |
| **# of Pairs** | 9 | 9 |
| **Lung** | 12% | 25% |
| **# of Pairs** | 40 | 8 |
| **Lymphoma** | 0% | 50% |
| **# of Pairs** | 4 | 2 |
| **Ovary** | 37% | 7% |
| **# of Pairs** | 19 | 15 |
| **Pancreas** | 58% | 33% |
| **# of Pairs** | 12 | 3 |
| **Prostate** | 8% | 27% |
| **# of Pairs** | 24 | 22 |
| **Skin** | 100% | 0% |
| **# of Pairs** | 7 | 2 |
| **Stomach** | 73% | 67% |
| **# of Pairs** | 11 | 3 |
| **Testis** | 88% | 0% |
| **# of Pairs** | 8 | 3 |
| **Thyroid Gland** | 43% | 11% |
| **# of Pairs** | 14 | 9 |
| **Uterus** | 22% | 12% |
| **# of Pairs** | 23 | 16 |

### VI. UP functional assays

RNAi experiments were carried out to knock down expression of UP (SEQ ID NOs:1 and 8) in various cell lines using small interfering RNAs (siRNA, Elbashir et al, *supra*).

Effect of UP RNAi on cell proliferation and growth. BrdU assay, as described above, was employed to study the effects of decreased UP expression on cell proliferation. The results of these experiments indicated that RNAi of SEQ ID NO:1 decreased proliferation in SW480 colon cancer and PC3 prostate cancer cells. RNAi of SEQ ID NO:8 decreased proliferation in PC3 cells . [3]H-Thymidine incorporation assay, as described above, was also employed to study the effects of decreased UP expression on cell proliferation. Results indicated that RNAi of both SEQ ID NO:1 and SEQ ID NO:8 decreased cell proliferation in LOVO and HCT116 colon cancer cells, and in PC3 cells. Standard colony growth assays, as described above, were employed to study the effects of decreased UP expression on cell growth. Results indicated that RNAi of SEQ ID NO:1 decreased proliferation in PC3 and SW480 cells.

Effect of UP RNAi on apoptosis. Phospho Histone H2B assay, as described above, was employed to to study the effects of decreased UP expression on apoptosis. Results indicated that RNAi of both SEQ ID NOs:1 and 8 increased apoptosis in SW480 and PC3 cells. Further, results indicated that RNAi of SEQ ID NO:1 decreased cell count in SW480 and HCT116 colon cancer cells, and also in PC3 cells. Multi-parameter apoptosis assays, as described above, was also employed to study the effects of decreased UP expression on apoptosis. Results indicated that RNAi of SEQ ID NO:1 caused apoptosis via affecting caspase activity, membrane permeability, and nuclear swelling in A549 lung cancer and PC3 prostate cancer cells. RNAi of SEQ ID NO:8 caused apoptosis via affecting caspase activity and nuclear swelling in A549 cells.

UP overexpression analysis. UP (SEQ ID NOs:1 and 8) were overexpressed and tested in colony growth assays as described above. Overexpressed SEQ ID NO:1 in combination with TRKa oncogene caused an increase in cell growth and formation of foci as compared with normal controls when transfected into RIE cells. Overexpressed SEQ ID NO:8 in combination with TRKa oncogene, and also in combination with Ras oncogene, caused an increase in cell growth and formation of foci as compared with normal controls when transfected into RIE cells. Further, overexpressed SEQ ID NO:8 caused increased colony growth in MDCK canine kidney cells, and also in RKE rat kidney cells.

Transcriptional reporter assays. Effects of overexpressed UP on expression of various transcription factors was studied. In this assay, rat intestinal epithelial cells (RIEs) cells were co-transfected with reporter constructs containing various transcription factors and luciferase along with UP. Luciferase intensity was then measured as the readout for transcriptional activation due to overexpression of the UP. Overexpressed SEQ ID NO:1 caused an increased expression of SRE (Serum response element). Overexpressed SEQ ID NO:8 caused an increased expression of EGR (Early growth response) and AP1 (Activator protein 1) transcription factors.
Beta Catenin Transcriptional readout assay. This assay is an expanded TaqMan^{®} transcriptional readout assay monitoring changes in the mRNA levels of endogenous beta catenin regulated genes. This assay measures changes in expression of beta catenin regulated cellular genes as a readout for pathway signaling activity. We identified a panel of genes that were transcriptionally regulated by beta catenin signaling, then designed and tested TaqMan^{®} primer/probes sets. We reduced expression of beta catenin by RNAi, and tested its affect on the expression of the transcriptionally regulated genes in multiple cell types. The panel readout was then narrowed to the ten most robust probes. We then treated cancer cells with siRNAs of the target genes of interest, such as UP, and tested how the reduced levels of the target genes affected the expression levels of the beta catenin regulated gene panel. Genes that when knocked out via RNAi, demonstrated the same pattern of activity on at least one panel gene as a beta-catenin knockout, were identified as involved in the beta catenin pathway. TaqMan^{®} assays were performed on the RNAs in a 384 well format. RNAi of SEQ ID NO:1 showed the same pattern of activity as beta catenin RNAi for many of the transcriptionally regulated genes. Active nuclear beta catenin measurement assay. Beta catenin is a cytoplasmic gene, which when activated, moves into the nucleus. This assay was designed to measure the amount of active beta catenin protein in the nucleus using an anti active beta catenin antibody and a nuclear staining dye. Using this assay, we looked for genes that when knocked out, decrease beta catenin activity, and hence, the amount of active beta catenin in the nucleus. This assay was performed using Cellomics Inc. instrumentation.
For this assay, cells were transfected in quadruplicate with siRNAs in 96 well format and stained 72 hours post transfection. The amount of nuclear beta catenin was measured using two different methods. RNAi of SEQ ID NO:1 caused a decrease in the nuclear beta catenin in SW480 cells.
TOPFLASH beta-catenin reporter assay. Factors of the TCF/LEF HMG domain family (TCFs) exist in vertebrates, Drosophila melanogaster and Caenorhabditis elegans. Upon Wingless/Wnt signaling, Armadillo/beta-catenin associate with nuclear TCFs and contribute a trans-activation domain to the resulting bipartite transcription factor. So, transcriptional activation of TCF target genes by beta-catenin appears to be a central event in development and cellular transformation. Topflash beta-catenin luciferase gene reporter assay is used as a tool to measures activity of various genes in the beta-catenin pathway by transcriptional activation of TCFs (Korinek, V, et al. (1998) Molecular and Cellular Biology 18: 1248-1256). Briefly, cells are co-transfected with TOPFLASH plasmids containing TCF binding sites driving luciferase, and gene of interest. Transfected cells are then analyzed for luciferase activity. RNAi of SEQ ID NOs:1 and 8 caused decreased luciferase activity as compared with normal controls in LX1 lung cancer cells, and LOVO and SW480 colon cancer cells. As an extension of this assay, knockdown of beta catenin itself by RNAi caused an increase in the mRNA level of SEQ ID NO:1, suggesting that SEQ ID NO:1 is a transcriptional target of the beta catenin pathway.

## Claims

1. An *in vitro* method of identifying a candidate beta catenin pathway modulating agent, said method comprising the steps of:
(a) providing an assay system capable of detecting uridine phosphorylase (UP) expression and /or phosphorylase activity comprising a purified uridine phosphorylase (UP) polypeptide or nucleic acid or functionally active fragment or derivative thereof;
(b) contacting the assay system with a test agent; and
(c) determining the expression or phosphorylase activity of uridine phosphorylase (UP) in the system, wherein a change in uridine phosphorylase (UP) expression and /or phosphorylase activity between the presence and absence of said test agent identifies the test agent as a candidate modulator of beta catenin expression or activity.

2. The method of Claim 1 wherein the assay system comprises cultured cells that express the uridine phosphorylase (UP) polypeptide.

3. The method of Claim 2 wherein the cultured cells additionally have defective beta catenin function.

4. The method of Claim 1 wherein the assay system includes a screening assay comprising a uridine phosphorylase (UP) polypeptide and the candidate test agent is a small molecule modulator.

5. The method of Claim 1 wherein the assay is a phosphorylase assay system.

6. The method of Claim 1 wherein the assay system is selected from the group consisting of an apoptosis assay system, a cell proliferation assay system, an angiogenesis assay system, and a hypoxic induction assay system.

7. The method of Claim 1 wherein the assay system includes a binding assay comprising a uridine phosphorylase (UP) polypeptide and the candidate test agent is an antibody.

8. The method of Claim 1 wherein the assay system includes an expression assay comprising a uridine phosphorylase (UP) nucleic acid and the candidate test agent is a nucleic acid modulator.

9. The method of claim 8 wherein the nucleic acid modulator is an antisense oligomer.

10. The method of Claim 8 wherein the nucleic acid modulator is a phosphothioate morpholino oligomer.

11. The method of Claim 1 wherein the method additionally comprises:
(d) administering the test agent to an *in vitro* model system comprising cells defective in beta catenin function and detecting a phenotypic change in the model system that indicates that the beta catenin function is restored.

12. An *in vitro* method for modulating a beta catenin pathway in a cell comprising contacting the cell with an agent that specifically modulates the expression of uridine phosphorylase (UP) and/or phosphorylase activity, wherein said candidate modulator is selected from the group consisting of UP-specific antibodies, UP-specific antisense oligomers and UP-specific double stranded RNA.

13. An agent that specifically modulates the expression of uridine phosphorylase (UP) and/or phosphorylase activity for use in the treatment of a lymphoma, a pancreatic cancer, a skin cancer, a stomach cancer, a testicular cancer and a uterine cancer relating to a defect in beta catenin function, wherein the said agent is selected from the group consisting of UP-specific antibodies, UP-specific antisense oligomers and UP-specific double stranded RNA.

14. An agent according to claim 13 wherein the agent is a UP-specific antibody.

15. The *in vitro* method of claim 1, comprising the additional steps of:
(d) providing a secondary assay system capable of detecting beta catenin activity and/or expression of beta catenin pathway components, said assay comprising cultured cells expressing uridine phosphorylase (UP);
(e) contacting the secondary assay system with the test agent of (b) or an agent derived therefrom;
(f) determining beta catenin activity and/or expression of beta catenin pathway components in the secondary assay system, wherein a change in beta catenin activity and/or expression of beta catenin pathway components between the presence and absence of said test agent or agent derived therefrom confirms the candidate test agent as a beta catenin pathway modulating agent.

16. The method of Claim 15 wherein the cultured cells have defective beta catenin function.

17. A method according to claims 15 or 16 wherein the secondary assay system is a neovascular or angiogenic assay system.

18. An *in vitro* method for diagnosing a disorder or disease associated with a defect in the beta catenin pathway in a biological sample from a patient comprising:
(a) contacting the sample with a probe for uridine phosphorylase (UP) expression;
(b) comparing results from step (a) with a control;
(c) determining whether step (b) indicates a likelihood of a disease or disorder associated with a defect in the beta catenin pathway, wherein the disease or disorder is an angiogenic, apoptotic or cell proliferation disease or disorder.

19. The method of claim 18 wherein said disease is cancer.

20. The method according to claim 19, wherein said cancer is a cancer as shown in Table 1 as having > 25% expression level.

21. An agent according to claim 13, wherein said agent is for use in the treatment of a vertebrate animal predetermined to have said cancer.

22. The method according to claim 12, wherein said cell is a mammalian cell and said agent specifically binds a uridine phosphorylase (UP) polypeptide or nucleic acid.

23. An agent according to claim 13 or 21, wherein said treatment is treatment of a mammal.

## Patentansprüche

1. Ein *In-vitro-*Verfahren zum Identifizieren eines den Beta-Catenin-Weg modulierenden Wirkstoffkandidaten, wobei das Verfahren die folgenden Schritte beinhaltet:
(a) Bereitstellen eines Assaysystems, das in der Lage ist, die Expression und/oder die Phosphorylaseaktivität von Uridinphosphorylase (UP) zu erkennen, das ein gereinigtes Uridinphosphorylase(UP)-Polypeptid oder eine Uridinphosphorylase(UP)-Nucleinsäure oder ein funktionell aktives Fragment oder Derivat davon beinhaltet;
(b) In-Kontakt-Bringen des Assaysystems mit einem Testwirkstoff und
(c) Bestimmen der Expression oder der Phosphorylaseaktivität von Uridinphosphorylase (UP) in dem System, wobei eine Änderung der Expression und/oder Phosphorylaseaktivität von Uridinphosphorylase (UP) zwischen dem Vorhandensein und der Abwesenheit des Testwirkstoffs den Testwirkstoff als einen Modulatorkandidaten für die Beta-Catenin-Expression oder -Aktivität identifiziert.

2. Verfahren gemäß Anspruch 1, wobei das Assaysystem kultivierte Zellen beinhaltet, die das Uridinphosphorylase(UP)-Polypeptid exprimieren.

3. Verfahren gemäß Anspruch 2, wobei die kultivierten Zellen zusätzlich eine fehlerhafte Beta-Catenin-Funktion aufweisen.

4. Verfahren gemäß Anspruch 1, wobei das Assaysystem einen Screening-Assay umfasst, der ein Uridinphosphorylase(UP)-Polypeptid beinhaltet, und der Testwirkstoffkandidat ein Modulator aus einem kleinen Molekül ist.

5. Verfahren gemäß Anspruch 1, wobei der Assay ein Phosphorylase-Assaysystem ist.

6. Verfahren gemäß Anspruch 1, wobei das Assaysystem aus der Gruppe, bestehend aus einem Apoptose-Assaysystem, einem Zell-Proliferationsassaysystem, einem Angiogenese-Assaysystem und einem Hypoxieinduktions-Assaysystem, ausgewählt ist.

7. Verfahren gemäß Anspruch 1, wobei das Assaysystem einen Bindungsassay umfasst, der ein Uridinphosphorylase(UP)-Polypeptid beinhaltet, und der Testwirkstoffkandidat ein Antikörper ist.

8. Verfahren gemäß Anspruch 1, wobei das Assaysystem einen Expressionsassay umfasst, der eine Uridinphosphorylase(UP)-Nucleinsäure beinhaltet, und der Testwirkstoffkandidat ein Modulator aus Nucleinsäure ist.

9. Verfahren gemäß Anspruch 8, wobei der Modulator aus Nucleinsäure ein Antisense-Oligomer ist.

10. Verfahren gemäß Anspruch 8, wobei der Modulator aus Nucleinsäure ein Phosphothioatmorpholino-Oligomer ist.

11. Verfahren gemäß Anspruch 1, wobei das Verfahren zusätzlich Folgendes beinhaltet:
(d) Verabreichen des Testwirkstoffs an ein In-vitro-Modellsystem, das hinsichtlich der Beta-Catenin-Funktion fehlerhafte Zellen beinhaltet, und Erkennen einer phänotypischen Änderung in dem Modellsystem, die anzeigt, dass die Beta-Catenin-Funktion wiederhergestellt ist.

12. Ein In-vitro-Verfahren zum Modulieren eines Beta-Catenin-Wegs in einer Zelle, das das In-Kontakt-Bringen der Zelle mit einem Wirkstoff beinhaltet, der spezifisch die Expression von Uridinphosphorylase (UP) und/oder Phosphorylaseaktivität moduliert, wobei der Modulatorkandidat aus der Gruppe, bestehend aus UP-spezifischen Antikörpern, UP-spezifischen Antisense-Oligomeren und UP-spezifischer doppelsträngiger RNA, ausgewählt ist.

13. Ein Wirkstoff, der spezifisch die Expression von Uridinphosphorylase (UP) und/oder Phosphorylaseaktivität moduliert, zur Verwendung bei der Behandlung von einem Lymphknotentumor, einem Bauchspeicheldrüsenkrebs, einem Hautkrebs, einem Magenkrebs, einem Hodenkrebs und einem Gebärmutterkrebs, die mit einem Defekt in der Beta-Catenin-Funktion verbunden sind, wobei der Wirkstoff aus der Gruppe, bestehend aus UP-spezifischen Antikörpern, UP-spezifischen Antisense-Oligomeren und UP-spezifischer doppelsträngiger RNA, ausgewählt ist.

14. Wirkstoff gemäß Anspruch 13, wobei der Wirkstoff ein UP-spezifischer Antikörper ist.

15. *In-vitro*-Verfahren gemäß Anspruch 1, das die folgenden zusätzlichen Schritte beinhaltet:
(d) Bereitstellen eines sekundären Assaysystems, das in der Lage ist, Beta-Catenin-Aktivität und/oder Expression von Komponenten des Beta-Catenin-Wegs zu erkennen, wobei der Assay Uridinphosphorylase (UP) exprimierende kultivierte Zellen beinhaltet;
(e) In-Kontakt-Bringen des sekundären Assaysystems mit dem Testwirkstoff aus (b) oder einem davon abgeleiteten Wirkstoff;
(f) Bestimmen von Beta-Catenin-Aktivität und/oder Expression von Komponenten des Beta-Catenin-Wegs in dem sekundären Assaysystem, wobei eine Änderung der Beta-Catenin-Aktivität und/oder Expression von Komponenten des Beta-Catenin-Wegs zwischen dem Vorhandensein und der Abwesenheit des Testwirkstoffs oder des davon abgeleiteten Wirkstoffs den Testwirkstoffkandidaten als einen Beta-Catenin-Weg modulierenden Wirkstoff bestätigt.

16. Verfahren gemäß Anspruch 15, wobei die kultivierten Zellen eine fehlerhafte Beta-Catenin-Funktion aufweisen.

17. Verfahren gemäß Anspruch 15 oder 16, wobei das sekundäre Assaysystem ein neovaskuläres oder angiogenetisches Assaysystem ist.

18. Ein *In-vitro*-Verfahren zum Diagnostizieren einer Störung oder Krankheit, die mit einem Defekt in dem Beta-Catenin-Weg verbunden ist, in einer biologischen Probe von einem Patienten, das Folgendes beinhaltet:
(a) In-Kontakt-Bringen der Probe mit einer Sonde für Uridinphosphorylase(UP)-Expression;
(b) Vergleichen der Ergebnisse aus Schritt (a) mit einer Kontrolle;
(c) Bestimmen, ob Schritt (b) eine Wahrscheinlichkeit einer mit einem Defekt in dem Beta-Catenin-Weg verbundenen Krankheit oder Störung anzeigt, wobei die Krankheit oder Störung eine Angiogenese, Apoptose oder Zellproliferation betreffende Krankheit oder Störung ist.

19. Verfahren gemäß Anspruch 18, wobei die Krankheit Krebs ist.

20. Verfahren gemäß Anspruch 19, wobei der Krebs ein wie in Tabelle 1 mit einem Expressionsgrad von >25 % gezeigter Krebs ist.

21. Wirkstoff gemäß Anspruch 13, wobei der Wirkstoff zur Verwendung bei der Behandlung eines Wirbeltiers, von dem zuvor bestimmt wurde, dass es an dieser Art von Krebs leidet, vorgesehen ist.

22. Verfahren gemäß Anspruch 12, wobei die Zelle eine Säugetierzelle ist und der Wirkstoff spezifisch ein Uridinphosphorylase(UP)-Polypeptid oder eine Uridinphosphorylase(UP)-Nucleinsäure bindet.

23. Wirkstoff gemäß Anspruch 13 oder 21, wobei die Behandlung die Behandlung eines Säugetiers ist.

## Revendications

1. Une méthode *in vitro* pour identifier un agent de modulation de voie bêta-caténine candidat, ladite méthode comprenant les étapes consistant à :
(a) fournir un système d'épreuve capable de détecter une expression de l'uridine phosphorylase (UP) et / ou une activité phosphorylase comprenant un polypeptide ou un acide nucléique ou un fragment ou un dérivé fonctionnellement actifs de ceux-ci purifiés d'uridine phosphorylase (UP) ;
(b) mettre en contact le système d'épreuve avec un agent de test ; et
(c) déterminer l'expression ou l'activité phosphorylase de l'uridine phosphorylase (UP) dans le système, où un changement dans l'expression de l'uridine phosphorylase (UP) et / ou l'activité phosphorylase entre la présence et l'absence dudit agent de test identifie l'agent de test comme étant un modulateur candidat de l'expression ou de l'activité de la bêta-caténine.

2. La méthode de la revendication 1 où le système d'épreuve comprend des cellules cultivées qui expriment le polypeptide d'uridine phosphorylase (UP).

3. La méthode de la revendication 2 où les cellules cultivées ont de plus une fonction bêta-caténine défectueuse.

4. La méthode de la revendication 1 où le système d'épreuve comporte une épreuve de criblage comprenant un polypeptide d'uridine phosphorylase (UP), et l'agent de test candidat est une petite molécule modulatrice.

5. La méthode de la revendication 1 où l'épreuve est un système d'épreuve de phosphorylase.

6. La méthode de la revendication 1 où le système d'épreuve est sélectionné dans le groupe consistant en un système d'épreuve d'apoptose, un système d'épreuve de prolifération cellulaire, un système d'épreuve d'angiogenèse, et un système d'épreuve d'induction hypoxique.

7. La méthode de la revendication 1 où le système d'épreuve comporte une épreuve de liaison comprenant un polypeptide d'uridine phosphorylase et l'agent de test candidat est un anticorps.

8. La méthode de la revendication 1 où le système d'épreuve comporte une épreuve d'expression comprenant un acide nucléique d'uridine phosphorylase (UP) et l'agent de test candidat est un acide nucléique modulateur.

9. La méthode de la revendication 8 où l'acide nucléique modulateur est un oligomère antisens.

10. La méthode de la revendication 8 où l'acide nucléique modulateur est un oligomère morpholino phosphothioate (PMO).

11. La méthode de la revendication 1 où la méthode comprend de plus :
(d) administrer l'agent de test à un système modèle *in vitro* comprenant des cellules défectueuses en terme de fonction bêta-caténine et détecter un changement phénotypique dans le système modèle qui indique que la fonction bêta-caténine est restaurée.

12. Une méthode *in vitro* pour moduler une voie bêta-caténine dans une cellule comprenant mettre en contact la cellule avec un agent qui module de façon spécifique l'expression de l'uridine phosphorylase (UP) et / ou l'activité phosphorylase, où ledit modulateur candidat est sélectionné dans le groupe consistant en anticorps spécifiques à UP, oligomères antisens spécifiques à UP et ARN à double brin spécifique à UP.

13. Un agent qui module de façon spécifique l'expression de l'uridine phosphorylase (UP) et / ou l'activité phosphorylase destiné à être utilisé dans le traitement d'un lymphome, d'un cancer pancréatique, d'un cancer de la peau, d'un cancer de l'estomac, d'un cancer testiculaire et d'un cancer utérin en rapport avec un défaut dans la fonction bêta-caténine, où ledit agent est sélectionné dans le groupe consistant en anticorps spécifiques à UP, oligomères antisens spécifiques à UP et ARN à double brin spécifique à UP.

14. Un agent selon la revendication 13 où l'agent est un anticorps spécifique à UP.

15. La méthode *in vitro* de la revendication 1, comprenant les étapes supplémentaires consistant à :
(d) fournir un système d'épreuve secondaire capable de détecter une activité bêta-caténine et / ou une expression de composants de voie bêta-caténine, ladite épreuve comprenant des cellules cultivées exprimant l'uridine phosphorylase (UP) ;
(e) mettre en contact le système d'épreuve secondaire avec l'agent de test de (b) ou un agent dérivé de celui-ci ;
(f) déterminer l'activité bêta-caténine et / ou l'expression de composants de voie bêta-caténine dans le système d'épreuve secondaire, où un changement dans l'activité bêta-caténine et / ou l'expression de composants de voie bêta-caténine entre la présence et l'absence dudit agent de test ou dudit agent dérivé de celui-ci confirme que l'agent de test candidat est un agent de modulation de voie bêta-caténine.

16. La méthode de la revendication 15 où les cellules cultivées ont une fonction bêta-caténine défectueuse.

17. Une méthode selon les revendications 15 ou 16 où le système d'épreuve secondaire est un système d'épreuve néovasculaire ou angiogénique.

18. Une méthode *in vitro* pour diagnostiquer un trouble ou une maladie associés à un défaut dans la voie bêta-caténine dans un échantillon biologique provenant d'un patient comprenant :
(a) mettre en contact l'échantillon avec une sonde pour l'expression de l'uridine phosphorylase (UP) ;
(b) comparer les résultats de l'étape (a) à un témoin ;
(c) déterminer si l'étape (b) indique un risque d'un trouble ou d'une maladie associés à un défaut dans la voie bêta-caténine, où la maladie ou le trouble sont une maladie ou un trouble angiogénique, apoptotique ou à prolifération cellulaire.

19. La méthode de la revendication 18 où ladite maladie est un cancer.

20. La méthode selon la revendication 19, où ledit cancer est un cancer tel que montré sur le Tableau 1 ayant un niveau d'expression > 25 %.

21. Un agent selon la revendication 13, où ledit agent est destiné à être utilisé dans le traitement d'un animal vertébré chez lequel ledit cancer a été prédéterminé.

22. La méthode selon la revendication 12, où ladite cellule est une cellule mammalienne et ledit agent lie de façon spécifique un polypeptide ou un acide nucléique d'uridine phosphorylase (UP).

23. Un agent selon la revendication 13 ou la revendication 21, où ledit traitement est le traitement d'un mammifère.
